# EUROPEAN PATENT APPLICATION

(11) **EP 4 810 519 A2**
(43) Date of publication of application: **23.09.2026**
(21) Application number: 26183288.5
(22) Date of filing: 13.04.2023
(51) Int. Cl.: C07K 16/32

(54) **PHARMACEUTICAL COMPOSITIONS OF THERAPEUTIC PROTEINS AND METHODS OF USE**

(30) Priority: 13.04.2022 US 202263330759 P
(62) Document number(s) of the earlier application(s) in accordance with Art. 76 EPC: 23723780.5 / 4 508 087
(71) Applicant: GENENTECH, INC., San Francisco, CA 94080-4990 (US); F. Hoffmann-La Roche AG, 4070 Basel (CH)
(72) Inventor: QUIVET NÉÉ BOILLON, Adeline Valentine, 4070 Basel (CH); JI, Junyan, San Francisco, CA 94080-4990 (US); MEUX, Ellen Dorothee, 4070 Basel (CH); RAVURI, Satya Krishna Kishore, 4070 Basel (CH); SCHOENHAMMER, Karin, 4070 Basel (CH); TYLER, Jacqueline Yvonne, San Francisco, CA 94080-4990 (US); VOLLRATH, Ilona Elisabeth, 4070 Basel (CH); BHARGAVA, Adithi Chandrasekhara, San Francisco, CA 94080-4990 (US); DUBOEUF, Jérémy Jean-Pierre, 4070 Basel (CH); FAST, Lars Jonas, 4070 Basel (CH); PRINTZ, Miriam, 4070 Basel (CH); IRNGARTINGER, Meike, 4070 Basel (CH)
(74) Representative: Mewburn Ellis LLP

(57) **Abstract**

The disclosure provides pharmaceutical compositions of therapeutic proteins and methods of using the same.

## Description

### SEQUENCE LISTING

The instant application contains a Sequence Listing which has been submitted electronically in XML format and is hereby incorporated by reference in its entirety. Said XML copy, created on March 22, 2023, is named 50474-276WO2_Sequence_Listing_3_22_23 and is 119,559 bytes in size.

### FIELD OF THE INVENTION

The present disclosure relates to compositions (e.g., pharmaceutical compositions) comprising therapeutic proteins (e.g., T cell-dependent bispecific antibodies or T cell engaging bispecific antibodies) and methods of using the same.

### BACKGROUND

Therapeutic proteins, such as antibodies (e.g., bispecific antibodies; e.g., T cell-dependent bispecific antibodies (TDBs) or T cell engaging bispecific antibodies (TCBs); e.g., anti-CD20/anti-CD3, anti-FcRH5/anti-CD3, or anti-HER2/anti-CD3 TDBs or TCBs; e.g., mosunetuzumab, glofitamab, cevostamab, or runimotamab), are being increasingly used as treatments for a variety of diseases and disorders, such as cancer and autoimmunity. Such therapeutic proteins can be formulated in an aqueous carrier for administration to a subject, e.g., by intravenous or subcutaneous administration. During storage, handling, and administration of such pharmaceutical compositions, it is necessary to mitigate loss of the therapeutic protein, which can occur through degradation and surface adsorption, such as protein adsorption to surfaces of filters, storage canisters, tubing, syringes, intravenous fluid bags, and other containers. In instances in which the pharmaceutical composition contains a relatively low concentration of therapeutic protein, protein loss can be dramatically increased by these factors, resulting in reduced therapeutic efficacy of the pharmaceutical composition.

Thus, there is a need in the field to develop pharmaceutical formulations in which a therapeutic protein (e.g., low-dose therapeutic protein) is stable and protected from loss due to adsorption.

### SUMMARY OF THE DISCLOSURE

The present disclosure relates to compositions (e.g., pharmaceutical compositions) comprising therapeutic proteins (e.g., low concentrations of therapeutic proteins, such as bispecific antibodies (e.g., anti-CD3 bispecific antibodies; e.g., anti-CD20/anti-CD3, anti-FcRH5/anti-CD3, or anti-HER2/anti-CD3 bispecific antibodies; e.g., T cell-dependent bispecific antibodies (TDBs) or T cell engaging bispecific antibodies (TCBs); e.g., anti-CD20/anti-CD3, anti-FcRH5/anti-CD3, or anti-HER2/anti-CD3 TDBs or TCBs; e.g., mosunetuzumab, glofitamab, cevostamab, or runimotamab)) and methods of using the same. The disclosed compositions and related methods address the problem of delivering therapeutic proteins that are formulated at low concentration, ensuring that patients receive the intended dose of the therapeutic protein with little to no loss of the protein during storage and administration.

In one aspect, the disclosure provides a pharmaceutical composition that includes a therapeutic protein (e.g., an antibody; e.g., a bispecific antibody; e.g., an anti-CD3 bispecific antibody; e.g., a T cell-dependent bispecific antibody (TDB) or T cell engaging bispecific antibody (TCB); e.g., mosunetuzumab, glofitamab, cevostamab, or runimotamab), a surfactant (e.g., polysorbate 20 (PS20) or poloxamer 188 (P188)), methionine, a buffering agent, and a carrier. In some embodiments, the molar ratio of the surfactant (e.g., PS20 or P188) to the therapeutic protein is 100 or less, e.g., 90 or less, 80 or less, 70 or less, 60 or less, 50 or less, 40 or less, 30 or less, 20 or less, 10 or less, 9 or less, 8 or less, 7 or less, 6 or less, 5 or less, 4 or less, 3 or less, 2 or less, or 1 or less, e.g., from 0.5 to 100, from 0.5 to 50, from 0.5 to 10, from 0.5 to 5, from 0.5 to 1, from 1 to 5, from 2 to 4, from 5 to 100, from 10 to 70, from 10 to 50, from 10 to 30, from 50 to 100, from 60 to 80, from 70 to 80, or from 40 to 50. In certain embodiments, the molar ratio of the surfactant (e.g., PS20 or P188) to the therapeutic protein is from 1 to 100. In some embodiments, the molar ratio of the surfactant (e.g., PS20) to the therapeutic protein is from 45 to 100, from 45 to 55, from 50 to 100, from 60 to 90, from 70 to 90, from 60 to 80, from 70 to 80, from 65 to 75, or from 75 to 85, e.g., about 45, 46, 47, 48, 49, 50, 51, 52, 53, 54, 55, 65, 66, 67, 68, 69, 70, 71, 72, 73, 74, 75, 76, 77, 78, 79, or 80. In a particular embodiment, the molar ratio of the surfactant to the therapeutic protein is about 71. In another particular embodiment, the molar ratio of the surfactant to the therapeutic protein is about 79. In yet another particular embodiment, the molar ratio of the surfactant to the therapeutic protein is about 48. In other embodiments, the molar ratio of the surfactant (c.g., P188) to the therapeutic protein is from 5 to 50, from 5 to 25, from 10 to 15, or from 15 to 20, e.g., about 10, 11, 12, 13, 14, 15, 16, 17, 18, 19, or 20. In a particular embodiment, the molar ratio of surfactant (e.g., P188) to the therapeutic protein is about 14. In another particular embodiment, the molar ratio of surfactant (e.g., P188) to the therapeutic protein is about 11.5.

In some embodiments, the concentration of the surfactant (e.g., PS20 or P188) is from 0.01% to 0.12% weight-by-volume (w/v) (e.g., from 0.01% to 0.025%, from 0.025% to 0.5%, from 0.05% to 0.075%, or from 0.075% to 0.12% (w/v), e.g., from 0.01% to 0.02%, from 0.02% to 0.03%, from 0.03% to 0.04%, from 0.04% to 0.05%, from 0.05% to 0.06%, from 0.06% to 0.07%, from 0.07% to 0.08%, from 0.08% to 0.09%, or from 0.09% to 0.12% (w/v); e.g., about 0.01%, about 0.015%, about 0.02%, about 0.025%, about 0.03%, about 0.035%, about 0.04%, about 0.045%, about 0.05%, about 0.055%, about 0.06%, about 0.065%, about 0.07%, about 0.075%, about 0.08%, about 0.085%, about 0.09%, about 0.095%, or about 0.1%, about 0.115%, or about 0.12% (w/v)).

In some embodiments, the concentration of the methionine is from 1 mM to 50 mM (e.g., from 1 mM to 10 mM, from 10 mM to 20 mM, from 20 mM to 30 mM, from 30 mM to 40 mM, or from 40 mM to 50 mM, e.g., from 5 mM to 45 mM, from 10 mM to 40 mM, from 15 mM to 35 mM, or from 20 mM to 30 mM, e.g., about 1 mM, about 2 mM, about 3 mM, about 4 mM, about 5 mM, about 6 mM, about 7 mM, about 8 mM, about 9 mM, about 10 mM, about 11 mM, about 12 mM, about 13 mM, about 14 mM, about 15 mM, **about 16** mM, about 17 mM, about 18 mM, about 19 mM, about 20 mM, about 21 mM, about 22 mM, about 23 mM, about 24 mM, about 25 mM, about 26 mM, about 27 mM, about 28 mM, about 29 mM, about 30 mM, about 31 mM, about 32 mM, about 33 mM, about 34 mM, about 35 mM, about 36 mM, about 37 mM, about 38 mM, about 39 mM about 40 mM, about 41 mM, about 42 mM, about 43 mM, about 44 mM, about 45 mM, about 46 mM, about 47 mM, about 48 mM, about 49 mM, or about 50 mM). In some embodiments, the concentration of methionine is about 10 mM.

In some embodiments, the concentration of the buffering agent is from 5 mM to 20 mM (e.g., from 5 mM to 10 mM, from 10 mM to 15 mM, or from 15 mM to 20 mM, e.g., from 6 mM to 18 mM, from 7 mM to 16 mM, from 8 mM to 15 mM, or from 9 mM to 12 mM, e.g., about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, or about 20 mM).

In some embodiments, the concentration of the therapeutic protein (e.g., antibody; e.g., bispecific antibody; e.g., anti-CD3 bispecific antibody; e.g., T cell-dependent bispecific antibody or T cell engaging bispecific antibody; e.g., mosunetuzumab, glofitamab, cevostamab, or runimotamab) is 10 mg/ml or less (e.g., 9 mg/ml or less, 8 mg/ml or less, 7.5 mg/ml or less, 7 mg/ml or less, 6 mg/ml or less, 5 mg/ml or less, 4 mg/ml or less, 3 mg/ml or less, 2.5 mg/ml or less, 2 mg/ml or less, 1.5 mg/ml or less, 1.0 mg/ml or less, 0.5 mg/ml or less, 0.25 mg/ml or less, 0.2 mg/ml or less, or 0.1 mg/ml or less, e.g., from 0.01 mg/ml to 0.1 mg/ml, from 0.1 mg/ml to 1 mg/ml, from 1 mg/ml to 5 mg/ml, or from 5 mg/ml to 10 mg/ml, e.g., about 0.1 mg/ml, about 0.2 mg/ml, about 0.3 mg/ml, about 0.4 mg/ml, about 0.5 mg/ml, about 0.6 mg/ml, about 0.7 mg/ml, about 0.8 mg/ml, about 0.9 mg/ml, about 1.0 mg/ml, about 1.5 mg/ml, about 2 mg/ml, about 2.5 mg/ml, about 3 mg/ml, 4 mg/ml, about 5 mg/ml, about 6 mg/ml, about 7 mg/ml, about 8 mg/ml, about 9 mg/ml, or about 10 mg/ml). In a particular embodiment, the concentration of the therapeutic protein is about 1 mg/ml. In another particular embodiment, the concentration of the therapeutic protein is about 3 mg/ml.

In some embodiments, the pharmaceutical composition is formulated as a drug product (DP). In some embodiments, the pharmaceutical composition formulated as a DP has a therapeutic protein (e.g., antibody; e.g., bispecific antibody; e.g., anti-CD3 bispecific antibody; e.g., T cell-dependent bispecific antibody or T cell engaging bispecific antibody; e.g., mosunetuzumab, glofitamab, cevostamab, or runimotamab) concentration of about 1 mg/ml and/or a molar ratio of surfactant (e.g., PS20) to therapeutic protein from 45 to 100, from 45 to 55, from 50 to 100, from 60 to 90, from 70 to 90, from 60 to 80, from 70 to 80, from 65 to 75, or from 75 to 85, e.g., about 45, 46, 47, 48, 49, 50, 51, 52, 53, 54, 55, 65, 66, 67, 68, 69, 70, 71, 72, 73, 74, 75, 76, 77, 78, 79, or 80. In a particular embodiment, the molar ratio of the surfactant to the therapeutic protein (e.g., mosunetuzumab or runimotamab) is about 71. In another particular embodiment, the molar ratio of the surfactant to the therapeutic protein (e.g., glofitamab) is about 79. In some embodiments, the molar ratio of the surfactant to the therapeutic protein (e.g., runimotamab) is about 48.

In some embodiments, the buffering agent is a histidine, a phosphate, a succinate, an acetate, or a combination thereof. For example, in some embodiments, the buffering agent is a histidine, such as histidine acetate or histidine hydrochloride (HCl). The concentration of the buffering agent (e.g., histidine, e.g., histidine acetate or histidine HCl, e.g., L-histidine acetate or L-histidine HCl) can be, e.g., from 8 mM to 12 mM, e.g., about 8 mM, about 9 mM, about 10 mM, about 11 mM, or about 12 mM. In some embodiments, the concentration of the buffering agent (e.g., histidine, e.g., histidine acetate or histidine HCl, e.g., L-histidine acetate or L-histidine HCl) is about 10 mM or 20 mM. In a particular embodiment, the buffering agent is histidine HCl at a concentration of about 20 mM. In another particular embodiment, the buffering agent is histidine acetate at a concentration of about 20 mM. In yet another particular embodiment, the buffering agent is histidine acetate at a concentration of about 10 mM.

In some embodiments, the pharmaceutical composition further includes a tonicity agent, such as a sugar, an amino acid, or a salt. In embodiments in which the tonicity agent is a sugar, the sugar can be, e.g., sucrose, glucose, glycerol, or trehalose. In a particular embodiment, the sugar is sucrose. In some embodiments, the tonicity agent (e.g., sugar, e.g., sucrose) is at a concentration from 100 mM to 500 mM (e.g., from 100 mM to 120 mM, from 120 mM to 140 mM, from 140 mM to 160 mM, from 160 mM to 180 mM, from 180 mM to 200 mM, from 200 mM to 220 mM, from 220 mM to 240 mM, from 240 mM to 260 mM, from 260 mM to 280 mM, from 280 mM to 300 mM, from 300 mM to 320 mM, from 320 mM to 340 mM, from 340 mM to 360 mM, from 360 mM to 380 mM, from 380 mM to 400 mM, from 400 mM to 420 mM, from 420 mM to 440 mM, from 440 mM to 460 mM, from 460 mM to 480 mM, or from 480 mM to 500 mM, e.g., from 100 mM to 400 mM, from 150 mM to 350 mM, or from 200 mM to 300 mM, e.g., about 100 mM, about 150 mM, about 200 mM, about 210 mM, about 220 mM, about 230 mM, about 240 mM, about 250 mM, about 260 mM, about 270 mM, about 280 mM, about 290 mM, about 300 mM, about 350 mM, about 400 mM, about 450 mM, or about 500 mM). In a particular embodiment, the concentration of the tonicity agent (e.g., sugar, e.g., sucrose) is about 240 mM.

In some embodiments, the pharmaceutical composition further includes an antioxidant. In some embodiments, the antioxidant is N-acetyl-DL-tryptophan. In some embodiments, the concentration of N-acetyl-DL-tryptophan is from 0.1 mM to 0.5 mM (e.g., about 0.1 mM, about 0.15 mM, about 0.2 mM, about 0.25 mM, about 0.3 mM, about 0.35 mM, about 0.4 mM, about 0.45 mM, or about 0.5 mM). In some embodiments, the concentration of N-acetyl-DL-tryptophan is about 0.3 mM.

In some embodiments, the pharmaceutical composition has a pH from 4.5 to 8 (e.g., from 4.5 to 5.0, from 5.0 to 5.5, from 5.5 to 6.0, from 6.0 to 6.5, from 6.5 to 7.0, from 7.0 to 7.5, or from 7.5 to 8.0, e.g., about 4.5, about 4.6, about 4.7, about 4.8, about 4.9, about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, about 6.5, about 6.6, about 6.7, about 6.8, about 6.9, about 7.0, about 7.1, about 7.2, about 7.3, about 7.4, about 7.5, about 7.6, about 7.7, about 7.8, about 7.9, or about 8.0). In some embodiments, the pH of the pharmaceutical composition is from 5.1 to 6.1. In a particular embodiment, the pH of the pharmaceutical composition is about 5.8. In another particular embodiment, the pH of the pharmaceutical composition is about 5.5.

In some embodiments, the therapeutic protein is an antibody. In some embodiments, the therapeutic protein is an anti-CD3 antibody. In some embodiments, the therapeutic protein is a bispecific antibody. For example, the bispecific antibody can be a T cell-dependent bispecific antibody (TDB) or T cell engaging bispecific antibody (TCB), such as a bispecific antibody having at least one target antigen-binding moiety and one CD3-binding moiety. In some embodiments, the target antigen-binding moiety of the bispecific antibody (e.g., TDB or TCB) can bind to CD20, FcRH5, or HER2. In some embodiments, the target antigen-binding moiety is a Fab or a crossover Fab. In some embodiments, the therapeutic protein is mosunetuzumab, glofitamab, cevostamab, or runimotamab

In some embodiments, the bispecific antibody (e.g., anti-CD3 bispecific antibody; e.g., T cell-dependent bispecific antibody; e.g., mosunetuzumab) has a methionine at position 257 of the Fc region (as in the EU index). In some embodiments, oxidation of the methionine at position 257 of the Fc region is less than 10% over two weeks at 40 °C (e.g., less than 9%, less than 8%, less than 7%, less than 6%, less than 5%, less than 4%, less than 3%, less than 2%, or less than 1% over two weeks at 40%). In some embodiments, the oxidation of the methionine at position 257 of the Fc region is no more than about 6% over two weeks at 40 °C.

In another aspect, the pharmaceutical composition includes a bispecific antibody (e.g., anti-CD3 bispecific antibody; e.g., anti-CD20/anti-CD3, anti-FcRH5/anti-CD3, or anti-HER2/anti-CD3 bispecific antibody; e.g., TDB or TCB; e.g., anti-CD20/anti-CD3, anti-FcRH5/anti-CD3, or anti-HER2/anti-CD3 TDB or TCB; e.g., mosunetuzumab, glofitamab, cevostamab, or runimotamab), a surfactant (e.g., PS20 or poloxamer 188 (P188)), methionine, and a carrier, wherein the pharmaceutical composition has a pH of about 5.5 or 5.8. In some embodiments, the bispecific antibody includes at least one target antigen-binding moiety and one CD3-binding moiety and is at a concentration of about 10 mg/ml or less (e.g., 9 mg/ml or less, 8 mg/ml or less, 7.5 mg/ml or less, 7 mg/ml or less, 6 mg/ml or less, 5 mg/ml or less, 4 mg/ml or less, 3 mg/ml or less, 2.5 mg/ml or less, 2 mg/ml or less, 1.5 mg/ml or less, 1.0 mg/ml or less, 0.5 mg/ml or less, 0.25 mg/ml or less, 0.2 mg/ml or less, or 0.1 mg/ml or less, e.g., from 0.01 mg/ml to 0.1 mg/ml, from 0.1 mg/ml to 1 mg/ml, from 1 mg/ml to 5 mg/ml, or from 5 mg/ml to 10 mg/ml, e.g., about 0.1 mg/ml, about 0.2 mg/ml, about 0.3 mg/ml, about 0.4 mg/ml, about 0.5 mg/ml, about 0.6 mg/ml, about 0.7 mg/ml, about 0.8 mg/ml, about 0.9 mg/ml, about 1.0 mg/ml, about 1.5 mg/ml, about 2 mg/ml, about 2.5 mg/ml, about 3 mg/ml, 4 mg/ml, about 5 mg/ml, about 6 mg/ml, about 7 mg/ml, about 8 mg/ml, about 9 mg/ml, or about 10 mg/ml). In some embodiments, the surfactant is at a concentration from 0.05% to 0.12% w/v (e.g., from 0.05% to 0.075% or from 0.075% to 0.12% w/v, e.g., from 0.05% to 0.06%, from 0.06% to 0.07%, from 0.07% to 0.08%, from 0.08% to 0.09%, or from 0.09% to 0.12% w/v, e.g., about 0.05%, about 0.055%, about 0.06%, about 0.065%, about 0.07%, about 0.075%, about 0.08%, about 0.085%, about 0.09%, about 0.095%, or about 0.1 % w/v), and the methionine is at a concentration of about 10 mM.

In some embodiments, the molar ratio of the surfactant (e.g., PS20 or P188) to the bispecific antibody (e.g., anti-CD3 bispecific antibody; e.g., T cell-dependent bispecific antibody or T cell engaging bispecific antibody; e.g., mosunetuzumab, glofitamab, cevostamab, or runimotamab) is 100 or less, e.g., 90 or less, 80 or less, 70 or less, 60 or less, 50 or less, 40 or less, 30 or less, 20 or less. 10 or less, 9 or less, 8 or less, 7 or less, 6 or less, 5 or less, 4 or less, 3 or less, 2 or less, or 1 or less, e.g., from 0.5 to 100, from 0.5 to 50, from 0.5 to 10, from 0.5 to 5, from 0.5 to 1, from 1 to 5, from 2 to 4, from 5 to 100, from 10 to 70, from 10 to 50, from 10 to 30, from 50 to 100, from 60 to 80, from 70 to 80, or from 40 to 50. In certain embodiments, the molar ratio of the surfactant (e.g., PS20 or P188) to the bispecific antibody is from 1 to 100. In some embodiments, the molar ratio of the surfactant (e.g., PS20) to the bispecific antibody is from 45 to 100, from 45 to 55, from 50 to 100, from 60 to 90, from 70 to 90, from 60 to 80, from 70 to 80, from 65 to 75, or from 75 to 85, e.g., about 45, 46, 47, 48, 49, 50, 51, 52, 53, 54, 55, 65, 66, 67, 68, 69, 70, 71, 72, 73, 74, 75, 76, 77, 78, 79, or 80. In a particular embodiment, the molar ratio of the surfactant to the bispecific antibody is about 71. In another particular embodiment, the molar ratio of the surfactant to the bispecific antibody is about 79. In some another particular embodiment, the molar ratio of the surfactant to the bispecific antibody is about 48. In some embodiments, the surfactant is PS20 and the concentration of the PS20 is about 0.06% (w/v).

In some embodiments, the concentration of the bispecific antibody (e.g., anti-CD3 bispecific antibody; e.g., TDB or TCB; e.g., mosunetuzumab, glofitamab, cevostamab, or runimotamab) is between about 0.1 mg/ml to about 30 mg/ml, e.g., about 0.1 mg/ml to about 10 mg/ml, about 0.1 mg/ml to about 5 mg/ml, about 0.1 mg/ml to about 3 mg/ml, about 0.1 mg/ml to about 2 mg/ml, about 0.1 mg/ml to about 1.5 mg/ml, about 0.3 mg/ml to about 2 mg/ml, about 0.5 mg/ml to about 2 mg/ml, about 0.5 mg/ml to about 5 mg/ml, about 0.5 mg/ml to about 2 mg/ml, about 0.6 mg/ml to about 2 mg/ml, about 0.7 mg/ml to about 2 mg/ml, about 0.8 mg/ml to about 2 mg/ml, about 0.9 mg/ml to about 10 mg/ml, about 0.5 mg/ml to about 1.4 mg/ml, about 0.5 mg/ml to about 1.3 mg/ml, about 0.5 mg/ml to about 1.2 mg/ml, about 0.5 mg/ml to about 1.1 mg/ml, about 0.5 mg/ml to about 1.5 mg/ml, about 0.8 mg/ml to about 1.2 mg/ml, 1 mg/ml to about 30 mg/ml, about 2 mg/ml to about 30 mg/ml, about 3 mg/ml to about 30 mg/ml, about 4 mg/ml to about 30 mg/ml, about 5 mg/ml to about 30 mg/ml, about 6 mg/ml to about 30 mg/ml, about 7 mg/ml to about 30 mg/ml, about 8 mg/ml to about 30 mg/ml, about 9 mg/ml to about 30 mg/ml, about 10 mg/ml to about 30 mg/ml, about 11 mg/ml to about 30 mg/ml, about 12 mg/ml to about 30 mg/ml, about 13 mg/ml to about 30 mg/ml, about 14 mg/ml to about 30 mg/ml, about 15 mg/ml to about 30 mg/ml, about 20 mg/ml to about 30 mg/ml, about 25 mg/ml to about 30 mg/ml, about 1 mg/ml to about 20 mg/ml, about 2 mg/ml to about 20 mg/ml, about 3 mg/ml to about 20 mg/ml, about 4 mg/ml to about 20 mg/ml, about 5 mg/ml to about 20 mg/ml, about 6 mg/ml to about 20 mg/ml, about 7 mg/ml to about 20 mg/ml, about 8 mg/ml to about 20 mg/ml, about 9 mg/ml to about 20 mg/ml, about 10 mg/ml to about 20 mg/ml, about 11 mg/ml to about 20 mg/ml, about 12 mg/ml to about 20 mg/ml, about 13 mg/ml to about 20 mg/ml, about 14 mg/ml to about 20 mg/ml, about 15 mg/ml to about 20 mg/ml about 1 mg/ml to about 15 mg/ml, about 2 mg/ml to about 15 mg/ml, about 3 mg/ml to about 15 mg/ml, about 4 mg/ml to about 15 mg/ml, about 5 mg/ml to about 15 mg/ml, about 6 mg/ml to about 15 mg/ml, about 7 mg/ml to about 15 mg/ml, about 8 mg/ml to about 15 mg/ml, about 9 mg/ml to about 15 mg/ml, about 10 mg/ml to about 15 mg/ml, about 11 mg/ml to about 15 mg/ml, about 12 mg/ml to about 15 mg/ml, about 12 mg/ml to about 14 mg/ml, about 13 mg/ml to about 15 mg/ml, about 13 mg/ml to about 14 mg/ml, about 14 mg/ml to about 15 mg/ml, about 1 mg/ml to about 10 mg/ml, about 2 mg/ml to about 10 mg/ml, about 3 mg/ml to about 10 mg/ml, about 4 mg/ml to about 10 mg/ml, about 5 mg/ml to about 10 mg/ml, about 6 mg/ml to about 10 mg/ml, about 7 mg/ml to about 10 mg/ml, about 8 mg/ml to about 10 mg/ml, about 9 mg/ml to about 10 mg/ml, about 1 mg/ml to about 5 mg/ml, about 1 mg/ml to about 3 mg/ml, about 1.5 mg/ml to about 2.5 mg/ml, about 1.8 mg/ml to about 2.2 mg/ml, about 2 mg/ml to about 5 mg/ml, about 3 mg/ml to about 5 mg/ml, or about 4 mg/ml to about 5 mg/ml. In a particular embodiment, the concentration of the bispecific antibody is about 1 mg/ml. In some embodiments, the concentration of the bispecific antibody is about 3 mg/ml. In some embodiments, the pharmaceutical composition is formulated as a DP.

In some embodiments, the surfactant is P188 and the concentration of the P188 is about 0.08% (w/v). In other embodiments, the molar ratio of the surfactant (e.g., P188) to the bispecific antibody is from 5 to 50, from 5 to 25, from 10 to 15, or from 15 to 20, e.g., about 10, 11, 12, 13, 14, 15, 16, 17, 18, 19, or 20. In a particular embodiment, the molar ratio of surfactant (e.g., P188) to the bispecific antibody is about 14. In another particular embodiment, the molar ratio of surfactant (e.g., P188) to the bispecific antibody about 11.5

In some embodiments, the pharmaceutical composition further includes histidine acetate or histidine HCl at a concentration of about 10 mM or 20 mM and/or sucrose at a concentration of about 240 mM.

In some embodiments, the bispecific antibody (e.g., anti-CD3 bispecific antibody; e.g., T cell-dependent bispecific antibody) includes an anti-target arm and an anti-CD3 arm. In some embodiments, the anti-target arm of the bispecific antibody (e.g., anti-CD3 bispecific antibody; e.g., T cell-dependent bispecific antibody) is an anti-CD20 arm, an anti-FcRH5 arm, or an anti-HER2 arm.

In some embodiments, the anti-CD3 arm includes a CD3-binding domain including a hypervariable region (HVR)-H1 comprising the amino acid sequence of SEQ ID NO: 9; an HVR-H2 comprising the amino acid sequence of SEQ ID NO: 10; an HVR-H3 comprising the amino acid sequence of SEQ ID NO: 11; an HVR-L1 comprising the amino acid sequence of SEQ ID NO: 12; an HVR-L2 comprising the amino acid sequence of SEQ ID NO: 13; and an HVR-L3 comprising the amino acid sequence of SEQ ID NO: 14. In some embodiments, the CD3-binding domain comprises (a) a heavy chain variable (VH) domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 15; (b) a light chain variable (VL) domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 16; or (c) a VH domain as in (a) and a VL domain as in (b). In certain embodiments, the VH domain of the CD3-binding domain comprises the amino acid sequence of SEQ ID NO: 15 and the VL domain of the CD3-binding domain comprises the amino acid sequence of SEQ ID NO: 16. For example, in some embodiments in which the VH domain of the CD3-binding domain comprises the amino acid sequence of SEQ ID NO: 15 and the VL domain of the CD3-binding domain comprises the amino acid sequence of SEQ ID NO: 16, the bispecific antibody is mosunetuzumab.

In some embodiments, the anti-target arm is an anti-CD20 arm comprising a CD20-binding domain including an HVR-H1 comprising the amino acid sequence of SEQ ID NO: 1; an HVR-H2 comprising the amino acid sequence of SEQ ID NO: 2; an HVR-H3 comprising the amino acid sequence of SEQ ID NO: 3; an HVR-L1 comprising the amino acid sequence of SEQ ID NO: 4; an HVR-L2 comprising the amino acid sequence of SEQ ID NO: 5; and an HVR-L3 comprising the amino acid sequence of SEQ ID NO: 6. In some embodiments, the CD20-binding domain comprises (a) a VH domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 7; (b) a VL domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 8; or (c) a VH domain as in (a) and a VL domain as in (b). In certain embodiments, the VH domain of the CD20-binding domain comprises the amino acid sequence of SEQ ID NO: 7 and the VL domain of the CD20-binding domain comprises the amino acid sequence of SEQ ID NO: 8. For example, in some embodiments in which the VH domain of the CD20-binding domain comprises the amino acid sequence of SEQ ID NO: 7 and the VL domain of the CD20-binding domain comprises the amino acid sequence of SEQ ID NO: 8, the bispecific antibody is mosunetuzumab.

In another aspect, the disclosure features a pharmaceutical composition comprising a bispecific antibody, PS20, and a carrier, wherein the molar ratio of the PS20 to the bispecific antibody is 100 or less, the PS20 is at a concentration from 0.01% to 0.12% weight-by-volume (w/v), and the bispecific antibody comprises an anti-CD3 arm and an anti-CD20 arm, wherein the anti-CD3 arm comprises a CD3-binding domain comprising: an HVR-H1 comprising the amino acid sequence of SEQ ID NO: 9, an HVR-H2 comprising the amino acid sequence of SEQ ID NO: 10, an HVR-H3 comprising the amino acid sequence of SEQ ID NO: 11, an HVR-L1 comprising the amino acid sequence of SEQ ID NO: 12, an HVR-L2 comprising the amino acid sequence of SEQ ID NO: 13, and an HVR-L3 comprising the amino acid sequence of SEQ ID NO: 14; and the anti-CD20 arm comprises a CD20-binding domain comprising: an HVR-H1 comprising the amino acid sequence of SEQ ID NO: 1, an HVR-H2 comprising the amino acid sequence of SEQ ID NO: 2, an HVR-H3 comprising the amino acid sequence of SEQ ID NO: 3, an HVR-L1 comprising the amino acid sequence of SEQ ID NO: 4, an HVR-L2 comprising the amino acid sequence of SEQ ID NO: 5, and an HVR-L3 comprising the amino acid sequence of SEQ ID NO: 6.

In some embodiments, the CD3-binding domain comprises (a) a VH domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 15; (b) a VL domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 16; or (c) a VH domain as in (a) and a VL domain as in (b). In certain embodiments, the VH domain of the CD3-binding domain comprises the amino acid sequence of SEQ ID NO: 15 and the VL domain of the CD3-binding domain comprises the amino acid sequence of SEQ ID NO: 16. Additionally or alternatively, in some embodiments, the CD20-binding domain comprises (a) a VH domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 7; (b) a VL domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 8; or (c) a VH domain as in (a) and a VL domain as in (b). In certain embodiments, the VH domain of the CD20-binding domain comprises the amino acid sequence of SEQ ID NO: 7 and the VL domain of the CD20-binding domain comprises the amino acid sequence of SEQ ID NO: 8. For example, in some embodiments of the pharmaceutical composition, the bispecific antibody is mosunetuzumab.

In some embodiments, the anti-CD3 arm includes a CD3-binding domain including a hypervariable region (HVR)-H1 comprising the amino acid sequence of SEQ ID NO: 65; an HVR-H2 comprising the amino acid sequence of SEQ ID NO: 66; an HVR-H3 comprising the amino acid sequence of SEQ ID NO: 67; an HVR-L1 comprising the amino acid sequence of SEQ ID NO: 68; an HVR-L2 comprising the amino acid sequence of SEQ ID NO: 69; and an HVR-L3 comprising the amino acid sequence of SEQ ID NO: 70. In some embodiments, the CD3-binding domain comprises (a) a heavy chain variable (VH) domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 71; (b) a light chain variable (VL) domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 72; or (c) a VH domain as in (a) and a VL domain as in (b). In certain embodiments, the VH domain of the CD3-binding domain comprises the amino acid sequence of SEQ ID NO: 71 and the VL domain of the CD3-binding domain comprises the amino acid sequence of SEQ ID NO: 72. For example, in some embodiments in which the VH domain of the CD3-binding domain comprises the amino acid sequence of SEQ ID NO: 71 and the VL domain of the CD3-binding domain comprises the amino acid sequence of SEQ ID NO: 72, the bispecific antibody is cevostamab.

In some embodiments, the anti-target arm is an anti-FcRH5 arm comprising a FcRH5-binding domain including an HVR-H1 comprising the amino acid sequence of SEQ ID NO: 57; an HVR-H2 comprising the amino acid sequence of SEQ ID NO: 58; an HVR-H3 comprising the amino acid sequence of SEQ ID NO: 59; an HVR-L1 comprising the amino acid sequence of SEQ ID NO: 60; an HVR-L2 comprising the amino acid sequence of SEQ ID NO: 61; and an HVR-L3 comprising the amino acid sequence of SEQ ID NO: 62. In some embodiments, the FcRH5-binding domain comprises (a) a VH domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 63; (b) a VL domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 64; or (c) a VH domain as in (a) and a VL domain as in (b). In certain embodiments, the VH domain of the FcRH5-binding domain comprises the amino acid sequence of SEQ ID NO: 63 and the VL domain of the FcRH5-binding domain comprises the amino acid sequence of SEQ ID NO: 64. For example, in some embodiments in which the VH domain of the FcRH5-binding domain comprises the amino acid sequence of SEQ ID NO: 63 and the VL domain of the FcRH5-binding domain comprises the amino acid sequence of SEQ ID NO: 64, the bispecific antibody is cevostamab.

In another aspect, the disclosure features a pharmaceutical composition comprising a bispecific antibody, PS20, and a carrier, wherein the molar ratio of the PS20 to the bispecific antibody is 100 or less, the PS20 is at a concentration from 0.01% to 0.12% weight-by-volume (w/v), and the bispecific antibody comprises an anti-CD3 arm and an anti-FcRH5 arm, wherein the anti-CD3 arm comprises a CD3-binding domain comprising: an HVR-H1 comprising the amino acid sequence of SEQ ID NO: 65, an HVR-H2 comprising the amino acid sequence of SEQ ID NO: 66, an HVR-H3 comprising the amino acid sequence of SEQ ID NO: 67, an HVR-L1 comprising the amino acid sequence of SEQ ID NO: 68, an HVR-L2 comprising the amino acid sequence of SEQ ID NO: 69, and an HVR-L3 comprising the amino acid sequence of SEQ ID NO: 70; and the anti-FcRH5 arm comprises a FcRH5-binding domain comprising: an HVR-H1 comprising the amino acid sequence of SEQ ID NO: 57, an HVR-H2 comprising the amino acid sequence of SEQ ID NO: 58, an HVR-H3 comprising the amino acid sequence of SEQ ID NO: 59, an HVR-L1 comprising the amino acid sequence of SEQ ID NO: 60, an HVR-L2 comprising the amino acid sequence of SEQ ID NO: 61, and an HVR-L3 comprising the amino acid sequence of SEQ ID NO: 62.

In some embodiments, the CD3-binding domain comprises (a) a VH domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 71; (b) a VL domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 72; or (c) a VH domain as in (a) and a VL domain as in (b). In certain embodiments, the VH domain of the CD3-binding domain comprises the amino acid sequence of SEQ ID NO: 71 and the VL domain of the CD3-binding domain comprises the amino acid sequence of SEQ ID NO: 72. Additionally or alternatively, in some embodiments, the FcRH5-binding domain comprises (a) a VH domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 63; (b) a VL domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 64; or (c) a VH domain as in (a) and a VL domain as in (b). In certain embodiments, the VH domain of the FcRH5-binding domain comprises the amino acid sequence of SEQ ID NO: 63 and the VL domain of the FcRH5-binding domain comprises the amino acid sequence of SEQ ID NO: 64. For example, in some embodiments of the pharmaceutical composition, the bispecific antibody is cevostamab.

In some embodiments, the anti-CD3 arm includes a CD3-binding domain including a hypervariable region (HVR)-H1 comprising the amino acid sequence of SEQ ID NO: 109; an HVR-H2 comprising the amino acid sequence of SEQ ID NO: 110; an HVR-H3 comprising the amino acid sequence of SEQ ID NO: 111; an HVR-L1 comprising the amino acid sequence of SEQ ID NO: 112; an HVR-L2 comprising the amino acid sequence of SEQ ID NO: 113; and an HVR-L3 comprising the amino acid sequence of SEQ ID NO: 114. In some embodiments, the CD3-binding domain comprises (a) a heavy chain variable (VH) domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 115; (b) a light chain variable (VL) domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 116; or (c) a VH domain as in (a) and a VL domain as in (b). In certain embodiments, the VH domain of the CD3-binding domain comprises the amino acid sequence of SEQ ID NO: 115 and the VL domain of the CD3-binding domain comprises the amino acid sequence of SEQ ID NO: 116. For example, in some embodiments in which the VH domain of the CD3-binding domain comprises the amino acid sequence of SEQ ID NO: 115 and the VL domain of the CD3-binding domain comprises the amino acid sequence of SEQ ID NO: 116, the bispecific antibody is runimotamab.

In some embodiments, the anti-target arm is an anti-HER2 arm comprising a HER2-binding domain including an HVR-H1 comprising the amino acid sequence of SEQ ID NO: 93; an HVR-H2 comprising the amino acid sequence of SEQ ID NO: 94; an HVR-H3 comprising the amino acid sequence of SEQ ID NO: 95; an HVR-L1 comprising the amino acid sequence of SEQ ID NO: 96; an HVR-L2 comprising the amino acid sequence of SEQ ID NO: 97; and an HVR-L3 comprising the amino acid sequence of SEQ ID NO: 98. In some embodiments, the HER2-binding domain comprises (a) a VH domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 99; (b) a VL domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 100; or (c) a VH domain as in (a) and a VL domain as in (b). In certain embodiments, the VH domain of the HER2-binding domain comprises the amino acid sequence of SEQ ID NO: 99 and the VL domain of the HER2-binding domain comprises the amino acid sequence of SEQ ID NO: 100. For example, in some embodiments in which the VH domain of the HER2-binding domain comprises the amino acid sequence of SEQ ID NO: 99 and the VL domain of the HER2-binding domain comprises the amino acid sequence of SEQ ID NO: 100, the bispecific antibody is runimotamab.

In another aspect, the disclosure features a pharmaceutical composition comprising a bispecific antibody, PS20, and a carrier, wherein the molar ratio of the PS20 to the bispecific antibody is 100 or less, the PS20 is at a concentration from 0.01% to 0.12% weight-by-volume (w/v), and the bispecific antibody comprises an anti-CD3 arm and an anti-HER2 arm, wherein the anti-CD3 arm comprises a CD3-binding domain comprising: an HVR-H1 comprising the amino acid sequence of SEQ ID NO: 109, an HVR-H2 comprising the amino acid sequence of SEQ ID NO: 110, an HVR-H3 comprising the amino acid sequence of SEQ ID NO: 111, an HVR-L1 comprising the amino acid sequence of SEQ ID NO: 112, an HVR-L2 comprising the amino acid sequence of SEQ ID NO: 113, and an HVR-L3 comprising the amino acid sequence of SEQ ID NO: 114; and the anti-HER2 arm comprises a HER2-binding domain comprising: an HVR-H1 comprising the amino acid sequence of SEQ ID NO: 93, an HVR-H2 comprising the amino acid sequence of SEQ ID NO: 94, an HVR-H3 comprising the amino acid sequence of SEQ ID NO: 95, an HVR-L1 comprising the amino acid sequence of SEQ ID NO: 96, an HVR-L2 comprising the amino acid sequence of SEQ ID NO: 97, and an HVR-L3 comprising the amino acid sequence of SEQ ID NO: 98.

In some embodiments, the CD3-binding domain comprises (a) a VH domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 115; (b) a VL domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 116; or (c) a VH domain as in (a) and a VL domain as in (b). In certain embodiments, the VH domain of the CD3-binding domain comprises the amino acid sequence of SEQ ID NO: 115 and the VL domain of the CD3-binding domain comprises the amino acid sequence of SEQ ID NO: 116. Additionally or alternatively, in some embodiments, the HER2-binding domain comprises (a) a VH domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 99; (b) a VL domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 100; or (c) a VH domain as in (a) and a VL domain as in (b). In certain embodiments, the VH domain of the HER2-binding domain comprises the amino acid sequence of SEQ ID NO: 99 and the VL domain of the HER2-binding domain comprises the amino acid sequence of SEQ ID NO: 100. For example, in some embodiments of the pharmaceutical composition, the bispecific antibody is runimotamab.

In some embodiments, the bispecific antibody comprises an aglycosylation site mutation, e.g., a substitution mutation. In some embodiments, the antibody (e.g., bispecific antibody, e.g., TDB or TCB) includes one or more substitution mutations in the Fc region. In certain embodiments, the one or more substitution mutations can reduce effector function of the bispecific antibody.

In embodiments in which the therapeutic protein is an antibody (e.g., bispecific antibody, e.g., TDB or TCB), the antibody can be an IgG antibody (e.g., an IgG₁ antibody or an IgG₄ antibody). In particular instances, the antibody is a bispecific antibody which is an IgG₁ antibody. In some embodiments, the one or more substitution mutations is at one or more amino acid residues selected from the group consisting of N297, L234, L235, D265, and/or P329 (EU numbering). In some embodiments the one or more substitution mutations in the Fc region comprises one or more knob-in-hole mutations. In some embodiments, the anti-target arm (e.g., the anti-CD20 arm, the anti-FcRH5 arm, or the anti-HER2 arm) includes T366W and N297G substitution mutations, and the anti-CD3 arm includes T366S, L368A, Y407V, and N297G substitution mutations.

At least one of the arms of the bispecific antibody can be monoclonal (e.g., the anti-CD3 arm can be monoclonal, the anti-target arm can be monoclonal, or both the anti-CD3 arm and the anti-target arm can be monoclonal). In some embodiments, at least one of the arms of the bispecific antibody is human, humanized, or chimeric (e.g., the anti-CD3 arm is human, humanized, or chimeric; the anti-target arm is human, humanized, or chimeric; or both the anti-CD3 arm and the anti-target arm are human, humanized, or chimeric).

In some embodiments, the bispecific antibody (e.g., anti-CD20/anti-CD3 bispecific antibody; e.g., T-cell engaging bispecific antibody) comprises at least one Fab molecule which specifically binds to CD20 comprising the following six hypervariable regions (HVRs): (a) an HVR-H1 comprising the amino acid sequence of SEQ ID NO: 37; (b) an HVR-H2 comprising the amino acid sequence of SEQ ID NO: 38; (c) an HVR-H3 comprising the amino acid sequence of (SEQ ID NO: 39); (d) an HVR-L1 comprising the amino acid sequence of SEQ ID NO: 40; (e) an HVR-L2 comprising the amino acid sequence of SEQ ID NO: 41; and (f) an HVR-L3 comprising the amino acid sequence of SEQ ID NO: 42. In some embodiments, the bispecific antibody comprises at least one Fab molecule which specifically binds to CD20 comprising (a) a heavy chain variable VH domain comprising an amino acid sequence having at least 95% (e.g., at least 95%, at least 96%, at least 97%, at least 98%, or at least 99%; e.g., 95%, 96%, 97%, 98%, 99%, or 100%) sequence identity to the amino acid sequence of SEQ ID NO: 43; (b) a variable light (VL) domain comprising an amino acid sequence having at least 95% (e.g., at least 95%, at least 96%, at least 97%, at least 98%, or at least 99%; e.g., 95%, 96%, 97%, 98%, 99%, or 100%) sequence identity to the amino acid sequence of SEQ ID NO: 44; or (c) a VH domain as in (a) and a VL domain as in (b). In some embodiments, the Fab molecule which specifically binds to CD20 comprises (a) a VH domain comprising an amino acid sequence of SEQ ID NO: 43 and (b) a VL domain comprising an amino acid sequence of SEQ ID NO: 44.

In some embodiments, the bispecific antibody comprises at least one Fab molecule which specifically binds to CD3 comprising the following six HVRs: (a) an HVR-H1 comprising the amino acid sequence of SEQ ID NO: 45; (b) an HVR-H2 comprising the amino acid sequence of SEQ ID NO: 46; (c) an HVR-H3 comprising the amino acid sequence of SEQ ID NO: 47; (d) an HVR-L1 comprising the amino acid sequence of SEQ ID NO: 48; (e) an HVR-L2 comprising the amino acid sequence of SEQ ID NO: 49; and (f) an HVR-L3 comprising the amino acid sequence of SEQ ID NO: 50. In some embodiments, the bispecific antibody comprises at least one Fab molecule which specifically binds to CD3 comprising (a) a heavy chain variable VH domain comprising an amino acid sequence having at least 95% (e.g., at least 95%, at least 96%, at least 97%, at least 98%, or at least 99%; e.g., 95%, 96%, 97%, 98%, 99%, or 100%) sequence identity to the amino acid sequence of SEQ ID NO: 51; (b) a variable light (VL) domain comprising an amino acid sequence having at least 95% (e.g., at least 95%, at least 96%, at least 97%, at least 98%, or at least 99%; e.g., 95%, 96%, 97%, 98%, 99%, or 100%) sequence identity to the amino acid sequence of SEQ ID NO: 52; or (c) a VH domain as in (a) and a VL domain as in (b). In some embodiments, the Fab molecule which specifically binds to CD3 comprises (a) a VH domain comprising an amino acid sequence of SEQ ID NO: 51 and (b) a VL domain comprising an amino acid sequence of SEQ ID NO: 52.

In another aspect, the disclosure features a pharmaceutical composition comprising a bispecific antibody, PS20, and a carrier, wherein the molar ratio of the PS20 to the bispecific antibody is 100 or less, the PS20 is at a concentration from 0.01% to 0.12% weight-by-volume (w/v), and the bispecific antibody comprises one Fab molecule which specifically binds CD3 and two Fab molecules which each specifically binds CD20, wherein the Fab molecule that specifically binds to CD3 comprises a CD3-binding domain comprising: an HVR-H1 comprising the amino acid sequence of SEQ ID NO: 45, an HVR-H2 comprising the amino acid sequence of SEQ ID NO: 46, an HVR-H3 comprising the amino acid sequence of SEQ ID NO: 47, an HVR-L1 comprising the amino acid sequence of SEQ ID NO: 48, an HVR-L2 comprising the amino acid sequence of SEQ ID NO: 49, and an HVR-L3 comprising the amino acid sequence of SEQ ID NO: 50; and the two Fabs which specifically bind to CD20 each comprises a CD20-binding domain comprising: an HVR-H1 comprising the amino acid sequence of SEQ ID NO: 37, an HVR-H2 comprising the amino acid sequence of SEQ ID NO: 38, an HVR-H3 comprising the amino acid sequence of SEQ ID NO: 39, an HVR-L1 comprising the amino acid sequence of SEQ ID NO: 40, an HVR-L2 comprising the amino acid sequence of SEQ ID NO: 41, and an HVR-L3 comprising the amino acid sequence of SEQ ID NO: 42.

In some embodiments, the CD3-binding domain comprises (a) a VH domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 51; (b) a VL domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 52; or (c) a VH domain as in (a) and a VL domain as in (b). In certain embodiments, the VH domain of the CD3-binding domain comprises the amino acid sequence of SEQ ID NO: 51 and the VL domain of the CD3-binding domain comprises the amino acid sequence of SEQ ID NO: 52. Additionally or alternatively, in some embodiments, each CD20-binding domain comprises (a) a VH domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 43; (b) a VL domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 44; or (c) a VH domain as in (a) and a VL domain as in (b). In certain embodiments, the VH domain of each CD20-binding domain comprises the amino acid sequence of SEQ ID NO: 43 and the VL domain of each CD20-binding domain comprises the amino acid sequence of SEQ ID NO: 44. For example, in some embodiments of the pharmaceutical composition, the bispecific antibody is glofitamab.

In some embodiments, the bispecific antibody comprises a Fab molecule which specifically binds to CD3, wherein (a) the variable domains of the Fab heavy and light chain are exchanged or (b) the constant domains of the Fab heavy and light chain are exchanged. In some embodiments, the bispecific antibody comprises at least one Fab molecule which specifically binds to CD20, wherein in the constant domain CL of the Fab molecule the amino acid at position 124 is substituted by lysine (K) (numbering according to Kabat) and the amino acid at position 123 is substituted by arginine (R) or lysine (K) (numbering according to Kabat), and wherein in the constant domain CH1 of the Fab molecule the amino acid at position 147 is substituted by glutamic acid (E) (EU numbering) and the amino acid at position 213 is substituted by glutamic acid (E) (EU numbering). In some embodiments, the bispecific antibody is bivalent for CD20 and monovalent for CD3. In some embodiments, the bispecific antibody comprises two Fab molecule which specifically bind to CD20 and one Fab molecule which specifically binds to CD3.

In some embodiments, the bispecific antibody comprises (a) a first Fab molecule which specifically binds to CD20; (b) a second Fab molecule which specifically binds to CD3; (c) a third Fab molecule which specifically binds to CD20; and (d) an Fc domain composed of a first and a second subunit capable of stable association; wherein the third Fab molecule under (c) is identical to the first Fab molecule under (a); wherein in the constant domain CL of the first Fab molecule under (a) and the third Fab molecule under (c) the amino acid at position 124 is substituted by lysine (K) (numbering according to Kabat) and the amino acid at position 123 is substituted by arginine (R) or lysine (K) (numbering according to Kabat); and wherein in the constant domain CH1 of the first Fab molecule under (a) and the third Fab molecule under (c) the amino acid at position 147 is substituted by glutamic acid (E) (EU numbering) and the amino acid at position 213 is substituted by glutamic acid (E) (EU numbering); and wherein the first Fab molecule under (a) is fused at the C-terminus of the Fab heavy chain to the N-terminus of the Fab heavy chain of the second Fab molecule under (b), and the second Fab molecule under (b) and the third Fab molecule under (c) are each fused at the C-terminus of the Fab heavy chain to the N-terminus of one of the subunits of the Fc domain under (d).

In some embodiments, the bispecific antibody is a humanized antibody. In some embodiments, the bispecific antibody is a chimeric antibody.

In some embodiments, the bispecific antibody comprises an Fc domain, wherein the Fc domain is an IgG Fc domain. In some embodiments, the IgG Fc domain is an IgG1 Fc domain. In some embodiments, the IgG Fc domain comprises a mutation at amino acid residue N297 (EU numbering) that results in the absence of glycosylation. In some embodiments, the mutation at amino acid residue N297 is a substitution mutation. In some embodiments, the mutation at amino acid residue N297 reduces effector function of the Fc region. In some embodiments, the mutation is an N297G or N297A mutation. In some embodiments, the bispecific antibody comprises a mutation in the Fc region that reduces effector function. In some embodiments, the mutation is a substitution mutation. In some embodiments, the substitution mutation is at amino acid residue L234, L235, D265, and/or P329 (EU numbering). In some embodiments, mutation is selected from the group consisting of L234A, L235A, D265A, and P329G.

In some embodiments, the bispecific antibody comprises one or more heavy chain constant domains, wherein the one or more heavy chain constant domains are selected from a first CH1 (CH1*₁*) domain, a first CH2 (CH2*₁*) domain, a first CH3 (CH3*₁*) domain, a second CH1 (CH1*₂*) domain, second CH2 (CH2*₂*) domain, and a second CH3 (CH3*₂*) domain. In some embodiments, at least one of the one or more heavy chain constant domains is paired with another heavy chain constant domain. In some embodiments, the CH3*₁* and CH3*₂* domains each comprise a protuberance or cavity, and wherein the protuberance or cavity in the CH3*₁* domain is positionable in the cavity or protuberance, respectively, in the CH3*₂* domain. In some embodiments, the CH3, and CH3*₂* domains meet at an interface between the protuberance and cavity. In some embodiments, the CH2*₁* and CH2*₂* domains each comprise a protuberance or cavity, and wherein the protuberance or cavity in the CH2*₁* domain is positionable in the cavity or protuberance, respectively, in the CH2*₂* domain. In some embodiments, the CH2*₁* and CH2*₂* domains meet at an interface between said protuberance and cavity.

In some embodiments of any of the preceding embodiments, the CD3-binding domain binds to a human CD3 polypeptide or a cynomolgus monkey (cyno) CD3 polypeptide. The human CD3 polypeptide or the cyno CD3 polypeptide can be, e.g., a human CD3ε polypeptide or a cyno CD3ε polypeptide, respectively. Alternatively, the human CD3 polypeptide or the cyno CD3 polypeptide can be a human CD3y polypeptide or a cyno CD3γ polypeptide, respectively.

In some embodiments, the pharmaceutical composition is in a unit dosage form (e.g., liquid formulation for infusion, liquid formulation for injection, or liquid formulation for dilution). In a particular embodiment, the pharmaceutical composition is a liquid formulation for dilution. In a particular embodiment, the liquid formulation for dilution is supplied in a container having a volume of about 50 ml (e.g., about 40 ml, about 45 ml, about 46 ml, about 47 ml, about 48 ml, about 49 ml, about 50 ml, about 51 ml, about 52 ml, about 53 ml, about 54 ml, about 55 ml, or about 60 ml). In some embodiments, the volume of the liquid formulation for dilution is between 20-40 ml (e.g., between 20-30 ml, between 30-40 ml, between 20-35 ml, between 25-40 ml, between 25-35 ml, or between 28-32 ml; e.g., about 20 ml, about 25 ml, about 26 ml, about 27 ml, about 28 ml, about 29 ml, about 30 ml, about 31 ml, about 32 ml, about 33 ml, about 34 ml, about 35 ml, or about 40 ml). In a particular embodiment, the volume of the liquid formulation for dilution is about 30 ml. In some embodiments, the volume of the liquid formulation for dilution is between 10-20 ml (e.g., between 10-15 ml, between 15-20 ml, between 13-20 ml, between 10-17 ml, between 13-17 ml, or between 14-16 ml; e.g., about 10 ml, about 11 ml, about 12 ml, about 13 ml, about 14 ml, about 15 ml, about 16 ml, about 17 ml, about 18 ml, about 19 ml, or about 20 ml). In a particular embodiment, the volume of the liquid formulation for dilution is about 15 ml.

In another particular embodiment, the liquid formulation for dilution is supplied in a container having a volume of about 1 ml or about 2 ml (e.g., about 0.5 ml, about 0.6 ml, about 0.7 ml, about 0.8 ml, about 0.9 ml, about 1 ml, about 1.5 ml, about 1.6 ml, about 1.7 ml, about 1.8 ml, about 1.9 ml, about 2 ml, about 2.1 ml, about 2.2 ml, about 2.3 ml, about 2.4 ml, about 2.5 ml, or about 3 ml). In some embodiments, the liquid formulation for dilution is supplied in a container having a volume of about 2.5 ml. In some embodiments, the volume of the liquid formulation for dilution is between 0.2-2 ml (e.g., between 0.2-1.5 ml, between 0.5-2 ml, between 0.5-1 ml, or between 0.8-1.2 ml; e.g., about 0.2 ml, about 0.5 ml, about 0.6 ml, about 0.7 ml, about 0.8 ml, about 0.9 ml, about 1 ml, about 1.1 ml, about 1.2 ml, about 1.3 ml, about 1.4 ml, about 1.5 ml, or about 2 ml). In a particular embodiment, the volume of the liquid formulation for dilution is about 0.5 ml, about 0.9 ml, or about 1 ml.

In yet another particular embodiment, the liquid formulation for dilution is supplied in a container having a volume of about 15 ml (e.g., about 10 ml, about 11 ml, about 12 ml, about 13 ml, about 14 ml, about 15 ml, about 16 ml, about 17 ml, about 18 ml, about 19 ml, or about 20 ml). In some embodiments, the liquid formulation for dilution is supplied in a container having a volume of about 10 ml. In some embodiments, the volume of the liquid formulation for dilution is between 4-12 ml (e.g., between 4-8 ml, between 8-12 ml, between 4-10 ml, between 6-12 ml, between 6-10 ml, or between 7-9 ml; e.g., about 4 ml, about 5 ml, about 6 ml, about 7 ml, about 8 ml, about 9 ml, about 10 ml, about 11 ml, or about 12 ml). In a particular embodiment, the volume of the liquid formulation for dilution is about 8 ml.

In some embodiments, the liquid formulation is for dilution with a diluent. In some embodiments, the liquid formulation is for dilution with a saline solution. In some embodiments, the liquid formulation is for dilution with a normal saline solution. In some embodiments, the normal saline solution comprises sodium chloride (NaCl). In some embodiments, the normal saline solution comprises between 0.1-1.5% (e.g., between 0.1-1.2%, between 0.3-1.5%, between 0.4-0.5%, between 0.3-1%, between 0.8-1%, between 0.85-0.95%; e.g., about 0.1%, about 0.3%, about 0.4%, about 0.45%, about 0.5%, about 0.6%, about 0.7%, about 0.8%, about 0.85%, about 0.9%, about 0.95%, about 1%, or about 1.2%) (w/v) NaCl). In some embodiments, the liquid formulation (e.g., comprising an anti-FcRH5/anti-CD3 bispecific antibody; e.g., anti-FcRH5/anti-CD3 TDB; e.g., cevostamab) is not diluted.

In some embodiments, the pharmaceutical composition is in a container (e.g., a stainless steel container or a nickel-steel alloy container (e.g., HASTELLOY^{®}), such as a tank (e.g., mini-tank), or can (e.g., mini-can) or a glass container (e.g., a glass vial or a glass ampule).

In some embodiments, the pharmaceutical composition comprises no more than 1,000 particles having a diameter ≥ 2 µm per ml (e.g., 900 or fewer, 800 or fewer, 700 or fewer, 600 or fewer, 500 or fewer, 400 or fewer, 300 or fewer, 200 or fewer, or 100 or fewer particles having a diameter ≥ 2 µm per ml, e.g., from 0 to 100, from 100 to 200, from 200 to 300, from 300 to 400, from 400 to 500, from 500 to 600, from 600 to 700, from 700 to 800, from 800 to 900, or from 900 to 1,000 particles having a diameter ≥ 2 µm per ml). In some embodiments, the carrier is water.

In some embodiments, the pharmaceutical composition has a shelf-life of at least 36 months when stored at 5 °C ± 3 °C and protected from light (e.g., at least 38 months, at least 40 months, at least 42 months, at least 44 months, at least 46 months, at least 48 months, at least 60 months, at least 72 months, or at least 96 months). In some embodiments, the pharmaceutical composition is stable through one or more freeze-thaw cycles (e.g., two or more freeze-thaw cycles, three or more freeze-thaw cycles, four or more freeze-thaw cycles, five or more freeze-thaw cycles, six or more freeze-thaw cycles, eight or more freeze-thaw cycles, or more). In a particular embodiment, the pharmaceutical composition is stable through three or more freeze-thaw cycles. In some embodiments, the pharmaceutical composition is stable for about two weeks or longer at about 25 °C (e.g., about three weeks, about four weeks, about six weeks, about eight weeks, about 10 weeks, about 12 weeks, about 24 weeks, or longer at about 25 °C). In a particular embodiment, the pharmaceutical composition is stable for about four weeks or longer at about 25 °C. In some embodiments, the pharmaceutical composition is stable for about 48 months or longer at -20 °C (e.g., about 48 months, about 60 months, about 72 months, about 84 months, about 96 months, or longer at -20 °C).

In some embodiments of any of the aspects and embodiments recited above or herein, the pharmaceutical composition has a purity of about 85% or higher, e.g., as assessed by size-exclusion high-performance liquid chromatography (SE-HPLC). In some embodiments, the purity is about 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, e.g., from 85% to 90%, from 90% to 95%, or from 95% to 100%, e.g., as assessed by SE-HPLC. In a particular embodiment, the pharmaceutical composition has a purity of about 90% or higher as assessed by SE-HPLC, or about 95% or higher as assessed by SE-HPLC. In some embodiments, the pharmaceutical composition has a purity of about 95% or higher as assessed by SE-HPLC for about 36 months or longer at about 5 °C (e.g., 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, as assessed by SE-HPLC for about 36 months or longer at about 5 °C, e.g., from 85% to 90%, from 90% to 95%, or from 95% to 100%, as assessed by SE-HPLC for about 36 months or longer at about 5 °C). In a particular embodiment, the pharmaceutical composition has a purity of about 95% or higher as assessed by SE-HPLC for about 42 months or longer at about 5 °C, e.g., for about 42 months, for about 60 months, for about 72 months, for about 84 months, for about 96 months, or longer, at about 5 °C.

In any of the preceding aspects and embodiments, the pharmaceutical composition has a purity of about 75% or higher as assessed by non-reduced capillary electrophoresis sodium dodecyl sulfate (CE-SDS) assay (e.g., about 76% or higher, about 77% or higher, about 78% or higher, about 79% or higher, about 80% or higher, about 81% or higher, about 82% or higher, about 83% or higher, about 84% or higher, 85% or higher, about 86% or higher, about 87% or higher, about 88% or higher, about 89% or higher, about 90% or higher, about 91% or higher, about 92% or higher, about 93% or higher, about 94% or higher, about 95% or higher, about 96% or higher, about 97% or higher, about 98% or higher, about 99% or higher, as assessed by non-reduced CE-SDS assay, e.g., from 75% to 80%, from 80% to 85%, from 85% to 90%, from 90% to 95%, or from 95% to 100%, as assessed by non-reduced CE-SDS assay), In a particular embodiment, the pharmaceutical composition has a purity of about 80% or higher as assessed by non-reduced CE-SDS assay. For example, in some embodiments, the pharmaceutical composition has a purity of about 85% or higher as assessed by non-reduced CE-SDS assay. In some embodiments, the pharmaceutical composition has a purity of about 85% or higher as assessed by non-reduced CE-SDS assay for about 36 months or longer at about 5 °C (e.g., 85% or higher, about 86% or higher, about 87% or higher, about 88% or higher, about 89% or higher, about 90% or higher, about 91% or higher, about 92% or higher, about 93% or higher, about 94% or higher, about 95% or higher, about 96% or higher, about 97% or higher, about 98% or higher, about 99% or higher, as assessed by non-reduced CE-SDS assay, e.g., from 85% to 90%, from 90% to 95%, or from 95% to 100%, as assessed by non-reduced CE-SDS assay for about 36 months or longer at about 5 °C). In some embodiments, the pharmaceutical composition has a purity of about 85% or higher as assessed by non-reduced CE-SDS assay for about 42 months or longer at about 5 °C (e.g., 85% or higher, about 86% or higher, about 87% or higher, about 88% or higher, about 89% or higher, about 90% or higher, about 91% or higher, about 92% or higher, about 93% or higher, about 94% or higher, about 95% or higher, about 96% or higher, about 97% or higher, about 98% or higher, about 99% or higher, as assessed by non-reduced CE-SDS assay, e.g., from 85% to 90%, from 90% to 95%, or from 95% to 100%, as assessed by non-reduced CE-SDS assay for about 42 months or longer at about 5 °C). In some embodiments, the non-reduced CE-SDS assay is a microchip CE-SDS (mCE-SDS) assay.

In some embodiments, the pharmaceutical composition having the any of the above shelf-life, purity, or stability properties recited above is a DS. In other embodiments, the pharmaceutical composition having the any of the shelf-life, purity, or stability properties recited above is a DP. In some embodiments, the pharmaceutical composition having the any of the shelf-life or stability properties recited above is frozen (e.g., stored at a temperature between -80 °C and 2 °C (e.g., about -40 °C or -20 °C).

In some embodiments of any of the preceding aspects and embodiments, the pharmaceutical composition is formulated for intravenous, subcutaneous, intramuscular, topical, oral, transdermal, intraperitoneal, intraorbital, intranasal, intrathecal, or intraventricular administration. For example, in a particular embodiment, the pharmaceutical composition is formulated for intravenous administration. In other embodiments, the pharmaceutical composition is formulated for subcutaneous administration. In some embodiments, the pharmaceutical composition does not contain a preservative. In some embodiments, the pharmaceutical composition (e.g., comprising an anti-FcRH5/anti-CD3 bispecific antibody; e.g., anti-FcRH5/anti-CD3 TDB; e.g., cevostamab) is formulated for administration by infusion without dilution. In some embodiments, the pharmaceutical composition is formulated for administration by infusion after dilution with saline or a diluent (e.g., a saline solution; e.g., a normal saline solution; e.g., a normal saline solution comprising 0.45% or 0.9% (w/v) NaCl). In some embodiments, the liquid formulation is for dilution with an aqueous solution. In some embodiments, the liquid formulation is for dilution with a saline solution. In some embodiments, the liquid formulation is for dilution with a normal saline solution. In some embodiments, the normal saline solution comprises sodium chloride (NaCl). In some embodiments, the normal saline solution comprises between 0.1-1.5% (e.g., between 0.1-1.2%, between 0.3-1.5%, between 0.4-0.5%, between 0.3-1%, between 0.8-1%, between 0.85-0.95%; e.g., about 0.1%, about 0.3%, about 0.4%, about 0.45%, about 0.5%, about 0.6%, about 0.7%, about 0.8%, about 0.85%, about 0.9%, about 0.95%, about 1%, or about 1.2%) (w/v) NaCl).

In another aspect, a pharmaceutical composition of any of the preceding aspects and embodiments is for use as a medicament.

In another aspect, a pharmaceutical composition of any of the preceding aspects and embodiments is for use in a treating or delaying progression of a cell proliferative disorder in a subject in need thereof (e.g., a human subject in need thereof).

In yet another aspect, a pharmaceutical composition of any of the preceding aspects and embodiments is for use in enhancing immune function in a subject having a cell proliferative disorder.

In some embodiments, the cell proliferative disorder is a cancer. In some embodiments, the therapeutic protein is a bispecific antibody formulated to bind to a CD3 molecule located on an immune effector cell and a target molecule located on a target cell other than the immune effector cell (e.g., a CD20 molecule located on (e.g., expressed by) a target cell, such as a B cell. In some embodiments, the bispecific antibody activates the immune effector cell following binding to the CD3 molecule and to the target molecule. In some embodiments, the activated immune effector cell is capable of exerting a cytotoxic effect and/or an apoptotic effect on the target cell.

In some embodiments, e.g., in which the therapeutic protein is a bispecific antibody having an anti-CD20 arm, the cell proliferative disorder is a cancer that is a non-Hodgkin's lymphoma (NHL). In some embodiments, the NHL is selected from the group consisting of chronic lymphoid leukemia (CLL), B cell lymphoma, splenic diffuse red pulp small B cell lymphoma, B cell lymphoma with features intermediate between diffuse large B-cell lymphoma and Burkitt lymphoma, Burkitt-like lymphoma with 11q aberration, B cell lymphoma with features intermediate between diffuse large B-cell lymphoma and classical Hodgkin lymphoma, diffuse large B cell lymphoma (DLBCL), germinal center B cell-like (GCB) diffuse large B cell lymphoma (DLBCL), activated B cell-like (ABC) DLBCL, primary cutaneous follicle center lymphoma, T-cell/histiocyte-rich large B-cell lymphoma, primary DLBCL of the central nervous system, primary cutaneous DLBCL (leg type), Epstein-Barr virus (EBV)-positive DLBCL of the elderly, DLBCL associated with chronic inflammation, primary mediastinal (thymic) large B cell lymphoma, intravascular large B cell lymphoma, ALK-positive large B-cell lymphoma, large B-cell lymphoma arising in HHV8-associated multicentric Castleman disease, B cell leukemia, follicular lymphoma (FL), in situ follicular neoplasia, mantle cell lymphoma (MCL), in situ mantle cell neoplasia, acute myeloid leukemia (AML), marginal zone lymphoma (MZL), small lymphocytic leukemia (SLL), lymphoplasmacytic lymphoma (LL), Waldenstrom macroglobulinemia (WM), central nervous system lymphoma (CNSL), Burkitt's lymphoma (BL), B cell prolymphocytic leukemia, splenic marginal zone lymphoma, hairy cell leukemia, splenic lymphoma/leukemia, hairy cell leukemia variant, α heavy chain disease, γ heavy chain disease, µ heavy chain disease, plasma cell myeloma, solitary plasmacytoma of bone, extraosseous plasmacytoma, extranodal marginal zone lymphoma of mucosa-associated lymphoid tissue (MALT lymphoma), nodal marginal zone lymphoma, pediatric nodal marginal zone lymphoma, pediatric follicular lymphoma, lymphomatoid granulomatosis, plasmablastic lymphoma, and primary effusion lymphoma. In a particular embodiment, the cancer is diffuse large B cell lymphoma (DLBCL), germinal center B cell-like (GCB) DLBCL, activated B-cell-like (ABC) DLBCL, follicular lymphoma (FL), mantle cell lymphoma (MCL), acute myeloid leukemia (AML), chronic lymphoid leukemia (CLL), marginal zone lymphoma (MZL), small lymphocytic leukemia (SLL), lymphoplasmacytic lymphoma (LL), Waldenstrom macroglobulinemia (WM), central nervous system lymphoma (CNSL), or Burkitt's lymphoma (BL).

In some embodiments, the cancer is selected from the group consisting of breast cancer, colorectal cancer, gastric cancer, non-small cell lung cancer (NSCLC), multiple myeloma, renal cancer, prostate cancer, liver cancer, head and neck cancer, melanoma, ovarian cancer, mesothelioma, and glioblastoma. In some embodiments, the cancer is a HER2-positive cancer.

In some embodiments, the therapeutic protein is a bispecific antibody (e.g., a TDB or TCB) formulated for administration to the subject at a dosage from about 10 µg to about 100 mg (e.g., from 100 µg to 80 mg, from 500 µg to 50 mg, or from 1 mg to 20 mg, e.g., from 10 µg to 50 µg, from 50 µg to 100 µg, from 100 µg to 200 µg, from 200 µg to 500 µg, from 500 µg to 1 mg, from 1 mg to 5 mg, from 5 mg to 10 mg, from 10 mg to 20 mg, to 20 mg to 30 mg, from 30 mg to 40 mg, from 40 mg to 50 mg, from 50 mg to 60 mg, from 60 mg to 70 mg, from 70 mg to 80 mg, from 80 mg to 90 mg, or from 90 to 100 mg, e.g., about 10 µg, about 20 µg about 25 µg, about 30 µg, about 40 µg, about 50 µg, about 60 µg, about 70 µg, about 75 µg, about 80 µg, about 90 µg, about 100 µg, about 200 µg, about 250 µg, about 300 µg, about 400 µg, about 500 µg, about 600 µg, about 700 µg, about 750 µg, about 800 µg, about 900 µg, about 1 mg, about 2 mg, about 2.5 mg, about 3 mg, about 4 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 25 mg, about 30 mg, about 40 mg, about 50 mg, about 60 mg, about 70 mg, about 75 mg, about 80 mg, about 90 mg, or about 100 mg). In a particular embodiment, the therapeutic protein is a bispecific antibody formulated for administration to the subject at a dosage from about 1 mg to about 60 mg.

In some embodiments, the pharmaceutical composition (e.g., comprising an anti-FcRH5/anti-CD3 bispecific antibody; e.g., anti-FcRH5/anti-CD3 TDB; e.g., cevostamab) is administered to the subject without dilution (e.g., at a concentration of about 1 mg/ml or about 3 ml/ml). In some embodiments, the pharmaceutical composition is administered to the subject after dilution with a saline solution. In some embodiments, the saline solution is a normal saline solution. In some embodiments, the normal saline solution comprises sodium chloride (NaCl). In some embodiments, the normal saline solution comprises between 0.1-1.5% (e.g., between 0.1-1.2%, between 0.3-1.5%, between 0.4-0.5%, between 0.3-1%, between 0.8-1%, between 0.85-0.95%; e.g., about 0.1%, about 0.3%, about 0.4%, about 0.45%, about 0.5%, about 0.6%, about 0.7%, about 0.8%, about 0.85%, about 0.9%, about 0.95%, about 1%, or about 1.2%) (w/v) NaCl). In particular embodiments, the normal saline solution comprises 0.45% or 0.9% (w/v) NaCl.

In some embodiments, after dilution with the normal saline solution, the concentration of the therapeutic protein (e.g., antibody; e.g., bispecific antibody; e.g., TDB or TCB; e.g., mosunetuzumab, glofitamab, cevostamab, or runimotamab) is from about 0.001 mg/ml to about 0.6 mg/ml (e.g., about 0.001 mg/ml, about 0.002 mg/ml, about 0.003 mg/ml, about 0.004 mg/ml, about 0.005 mg/ml, about 0.01 mg/ml, about 0.02 mg/ml, about 0.03 mg/ml, about 0.04 mg/ml, about 0.05 mg/ml, about 0.75 mg/ml, about 0.1 mg/ml, about 0.11 mg/ml, about 0.12 mg/ml, about 0.13 mg/ml, about 0.14 mg/ml, about 0.15 mg/ml, about 0.16 mg/ml, about 0.17 mg/ml, about 0.18 mg/ml, about 0.19 mg/ml, about 0.2 mg/ml, about 0.21 mg/ml, about 0.22 mg/ml, about 0.23 mg/ml, about 0.24 mg/ml about 0.25 mg/ml, about 0.26 mg/ml, about 0.27 mg/ml, about 0.28 mg/ml, about 0.29 mg/ml, about 0.3 mg/ml, about 0.35 mg/ml, about 0.4 mg/ml about 0.45 mg/ml, about 0.5 mg/ml, about 0.55 mg/ml, or about 0.6 mg/ml). In particular embodiments, after dilution with the normal saline solution, the concentration of therapeutic protein (e.g., antibody; e.g., bispecific antibody; e.g., TDB or TCB; e.g., mosunetuzumab, glofitamab, cevostamab, or runimotamab) is about 0.003 mg/ml, about 0.01 mg/ml, about 0.02 mg/ml, about 0.03 mg/ml, about 0.04 mg/ml, about 0.12 mg/ml, about 0.24 mg/ml, or about 0.3 mg/ml. In a particular embodiment, after dilution with the normal saline solution, the concentration of the therapeutic antibody (e.g., antibody; bispecific antibody; anti-CD20/anti-CD3 bispecific antibody; anti-CD20/anti-CD3 TDB; e.g., mosunetuzumab) is about 0.01 mg/ml, about 0.02 mg/ml, about 0.04 mg/ml, about 0.12 mg/ml, about 0.24 mg/ml or about 0.3 mg/ml. In a particular embodiment, after dilution with the normal saline solution, the concentration of the therapeutic antibody (e.g., antibody; bispecific antibody; anti-CD20/anti-CD3 bispecific antibody; anti-CD20/anti-CD3 TCB; e.g., glofitamab) is about 0.1 mg/ml or about 0.6 mg/ml. In a particular embodiment, after dilution with the normal saline solution, the concentration of the therapeutic antibody (e.g., antibody; bispecific antibody; anti-FcRH5/anti-CD3 bispecific antibody; anti-FcRH5/anti-CD3 TDB; e.g., cevostamab) is about 0.003 mg/ml, 0.03 mg/ml, or 0.3 mg/ml.

In some embodiments, the subject is to be co-administered with at least one additional therapeutic agent (e.g., one, two, three, four, or more additional therapeutic agents). In some embodiments, the at least one additional therapeutic agent includes a PD-1 axis binding antagonist. In some embodiments, the PD-1 axis binding antagonist is selected from the group consisting of a PD-L1 binding antagonist, a PD-1 binding antagonist, and a PD-L2 binding antagonist. In some embodiments, the PD-1 axis binding antagonist is a PD-L1 binding antagonist (e.g., atezolizumab (MPDL3280A), MDX-1105 (BMS-936559; described in WO 2016/201425), and MEDI4736 (durvalumab)). In some embodiments, the PD-1 axis binding antagonist is a PD-1 binding antagonist (e.g., MDX-1106 (nivolumab), MK-3475 (lambrolizumab), AMG 404, REGN2810 (cemiplimab; LIBTAYO^{®}), and AMP-224 (described in WO 2017/058780)). In some embodiments, the PD-1 axis binding antagonist is a PD-L2 binding antagonist (e.g., an antibody (e.g., an anti-PD-L2 antibody) or an immunoadhesin). In some embodiments, the at least one additional therapeutic agent comprises obinutuzumab, rituximab, an antibody-drug conjugate (ADC), a corticosteroid, or tocilizumab. In some embodiments, the therapeutic protein is a bispecific antibody comprising an anti-CD3 arm and an anti-CD20 arm, and the at least one additional therapeutic agent comprises an ADC (e.g., an anti-CD79b ADC, e.g., polatuzumab vedotin). In some embodiments, the subject is a human.

In another aspect, the disclosure features a method of treating or delaying the progression of a cell proliferative disorder in a subject in need thereof. In some embodiments, the method includes administering to the subject an effective amount of the pharmaceutical composition of any of the preceding aspects.

In another aspect, the disclosure features a method of enhancing immune function in a subject having a cell proliferative disorder, e.g., by administering to the subject an effective amount of the pharmaceutical composition of any of the preceding aspects.

In some embodiments, the cell proliferative disorder is a cancer. In some embodiments, the therapeutic protein of the pharmaceutical composition administered to the subject is a bispecific antibody formulated to bind to a CD3 molecule located on an immune effector cell and a target molecule located on a target cell other than the immune effector cell (e.g., a CD20 molecule located on (e.g., expressed by) a target cell, such as a B cell. **In** some embodiments, the bispecific antibody activates the immune effector cell following binding to the CD3 molecule and to the target molecule. In some embodiments, the activated immune effector cell is capable of exerting a cytotoxic effect and/or an apoptotic effect on the target cell.

In some embodiments, e.g., in which the therapeutic protein is a bispecific antibody having an anti-CD20 arm, the cell proliferative disorder is a cancer that is a non-Hodgkin's lymphoma (NHL). In some embodiments, the NHL is selected from the group consisting of chronic lymphoid leukemia (CLL), B cell lymphoma, splenic diffuse red pulp small B cell lymphoma, B cell lymphoma with features intermediate between diffuse large B-cell lymphoma and Burkitt lymphoma, Burkitt-like lymphoma with 11q aberration, B cell lymphoma with features intermediate between diffuse large B-cell lymphoma and classical Hodgkin lymphoma, diffuse large B cell lymphoma (DLBCL), germinal center B cell-like (GCB) diffuse large B cell lymphoma (DLBCL), activated B cell-like (ABC) DLBCL, primary cutaneous follicle center lymphoma, T-cell/histiocyte-rich large B-cell lymphoma, primary DLBCL of the central nervous system, primary cutaneous DLBCL (leg type), Epstein-Barr virus (EBV)-positive DLBCL of the elderly, DLBCL associated with chronic inflammation, primary mediastinal (thymic) large B cell lymphoma, intravascular large B cell lymphoma, ALK-positive large B-cell lymphoma, large B-cell lymphoma arising in HHV8-associated multicentric Castleman disease, B cell leukemia, follicular lymphoma (FL), in situ follicular neoplasia, mantle cell lymphoma (MCL), in situ mantle cell neoplasia, acute myeloid leukemia (AML), marginal zone lymphoma (MZL), small lymphocytic leukemia (SLL), lymphoplasmacytic lymphoma (LL), Waldenstrom macroglobulinemia (WM), central nervous system lymphoma (CNSL), Burkitt's lymphoma (BL), B cell prolymphocytic leukemia, splenic marginal zone lymphoma, hairy cell leukemia, splenic lymphoma/leukemia, hairy cell leukemia variant, α heavy chain disease, γ heavy chain disease, µ heavy chain disease, plasma cell myeloma, solitary plasmacytoma of bone, extraosseous plasmacytoma, extranodal marginal zone lymphoma of mucosa-associated lymphoid tissue (MALT lymphoma), nodal marginal zone lymphoma, pediatric nodal marginal zone lymphoma, pediatric follicular lymphoma, lymphomatoid granulomatosis, plasmablastic lymphoma, and primary effusion lymphoma. In a particular embodiment, the cancer is diffuse large B cell lymphoma (DLBCL), germinal center B cell-like (GCB) DLBCL, activated B-cell-like (ABC) DLBCL, follicular lymphoma (FL), mantle cell lymphoma (MCL), acute myeloid leukemia (AML), chronic lymphoid leukemia (CLL), marginal zone lymphoma (MZL), small lymphocytic leukemia (SLL), lymphoplasmacytic lymphoma (LL), Waldenstrom macroglobulinemia (WM), central nervous system lymphoma (CNSL), or Burkitt's lymphoma (BL).

In some embodiments, the cancer is selected from the group consisting of breast cancer, colorectal cancer, gastric cancer, non-small cell lung cancer (NSCLC), multiple myeloma, renal cancer, prostate cancer, liver cancer, head and neck cancer, melanoma, ovarian cancer, mesothelioma, and glioblastoma. In some embodiments, the cancer is a HER2-positive cancer.

In some embodiments, the therapeutic protein (e.g., the bispecific antibody) is administered to the subject at a dosage from about 10 µg to about 100 mg (e.g., from 100 µg to 80 mg, from 500 µg to 50 mg, or from 1 mg to 20 mg, e.g., from 10 µg to 50 µg, from 50 µg to 100 µg, from 100 µg to 200 µg, from 200 µg to 500 µg, from 500 µg to 1 mg, from 1 mg to 5 mg, from 5 mg to 10 mg, from 10 mg to 20 mg, to 20 mg to 30 mg, from 30 mg to 40 mg, from 40 mg to 50 mg, from 50 mg to 60 mg, from 60 mg to 70 mg, from 70 mg to 80 mg, from 80 mg to 90 mg, or from 90 to 100 mg, e.g., about 10 µg, about 20 µg about 25 µg, about 30 µg, about 40 µg, about 50 µg, about 60 µg, about 70 µg, about 75 µg, about 80 µg, about 90 µg, about 100 µg, about 200 µg, about 250 µg, about 300 µg, about 400 µg, about 500 µg, about 600 µg, about 700 µg, about 750 µg, about 800 µg, about 900 µg, about 1 mg, about 2 mg, about 2.5 mg, about 3 mg, about 4 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 25 mg, about 30 mg, about 40 mg, about 50 mg, about 60 mg, about 70 mg, about 75 mg, about 80 mg, about 90 mg, or about 100 mg). In a particular embodiment, the method includes administering the therapeutic protein (e.g., the bispecific antibody) to the subject at a dosage from about 1 mg to about 60 mg.

In some embodiments, the subject is co-administered with at least one additional therapeutic agent (e.g., one, two, three, four, or more additional therapeutic agents). In some embodiments, the at least one additional therapeutic agent includes a PD-1 axis binding antagonist. In some embodiments, the PD-1 axis binding antagonist is selected from the group consisting of a PD-L1 binding antagonist, a PD-1 binding antagonist, and a PD-L2 binding antagonist. In some embodiments, the PD-1 axis binding antagonist is a PD-L1 binding antagonist (e.g., atezolizumab (MPDL3280A), MDX-1105 (BMS-936559), and MEDI4736 (durvalumab)). In some embodiments, the PD-1 axis binding antagonist is a PD-1 binding antagonist (e.g., MDX-1106 (nivolumab), MK-3475 (lambrolizumab), AMG 404, REGN2810 (cemiplimab; LIBTAYO^{®}), and AMP-224 (described in WO 2017/058780). In some embodiments, the PD-1 axis binding antagonist is a PD-L2 binding antagonist (e.g., an antibody (e.g., an anti-PD-L2 antibody) or an immunoadhesin). In some embodiments, the at least one additional therapeutic agent comprises obinutuzumab, rituximab, an antibody-drug conjugate (ADC), a corticosteroid, or tocilizumab. In some embodiments, the therapeutic protein is a bispecific antibody comprising an anti-CD3 arm and an anti-CD20 arm, and the at least one additional therapeutic agent comprises an ADC (e.g., an anti-CD79b ADC, e.g., polatuzumab vedotin). In some embodiments, the pharmaceutical composition is administered intravenously, subcutaneously, intramuscularly, topically, orally, transdermally, intraperitoneally, intraorbitally, intranasally, intrathecally, or by intraventricular administration. In some embodiments, the subject is a human.

Each and every embodiment can be combined unless the context clearly suggests otherwise. Each and every embodiment can be applied to each and every aspect of the disclosure unless the context clearly suggests otherwise.

Specific embodiments of the present disclosure will become evident from the following more detailed description of certain preferred embodiments and the claims.

### BRIEF DESCRIPTION OF THE DRAWINGS

FIG. 1 is a table summarizing delivery and in-use considerations for various phase III drug product (DP) formulations.
**FIG. 2** is a graph showing minimum PS20 concentration (% w/v) as a function of dose and DP protein concentration, determined by IV bag shaking study in 100 ml PO bags. The x-axis shows DP protein concentration in mg/ml, while the y-axis shows dose in mg
**FIG. 3** is a graph showing the effect of various IV bag sizes and anti-CD20/anti-CD3 TDB quantities on minimum surfactant (PS20 or P188) concentrations required for prevention of aggregation and particle formation in 1 mg/ml DP. The first bar for each set of conditions on the x-axis (left to right) represents > 5 mg of the TDB, the second bar represents 2 mg of the TDB, and the third bar represents 1 mg of the TDB.
**FIG. 4A** is a graph showing kinetics of protein oxidation at methionine 257 (Met257) of mosunetuzumab formulated with PS20 at 1 mg/ml (triangles), 10 mg/ml (squares), or 60 mg/ml (diamonds) at 40 °C.
**FIG. 4B** is a graph showing kinetics of protein oxidation at methionine 257 of mosunetuzumab formulated with P188 at 1 mg/ml (triangles), 10 mg/ml (squares), or 60 mg/ml (diamonds) at 40 °C.
**FIG. 4C** is a graph showing kinetics of protein oxidation at methionine 257 of mosunetuzumab formulated with srPS20 at 1 mg/ml (triangles), 10 mg/ml (squares), or 60 mg/ml (diamonds) at 40 °C.
**FIG. 5** is a graph showing kinetics of protein oxidation at methionine 257 of mosunetuzumab formulated with 30 mM histidine (diamonds) or 10 mM histidine (squares) at 40 °C.
**FIG. 6** is a graph showing percent oxidation at methionine 257 of mosunetuzumab in various formulations after up to 300,000 lux-hours of ambient light exposure (at 5,500 lux light intensity). The first bar for each formulation on the x-axis (left to right) represents time = 0; the second bar, 24 hours; the third bar, 54 hours; and the last (fourth) bar, dark control.

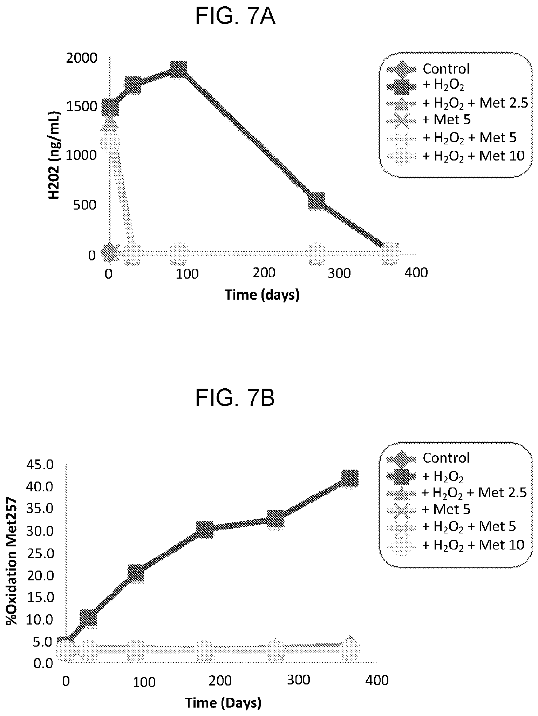

**FIG. 7A** is a graph showing kinetics of hydrogen peroxide (H₂O₂) concentration in various mosunetuzumab compositions stored at 5 °C for up to 12 months. BTCT4465A compositions tested included composition alone (1 mg/ml mosunetuzumab, 15 mM histidine acetate, 0.08% (w/v) PS20, 160 mM sucrose, pH 5.8; control, diamonds), composition +H₂O₂ (squares), composition +H₂O₂ +2.5 mM methionine (triangles), composition +5 mM methionine (dark X's), composition +H₂O₂+5 mM methionine (light X's), and composition +H₂O₂ +10 mM methionine (light X's). H₂O₂ concentrations were measured by AMPLEX^{®} Red assay.
**FIG. 7B** is a graph showing kinetics of oxidation at methionine 257 in various mosunetuzumab compositions stored at 5 °C for up to 12 months. Mosunetuzumab compositions tested included composition alone (1 mg/ml mosunetuzumab, 15 mM histidine acetate, 0.08% (w/v) PS20, 160 mM sucrose, pH 5.8; control, diamonds), composition +H₂O₂ (squares), composition +H₂O₂ +2.5 mM methionine (triangles), composition +5 mM methionine (dark X's), composition +H₂O₂+5 mM methionine (light X's), and composition +H₂O₂ +10 mM methionine (light X's). Oxidation was measured by peptide mapping.

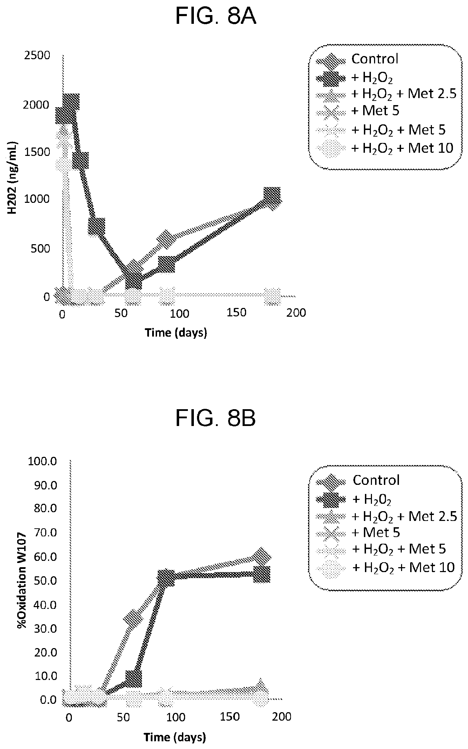

**FIG. 8A** is a graph showing kinetics of hydrogen peroxide (H₂O₂) concentration in various mosunetuzumab compositions stored at 25 °C for up to six months. Mosunetuzumab compositions tested included composition alone (1 mg/ml mosunetuzumab, 15 mM histidine acetate, 0.08% (w/v) PS20, 160 mM sucrose, pH 5.8; control, diamonds), composition +H₂O₂ (squares), composition +H₂O₂ +2.5 mM methionine (triangles), composition +5 mM methionine (dark X's), composition +H₂O₂ +5 mM methionine (light X's), and composition +H₂O₂ +10 mM methionine (light X's). H₂O₂ concentrations were measured by AMPLEX^{®} Red assay.
**FIG. 8B** is a graph showing kinetics of oxidation at tryptophan 107 in the CD20 arm of various mosunetuzumab compositions stored at 25 °C for up to six months. Mosunetuzumab compositions tested included composition alone (1 mg/ml mosunetuzumab, 15 mM histidine acetate, 0.08% (w/v) PS20, 160 mM sucrose, pH 5.8; control, diamonds), composition +H₂O₂ (squares), composition +H₂O₂ +2.5 mM methionine (triangles), composition +5 mM methionine (dark X's), composition +H₂O₂ +5 mM methionine (light X's), and composition +H₂O₂ +10 mM methionine (light X's). Oxidation was measured by peptide mapping.
**FIG. 8C** is a graph showing kinetics of oxidation at methionine 257 of various mosunetuzumab compositions stored at 25 °C for up to six months. Mosunetuzumab compositions tested included composition alone (1 mg/ml mosunetuzumab, 15 mM histidine acetate, 0.08% (w/v) PS20, 160 mM sucrose, pH 5.8; control, diamonds), composition +H₂O₂ (squares), composition +H₂O₂ +2.5 mM methionine (triangles), composition +5 mM methionine (dark X's), composition +H₂O₂+5 mM methionine (light X's), and composition +H₂O₂ +10 mM methionine (light X's). Oxidation was measured by peptide mapping.
**FIG. 8D** is a graph showing kinetics of high molecular weight species (HMWS) levels, measured by SEC, in various mosunetuzumab compositions stored at 25 °C for up to six months. Mosunetuzumab compositions tested included composition alone (1 mg/ml mosunetuzumab, 15 mM histidine acetate, 0.08% (w/v) PS20, 160 mM sucrose, pH 5.8; control, diamonds), composition +H₂O₂ (squares), composition +H₂O₂ +2.5 mM methionine (triangles), composition +5 mM methionine (dark X's), composition +H₂O₂+5 mM methionine (light X's), and composition +H₂O₂+10 mM methionine (light X's).
**FIG. 8E** is a graph showing kinetics of levels of low molecular weight species, measured by mCE-SDS, in various mosunetuzumab compositions stored at 25 °C for up to six months. Mosunetuzumab compositions tested included composition alone (1 mg/ml mosunetuzumab, 15 mM histidine acetate, 0.08% (w/v) PS20, 160 mM sucrose, pH 5.8; control, diamonds), composition +H₂O₂ (squares), composition +H₂O₂ +2.5 mM methionine (triangles), composition +5 mM methionine (dark X's), composition +H₂O₂+5 mM methionine (light X's), and composition +H₂O₂+10 mM methionine (light X's).
**FIG. 9A** is a graph showing change in HMWS levels over time, measured by SE-HPLC, in various mosunetuzumab formulations stored at 40 °C and 75% relative humidity (RH) for up to one month. Formulations F1-F5 are characterized in Table 5.
**FIG. 9B** is a graph showing change in monomer levels over time, measured by SE-HPLC, in various mosunetuzumab formulations stored at 40 °C and 75% RH for up to one month. Formulations F1-F5 are characterized in Table 5.
**FIG. 9C** is a graph showing change in LMWS levels over time, measured by SE-HPLC, in various mosunetuzumab formulations stored at 40 °C and 75% RH for up to one month. Formulations F1-F5 are characterized in Table 5.
**FIG. 10A** is a graph showing change in acidic variants over time, measured by icIEF, in various mosunetuzumab formulations stored at 40 °C and 75% RH for up to one month. Formulations F1-F5 are characterized in Table 5.
**FIG. 10B** is a graph showing change in main peak over time, measured by icIEF, in various mosunetuzumab formulations stored at 40 °C and 75% RH for up to one month. Formulations F1-F5 are characterized in Table 5.
**FIG. 10C** is a graph showing change in basic variants over time, measured by icIEF, in various mosunetuzumab formulations stored at 40 °C and 75% RH for up to one month. Formulations F1-F5 are characterized in Table 5.
**FIG. 11A** is a graph showing change of sum of pre-peaks over time, measured by mCE-SDS, in various mosunetuzumab formulations stored at 40 °C and 75% RH for up to one month. Formulations F1-F5 are characterized in Table 5.
**FIG. 11B** is a graph showing change of sum of main peaks over time, measured by mCE-SDS, in various mosunetuzumab formulations stored at 40 °C and 75% RH for up to one month. Formulations F1-F5 are characterized in Table 5.
**FIG. 12** is a graph showing the Donnan Effect on pH values of mosunetuzumab compositions.
**FIG. 13****:** Schematic diagram showing the structure of glofitamab.
**FIG. 14****:** Formulation Development GLP Tox and Entry into Human Study. Surfactant content of formulations F1 to F5, initial vs. after 6 weeks of storage at 5, 25 or 40°C.
**FIG. 15A** **-** **FIG. 15C****:** Formulation Development GLP Tox and Entry into Human Study, size exclusion chromatography (SEC) of formulations F1 to F5, initial vs. after 6 weeks of storage at 5, 25 or 40°C. FIG 15A: Main Peak, FIG 15B: high molecular weight (HMW); FIG 15C: low molecular weight (LMW).
**FIG. 16A** **-** **FIG. 16C****:** Formulation Development GLP Tox and Entry into Human Study, ion exchange chromatography (IEC) of formulations F1 to F5, initial vs. after 6 weeks of storage at 5, 25 or 40°C. FIG 16A: Main Peak, FIG 16B: HMW; FIG 16C: LMW
**FIG. 17****:** Formulation Development - analytical results of formulation F1 up to 84 weeks. F1 = 5 mg/ml RO7022859 (i.e., glofitamab), 20 mM Histidine-HCl pH 5.5, 240 mM Sucrose, 10 mM Methionine, 0.05% (w/v) Polysorbate 20.
**FIG. 18A****:** Formulation Development GLP Tox and Entry into Human Study, huCD20 binding of formulations F1 to F5, initial vs. after 3 and 6 weeks of storage at 5, 25 or 40°C.
**FIG. 18B****:** Formulation Development GLP Tox and Entry into Human Study, huCD3 binding of formulations F1 to F5, initial vs. after 3 and 6 weeks of storage at 5, 25, or 40°C.
**FIG. 19A** **-** **FIG. 19B****:** Development Studies for Phase III and commercial formulation. Glofitamab size exclusion (SE)-HPLC % HMWS (FIG. 19A) and ion exchange (IE)-HPLC % Acidic Region (FIG. 19B) as a Function of Protein Concentration after 104 Weeks Storage at 5°C.
**FIG. 20A** **-** **FIG. 20B****:** Development Studies for Phase III and commercial formulation. Glofitamab SE-HPLC % HMWS (FIG. 20A) and IE-HPLC % Acidic Region (FIG. 20B) as Function of pH and Stabilizer (Methionine) Addition after 6w Storage at 40°C.
**FIG. 21****:** Development Studies for Phase III and commercial formulation. Glofitamab SE-HPLC % HMWS including Visible Particle Formation and IE-HPLC % Acidic Region as Function of Tonicity Agent after 26 Weeks Storage at 25°C.
**FIG. 22****:** Development Studies for Phase III and commercial formulation. Glofitamab SE-HPLC % HMWS including Visible Particle Formation and IE-HPLC % Acidic Region as Function of Surfactant after 7 Days of Shaking at 25°C.
**FIG. 23****:** Development Studies for Phase III and commercial formulation. Glofitamab PS20 Content [mg/ml] and Visible Particle Formation as Function of Protein Concentration Initially and after 104 Weeks of Storage at 5°C.
**FIG. 24****:** Long-term stability data: PS20 Content of Example Glofitamab DP Batches on Stability (Storage at 2-8°C).

### DETAILED DESCRIPTION OF EMBODIMENTS OF THE DISCLOSURE

### I. Definitions

Unless otherwise defined, all terms of art, notations and other scientific terminology used herein are intended to have the meanings commonly understood by those of skill in the art to which this disclosure pertains. In some cases, terms with commonly understood meanings are defined herein for clarity and/or for ready reference, and the inclusion of such definitions herein should not necessarily be construed to represent a substantial difference over what is generally understood in the art.

The term "about" as used herein refers to the usual error range for the respective value readily known to the skilled person in this technical field. Reference to "about" a value or parameter herein includes (and describes) embodiments that are directed to that value or parameter per se.

As used herein, the singular forms "a," "an," and "the" include plural referents unless the context clearly dictates otherwise. For example, reference to "an isolated peptide" means one or more isolated peptides.

Throughout this specification and claims, the word "comprise," or variations such as "comprises" or "comprising" will be understood to imply the inclusion of a stated integer or group of integers but not the exclusion of any other integer or group of integers.

The terms "pharmaceutical composition" and "pharmaceutical formulation" are used interchangeably herein and refer to a preparation which is in such form as to permit the biological activity of an active ingredient contained therein to be effective, and which contains no additional components which are unacceptably toxic to a subject to which the formulation would be administered.

The term "therapeutic protein" refers to a protein that can be administered to a subject to elicit a biological response associated with treatment or improvement of a disease or pathological condition. Therapeutic proteins include biologic drugs, such as peptides, antibodies (e.g., bispecific antibodies, e.g., T cell-dependent bispecific antibodies (TDBs), e.g., anti-CD20/anti-CD3 TDBs, e.g., mosunetuzumab, e.g., T cell engaging bispecific antibodies (TCBs), e.g., anti-CD20/anti-CD3 TCBs, e.g., glofitamab), immunoconjugates

A "pharmaceutically acceptable carrier" or "carrier" refers to an ingredient in a pharmaceutical formulation, other than an active ingredient, which is nontoxic to a subject. A pharmaceutically acceptable carrier or carrier includes, but is not limited to, a buffer, excipient, stabilizer, or preservative.

The term "shelf-life" refers to the length of time that a product (e.g., a therapeutic protein (e.g., a bispecific antibody, e.g., a TDB, e.g., an anti-CD20/anti-CD3 TDB, e.g., mosunetuzumab, e.g., a TCB, e.g., an anti-CD20/anti-CD3 TCB, e.g., glofitamab)) may be stored without becoming unfit for use (e.g., by administration to a subject) or sale. In some embodiments, the shelf-life is the length of time in which a composition (e.g., a pharmaceutical composition) is stable. For example, in some embodiments, a composition herein has a shelf-life of at least 36 months when stored at 5 °C ± 3 °C and protected from light.

A "stable" pharmaceutical formulation is one in which the protein (e.g., the therapeutic protein) therein essentially retains its physical stability and/or chemical stability and/or biological activity upon storage. Preferably, the formulation essentially retains its physical and chemical stability, as well as its biological activity upon storage (e.g., frozen storage). The storage period is generally selected based on the intended shelf-life of the formulation. Various analytical techniques for measuring protein stability are available in the art and are reviewed in Peptide and Protein Drug Delivery, 247-301, Vincent Lee Ed., Marcel Dekker, Inc., New York, N.Y., Pubs. (1991) and Jones. A. Adv. Drug Delivery Rev. 10: 29-90 (1993), for example. Stability can be measured at a selected amount of light exposure and/or temperature for a selected time period. Stability can be evaluated qualitatively and/or quantitatively in a variety of different ways, including evaluation of aggregate formation (for example, using size exclusion chromatography, by measuring turbidity, and/or by visual inspection); evaluation of ROS formation (for example, by using a light stress assay or an 2,2'-azobis(2-amidinopropane) dihydrochloride (AAPH) stress assay); oxidation of specific amino acid residues of the protein (for example, a Met residue of an antibody); by assessing charge heterogeneity using cation exchange chromatography, image capillary isoelectric focusing (icIEF) or capillary zone electrophoresis; amino-terminal or carboxy-terminal sequence analysis; mass spectrometric analysis; SDS-PAGE analysis to compare reduced and intact polypeptides (e.g., therapeutic proteins); peptide map (for example, tryptic or LYS-C) analysis; evaluating biological activity or target binding function of the protein (e.g., binding of an antibody to its antigen, e.g., binding of a TDB to a T cell and/or a target cell); and the like. Instability may involve any one or more of: aggregation, deamidation (e.g., Asn deamidation), oxidation (e.g., Met oxidation and/or Trp oxidation), isomerization (e.g., Asp isomerization), clipping/hydrolysis/fragmentation (e.g., hinge region fragmentation), succinimide formation, unpaired cysteine(s), N-terminal extension, C-terminal processing, glycosylation differences, and the like.

A protein (e.g., a therapeutic protein, such as a bispecific antibody) "retains its physical stability" in a pharmaceutical formulation if it shows no signs or very little of aggregation, precipitation, fragmentation, and/or denaturation upon visual examination of color and/or clarity, or as measured by UV light scattering or by size exclusion chromatography.

A protein (e.g., a therapeutic protein, such as a bispecific antibody) "retains its chemical stability" in a pharmaceutical formulation, if the chemical stability at a given time is such that the protein (e.g., the therapeutic protein (e.g., a bispecific antibody, e.g., a TDB or TCB)) is considered to still retain its biological activity as defined below. Chemical stability can be assessed by detecting and quantifying chemically altered forms of the protein (e.g., the therapeutic protein (e.g., a bispecific antibody, e.g., a TDB or TCB, e.g., an anti-CD20/anti-CD3 TDB or TCB). Chemical alteration may involve protein oxidation, which can be evaluated using tryptic peptide mapping, reverse-phase high-performance liquid chromatography (HPLC) and liquid chromatography-mass spectrometry (LC/MS), for example. Other types of chemical alteration include charge alteration of the protein (e.g., the therapeutic protein (e.g., a bispecific antibody, e.g., a TDB or TCB, e.g., an anti-CD20/anti-CD3 TDB or TCB), which can be evaluated by ion-exchange chromatography or icIEF, for example.

A protein (e.g., a therapeutic protein) "retains its biological activity" in a pharmaceutical formulation, if the biological activity of the protein (e.g., the therapeutic protein (e.g., a bispecific antibody, e.g., a TDB or TCB)) at a given time is within about 20% (such as within about 10%) of the biological activity exhibited at the time the pharmaceutical formulation was prepared (within the errors of the assay), as determined, for example, in a receptor binding assay.

As used herein, "biological activity" of protein (e.g., the therapeutic protein (e.g., a bispecific antibody, e.g., a TDB or TCB)) refers to the ability of the protein (e.g., the therapeutic protein (e.g., a bispecific antibody, e.g., a TDB or TCB)) to bind its target, for example, the ability of an antibody to bind its antigen (e.g., the ability of a TDB or TCB to bind a T cell and/or a target cell). It can further include a biological response, which can be measured in vitro or in vivo. Such activity may be antagonistic or agonistic.

A protein (e.g., the therapeutic protein, such as a bispecific antibody, e.g., a TDB or TCB) which is "susceptible to oxidation" is one comprising one or more residue(s) that has been found to be prone to oxidation such as, but not limited to, methionine (Met), cysteine (Cys), histidine (His), tryptophan (Trp), and tyrosine (Tyr). For example, one or more methionine residues in a therapeutic protein, such as a TDB or TCB, may be susceptible to oxidation.

The term "percent oxidation" refers to the percentage of proteins (e.g., therapeutic proteins) in a formulation (e.g., a pharmaceutical composition) that are oxidized at a particular amino acid residue, for example, a Met residue. Percent oxidation can be determined by, e.g., mass spectrometry (MS) analysis of one or more tryptic peptides, in which one or more particular oxidation-prone amino acid residues reside. Percent oxidation may be determined, for example, following an AAPH stress test, within 9 months, 12 months, 18 months, or two years from the initial production of a protein (e.g., the therapeutic protein) or pharmaceutical composition thereof.

The term "as assessed by an AAPH stress test," as used herein, means that the percent oxidation at a particular amino acid residue (for example, a Met residue) is determined by mass spectrometry analysis of tryptic peptides following formulating the protein (e.g., a therapeutic protein, e.g., a TDB or TCB) with AAPH (e.g., about 0 mM AAPH, about 1 mM AAPH, about 3 mM AAPH, about 3.5 mM AAPH, or about 5 mM AAPH), for example, in a formulation of about 10 mg/ml therapeutic protein, about 10 mM histidine acetate, about 240 mM sucrose, about 0.06 (w/v) polysorbate 20, pH about 5.8 for about 24 hours at about 40 °C. The stressed protein (e.g., therapeutic protein, e.g., TDB or TCB) is digested with trypsin and the digested peptides are subjected to LC-MS-MS to determine the percentage of oxidation.

As used herein, "buffer" refers to a buffered solution that resists changes in pH by the action of its acid-base conjugate components (also referred to herein as "buffering agents"). In some embodiments, the buffer of this disclosure has a pH in the range of from about 4.5 to about 8. In some embodiments, the buffer has a pH in the range from about 5.1 to 6.1 (e.g., about 5.5, about 5.6, about 5.7, about 5.8, about 5.9, about 6.0, or about 6.1), e.g., about pH 5.5 or 5.8. In a particular embodiment, the buffer is about pH 5.5. In another particular embodiment, the buffer is about pH 5.8. Exemplary buffering agents for use in the disclosure include, but are not limited to, histidine (e.g., histidine acetate or histidine hydrochloride (HCl)), an acetate, a phosphate, a succinate, or a combination thereof. In some embodiments, the histidine is histidine acetate or histidine HCl, sodium phosphate monobasic, sodium phosphate dibasic, sodium phosphate tribasic, potassium phosphate monobasic, potassium phosphate dibasic, potassium phosphate tribasic, or a mixture thereof. In particular embodiments, the buffering agent is histidine HCl or histidine acetate.

As used herein, a "tonicity agent" refers to an agent that can be added to a liquid (e.g., an aqueous solution) to adjust the tonicity of said liquid. Tonicity refers to a measure of an osmotic pressure gradient between two solutions. In some embodiments, tonicity agents cannot cross a semipermeable membrane (e.g., a semi-permeable cell membrane) that otherwise permits the liquid (e.g., an aqueous solution) or other components of the liquid (e.g., other solutes) to cross. In some embodiments, tonicity agents are used to reduce local irritation by preventing osmotic shock at the site of application (e.g., upon subcutaneous administration). Exemplary tonicity agents include carbohydrates (e.g., sucrose, glucose, dextrose, glycerol, glycerin, mannitol, and trehalose), amino acids, and salts (e.g., sodium chloride and potassium chloride).

As used herein, a "surfactant" refers to a surface-active agent, preferably a nonionic surfactant. Examples of surfactants herein include polysorbate (for example, polysorbate 20, polysorbate 40, polysorbate 60, polysorbate 65, polysorbate 80, polysorbate 85); poloxamer (e.g., poloxamer 188); TRITON^{®}; sodium octyl glycoside; lauryl-, myristyl-, linoleyl-, or stearyl-sulfobetaine; lauryl-, myristyl-, linoleyl- or stearyl-sarcosine; linoleyl-, myristyl-, or cetyl-betaine; lauroamidopropyl-, cocamidopropyl-, linoleamidopropyl-, myristamidopropyl-, palmidopropyl-, or isostearamidopropyl-betaine (e.g., lauroamidopropyl); myristamidopropyl-, palmidopropyl-, or isostearamidopropyl-dimethylamine; sodium methyl cocoyl-, or disodium methyl oleyl-taurate; and the MONAQUAT^{™} series (Mona Industries, Inc., Paterson, N.J.); polyethyl glycol, polypropyl glycol, and copolymers of ethylene and propylene glycol (e.g., PLURONIC^{®} type block copolymers, e.g., PLURONIC^{®} F-68); and the like. In one embodiment, the surfactant herein is polysorbate 20 (PS20). In yet another embodiment, the surfactant herein is poloxamer 188 (P188).

A "preservative" is a compound which can be optionally included in the formulation to essentially reduce bacterial action therein, thus facilitating the production of a multi-use formulation, for example. Examples of potential preservatives include octadecyldimethylbenzyl ammonium chloride, hexamethonium chloride, benzalkonium chloride (a mixture of alkylbenzyldimethylammonium chlorides in which the alkyl groups are long-chain compounds), and benzethonium chloride. Other types of preservatives include aromatic alcohols such as phenol, butyl, and benzyl alcohol; alkyl parabens such as methyl or propyl paraben; catechol; resorcinol; cyclohexanol; 3-pentanol, and m-cresol. In one embodiment, the preservative herein is benzyl alcohol. In some embodiments, the formulation does not include a preservative.

The "molar ratio of the surfactant to a therapeutic protein" (surfactant:protein) is the ratio of surfactant to the therapeutic protein, where each component is expressed in molarity (also referred to as molar concentration). Equation (1) depicts this ratio: $Molar ratio \left(surfactant:protein\right) = \frac{Surfactant M}{Therapeutic protein M}$

As used herein, a "drug substance" or "DS" refers to a pharmaceutical composition formulated for storage prior to administration to a subject, e.g., frozen storage. A DS may have a concentration of a therapeutic protein that is greater than the concentration of therapeutic protein to be administered to the subject. Accordingly, in some instances, the DS is diluted prior to administration to the subject.

As used herein, a "drug product" or "DP" refers to a pharmaceutical composition in its final configuration such that it is ready to be administered to a subject (e.g., in final vial configuration). The concentration of the therapeutic protein in a DP may be the concentration at which it is to be administered to the subject. Alternatively, if the DP is to be administered with a diluent, or if it is intended to be administered in combination with other therapeutic reagents, the DP may be at a higher concentration than the concentration at which it is to be administered to the subject

Within this application, unless otherwise stated, the techniques utilized may be found in any of several well-known references such as: Molecular Cloning: A Laboratory Manual (Sambrook, et al., 1989, Cold Spring Harbor Laboratory Press), PCR Protocols: A Guide to Methods and Applications (Innis, et al. 1990. Academic Press, San Diego, CA), and Harlow and Lane (1988) Antibodies: A Laboratory Manual ch.14 (Cold Spring Harbor Laboratory, Cold Spring Harbor, NY).

As appropriate, procedures involving the use of commercially available kits and reagents are generally carried out in accordance with manufacturer defined protocols and/or parameters unless otherwise noted. Before the present methods and uses therefore are described, it is to be understood that this disclosure is not limited to the particular methodology, protocols, cell lines, animal species or genera, constructs, and reagents described as such can, of course, vary. It is also to be understood that the terminology used herein is for the purpose of describing particular embodiments only, and is not intended to limit the scope of the present disclosure which will be limited only by the appended claims.

As used herein, term "polypeptide" refers to a molecule composed of monomers (amino acids) linearly linked by amide bonds (also known as peptide bonds). The term "polypeptide" refers to any chain of two or more amino acids, and does not refer to a specific length of the product. Thus, peptides, dipeptides, tripeptides, oligopeptides, "protein," "amino acid chain," or any other term used to refer to a chain of two or more amino acids, are included within the definition of "polypeptide," and the term "polypeptide" may be used instead of, or interchangeably with any of these terms. The term "polypeptide" is also intended to refer to the products of post-expression modifications of the polypeptide, including without limitation glycosylation, acetylation, phosphorylation, amidation, derivatization by known protecting/blocking groups, proteolytic cleavage, or modification by non-naturally occurring amino acids. A polypeptide may be derived from a natural biological source or produced by recombinant technology, but is not necessarily translated from a designated nucleic acid sequence. It may be generated in any manner, including by chemical synthesis. A polypeptide of the disclosure may be of a size of about 3 or more, 5 or more, 10 or more, 20 or more, 25 or more, 50 or more, 75 or more, 100 or more, 200 or more, 500 or more, 1,000 or more, or 2,000 or more amino acids. Polypeptides may have a defined three-dimensional structure, although they do not necessarily have such structure. Polypeptides with a defined three-dimensional structure are referred to as folded, and polypeptides which do not possess a defined three-dimensional structure, but rather can adopt a large number of different conformations, and are referred to as unfolded.

By an "isolated" polypeptide or a variant, or derivative thereof is intended a polypeptide that is not in its natural milieu. No particular level of purification is required. For example, an isolated polypeptide can be removed from its native or natural environment. Recombinantly produced polypeptides and proteins expressed in host cells are considered isolated for the purpose of the disclosure, as are native or recombinant polypeptides which have been separated, fractionated, or partially or substantially purified by any suitable technique.

The term "antibody" herein is used in the broadest sense and encompasses various antibody structures, including but not limited to monoclonal antibodies, polyclonal antibodies, multispecific antibodies (e.g., bispecific antibodies, e.g., TDBs or TCBs), and antibody fragments, so long as they exhibit the desired antigen-binding activity (e.g., an antigen-binding fragment of an antibody).

As used herein, the term "antigen binding molecule" refers in its broadest sense to a molecule that specifically binds an antigenic determinant. Examples of antigen binding molecules are immunoglobulins and derivatives, e.g., fragments, thereof.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g., scFv); and multispecific antibodies formed from antibody fragments.

By "binding domain" is meant a part of a compound or a molecule that specifically binds to a target epitope, antigen, ligand, or receptor. Binding domains can be part of a molecule such as an antibody (e.g., a monoclonal, polyclonal, recombinant, humanized, or chimeric antibody), an antibody fragment or portion thereof (e.g., a Fab fragment, Fab'₂, scFv antibody, SMIP, domain antibody, diabody, minibody, scFv-Fc, affibody, nanobody and a VH and/or VL domain of an antibody), receptor, ligand, aptamer, or other molecule having an identified binding partner.

As used herein, the term "antigen binding moiety" refers to a polypeptide molecule that specifically binds to an antigenic determinant. In one embodiment, an antigen binding moiety is able to direct the entity to which it is attached (e.g., a cytokine or a second antigen binding moiety) to a target site, for example to a specific type of tumor cell or tumor stroma bearing the antigenic determinant. Antigen binding moieties include antibodies and fragments thereof as further defined herein. Preferred antigen binding moieties include an antigen binding domain of an antibody, comprising an antibody heavy chain variable region and an antibody light chain variable region. In certain embodiments, the antigen binding moieties may include antibody constant regions as further defined herein and known in the art. Useful heavy chain constant regions include any of the five isotypes: α, δ, ε, γ, or µ. Useful light chain constant regions include any of the two isotypes: κ and λ.

By a "crossover" Fab molecule (also termed "Crossfab") is meant a Fab molecule wherein the variable domains or the constant domains of the Fab heavy and light chain are exchanged (i.e., replaced by each other), i.e., the crossover Fab molecule comprises a peptide chain composed of the light chain variable domain VL and the heavy chain constant domain 1 CH1 (VL-CH1, in N- to C-terminal direction), and a peptide chain composed of the heavy chain variable domain VH and the light chain constant domain CL (VH-CL, in N- to C-terminal direction). For clarity, in a crossover Fab molecule wherein the variable domains of the Fab light chain and the Fab heavy chain are exchanged, the peptide chain comprising the heavy chain constant domain 1 CH1 is referred to herein as the "heavy chain" of the (crossover) Fab molecule. Conversely, in a crossover Fab molecule wherein the constant domains of the Fab light chain and the Fab heavy chain are exchanged, the peptide chain comprising the heavy chain variable domain VH is referred to herein as the "heavy chain" of the (crossover) Fab molecule.

In contrast thereto, by a "conventional" Fab molecule is meant a Fab molecule in its natural format, i.e., comprising a heavy chain composed of the heavy chain variable and constant domains (VH-CH1, in N- to C-terminal direction), and a light chain composed of the light chain variable and constant domains (VL-CL, in N- to C-terminal direction).

The term "hypervariable region" or "HVR" as used herein refers to each of the regions of an antibody variable domain which are hypervariable in sequence ("complementarity determining regions" or "CORs") and/or form structurally defined loops ("hypervariable loops") and/or contain the antigen-contacting residues ("antigen contacts"). Generally, antibodies comprise six HVRs: three in the VH (H1, H2, H3), and three in the VL (L1, L2, L3). Exemplary HVRs herein include:
(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (H1), 50-65 (H2), and 95-102 (H3) (Kabat et al., *supra);*
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., *supra.*

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., *supra.*

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

"Percent (%) amino acid sequence identity" or "percent (%) sequence identity" with respect to a reference polypeptide sequence is defined as the percentage of amino acid residues in a candidate sequence that are identical with the amino acid residues in the reference polypeptide sequence after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or MEGALIGN^{®} (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX^{®} operating system, including digital UNIX^{®} V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows: $100 times the fraction X / Y$ where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "chimeric" antibody refers to an antibody in which a portion of the heavy and/or light chain is derived from a particular source or species, while the remainder of the heavy and/or light chain is derived from a different source or species.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., *supra.* In one embodiment, for the VL, the subgroup is subgroup kappa I as in Kabat et al., *supra.* In one embodiment, for the VH, the subgroup is subgroup III as in Kabat et al., *supra.*

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The terms "full-length antibody," "intact antibody," and "whole antibody" are used herein interchangeably to refer to an antibody having a structure substantially similar to a native antibody structure or having heavy chains that contain an Fc region as defined herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues. Human antibodies can be produced using various techniques known in the art, including phage-display libraries. Hoogenboom and Winter, J. Mol. Biol., 227:381 (1991); Marks et al., J. Mol. Biol., 222:581 (1991). Also available for the preparation of human monoclonal antibodies are methods described in Cole et al., Monoclonal Antibodies and Cancer Therapy, Alan R. Liss, p. 77 (1985); Boerner et al., J. Immunol., 147(1):86-95 (1991). See also van Dijk and van de Winkel, Curr. Opin. Pharmacol., 5: 368-74 (2001). Human antibodies can be prepared by administering the antigen to a transgenic animal that has been modified to produce such antibodies in response to antigenic challenge, but whose endogenous loci have been disabled, *e.g.,* immunized xenomice (see, *e.g.,* U.S. Patent Nos. 6,075,181 and 6,150,584 regarding XENOMOUSE^{™} technology). See also, for example, Li et al., Proc. Natl. Acad. Sci. USA, 103:3557-3562 (2006) regarding human antibodies generated via a human B-cell hybridoma technology.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical composition.

"Native antibodies" refer to naturally occurring immunoglobulin molecules with varying structures. For example, native IgG antibodies are heterotetrameric glycoproteins of about 146,000 Daltons, composed of two identical light chains and two identical heavy chains that are disulfide-bonded. From N-to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

As used herein, the term "half antibody" refers to one immunoglobulin heavy chain associated with one immunoglobulin light chain.

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively.

The term "cluster of differentiation 3" or "CD3," as used herein, refers to any native CD3 from any vertebrate source, including mammals such as primates (e.g., humans and cynomolgus monkeys (cyno)) and rodents (e.g., mice and rats), unless otherwise indicated, including, for example, CD3ε, CD3y, CD3α, and CD3β chains. The term encompasses "full-length," unprocessed CD3 (e.g., unprocessed or unmodified CD3ε or CD3y), as well as any form of CD3 that results from processing in the cell. The term also encompasses naturally occurring variants of CD3, including, for example, splice variants or allelic variants. CD3 includes, for example, human CD3ε protein (NCBI RefSeq No. NP_000724), which is 207 amino acids in length, human CD3y protein (NCBI RefSeq No. NP_000064), which is 182 amino acids in length, cyno CD3ε protein (NCBI RefSeq No. NP_001270544.1), which is 198 amino acids in length, and cyno CD3y protein (NCBI RefSeq No. NP_001270839.1), which is 181 amino acids in length.

The term "cluster of differentiation 20" or "CD20," as used herein, refers to any native CD20 from any vertebrate source, including mammals such as primates (e.g., humans) and rodents (e.g., mice and rats), unless otherwise indicated. The term encompasses "full-length," unprocessed CD20, as well as any form of CD20 that results from processing in the cell. The term also encompasses naturally occurring variants of CD20, including, for example, splice variants or allclic variants. CD20 includes, for example, human CD20 protein (see, e.g., NCBI RefSeq Nos. NP_068769.2 and NP_690605.1), which is 297 amino acids in length and may be generated, for example, from variant mRNA transcripts that lack a portion of the 5' UTR (see, e.g., NCBI RefSeq No. NM_021950.3) or longer variant mRNA transcripts (see, e.g., NCBI RefSeq No. NM_152866.2).

The term "FcRH5" or "fragment crystallizable receptor-like 5," as used herein, refers to any native FcRH5 from any vertebrate source, including mammals such as primates (e.g., humans) and rodents (e.g., mice and rats), unless otherwise indicated, and encompasses "full-length," unprocessed FcRH5, as well as any form of FcRH5 that results from processing in the cell. The term also encompasses naturally occurring variants of FcRH5, including, for example, splice variants or allelic variants. FcRH5 includes, for example, human FcRH5 protein (UniProtKB/Swiss-Prot ID: Q96RD9.3), which is 977 amino acids in length.

The term "HER2," as used herein, refers to any native HER2 from any vertebrate source, including mammals such as primates (e.g., humans) and rodents (e.g., mice and rats), unless otherwise indicated. The term encompasses "full-length," unprocessed HER2, as well as any form of HER2 that results from processing in the cell. The term also encompasses naturally occurring variants of HER2, including, for example, splice variants or allelic variants. HER2 includes, for example, human HER2 protein (see, e.g., NCBI RefSeq No. NP_001276865), which is 1240 amino acids in length. Domain IV of HER2 is the extracellular protein region that is positioned closest to the cellular membrane. Domain IV has the amino acid sequence of SEQ ID NO: 17.

The terms "anti-CD20/anti-CD3 antibody," "anti-CD20/anti-CD3 antibody," "anti-CD20/anti-CD3 TDB," and "anti-CD20/anti-CD3 TCB," or variants thereof, refer to a multispecific antibody (e.g., a bispecific antibody) that is capable of binding to CD20 and CD3 with sufficient affinity such that the antibody is useful as a diagnostic and/or therapeutic agent in targeting CD20 and/or CD3. In one embodiment, the extent of binding of an anti-CD20/anti-CD3 antibody to an unrelated, non-CD3 protein and/or non-CD20 protein is less than about 10% of the binding of the antibody to CD3 and/or CD20 as measured, e.g., by a radioimmunoassay (RIA). In certain embodiments, the anti-CD20/anti-CD3 bispecific antibody binds to each of CD20 and/or CD3 with a dissociation constant (K_{D}) of ≤ 1 µM, ≤ 100 nM, ≤ 10 nM, ≤ 1 nM, ≤ 0.1 nM, ≤ 0.01 nM, or ≤ 0.001 nM (e.g., 10⁻⁸M or less, e.g., from 10⁻⁵M to 10⁻¹³M, e.g., from 10⁻⁹M to 10⁻¹³M). In certain embodiments, an anti-CD20/anti-CD3 antibody binds to an epitope of CD3 that is conserved among CD3 from different species and/or an epitope of CD20 that is conserved among CD20 from different species. Anti-CD20/anti-CD3 bispecific antibodies useful in the methods of the present disclosure include any of the anti-CD20/anti-CD3 bispecific antibodies described in PCT Pub. No. WO 2015/095392, which is incorporated herein by reference in its entirety. In some instances, the anti-CD20/anti-CD3 bispecific antibody is an anti-CD20/anti-CD3 T-cell dependent bispecific antibody (TDB). In a particular embodiment, the anti-CD20/anti-CD3 bispecific antibody is mosunetuzumab (also known as BTCT4465A or RG 7828), as defined by International Nonproprietary Names for Pharmaceutical Substances (INN) List 117 (WHO Drug Information, Vol. 31, No. 2, 2017, p. 304-305). In other embodiments, an anti-CD20/anti-CD3 bispecific antibody is a T cell activating bispecific antigen-binding molecule (e.g., a 2+1 TCB) described in U.S. Patent No. 9,914,776, which is incorporated herein by reference in its entirety. In a particular embodiment, the anti-CD20/anti-CD3 bispecific antibody is glofitamab (also known as RO 7082859 or RG6026), as defined by International Nonproprietary Names for Pharmaceutical Substances (INN) List 121 (WHO Drug Information (International Nonproprietary Names for Pharmaceutical Substances), Recommended INN: List 83, 2020, vol. 34, no. 1, p. 39), and with CAS registry number 2229047-91-8.

The terms "anti-FcRH5/anti-CD3 antibody," "anti-FcRH5/anti-CD3 bispecific antibody," and "anti-FcRH5/anti-CD3 TDB," or variants thereof, refer to a multispecific antibody (e.g., a bispecific antibody) that is capable of binding to FcRH5 and CD3 with sufficient affinity such that the antibody is useful as a diagnostic and/or therapeutic agent in targeting FcRH5 and/or CD3. In one embodiment, the extent of binding of an anti-FcRH5/anti-CD3 bispecific antibody to an unrelated, non-CD3 protein and/or non-FcRH5 protein is less than about 10% of the binding of the antibody to CD3 and/or FcRH5 as measured, e.g., by a radioimmunoassay (RIA). In certain embodiments, that the anti-FcRH5/anti-CD3 bispecific antibody binds to each of FcRH5 and/or CD3 with a dissociation constant (K_{D}) of ≤ 1 µM, ≤ 100 nM, ≤ 10 nM, ≤ 1 nM, ≤ 0.1 nM, ≤ 0.01 nM, or ≤ 0.001 nM (e.g., 10⁻⁸M or less, e.g., from 10⁻⁸M to 10⁻¹³M, e.g., from 10⁻⁹M to 10⁻¹¹M). In certain embodiments, an anti-FcRH5/anti-CD3 antibody binds to an epitope of CD3 that is conserved among CD3 from different species and/or an epitope of FcRH5 that is conserved among FcRH5 from different species. Anti-FcRH5/anti-CD3 bispecific antibodies useful in the methods of the present disclosure include any of the anti-FcRH5/anti-CD3 bispecific antibodies described in PCT Pub. Nos. WO 2016/205520, WO 2015/095392, and WO 2014/210064, and U.S. Patent No. 10,323,094, which are incorporated herein by reference in their entirety. In some instances, the anti-FcRH5/anti-CD3 bispecific antibody is an anti-FcRH5/anti-CD3 T-cell dependent bispecific antibody. In a particular embodiment, the anti-FcRH5 antibody is cevostamab (also known as BFCR4350A or RO7187797), is described in WHO Drug Information (International Nonproprietary Names for Pharmaceutical Substances), Recommended INN: List 84, Vol. 34, No. 3, published 2020 (see page 701) and has the CAS Registry No. 2249888-53-5. Cevostamab is an Fc-engineered, humanized, full-length non-glycosylated IgG1 kappa T-cell-dependent bispecific antibody (TDB) that binds FcRH5 and CD3.

The terms "anti-HER2/anti-CD3 antibody," "anti-HER2/anti-CD3 bispecific antibody," and "anti-HER2/anti-CD3 TDB," or variants thereof, refer to a multispecific antibody (e.g., a bispecific antibody) that is capable of binding to HER2 and CD3 with sufficient affinity such that the antibody is useful as a diagnostic and/or therapeutic agent in targeting HER2 and/or CD3. In one embodiment, the extent of binding of an anti-HER2/anti-CD3 bispecific antibody to an unrelated, non-CD3 protein and/or non-HER2 protein is less than about 10% of the binding of the antibody to CD3 and/or HER2 as measured, e.g., by a radioimmunoassay (RIA). In certain embodiments, that the anti-HER2/anti-CD3 bispecific antibody binds to each of HER2 and/or CD3 with a dissociation constant (K_{D}) of ≤ 1 µM, ≤ 100 nM, ≤ 10 nM, ≤ 1 nM, ≤ 0.1 nM, ≤ 0.01 nM, or ≤ 0.001 nM (e.g., 10⁻⁸M or less, c.g., from 10⁻⁸M to 10⁻¹³M, c.g., from 10⁻⁹M to 10⁻¹³M). In certain embodiments, an anti-HER2/anti-CD3 antibody binds to an epitope of CD3 that is conserved among CD3 from different species and/or an epitope of HER2 that is conserved among HER2 from different species. Anti-HER2/anti-CD3 bispecific antibodies useful in the methods of the present disclosure include any of the anti-HER2/anti-CD3 bispecific antibodies described in PCT Pub. No. WO 2015/095392, which is incorporated herein by reference in its entirety. In some instances, the anti-HER2/anti-CD3 bispecific antibody is an anti-HER2/anti-CD3 T-cell dependent bispecific antibody. In a particular embodiment, the anti-HER2/anti-CD3 bispecific antibody (anti-HER2/anti-CD3 TDB) is runimotamab, as defined by the International Nonproprietary Names for Pharmaceutical Substances (INN) List 124 (WHO Drug Information, Vol. 34, No. 4, 2020, p. 1031), and with CAS Registry No. 2361325-98-4.

The term "cluster of differentiation 79b" or "CD79b," as used herein, refers to any native CD79b from any vertebrate source, including mammals such as primates (e.g., humans) and rodents (e.g., mice and rats), unless otherwise indicated. The term encompasses "full-length," unprocessed CD79b, as well as any form of CD79b that results from processing in the cell. The term also encompasses naturally occurring variants of CD79b, including, for example, splice variants or allelic variants. CD79b includes, for example, human CD79b protein (NCBI RefSeq No. NP_000617), which is 229 amino acids in length.

The terms "anti-CD79b antibody" and "an antibody that binds to CD79b" refer to an antibody that is capable of binding CD79b with sufficient affinity such that the antibody is useful as a diagnostic and/or therapeutic agent in targeting CD79b. In one embodiment, the extent of binding of an anti-CD79b antibody to an unrelated, non-CD79b protein is less than about 10% of the binding of the antibody to CD79b as measured, e.g., by a radioimmunoassay (RIA). **In** certain embodiments, an antibody that binds to CD79b has a dissociation constant (K_{D}) of ≤ 1µM, ≤ 100 nM, ≤ 10 nM, ≤ 1 nM, ≤ 0.1 nM, ≤ 0.01 nM, or ≤ 0.001 nM (e.g., 10⁻⁸M or less, e.g., from 10⁻⁸M to 10⁻¹³M, e.g., from 10⁻⁹M to 10⁻¹³M). In certain embodiments, an anti-CD79b antibody binds to an epitope of CD79b that is conserved among CD79b from different species.

As used herein, the term "polatuzumab vedotin" refers to an anti-CD79b antibody drug conjugate having the IUPHAR/BPS Number 8404, the KEGG Number D10761, or the CAS Registry Number 1313206-42-6. Polatuzumab vedotin-piiq is also interchangeably referred to as "polatuzumab vedotinpiiq", "huMA79bv28-MC-vc-PAB-MMAE", "DCDS4501A", or "RG7596."

In some embodiments, an antibody drug conjugate is used that comprises the formula: 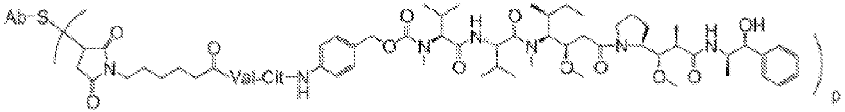 wherein Ab is polatuzumab, and wherein p is between 1 and 8. In some embodiments, the anti-CD79b antibody drug conjugate is huMA79bv28-MC-vc-PAB-MMAE. In some embodiments, the antibody drug conjugate is polatuzumab vedotin-piiq (CAS Registry Number 1313206-42-6). In some embodiments, the antibody drug conjugate is polatuzumab vedotin-piiq, as described in WHO Drug Information, Vol. 26, No. 4, 2012 (Proposed INN: List 108), which is expressly incorporated by reference herein in its entirety. As shown in WHO Drug Information, Vol. 26, No. 4, 2012, polatuzumab vedotin-piiq has the following structure: immunoglobulin G1-kappa auristatin E conjugate, anti-[Homo sapiens CD79B (immunoglobulin-associated CD79 beta)], humanized monoclonal antibody conjugated to auristatin E; gamma1 heavy chain (1-447) [humanized VH (Homo sapiens IGHV3-66*01 (79.60%) - (IGHO)-IGHJ4*01) [8.8.13] (1-120) -Homo sapiens IGHG1*03 (CH1 R120>K (214) (121- 218), hinge (219-233), CH2 (234-343), CH3 (344-448), CHS (449-450)) (121-450)], (220-218')-disulfide (if not conjugated) with kappa light chain (1'-218')[humanized V-KAPPA (Homo sapiens IGKV1-39*01 (80.00%) -IGKJ1*01) [11.3.9] (1'-112') -Homo sapiens IGKC*01 (113'-218')]; dimer (226-226":229- 229")-bisdisulfide; conjugated, on an average of 3 to 4 cysteinyl, to monomethylauristatin E (MMAE) via a cleavable maleimidecaproyl-valyl-citrullinyl-p-aminobenzylcarbamate (mc-val-cit-PABC) linker.

A "subject" or an "individual" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the subject or individual is a human.

As used herein, "administering" is meant a method of giving a dosage of a therapeutic protein (e.g., a TDB or TCB) or a composition (e.g., a pharmaceutical composition, e.g., a pharmaceutical composition including a therapeutic protein of the disclosure (e.g., a TDB or TCB)) to a subject. The pharmaceutical compositions utilized in the methods described herein can be administered, for example, intravenously, subcutaneously, intradermally, intramuscularly, percutaneously, intraarterially, intraperitoneally, intralesionally, intracranially, intraarticularly, intraprostatically, intrapleurally, intratracheally, intranasally, intravitreally, intravaginally, intrarectally, topically, intratumorally, peritoneally, subconjunctivally, intravesicularlly, mucosally, intrapericardially, intraumbilically, intraocularly, orally, topically, locally, by inhalation, by injection, by infusion, by continuous infusion, by localized perfusion bathing target cells directly, by catheter, by lavage, in cremes, or in lipid compositions. The method of administration can vary depending on various factors (e.g., the pharmaceutical composition being administered and the severity of the condition, disease, or disorder being treated).

As used herein, a "week" is 7 days ± 2 days.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, pharmaceutical compositions of the disclosure are used to delay development of a disease or to slow the progression of a disease.

As used herein, "enhancing immune function" in a subject means to induce, cause, stimulate, sustain, or amplify the innate or adaptive immune response. **In** some embodiments, enhancing immune function includes enhancing T-cell function. **In** some embodiments, the level of enhancement is as least 50%, alternatively 60%, 70%, 80%, 90%, 100%, 120%, 150%, 200%. The manner of measuring this enhancement is known to one of ordinary skill in the art.

As used herein, "enhancing T-cell function" means to induce, cause, or stimulate a T-cell to have a sustained or amplified biological function, or renew or reactivate exhausted or inactive T-cells. Examples of enhancing T-cell function include: increased secretion of γ-interferon from CD8+ T-cells, increased proliferation, increased antigen responsiveness (e.g., viral, pathogen, or tumor clearance) relative to such levels before the intervention. In some embodiment, the level of enhancements is as least 50%, alternatively 60%, 70%, 80%, 90%, 100%, 120%, 150%, 200%, 300%, 400%, 500%. The manner of measuring this enhancement is known to one of ordinary skill in the art.

As used herein, "delaying progression" of a disorder or disease means to defer, hinder, slow, retard, stabilize, and/or postpone development of the disease or disorder (e.g., a cell proliferative disorder, e.g., cancer). This delay can be of varying lengths of time, depending on the history of the disease and/or individual being treated. As is evident to one skilled in the art, a sufficient or significant delay can, in effect, encompass prevention, in that the individual does not develop the disease. For example, a late stage cancer, such as development of metastasis, may be delayed.

By "reduce" or "inhibit" is meant the ability to cause an overall decrease, for example, of 20% or greater, of 50% or greater, or of 75%, 85%, 90%, 95%, or greater. **In** certain embodiments, reduce or inhibit can refer to the effector function of an antibody that is mediated by the antibody Fc region, such effector functions specifically including complement-dependent cytotoxicity (CDC), antibody-dependent cellular cytotoxicity (ADCC), and antibody-dependent cellular phagocytosis (ADCP).

A "disorder" is any condition that would benefit from treatment including, but not limited to, chronic and acute disorders or diseases including those pathological conditions which predispose the mammal to the disorder in question.

The terms "cell proliferative disorder" and "proliferative disorder" refer to disorders that are associated with some degree of abnormal cell proliferation. In one embodiment, the cell proliferative disorder is cancer. In one embodiment, the cell proliferative disorder is a tumor.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia or lymphoid malignancies. More particular examples of such cancers include, but not limited to, squamous cell cancer (e.g., epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer and gastrointestinal stromal cancer, pancreatic cancer, glioblastoma, cervical cancer, ovarian cancer, liver cancer, bladder cancer, cancer of the urinary tract, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, melanoma, superficial spreading melanoma, lentigo maligna melanoma, acral lentiginous melanomas, nodular melanomas, multiple myeloma and B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom Macroglobulinemia); chronic lymphocytic leukemia (CLL); acute lymphoblastic leukemia (ALL); hairy cell leukemia; chronic myeloblastic leukemia; and post-transplant lymphoproliferative disorder (PTLD), as well as abnormal vascular proliferation associated with phakomatoses, edema (such as that associated with brain tumors), Meigs' syndrome, brain, as well as head and neck cancer, and associated metastases. In certain embodiments, cancers that are amenable to treatment by the antibodies of the disclosure include breast cancer, colorectal cancer, gastric cancer, rectal cancer, non-small cell lung cancer, glioblastoma, non-Hodgkins lymphoma (NHL), renal cell cancer, prostate cancer, liver cancer, pancreatic cancer, soft-tissue sarcoma, kaposi's sarcoma, carcinoid carcinoma, head and neck cancer, ovarian cancer, mesothelioma, and multiple myeloma. In some embodiments, the cancer is selected from: small cell lung cancer, gliblastoma, neuroblastomas, melanoma, breast carcinoma, gastric cancer, colorectal cancer (CRC), and hepatocellular carcinoma. Yet, in some embodiments, the cancer is selected from: non-small cell lung cancer, colorectal cancer, gastric cancer, glioblastoma, and breast carcinoma, including metastatic forms of those cancers. In other embodiments, the cancer is selected from a class of mature B cell cancers excluding Hodgkin's Lymphoma but including diffuse large B cell lymphoma (DLBCL), germinal-center B-cell-like (GCB) DLBCL, activated B-cell-like (ABC) DLBCL, follicular lymphoma (FL), mantle cell lymphoma (MCL), acute myeloid leukemia (AML), chronic lymphoid leukemia (CLL), marginal zone lymphoma (MZL), small lymphocytic leukemia (SLL), lymphoplasmacytic lymphoma (LL), Waldenstrom macroglobulinemia (WM), central nervous system lymphoma (CNSL), Burkitt's lymphoma (BL), B-cell prolymphocytic leukemia, Splenic marginal zone lymphoma, Hairy cell leukemia, Splenic lymphoma/leukemia, unclassifiable, Splenic diffuse red pulp small B-cell lymphoma, Hairy cell leukemia variant, Heavy chain diseases, α Heavy chain disease, γ Heavy chain disease, µ Heavy chain disease, Plasma cell myeloma, Solitary plasmacytoma of bone, Extraosseous plasmacytoma, Extranodal marginal zone lymphoma of mucosa-associated lymphoid tissue (MALT lymphoma), Nodal marginal zone lymphoma, Pediatric nodal marginal zone lymphoma, Pediatric follicular lymphoma, Primary cutaneous follicle center lymphoma, T-cell/histiocyte rich large B-cell lymphoma, Primary DLBCL of the CNS, Primary cutaneous DLBCL, leg type, EBV-positive DLBCL of the elderly, DLBCL associated with chronic inflammation, Lymphomatoid granulomatosis, Primary mediastinal (thymic) large B-cell lymphoma, Intravascular large B-cell lymphoma, ALK-positive large B-cell lymphoma, Plasmablastic lymphoma, Large B-cell lymphoma arising in HHV8-associated multicentric Castleman disease, Primary effusion lymphoma: B-cell lymphoma, unclassifiable, with features intermediate between diffuse large B-cell lymphoma and Burkitt lymphoma, and B-cell lymphoma, unclassifiable, with features intermediate between diffuse large B-cell lymphoma and classical Hodgkin lymphoma.

The term "HER2-positive" cancer comprises cancer cells which have higher than normal levels of HER2. Examples of HER2-positive cancer include HER2-positive breast cancer and HER2-positive gastric cancer. Optionally, HER2-positive cancer has an immunohistochemistry (IHC) score of 2+ or 3+ and/or an in situ hybridization (ISH) amplification ratio ≥2.0. **In** some instances, the HER2-positive cancer is a HER2-positive solid tumor. Additionally or alternatively, the HER2-positive cancer may be a locally advanced or metastatic HER2-positive cancer. In some instances, the HER2-positive cancer is a HER2-positive breast cancer or a HER2-positive gastric cancer. In some embodiments, the HER2-positive cancer is selected from the group consisting of a HER2-positive gastroesophageal junction cancer, a HER2-positive colorectal cancer, a HER2-positive lung cancer (e.g., a HER2-positive non-small cell lung carcinoma), a HER2-positive pancreatic cancer, a HER2-positive bladder cancer, a HER2-positive salivary duct cancer, a HER2-positive ovarian cancer (e.g., a HER2-positive epithelial ovarian cancer), or a HER2-positive endometrial cancer.

The term "FcRH5-positive cancer" refers to a cancer comprising cells that express FcRH5 on their surface. For the purposes of determining whether a cell expresses FcRH5 on the surface, FcRH5 mRNA expression is considered to correlate to FcRH5 expression on the cell surface. In some embodiments, expression of FcRH5 mRNA is determined by a method selected from in situ hybridization and RT-PCR (including quantitative RT-PCR). Alternatively, expression of FcRH5 on the cell surface can be determined, for example, using antibodies to FcRH5 in a method such as immunohistochemistry, FACS, etc. In some embodiments, FcRH5 is one or more of FcRH5a, FcRH5b, FcRH5c, UniProt Identifier Q96RD9-2, and/or FcRH5d. In some embodiments, the FcRH5 is FcRH5c. Particular examples of FcRH5-positive cancers include FcRH5-positive multiple myeloma (MM), FcRH5-positive chronic lymphoid leukemia (CLL), FcRH5-positive mantle cell lymphoma (MCL), FcRH5-positive diffuse large B-cell lymphoma (DLBCL), FcRH5-positive follicular lymphoma (FL), FcRH5-positive acute myeloid leukemia (AML), FcRH5-positive myelodysplastic syndrome (MDS), FcRH5-positive chronic myelogenous leukemia (CML), FcRH5-positive chronic myelomonocytic leukemia, FcRH5-positive acute promyelocytic leukemia (APL), FcRH5-positive chronic myeloproliferative disorder, FcRH5-positive thrombocytic leukemia, FcRH5-positive precursor B-cell acute lymphoblastic leukemia (pre-B-ALL), FcRH5-positive precursor T cell acute lymphoblastic leukemia (pre-T-ALL), FcRH5-positive mast cell disease, FcRH5-positive mast cell leukemia, FcRH5-positive mast cell sarcoma, FcRH5-positive myeloid sarcomas, FcRH5-positive lymphoid leukemia, and FcRH5-positive undifferentiated leukemia.

"Tumor," as used herein, refers to all neoplastic cell growth and proliferation, whether malignant or benign, and all pre-cancerous and cancerous cells and tissues. The terms "cancer", "cancerous", "cell proliferative disorder", "proliferative disorder", and "tumor" are not mutually exclusive as referred to herein.

The term "tumor antigen," as used herein, may be understood as those antigens that are presented on tumor cells. These antigens can be presented on the cell surface with an extracellular part, which is often combined with a transmembrane and cytoplasmic part of the molecule. These antigens can sometimes be presented only by tumor cells and never by the normal ones. Tumor antigens can be exclusively expressed on tumor cells or might represent a tumor specific mutation compared to normal cells. In this case, they are called tumor-specific antigens. More common are tumor antigens that are presented by tumor cells and normal cells, and they are called tumor-associated antigens. These tumor-associated antigens can be overexpressed compared to normal cells or are accessible for antibody binding in tumor cells due to the less compact structure of the tumor tissue compared to normal tissue.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g., B cell receptor); and B cell activation.

As used herein, the term "effector cells" refers to a population of lymphocytes that display effector moiety receptors, e.g., cytokine receptors, and/or Fc receptors on their surface through which they bind an effector moiety, e.g., a cytokine, and/or an Fc region of an antibody and contribute to the destruction of target cells, e.g., tumor cells. Effector cells may for example mediate cytotoxic or phagocytic effects. Effector cells include, but are not limited to, effector T cells such as CD8⁺ cytotoxic T cells, CD4⁺ helper T cells, yδ T cells, NK cells, lymphokine-activated killer (LAK) cells and macrophages/monocytes.

A "modification promoting the association of the first and the second subunit of the Fc domain" is a manipulation of the peptide backbone or the post-translational modifications of an Fc domain subunit that reduces or prevents the association of a polypeptide comprising the Fc domain subunit with an identical polypeptide to form a homodimer. A modification promoting association as used herein particularly includes separate modifications made to each of the two Fc domain subunits desired to associate (i.e., the first and the second subunit of the Fc domain), wherein the modifications are complementary to each other so as to promote association of the two Fc domain subunits. For example, a modification promoting association may alter the structure or charge of one or both of the Fc domain subunits so as to make their association sterically or electrostatically favorable, respectively. Thus, (hetero)dimerization occurs between a polypeptide comprising the first Fc domain subunit and a polypeptide comprising the second Fc domain subunit, which might be non-identical in the sense that further components fused to each of the subunits (e.g., antigen binding moieties) are not the same. In some embodiments the modification promoting association comprises an amino acid mutation in the Fc domain, specifically an amino acid substitution. In a particular embodiment, the modification promoting association comprises a separate amino acid mutation, specifically an amino acid substitution, in each of the two subunits of the Fc domain.

An "activating Fc receptor" is an Fc receptor that following engagement by an Fc region of an antibody elicits signaling events that stimulate the receptor-bearing cell to perform effector functions. Activating Fc receptors include FcyRllla (CD16a), FcγRI (CD64), FcyRlla (CD32), and FcaRI (CD89).

As used herein, the terms "engineer," "engineered," and "engineering" are considered to include any manipulation of the peptide backbone or the post-translational modifications of a naturally occurring or recombinant polypeptide or fragment thereof. Engineering includes modifications of the amino acid sequence, of the glycosylation pattern, or of the side chain group of individual amino acids, as well as combinations of these approaches. "Engineering", particularly with the prefix "glyco-", as well as the term "glycosylation engineering," includes metabolic engineering of the glycosylation machinery of a cell, including genetic manipulations of the oligosaccharide synthesis pathways to achieve altered glycosylation of glycoproteins expressed in cells. Furthermore, glycosylation engineering includes the effects of mutations and cell environment on glycosylation. In one embodiment, the glycosylation engineering is an alteration in glycosyltransferase activity. In a particular embodiment, the engineering results in altered glucosaminyltransferase activity and/or fucosyltransferase activity. Glycosylation engineering can be used to obtain a "host cell having increased GnTIII activity" (e.g., a host cell that has been manipulated to express increased levels of one or more polypeptides having β(1,4)-N-acetylglucosaminyltransferase **III** (GnTIII) activity), a "host cell having increased Manll activity" (e.g., a host cell that has been manipulated to express increased levels of one or more polypeptides having α-mannosidase II (Manll) activity), or a "host cell having decreased α(1,6) fucosyltransferase activity" (e.g., a host cell that has been manipulated to express decreased levels of α(1,6) fucosyltransferase).

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein. A host cell is any type of cellular system that can be used to generate proteins used for the present disclosure. In one embodiment, the host cell is engineered to allow the production of an antibody with modified oligosaccharides. In certain embodiments, the host cells have been manipulated to express increased levels of one or more polypeptides having β(1,4)-N-acetylglucosaminyltransferase III (GnTIII) activity. In certain embodiments the host cells have been further manipulated to express increased levels of one or more polypeptides having α-mannosidase II (Manll) activity. Host cells include cultured cells, e.g., mammalian cultured cells, such as CHO cells, BHK cells, NS0 cells, SP2/0 cells, YO myeloma cells, P3X63 mouse myeloma cells, PER cells, PER.C6 cells or hybridoma cells, yeast cells, insect cells, and plant cells, to name only a few, but also cells comprised within a transgenic animal, transgenic plant or cultured plant or animal tissue.

As used herein, the term "polypeptide having GnTIII activity" refers to a polypeptide that is able to catalyze the addition of a N-acetylglucosamine (GlcNAc) residue in β-1,4 linkage to the β-linked mannoside of the trimannosyl core of N-linked oligosaccharides. This includes fusion polypeptides exhibiting enzymatic activity similar to, but not necessarily identical to, an activity of β(1,4)-N-acetylglucosaminyltransferase **III,** also known as β-1,4-mannosyl-glycoprotein 4-beta-N-acetylglucosaminyl-transferase (EC 2.4.1.144), according to the Nomenclature Committee of the International Union of Biochemistry and Molecular Biology (NC-IUBMB), as measured in a particular biological assay, with or without dose dependency. In the case where dose dependency does exist, it need not be identical to that of GnTIII, but rather substantially similar to the dose-dependency in a given activity as compared to the GnTIII (i.e., the candidate polypeptide will exhibit greater activity or not more than about 25-fold less and, preferably, not more than about ten-fold less activity, and most preferably, not more than about three-fold less activity relative to the GnTIII). In certain embodiments the polypeptide having GnTIII activity is a fusion polypeptide comprising the catalytic domain of GnTIII and the Golgi localization domain of a heterologous Golgi resident polypeptide. Particularly, the Golgi localization domain is the localization domain of mannosidase II or GnTI, most particularly the localization domain of mannosidase II. Alternatively, the Golgi localization domain is selected from the group consisting of: the localization domain of mannosidase I, the localization domain of GnTll, and the localization domain of α1,6 core fucosyltransferase. Methods for generating such fusion polypeptides and using them to produce antibodies with increased effector functions are disclosed in WO2004/065540, U.S. Provisional Pat. Appl. No. 60/495,142 and U.S. Pat. Appl. Publ. No. 2004/0241817, the entire contents of which are expressly incorporated herein by reference.

As used herein, the term "Golgi localization domain" refers to the amino acid sequence of a Golgi resident polypeptide which is responsible for anchoring the polypeptide to a location within the Golgi complex. Generally, localization domains comprise amino terminal "tails" of an enzyme.

As used herein, the term "polypeptide having Manll activity" refers to polypeptides that are able to catalyze the hydrolysis of the terminal 1,3- and 1,6-linked α-D-mannose residues in the branched GlcNAcMan₅GlcNAc₂ mannose intermediate of N-linked oligosaccharides. This includes polypeptides exhibiting enzymatic activity similar to, but not necessarily identical to, an activity of Golgi α-mannosidase II, also known as mannosyl oligosaccharide 1,3-1,6-α-mannosidase II (EC 3.2.1.114), according to the Nomenclature Committee of the International Union of Biochemistry and Molecular Biology (NC-IUBMB).

An "effective amount" of a pharmaceutical composition, for example, a pharmaceutical composition including a therapeutic protein is at least the minimum amount required to achieve the desired therapeutic or prophylactic result, such as a measurable improvement of a particular disorder (e.g., a cell proliferative disorder, e.g., cancer). An effective amount herein may vary according to factors such as the disease state, age, sex, and weight of the patient, and the ability of the antibody to elicit a desired response in the individual. An effective amount is also one in which any toxic or detrimental effects of the treatment are outweighed by the therapeutically beneficial effects. For prophylactic use, beneficial or desired results include results such as reducing the risk, lessening the severity, or delaying the onset of the disease, including biochemical, histological and/or behavioral symptoms of the disease, its complications and intermediate pathological phenotypes presenting during development of the disease. For therapeutic use, beneficial or desired results include clinical results such as decreasing one or more symptoms resulting from the disease, increasing the quality of life of those suffering from the disease, decreasing the dose of other medications required to treat the disease, enhancing effect of another medication such as via targeting, delaying the progression of the disease, and/or prolonging survival. **In** the case of cancer or tumor, an effective amount of the drug may have the effect in reducing the number of cancer cells; reducing the tumor size; inhibiting (i.e., slow to some extent or desirably stop) cancer cell infiltration into peripheral organs; inhibit (i.e., slow to some extent and desirably stop) tumor metastasis; inhibiting to some extent tumor growth; and/or relieving to some extent one or more of the symptoms associated with the disorder. An effective amount can be administered in one or more administrations. For purposes of this disclosure, an effective amount of drug, compound, or pharmaceutical composition is an amount sufficient to accomplish prophylactic or therapeutic treatment either directly or indirectly. As is understood in the clinical context, an effective amount of a drug, compound, or pharmaceutical composition may or may not be achieved in conjunction with another drug, compound, or pharmaceutical composition. Thus, an "effective amount" may be considered in the context of administering one or more therapeutic agents, and a single agent may be considered to be given in an effective amount if, in conjunction with one or more other agents, a desirable result may be or is achieved.

The term "package insert" is used to refer to instructions customarily included in commercial packages of therapeutic products, that contain information about the indications, usage, dosage, administration, combination therapy, contraindications and/or warnings concerning the use of such therapeutic products.

The term "PD-1 axis binding antagonist" refers to a molecule that inhibits the interaction of a PD-1 axis binding partner with either one or more of its binding partner, so as to remove T-cell dysfunction resulting from signaling on the PD-1 signaling axis - with a result being to restore or enhance T-cell function (e.g., proliferation, cytokine production, target cell killing). As used herein, a PD-1 axis binding antagonist includes a PD-1 binding antagonist, a PD-L1 binding antagonist and a PD-L2 binding antagonist.

The term "PD-1 binding antagonist" refers to a molecule that decreases, blocks, inhibits, abrogates or interferes with signal transduction resulting from the interaction of PD-1 with one or more of its binding partners, such as PD-L1, PD-L2. In some embodiments, the PD-1 binding antagonist is a molecule that inhibits the binding of PD-1 to one or more of its binding partners. In a specific aspect, the PD-1 binding antagonist inhibits the binding of PD-1 to PD-L1 and/or PD-L2. For example, PD-1 binding antagonists include anti-PD-1 antibodies, antigen binding fragments thereof, immunoadhesins, fusion proteins, oligopeptides and other molecules that decrease, block, inhibit, abrogate or interfere with signal transduction resulting from the interaction of PD-1 with PD-L1 and/or PD-L2. In one embodiment, a PD-1 binding antagonist reduces the negative co-stimulatory signal mediated by or through cell surface proteins expressed on T lymphocytes mediated signaling through PD-1 so as render a dysfunctional T-cell less dysfunctional (e.g., enhancing effector responses to antigen recognition). In some embodiments, the PD-1 binding antagonist is an anti-PD-1 antibody. In a specific aspect, a PD-1 binding antagonist is MDX-1106 (nivolumab) described herein. In another specific aspect, a PD-1 binding antagonist is MK-3475 (lambrolizumab) described herein. In another specific aspect, a PD-1 binding antagonist is AMG 404 described herein. In another specific aspect, a PD-1 binding antagonist is REGN2810 (cemiplimab; LIBTAYO^{®}) described herein. In another specific aspect, a PD-1 binding antagonist is AMP-224 described herein and in PCT Pub. No. WO 2017/058780.

The term "PD-L1 binding antagonist" refers to a molecule that decreases, blocks, inhibits, abrogates or interferes with signal transduction resulting from the interaction of PD-L1 with either one or more of its binding partners, such as PD-1, B7-1. In some embodiments, a PD-L1 binding antagonist is a molecule that inhibits the binding of PD-L1 to its binding partners. In a specific aspect, the PD-L1 binding antagonist inhibits binding of PD-L1 to PD-1 and/or B7-1. In some embodiments, the PD-L1 binding antagonists include anti-PD-L1 antibodies, antigen binding fragments thereof, immunoadhesins, fusion proteins, oligopeptides and other molecules that decrease, block, inhibit, abrogate or interfere with signal transduction resulting from the interaction of PD-L1 with one or more of its binding partners, such as PD-1, B7-1. In one embodiment, a PD-L1 binding antagonist reduces the negative co-stimulatory signal mediated by or through cell surface proteins expressed on T lymphocytes mediated signaling through PD-L1 so as to render a dysfunctional T-cell less dysfunctional (e.g., enhancing effector responses to antigen recognition). In some embodiments, a PD-L1 binding antagonist is an anti-PD-L1 antibody. In a specific aspect, an anti-PD-L1 antibody is atezolizumab (MPDL3280A) described herein. In still another specific aspect, an anti-PD-L1 antibody is MDX-1105 (BMS-936559) described herein and in PCT Pub. No. WO 2016/201425. In still another specific aspect, an anti-PD-L1 antibody is MEDI4736 (durvalumab) described herein.

The term "PD-L2 binding antagonist" refers to a molecule that decreases, blocks, inhibits, abrogates or interferes with signal transduction resulting from the interaction of PD-L2 with either one or more of its binding partners, such as PD-1. In some embodiments, a PD-L2 binding antagonist is a molecule that inhibits the binding of PD-L2 to one or more of its binding partners. In a specific aspect, the PD-L2 binding antagonist inhibits binding of PD-L2 to PD-1. In some embodiments, the PD-L2 antagonists include anti-PD-L2 antibodies, antigen binding fragments thereof, immunoadhesins, fusion proteins, oligopeptides and other molecules that decrease, block, inhibit, abrogate or interfere with signal transduction resulting from the interaction of PD-L2 with either one or more of its binding partners, such as PD-1. In one embodiment, a PD-L2 binding antagonist reduces the negative co-stimulatory signal mediated by or through cell surface proteins expressed on T lymphocytes mediated signaling through PD-L2 so as render a dysfunctional T-cell less dysfunctional (e.g., enhancing effector responses to antigen recognition). In some embodiments, a PD-L2 binding antagonist is an immunoadhesin.

### II. Pharmaceutical Compositions

This disclosure provides pharmaceutical compositions that include therapeutic proteins, such as bispecific antibodies (e.g., anti-CD3 bispecific antibodies; e.g., anti-CD20/anti-CD3, anti-FcRH5/anti-CD3, or anti-HER2/anti-CD3 bispecific antibodies; e.g., T cell-dependent bispecific antibodies (TDBs) or T cell engaging bispecific antibodies (TCBs); e.g., anti-CD20/anti-CD3, anti-FcRH5/anti-CD3, or anti-HER2/anti-CD3 TDBs or TCBs; e.g., mosunetuzumab, glofitamab, cevostamab, or runimotamab), and uses thereof, for example, for treatment of cell-proliferative disorders (e.g., cancers, e.g., hematological cancers). Pharmaceutical compositions of the disclosure can be formulated to support a relatively low concentration of therapeutic protein (e.g., antibody; e.g., bispecific antibody; e.g., anti-CD3 bispecific antibody; e.g., T cell-dependent bispecific antibody or T cell engaging bispecific antibody; e.g., mosunetuzumab, glofitamab, cevostamab, or runimotamab).

Any of the therapeutic proteins described herein (e.g., anti-CD3 bispecific antibodies; e.g., TDBs or TCBs; e.g., mosunetuzumab, glofitamab, cevostamab, or runimotamab) can be used in the pharmaceutical compositions, but it is to be understood that other therapeutic proteins can also be used.

In one aspect, the disclosure provides a pharmaceutical composition that includes a therapeutic protein, a surfactant (e.g., polysorbate 20 (PS20)), a stabilizer (e.g., methionine), a buffering agent, and a carrier. In some embodiments, the molar ratio of the surfactant (e.g., PS20 or P188) to the therapeutic protein is 100 or less, e.g., 90 or less, 80 or less, 70 or less, 60 or less, 50 or less, 40 or less, 30 or less, 20 or less, 10 or less, 9 or less, 8 or less, 7 or less, 6 or less, 5 or less, 4 or less, 3 or less, 2 or less, or 1 or less, e.g., from 0.5 to 100, from 0.5 to 50, from 0.5 to 10, from 0.5 to 5, from 0.5 to 1, from 1 to 5, from 2 to 4, from 5 to 100, from 10 to 70, from 10 to 50, from 10 to 30, from 50 to 100, from 60 to 80, from 70 to 80, or from 40 to 50. In certain embodiments, the molar ratio of the surfactant (e.g., PS20 or P188) to the therapeutic protein is from 1 to 100. In some embodiments, the molar ratio of the surfactant (e.g., PS20) to the therapeutic protein is from 45 to 100, from 45 to 55, from 50 to 100, from 60 to 90, from 70 to 90, from 60 to 80, from 70 to 80, from 65 to 75, or from 75 to 85, e.g., about 45, 46, 47, 48, 49, 50, 51, 52, 53, 54, 55, 65, 66, 67, 68, 69, 70, 71, 72, 73, 74, 75, 76, 77, 78, 79, or 80. In a particular embodiment, the molar ratio of the surfactant (e.g., PS20) to the therapeutic protein (e.g., mosunetuzumab or runimotamab) is about 71, In another particular embodiment, the molar ratio of the surfactant (e.g., PS20) to the therapeutic protein (e.g., glofitamab) is about 79. In yet another particular embodiment, the molar ratio of the surfactant (e.g., PS20) to the therapeutic protein (e.g., cevostamab) is about 48. In other embodiments, the molar ratio of the surfactant (e.g., P188) to the therapeutic protein is from 5 to 50, from 5 to 25, from 10 to 15, or from 15 to 20, e.g., about 10, 11, 12, 13, 14, 15, 16, 17, 18, 19, or 20. In a particular embodiment, the molar ratio of surfactant (e.g., P188) to the therapeutic protein is about 14. In another particular embodiment, the molar ratio of surfactant (e.g., P188) to the therapeutic protein is about 11.5.

The pharmaceutical compositions may have any suitable concentration of the therapeutic protein (e.g., antibody; e.g., bispecific antibody; e.g., anti-CD3 bispecific antibody; e.g., T cell-dependent bispecific antibody or T cell engaging bispecific antibody; e.g., mosunetuzumab, glofitamab, cevostamab, or runimotamab). For example, in any of the preceding pharmaceutical compositions, the concentration of the therapeutic protein can be about 0.01 mg/ml to about 30 mg/ml, e.g., about 0.01 mg/ml, about 0.05 mg/ml, about 0.1 mg/ml, about 0.2 mg/ml, about 0.3 mg/ml, about 0.4 mg/ml, about 0.5 mg/ml, about 0.6 mg/ml, about 0.7 mg/ml, about 0.8 mg/ml, about 0.9 mg/ml, about 1 mg/ml, about 2 mg/ml, about 3 mg/ml, about 4 mg/ml, about 5 mg/ml, about 6 mg/ml, about 7 mg/ml, about 8 mg/ml, about 9 mg/ml, about 10 mg/ml, about 11 mg/ml, about 12 mg/ml, about 13 mg/ml, about 13.5 mg/ml, about 14 mg/ml, about 15 mg/ml, about 16 mg/ml, about 17 mg/ml, about 18 mg/ml, about 19 mg/ml, about 20 mg/ml, about 21 mg/ml, about 22 mg/ml, about 23 mg/ml, about 24 mg/ml, about 25 mg/ml, about 26 mg/ml, about 27 mg/ml, about 28 mg/ml, about 29 mg/ml, or about 30 mg/ml.

For example, in some embodiments, the therapeutic protein is an anti-CD20/anti-CD3 TDB, and the concentration of the anti-CD20/anti-CD3 TDB is about 0.1 mg/ml to about 10 mg/ml, about 0.1 mg/ml to about 5 mg/ml, about 0.1 mg/ml to about 3 mg/ml, about 0.1 mg/ml to about 2 mg/ml, about 0.1 mg/ml to about 1.5 mg/ml, about 0.3 mg/ml to about 2 mg/ml, about 0.5 mg/ml to about 2 mg/ml, about 0.5 mg/ml to about 5 mg/ml, about 0.5 mg/ml to about 2 mg/ml, about 0.6 mg/ml to about 2 mg/ml, about 0.7 mg/ml to about 2 mg/ml, about 0.8 mg/ml to about 2 mg/ml, about 0.9 mg/ml to about 10 mg/ml, about 0.5 mg/ml to about 1.4 mg/ml, about 0.5 mg/ml to about 1.3 mg/ml, about 0.5 mg/ml to about 1.2 mg/ml, about 0.5 mg/ml to about 1.1 mg/ml, about 0.5 mg/ml to about 1.5 mg/ml, about 0.8 mg/ml to about 1.2 mg/ml, about 1 mg/ml to about 10 mg/ml, about 2 mg/ml to about 10 mg/ml, about 3 mg/ml to about 10 mg/ml, about 4 mg/ml to about 10 mg/ml, about 5 mg/ml to about 10 mg/ml, about 6 mg/ml to about 10 mg/ml, about 7 mg/ml to about 10 mg/ml, about 8 mg/ml to about 10 mg/ml, about 9 mg/ml to about 10 mg/ml, about 1 mg/ml to about 5 mg/ml, about 1 mg/ml to about 3 mg/ml, about 1.5 mg/ml to about 2.5 mg/ml, about 1.8 mg/ml to about 2.2 mg/ml, about 2 mg/ml to about 5 mg/ml, about 3 mg/ml to about 5 mg/ml, or about 4 mg/ml to about 5 mg/ml. In a particular embodiment, the concentration of the anti-CD20/anti-CD3 TDB is about 1 mg/ml. In a particular embodiment, the concentration of the anti-CD20/anti-CD3 TDB is about 2 mg/ml.

In some embodiments, the therapeutic protein is an anti-HER2/anti-CD3 TDB, and the concentration of the anti-HER2/anti-CD3 TDB is about 0.1 mg/ml to about 10 mg/ml, about 0.1 mg/ml to about 5 mg/ml, about 0.1 mg/ml to about 3 mg/ml, about 0.1 mg/ml to about 2 mg/ml, about 0.1 mg/ml to about 1.5 mg/ml, about 0.3 mg/ml to about 2 mg/ml, about 0.5 mg/ml to about 2 mg/ml, about 0.5 mg/ml to about 5 mg/ml, about 0.5 mg/ml to about 2 mg/ml, about 0.6 mg/ml to about 2 mg/ml, about 0.7 mg/ml to about 2 mg/ml, about 0.8 mg/ml to about 2 mg/ml, about 0.9 mg/ml to about 10 mg/ml, about 0.5 mg/ml to about 1.4 mg/ml, about 0.5 mg/ml to about 1.3 mg/ml, about 0.5 mg/ml to about 1.2 mg/ml, about 0.5 mg/ml to about 1.1 mg/ml, about 0.5 mg/ml to about 1.5 mg/ml, about 0.8 mg/ml to about 1.2 mg/ml, about 1 mg/ml to about 5 mg/ml, about 1 mg/ml to about 3 mg/ml, about 1.5 mg/ml to about 2.5 mg/ml, about 1.8 mg/ml to about 2.2 mg/ml, about 2 mg/ml to about 5 mg/ml, about 3 mg/ml to about 5 mg/ml, or about 4 mg/ml to about 5 mg/ml. In some embodiments, the concentration of the anti-HER2/anti-CD3 TDB is about 0.5 mg/ml to about 1.5 mg/ml. In a particular embodiment, the concentration of the anti-HER2/anti-CD3 TDB is about 1 mg/ml. In another particular embodiment, the concentration of the anti-HER2/anti-CD3 TDB is about 2 mg/ml.

In some embodiments, the therapeutic protein is an anti-FcRH5/anti-CD3 TDB, and the concentration of the anti-FcRH5/anti-CD3 TDB is about 0.1 mg/ml to about 10 mg/ml, about 0.1 mg/ml to about 5 mg/ml, about 0.1 mg/ml to about 3 mg/ml, about 0.1 mg/ml to about 2 mg/ml, about 0.1 mg/ml to about 1.5 mg/ml, about 0.3 mg/ml to about 2 mg/ml, about 0.5 mg/ml to about 2 mg/ml, about 0.5 mg/ml to about 5 mg/ml, about 0.5 mg/ml to about 2 mg/ml, about 0.6 mg/ml to about 2 mg/ml, about 0.7 mg/ml to about 2 mg/ml, about 0.8 mg/ml to about 2 mg/ml, about 0.9 mg/ml to about 10 mg/ml, about 0.5 mg/ml to about 1.4 mg/ml, about 0.5 mg/ml to about 1.3 mg/ml, about 0.5 mg/ml to about 1.2 mg/ml, about 0.5 mg/ml to about 1.1 mg/ml, about 0.5 mg/ml to about 1.5 mg/ml, about 0.8 mg/ml to about 1.2 mg/ml, about 1 mg/ml to about 5 mg/ml, about 1 mg/ml to about 3 mg/ml, about 1.5 mg/ml to about 2.5 mg/ml, about 1.8 mg/ml to about 2.2 mg/ml, about 2 mg/ml to about 5 mg/ml, about 3 mg/ml to about 5 mg/ml, or about 4 mg/ml to about 5 mg/ml. In some embodiments, the concentration of the anti-FcRH5/anti-CD3 TDB is about 0.5 mg/ml to about 3 mg/ml. In a particular embodiment, the concentration of the anti-FcRH5/anti-CD3 TDB is about 1 mg/ml. In a particular embodiment, the concentration of the anti-FcRH5/anti-CD3 TDB is about 1.5 mg/ml. In a particular embodiment, the concentration of the anti-FcRH5/anti-CD3 TDB is about 3 mg/ml.

In some embodiments, the therapeutic protein is an anti-CD20/anti-CD3 TCB, and the concentration of the anti-CD20/anti-CD3 TCB is about 0.1 mg/ml to about 10 mg/ml, about 0.1 mg/ml to about 5 mg/ml, about 0.1 mg/ml to about 3 mg/ml, about 0.1 mg/ml to about 2 mg/ml, about 0.1 mg/ml to about 1.5 mg/ml, about 0.3 mg/ml to about 2 mg/ml, about 0.5 mg/ml to about 2 mg/ml, about 0.5 mg/ml to about 5 mg/ml, about 0.5 mg/ml to about 2 mg/ml, about 0.6 mg/ml to about 2 mg/ml, about 0.7 mg/ml to about 2 mg/ml, about 0.8 mg/ml to about 2 mg/ml, about 0.9 mg/ml to about 10 mg/ml, about 0.5 mg/ml to about 1.4 mg/ml, about 0.5 mg/ml to about 1.3 mg/ml, about 0.5 mg/ml to about 1.2 mg/ml, about 0.5 mg/ml to about 1.1 mg/ml, about 0.5 mg/ml to about 1.5 mg/ml, about 0.8 mg/ml to about 1.2 mg/ml, about 1 mg/ml to about 5 mg/ml, about 1 mg/ml to about 3 mg/ml, about 1.5 mg/ml to about 2.5 mg/ml, about 1.8 mg/ml to about 2.2 mg/ml, about 2 mg/ml to about 5 mg/ml, about 3 mg/ml to about 5 mg/ml, or about 4 mg/ml to about 5 mg/ml. In some embodiments, the concentration of the anti-CD20/anti-CD3 TCB is about 0.5 mg/ml to about 1.5 mg/ml. In a particular embodiment, the concentration of the anti-CD20/anti-CD3 TCB is about 1 mg/ml.

The disclosed pharmaceutical compositions include a surfactant. Any suitable surfactant can be used. In some embodiments, the surfactant is a nonionic surfactant (e.g., a polysorbate (a polyoxyethylene (n) sorbitan monolaurate), a poloxamer, a polyoxyethelene alkyl ether, an alkyl phenyl polyoxyethylene ether, or a combination thereof). In some embodiments, the nonionic surfactant is a polysorbate (e.g., polysorbate 20 (PS20; e.g., polyoxyethylene (20) sorbitan monolaurate, e.g., TWEEN 20^{®}; e.g., Super Refined^{™} PS20 (a PS20 that has been subjected to proprietary flash chromatographic process for greater purity and is available from Avantor Performance Materials, LLC (Center Valley, PA, U.S.)) or polysorbate 80 (PS80; e.g., polyoxyethylene (20) sorbitan monooleate, e.g., TWEEN 80^{®}; e.g., Super Refined^{™} PS80 (Avantor)). In a particular embodiment, the polysorbate is polysorbate 20 (PS20). In other embodiments, the nonionic surfactant is a poloxamer (e.g., poloxamer 188, poly(ethylene glycol)-block-poly(propylene glycol)-block-poly(ethylene glycol)).

Any suitable concentration of the surfactant may be used. The concentration of surfactant in the pharmaceutical composition can be selected based on the desired ratio of surfactant to therapeutic protein and the concentration of the therapeutic protein. In some embodiments of any of the pharmaceutical compositions described herein, the concentration of the surfactant (e.g., PS20 or P188) is about 0.001% (w/v) to about 2% (w/v), e.g., about 0.001%, about 0.005%, about 0.01%, about 0.02%, about 0.03%, about 0.04%, about 0.05%, about 0.06%, about 0.07%, about 0.08%, about 0.09%, about 0.1%, about 0.15%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, or about 2% (w/v). In some embodiments, the concentration of the surfactant (e.g., PS20 or P188) is about 0.01% (w/v) to about 0.12% (w/v). In some embodiments, the concentration of the surfactant (e.g., PS20 or P188) is about 0.05% (w/v) to about 0.12% (w/v). In a particular embodiment, the surfactant is PS20, and the concentration of PS20 is about 0.06% (w/v). In another particular embodiment, the surfactant is PS20 and the concentration of PS20 is about 0.0.05% (w/v). In yet another particular embodiment, the surfactant is PS20 and the concentration of PS20 is about 0.12% (w/v). In certain embodiments, the surfactant is P188, and the concentration of the P188 is about 0.08% (w/v).

Any of the pharmaceutical compositions described herein can include a stabilizer. Any suitable stabilizer can be used. For example, in some embodiments, the stabilizer is thiosorbitol, ascorbic acid, monothioglycerol, a cyclodextrin, Trolox ((±)-6-hydroxy-2,5,7,8-tetramethylchroman-2-carboxylic acid), pyridoxine, mannitol, a metal chelator, an amino acid, or a combination thereof. In some embodiments, the stabilizer is an amino acid. In some embodiments, the amino acid is methionine, cysteine, tryptophan, or a combination thereof. In a particular embodiment, the amino acid is methionine.

Any suitable concentration of the stabilizer (e.g., methionine) may be used. For example, in some embodiments of any of the preceding pharmaceutical compositions, the concentration of the stabilizer (e.g., methionine) is about 0.01 mM to about 50 mM, e.g., about 0.01 mM, about 0.05 mM, about 0.1 mM, about 0.2 mM, about 0.3 mM, about 0.4 mM, about 0.5 mM, about 0.6 mM, about 0.7 mM, about 0.8 mM, about 0.9 mM, about 1 mM, about 2 mM, about 3 mM, about 4 mM, about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 21 mM, about 22 mM, about 23 mM, about 24 mM, about 25 mM, about 26 mM, about 27 mM, about 28 mM, about 29 mM, about 30 mM, about 31 mM, about 32 mM, about 33 mM, about 34 mM, about 35 mM, about 36 mM, about 37 mM, about 38 mM, about 39 mM, about 40 mM, about 41 mM, about 42 mM, about 43 mM, about 44 mM, about 45 mM, about 46 mM, about 47 mM, about 48 mM, about 49 mM, or about 50 mM. In some embodiments, the concentration of the stabilizer (e.g., methionine) is about 1 mM to about 50 mM, about 2 mM to about 50 mM, about 3 mM to about 50 mM, about 4 mM to about 50 mM, about 5 mM to about 50 mM, about 6 mM to about 50 mM, about 7 mM to about 50 mM, about 8 mM to about 50 mM, about 9 mM to about 50 mM, about 10 mM to about 50 mM, about 15 mM to about 50 mM, about 20 mM to about 50 mM, about 25 mM to about 50 mM, about 30 mM to about 50 mM, about 1 mM to about 40 mM, about 2 mM to about 40 mM, about 3 mM to about 40 mM, about 4 mM to about 40 mM, about 5 mM to about 40 mM, about 5 mM to about 40 mM, about 6 mM to about 40 mM, about 7 mM to about 40 mM, about 8 mM to about 40 mM, about 9 mM to about 40 mM, about 10 mM to about 40 mM, about 15 mM to about 40 mM, about 20 mM to about 40 mM, about 25 mM to about 40 mM, about 30 mM to about 40 mM, about 1 mM to about 30 mM, about 2 mM to about 30 mM, about 3 mM to about 30 mM, about 4 mM to about 30 mM, about 5 mM to about 30 mM, about 6 mM to about 30 mM, about 7 mM to about 30 mM, about 8 mM to about 30 mM, about 9 mM to about 30 mM, about 10 mM to about 30 mM, about 11 mM to about 30 mM, about 12 mM to about 30 mM, about 13 mM to about 30 mM, about 14 mM to about 30 mM, about 15 mM to about 30 mM, about 20 mM to about 30 mM, about 25 mM to about 30 mM, about 1 mM to about 20 mM, about 2 mM to about 20 mM, about 3 mM to about 20 mM, about 4 mM to about 20 mM, about 5 mM to about 20 mM, about 6 mM to about 20 mM, about 7 mM to about 20 mM, about 8 mM to about 20 mM, about 9 mM to about 20 mM, about 10 mM to about 20 mM, about 11 mM to about 20 mM, about 12 mM to about 20 mM, about 13 mM to about 20 mM, about 14 mM to about 20 mM, about 15 mM to about 20 mM, about 1 mM to about 15 mM, about 2 mM to about 15 mM, about 3 mM to about 15 mM, about 4 mM to about 15 mM, about 5 mM to about 15 mM, about 6 mM to about 15 mM, about 7 mM to about 15 mM, about 8 mM to about 15 mM, about 9 mM to about 15 mM, about 10 mM to about 15 mM, about 11 mM to about 15 mM, about 12 mM to about 15 mM, about 13 mM to about 15 mM, about 14 mM to about 15 mM, about 1 mM to about 10 mM, about 2 mM to about 10 mM, about 3 mM to about 10 mM, about 4 mM to about 10 mM, about 5 mM to about 10 mM, about 6 mM to about 10 mM, about 7 mM to about 10 mM, about 8 mM to about 10 mM, about 9 mM to about 10 mM, about 1 mM to about 5 mM, about 2 mM to about 5 mM, about 3 mM to about 5 mM, or about 4 mM to about 5 mM.

In some embodiments, the concentration of the methionine is from 2.5 mM to 20 mM (e.g., from 2.5 mM to 5 mM, from 5 mM to 7.5 mM, from 7.5 mM to 10 mM, from 10 mM to 12.5 mM, from 12.5 mM to 15 mM, from 15 mM to 17.5 mM, or from 17.5 mM to 20 mM, e.g., from 3 mM to 18 mM, from 4 mM to 16 mM, from 5 mM to 14 mM, or from 8 mM to 12 mM, e.g., about 3 mM, about 4 mM, about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, or about 20 mM). In a particular embodiment, the concentration of methionine is about 10 mM.

In a pharmaceutical composition in which the therapeutic protein contains an Fc region (e.g., an antibody, e.g., a bispecific antibody, e.g., a TDB, e.g., an anti-CD20/anti-CD3 antibody, e.g., mosunetuzumab), oxidation of a methionine at position 257 of the Fc region is less than 10% over two weeks at 40 °C (e.g., less than 9%, less than 8%, less than 7%, less than 6%, less than 5%, less than 4%, less than 3%, less than 2%, or less than 1% over two weeks at 40%). In a particular embodiment, the oxidation of the methionine at position 257 of the Fc region is no more than about 6% over two weeks at 40 °C.

Any of the preceding compositions (e.g., pharmaceutical compositions) can further include a buffering agent. Any suitable buffering agent can be used. In some embodiments, the buffering agent is histidine, an acetate, a phosphate, a succinate, or a combination thereof. In some embodiments, the histidine is a histidine acetate. Alternative buffering agents include sodium phosphate monobasic, sodium phosphate dibasic, sodium phosphate tribasic, potassium phosphate monobasic, potassium phosphate dibasic, potassium phosphate tribasic, or a mixture thereof.

In certain instances, the buffering agent (e.g., histidine, e.g., histidine acetate) is at a concentration from 5 mM to 20 mM. For example, the buffering agent can be from 5 mM to 10 mM, from 10 mM to 15 mM, or from 15 mM to 20 mM, e.g., from 6 mM to 18 mM, from 7 mM to 16 mM, from 8 mM to 15 mM, or from 9 mM to 12 mM, e.g., about 5 mM, about 6 mM, about 7 mM, about 8 mM, about 9 mM, about 10 mM, about 11 mM about 12 mM, about 13 mM, about 14 mM, about 15 mM, about 16 mM, about 17 mM, about 18 mM, about 19 mM, or about 20 mM. In particular instances, the concentration of the buffering agent (e.g., histidine, e.g., histidine acetate) can be, e.g., from 8 mM to 12 mM, e.g., about 8 mM, about 9 mM, about 10 mM, about 11 mM, or about 12 mM. In some embodiments, the concentration of the buffering agent (e.g., histidine, e.g., histidine acetate or histidine HCl, e.g., L-histidine acetate or L-histidine HCl) is about 10 mM or 20 mM. In a particular embodiment, the buffering agent is histidine HCl at a concentration of about 20 mM. In another particular embodiment, the buffering agent is histidine acetate at a concentration of about 20 mM. In yet another particular embodiment, the buffering agent is histidine acetate at a concentration of about 10 mM.

In some embodiments, the pharmaceutical composition includes a tonicity agent, such as a carbohydrate (e.g., sucrose, glucose, dextrose, glycerol, glycerin, mannitol, and trehalose), an amino acid, or a salt (e.g., sodium chloride and potassium chloride). In embodiments in which the tonicity agent is a sugar, the sugar can be, e.g., sucrose, glucose, glycerol, or trehalose. In a particular embodiment, the sugar is sucrose. The tonicity agent (e.g., sugar, e.g., sucrose) can be at a concentration from about 100 mM to about 500 mM. For example, the tonicity agent (e.g., sugar, e.g., sucrose) can be at a concentration, such as from 100 mM to 120 mM, from 120 mM to 140 mM, from 140 mM to 160 mM, from 160 mM to 180 mM, from 180 mM to 200 mM, from 200 mM to 220 mM, from 220 mM to 240 mM, from 240 mM to 260 mM, from 260 mM to 280 mM, from 280 mM to 300 mM, from 300 mM to 320 mM, from 320 mM to 340 mM, from 340 mM to 360 mM, from 360 mM to 380 mM, from 380 mM to 400 mM, from 400 mM to 420 mM, from 420 mM to 440 mM, from 440 mM to 460 mM, from 460 mM to 480 mM, or from 480 mM to 500 mM, e.g., from 100 mM to 400 mM, from 150 mM to 350 mM, or from 200 mM to 300 mM, e.g., about 100 mM, about 150 mM, about 200 mM, about 210 mM, about 220 mM, about 230 mM, about 240 mM, about 250 mM, about 260 mM, about 270 mM, about 280 mM, about 290 mM, about 300 mM, about 350 mM, about 400 mM, about 450 mM, or about 500 mM). In some embodiments, the concentration of the tonicity agent is about 240 mM. In a particular embodiment, the tonicity agent is sucrose and present at a concentration of about 240 mM.

In some embodiments, the pharmaceutical composition further includes an antioxidant. In some embodiments, the antioxidant is N-acetyl-DL-tryptophan. In some embodiments, the concentration of N-acetyl-DL-tryptophan is from 0.1 mM to 0.5 mM (e.g., about 0.1 mM, about 0.15 mM, about 0.2 mM, about 0.25 mM, about 0.3 mM, about 0.35 mM, about 0.4 mM, about 0.45 mM, or about 0.5 mM). In some embodiments, the concentration of N-acetyl-DL-tryptophan is about 0.3 mM.

The pH of the pharmaceutical compositions can be any suitable pH. In some embodiments, the pharmaceutical composition has a pH range from about 4.5 to about 8 (e.g., from 4.5 to 5.0, from 5.0 to 5.5, from 5.5 to 6.0, from 6.0 to 6.5, from 6.5 to 7.0, from 7.0 to 7.5, or from 7.5 to 8.0, e.g., about 4.5, about 4.6, about 4.7, about 4.8, about 4.9, about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, about 6.5, about 6.6, about 6.7, about 6.8, about 6.9, about 7.0, about 7.1, about 7.2, about 7.3, about 7.4, about 7.5, about 7.6, about 7.7, about 7.8, about 7.9, or about 8.0). In some embodiments, the pH of the pharmaceutical composition is from 5.1 to 6.1. **In** a particular embodiment, the pH of the pharmaceutical composition is about 5.8. In another particular embodiment, the pH of the pharmaceutical composition is about 5.5.

In addition to the specific components described above, the pharmaceutical compositions of the disclosure can also be optionally prepared by mixing a therapeutic protein having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the used dosages and concentrations employed, and include, but are not limited to: buffers, such as phosphate, citrate, and other organic acids; antioxidants, including ascorbic acid and methionine; preservatives, such as octadecyldimethylbenzyl ammonium chloride, hexamethonium chloride, benzalkonium chloride, benzethonium chloride, phenol, butyl or benzyl alcohol, alkyl parabens such as methyl or propyl paraben, catechol, resorcinol, cyclohexanol, 3-pentanol, m-Cresol; low molecular weight (less than about 10 residues) polypeptides; proteins, such as serum albumin, gelatin, or immunoglobulins; hydrophilic polymers, such as polyvinylpyrrolidone; amino acids such as glycine, glutamine, asparagine, histidine, arginine, or lysine; carbohydrates, such as monosaccharides (e.g., glucose, mannose), disaccharides (e.g., sucrose, trehalose), or polysaccharides (e.g., dextrins), or sugar alcohols, such as mannitol or sorbitol; chelating agents such as ethylenediaminetetraacetic acid (EDTA); salt-forming counter-ions such as sodium; metal complexes (e.g., Zn-protein complexes); and/or non-ionic surfactants such as polyethylene glycol (PEG). Exemplary pharmaceutically acceptable carriers include interstitial drug dispersion agents, such as soluble neutral-active hyaluronidase glycoproteins (sHASEGP's), for example, human soluble PH-20 hyaluronidase glycoproteins, such as rHuPH20 (HYLENEX^{®}, Baxter International, Inc.). Certain exemplary sHASEGPs and methods of use, including rHuPH20, are described in U.S. Pat. Pub. Nos. 2005/0260186 and 2006/0104968. In one aspect, a sHASEGP is combined with one or more additional glycosaminoglycanases such as chondroitinases.

Exemplary lyophilized antibody formulations are described in U.S. Patent No. 6,267,958. Aqueous antibody formulations include those described in U.S. Patent No. 6,171,586 and WO 2006/044908, the latter formulations including a histidine-acetate buffer.

In some embodiments, the pharmaceutical composition is in a unit dosage form (e.g., liquid formulation for infusion, liquid formulation for injection, or liquid formulation for dilution). In a particular embodiment, the pharmaceutical composition is a liquid formulation for dilution. In a particular embodiment, the liquid formulation for dilution is supplied in a container having a volume of about 50 ml (e.g., about 40 ml, about 45 ml, about 46 ml, about 47 ml, about 48 ml, about 49 ml, about 50 ml, about 51 ml, about 52 ml, about 53 ml, about 54 ml, about 55 ml, or about 60 ml). In some embodiments, the volume of the liquid formulation for dilution is between 20-40 ml (e.g., between 20-30 ml, between 30-40 ml, between 20-35 ml, between 25-40 ml, between 25-35 ml, or between 28-32 ml; e.g., about 20 ml, about 25 ml, about 26 ml, about 27 ml, about 28 ml, about 29 ml, about 30 ml, about 31 ml, about 32 ml, about 33 ml, about 34 ml, about 35 ml, or about 40 ml). In a particular embodiment, the volume of the liquid formulation for dilution is about 30 ml. In some embodiments, the volume of the liquid formulation for dilution is between 10-20 ml (e.g., between 10-15 ml, between 15-20 ml, between 13-20 ml, between 10-17 ml, between 13-17 ml, or between 14-16 ml; e.g., about 10 ml, about 11 ml, about 12 ml, about 13 ml, about 14 ml, about 15 ml, about 16 ml, about 17 ml, about 18 ml, about 19 ml, or about 20 ml). In a particular embodiment, the volume of the liquid formulation for dilution is about 15 ml.

In another particular embodiment, the liquid formulation for dilution is supplied in a container having a volume of about 1 ml or about 2 ml (e.g., about 0.5 ml, about 0.6 ml, about 0.7 ml, about 0.8 ml, about 0.9 ml, about 1 ml, about 1.5 ml, about 1.6 ml, about 1.7 ml, about 1.8 ml, about 1.9 ml, about 2 ml, about 2.1 ml, about 2.2 ml, about 2.3 ml, about 2.4 ml, about 2.5 ml, or about 3 ml). In some embodiments, the liquid formulation for dilution is supplied in a container having a volume of about 2.5 ml. In some embodiments, the volume of the liquid formulation for dilution is between 0.2-2 ml (e.g., between 0.2-1.5 ml, between 0.5-2 ml, between 0.5-1 ml, or between 0.8-1.2 ml; e.g., about 0.2 ml, about 0.5 ml, about 0.6 ml, about 0.7 ml, about 0.8 ml, about 0.9 ml, about 1 ml, about 1.1 ml, about 1.2 ml, about 1.3 ml, about 1.4 ml, about 1.5 ml, or about 2 ml). In a particular embodiment, the volume of the liquid formulation for dilution is about 0.5 ml, about 0.9 ml, or about 1 ml.

In yet another particular embodiment, the liquid formulation for dilution is supplied in a container having a volume of about 15 ml (e.g., about 10 ml, about 11 ml, about 12 ml, about 13 ml, about 14 ml, about 15 ml, about 16 ml, about 17 ml, about 18 ml, about 19 ml, or about 20 ml). In some embodiments, the liquid formulation for dilution is supplied in a container having a volume of about 10 ml. In some embodiments, the volume of the liquid formulation for dilution is between 4-12 ml (e.g., between 4-8 ml, between 8-12 ml, between 4-10 ml, between 6-12 ml, between 6-10 ml, or between 7-9 ml; e.g., about 4 ml, about 5 ml, about 6 ml, about 7 ml, about 8 ml, about 9 ml, about 10 ml, about 11 ml, or about 12 ml). In a particular embodiment, the volume of the liquid formulation for dilution is about 8 ml.

In some embodiments, the liquid formulation is for dilution with a diluent. In some embodiments, the liquid formulation is for dilution with a saline solution. In some embodiments, the liquid formulation is for dilution with a normal saline solution. In some embodiments, the normal saline solution comprises sodium chloride (NaCl). In some embodiments, the normal saline solution comprises between 0.1-1.5% (e.g., between 0.1-1.2%, between 0.3-1.5%, between 0.4-0.5%, between 0.3-1%, between 0.8-1%, between 0.85-0.95%; e.g., about 0.1%, about 0.3%, about 0.4%, about 0.45%, about 0.5%, about 0.6%, about 0.7%, about 0.8%, about 0.85%, about 0.9%, about 0.95%, about 1%, or about 1.2%) (w/v) NaCl).

In some embodiments, after dilution with the normal saline solution, the concentration of the therapeutic protein (e.g., antibody; e.g., bispecific antibody; e.g., TDB or TCB; e.g., mosunetuzumab, glofitamab, cevostamab, or runimotamab) is from about 0.001 mg/ml to about 0.6 mg/ml (e.g., about 0.001 mg/ml, about 0.002 mg/ml, about 0.003 mg/ml, about 0.004 mg/ml, about 0.005 mg/ml, about 0.01 mg/ml, about 0.02 mg/ml, about 0.03 mg/ml, about 0.04 mg/ml, about 0.05 mg/ml, about 0.75 mg/ml, about 0.1 mg/ml, about 0.11 mg/ml, about 0.12 mg/ml, about 0.13 mg/ml, about 0.14 mg/ml, about 0.15 mg/ml, about 0.16 mg/ml, about 0.17 mg/ml, about 0.18 mg/ml, about 0.19 mg/ml, about 0.2 mg/ml, about 0.21 mg/ml, about 0.22 mg/ml, about 0.23 mg/ml, about 0.24 mg/ml, about 0.25 mg/ml, about 0.26 mg/ml, about 0.27 mg/ml, about 0.28 mg/ml, about 0.29 mg/ml, about 0.3 mg/ml, about 0.35 mg/ml, about 0.4 mg/ml about 0.45 mg/ml, about 0.5 mg/ml, about 0.55 mg/ml, or about 0.6 mg/ml). In particular embodiments, after dilution with the normal saline solution, the concentration of therapeutic protein (e.g., antibody; e.g., bispecific antibody; e.g., TDB or TCB; e.g., mosunetuzumab, glofitamab, cevostamab, or runimotamab) is about 0.003 mg/ml, about 0.01 mg/ml, about 0.02 mg/ml, about 0.03 mg/ml, about 0.04 mg/ml, about 0.12 mg/ml, about 0.24 mg/ml, or about 0.3 mg/ml. In a particular embodiment, after dilution with the normal saline solution, the concentration of the therapeutic antibody (e.g., antibody; bispecific antibody; anti-CD20/anti-CD3 bispecific antibody; anti-CD20/anti-CD3 TDB; e.g., mosunetuzumab) is about 0.01 mg/ml, about 0.02 mg/ml, about 0.04 mg/ml, about 0.12 mg/ml, about 0.24 mg/ml or about 0.3 mg/ml. In a particular embodiment, after dilution with the normal saline solution, the concentration of the therapeutic antibody (e.g., antibody; bispecific antibody; anti-CD20/anti-CD3 bispecific antibody; anti-CD20/anti-CD3 TCB; e.g., glofitamab) is about 0.1 mg/ml or about 0.6 mg/ml. In a particular embodiment, after dilution with the normal saline solution, the concentration of the therapeutic antibody (e.g., antibody; bispecific antibody; anti-FcRH5/anti-CD3 bispecific antibody; anti-FcRH5/anti-CD3 TDB; e.g., cevostamab) is about 0.003 mg/ml, 0.03 mg/ml, or 0.3 mg/ml.

The formulation herein may also contain more than one active ingredient as necessary for the particular indication being treated, preferably those with complementary activities that do not adversely affect each other. For example, it may be desirable to further provide an additional therapeutic agent (e.g., a chemotherapeutic agent, a cytotoxic agent, a growth inhibitory agent, and/or an anti-hormonal agent, such as those recited herein above). Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, for example, films, or microcapsules.

The formulations to be used for *in vivo* administration are generally sterile. Sterility may be readily accomplished, e.g., by filtration through sterile filtration membranes.

Any of the pharmaceutical compositions described herein can have a shelf-life of at least about 12 months (e.g., at least about 12 months, about 18 months, about 24 months, about 30 months, about 36 months, about 42 months, about 48 months, about 54 months, about 60 months, about 66 months, or about 72 months) when stored at 5 °C ± 3 °C and protected from light. In some embodiments, the pharmaceutical composition has a shelf-life of at least 36 months when stored at 5 °C ± 3 °C and protected from light. In some embodiments, the composition has a shelf-life of at least 42 months when stored at 5 °C ± 3 °C and protected from light. In some embodiments, the composition has a shelf-life of at least 48 months when stored at 5 °C ± 3 °C and protected from light.

In some embodiments, the shelf-life when stored at 5 °C ± 3 °C and protected from light is between about 1 month and about 72 months (e.g., about 1 month, about 5 months, about 10 months, about 15 months, about 20 months, about 24 months, about 25 months, about 30 months, about 35 months, about 40 months, about 45 months, about 48 months, about 50 months, about 55 months, about 60 months, about 65 months, about 70 months, or about 72 months). In some embodiments, the shelf-life when stored at 5 °C ± 3 °C and protected from light is between about 1 month and about 72 months, about 1 month and about 70 months, about 1 month and about 65 months, about 1 month and about 60 months, about 1 month and about 55 months, about 1 month and about 50 months, about 1 month and about 48 months, about 1 month and about 45 months, about 1 month and about 40 months, about 1 month and about 35 months, about 1 month and about 30 months, about 1 month and about 25 months, about 1 month and about 24 months, about 1 month and about 20 months, about 1 month and about 18 months, about 1 month and about 15 months, about 1 month and about 12 months, about 1 month and about 9 months, about 1 month and about 6 months, about 1 month and about 3 months, about 5 months and about 72 months, about 5 months and about 70 months, about 5 months and about 65 months, about 5 months and about 60 months, about 5 months and about 55 months, about 5 months and about 50 months, about 5 months and about 48 months, about 5 months and about 45 months, about 5 months and about 40 months, about 5 months and about 35 months, about 5 months and about 30 months, about 5 months and about 25 months, about 5 months and about 24 months, about 5 months and about 20 months, about 5 months and about 18 months, about 5 months and about 15 months, about 5 months and about 12 months, about 5 months and about 9 months, about 5 months and about 6 months, about 10 months and about 72 months, about 10 months and about 70 months, about 10 months and about 65 months, about 10 months and about 60 months, about 10 months and about 55 months, about 10 months and about 50 months, about 10 months and about 48 months, about 10 months and about 45 months, about 10 months and about 40 months, about 10 months and about 35 months, about 10 months and about 30 months, about 10 months and about 25 months, about 10 months and about 24 months, about 10 months and about 20 months, about 10 months and about 18 months, about 10 months and about 15 months, about 10 months and about 12 months, about 12 months and about 72 months, about 12 months and about 70 months, about 12 months and about 65 months, about 12 months and about 60 months, about 12 months and about 55 months, about 12 months and about 50 months, about 12 months and about 48 months, about 12 months and about 45 months, about 12 months and about 40 months, about 12 months and about 35 months, about 12 months and about 30 months, about 12 months and about 25 months, about 12 months and about 24 months, about 12 months and about 20 months, about 12 months and about 18 months, about 12 months and about 15 months, about 18 months and about 72 months, about 18 months and about 70 months, about 18 months and about 65 months, about 18 months and about 60 months, about 18 months and about 55 months, about 18 months and about 50 months, about 18 months and about 48 months, about 18 months and about 45 months, about 18 months and about 40 months, about 18 months and about 35 months, about 18 months and about 30 months, about 18 months and about 25 months, about 18 months and about 24 months, about 18 months and about 20 months, about 24 months and about 72 months, about 24 months and about 70 months, about 24 months and about 65 months, about 24 months and about 60 months, about 24 months and about 55 months, about 24 months and about 50 months, about 24 months and about 48 months, about 24 months and about 45 months, about 24 months and about 40 months, about 24 months and about 35 months, about 24 months and about 30 months, about 24 months and about 25 months, about 30 months and about 72 months, about 30 months and about 70 months, about 30 months and about 65 months, about 30 months and about 60 months, about 30 months and about 55 months, about 30 months and about 50 months, about 30 months and about 48 months, about 30 months and about 45 months, about 30 months and about 40 months, about 30 months and about 35 months, about 30 months and about 36 months, about 36 months and about 72 months, about 36 months and about 70 months, about 36 months and about 65 months, about 36 months and about 60 months, about 36 months and about 55 months, about 36 months and about 50 months, about 36 months and about 48 months, about 36 months and about 45 months, about 36 months and about 40 months, about 40 months and about 72 months, about 40 months and about 70 months, about 40 months and about 65 months, about 40 months and about 60 months, about 40 months and about 55 months, about 40 months and about 50 months, about 40 months and about 48 months, about 40 months and about 45 months, about 42 months and about 72 months, about 42 months and about 70 months, about 42 months and about 65 months, about 42 months and about 60 months, about 42 months and about 55 months, about 42 months and about 50 months, about 42 months and about 48 months, about 42 months and about 45 months, about 46 months and about 72 months, about 46 months and about 70 months, about 46 months and about 65 months, about 46 months and about 60 months, about 46 months and about 55 months, about 46 months and about 50 months, about 46 months and about 48 months, about 48 months and about 72 months, about 48 months and about 70 months, about 48 months and about 65 months, about 48 months and about 60 months, about 48 months and about 55 months, about 48 months and about 50 months, about 50 months and about 72 months, about 50 months and about 70 months, about 50 months and about 65 months, about 50 months and about 60 months, about 50 months and about 55 months, about 55 months and about 72 months, about 55 months and about 70 months, about 55 months and about 65 months, about 55 months and about 60 months, about 60 months and about 72 months, about 60 months and about 70 months, or about 60 months and about 65 months.

A stable pharmaceutical composition may include, e.g., no more than 1,000 particles having a diameter ≥ 2 µm per ml. For example, a pharmaceutical composition may have 900 or fewer, 800 or fewer, 700 or fewer, 600 or fewer, 500 or fewer, 400 or fewer, 300 or fewer, 200 or fewer, or 100 or fewer particles having a diameter ≥ 2 µm per ml (e.g., from 0 to 100, from 100 to 200, from 200 to 300, from 300 to 400, from 400 to 500, from 500 to 600, from 600 to 700, from 700 to 800, from 800 to 900, or from 900 to 1,000 particles having a diameter ≥ 2 µm per ml). In some embodiments, the carrier is water.

Additionally or alternatively, a stable pharmaceutical composition can have a purity of about 85% or higher. In some embodiments, the purity is about 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, e.g., from 85% to 90%, from 90% to 95%, or from 95% to 100%, e.g., as assessed by SE-HPLC. In a particular embodiment, the pharmaceutical composition has a purity of about 90% or higher as assessed by SE-HPLC, or about 95% or higher as assessed by SE-HPLC. In some embodiments, the pharmaceutical composition has a purity of about 95% or higher as assessed by SE-HPLC for about 36 months or longer at about 5 °C (e.g., 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, as assessed by SE-HPLC for about 36 months or longer at about 5 °C, e.g., from 85% to 90%, from 90% to 95%, or from 95% to 100%, as assessed by SE-HPLC for about 36 months or longer at about 5 °C. In particular instances, a pharmaceutical composition has a purity of about 95% or higher as assessed by SE-HPLC for about 42 months or longer at about 5 °C, e.g., for about 42 months, for about 60 months, for about 72 months, for about 84 months, for about 96 months, or longer, at about 5 °C.

In some instances, the disclosure provides a pharmaceutical composition having a purity of about 75% or higher as assessed by non-reduced capillary electrophoresis sodium dodecyl sulfate (CE-SDS) assay (e.g., about 76% or higher, about 77% or higher, about 78% or higher, about 79% or higher, about 80% or higher, about 81% or higher, about 82% or higher, about 83% or higher, about 84% or higher, 85% or higher, about 86% or higher, about 87% or higher, about 88% or higher, about 89% or higher, about 90% or higher, about 91% or higher, about 92% or higher, about 93% or higher, about 94% or higher, about 95% or higher, about 96% or higher, about 97% or higher, about 98% or higher, about 99% or higher, as assessed by non-reduced CE-SDS assay, e.g., from 75% to 80%, from 80% to 85%, from 85% to 90%, from 90% to 95%or from 95% to 100%, as assessed by non-reduced CE-SDS assay). In a particular embodiment, the pharmaceutical composition has a purity of about 80% or higher as assessed by non-reduced CE-SDS assay. For example, in some embodiments, the pharmaceutical composition has a purity of about 85% or higher as assessed by non-reduced CE-SDS assay. In some embodiments, the pharmaceutical composition has a purity of about 85% or higher as assessed by non-reduced CE-SDS assay for about 36 months or longer at about 5 °C (e.g., 85% or higher, about 86% or higher, about 87% or higher, about 88% or higher, about 89% or higher, about 90% or higher, about 91% or higher, about 92% or higher, about 93% or higher, about 94% or higher, about 95% or higher, about 96% or higher, about 97% or higher, about 98% or higher, about 99% or higher, as assessed by non-reduced CE-SDS assay, e.g., from 85% to 90%, from 90% to 95%, or from 95% to 100%, as assessed by non-reduced CE-SDS assay for about 36 months or longer at about 5 °C). In some embodiments, the pharmaceutical composition has a purity of about 85% or higher as assessed by non-reduced CE-SDS assay for about 42 months or longer at about 5 °C (e.g., 85% or higher, about 86% or higher, about 87% or higher, about 88% or higher, about 89% or higher, about 90% or higher, about 91% or higher, about 92% or higher, about 93% or higher, about 94% or higher, about 95% or higher, about 96% or higher, about 97% or higher, about 98% or higher, about 99% or higher, as assessed by non-reduced CE-SDS assay, e.g., from 85% to 90%, from 90% to 95%, or from 95% to 100%, as assessed by non-reduced CE-SDS assay for about 42 months or longer at about 5 °C).

### III. Exemplary Therapeutic Proteins

Therapeutic proteins in the pharmaceutical compositions of the disclosure include antibodies, such as bispecific antibodies, e.g., T cell-dependent bispecific antibodies (TDBs) or T cell engaging bispecific antibodies (TCBs). The antibody or bispecific antibody can bind to CD3 (e.g., an anti-CD3 antibody or an anti-CD3 bispecific antibody). TDBs can have an anti-CD3 arm having a binding domain that binds to CD3 and an anti-target arm that binds to a target antigen. The anti-target arm of such a TDB can be, for example, an anti-CD20 arm, an anti-FcRH5 arm, or an anti-HER2 arm. TCBs can have at least one CD3-binding moiety and at least one target antigen-binding moiety. In some embodiments, TCBs have one CD3-binding moiety and two target antigen-binding moieties. The target antigen-binding moiety of a TCB can be, e.g., CD20.

### Anti-CD3 Bispecific Antibodies

Anti-CD3 bispecific antibodies useful in the methods of the present disclosure include any of the anti-CD3 bispecific antibodies (e.g., TDBs) described in PCT Pub. No. WO 2015/095392, which is incorporated herein by reference in its entirety. In some embodiments, TDBs have an anti-CD3 arm. In some embodiments, the anti-CD3 arm includes a CD3-binding domain having one or more (e.g., one, two, three, four, five, or all six) hypervariable region (HVR) amino acid sequences of 40G5c. For example, in some embodiments, the CD3-binding domain has an HVR-H1 comprising the amino acid sequence of SEQ ID NO: 9; an HVR-H2 comprising the amino acid sequence of SEQ ID NO: 10; an HVR-H3 comprising the amino acid sequence of SEQ ID NO: 11; an HVR-L1 comprising the amino acid sequence of SEQ ID NO: 12; an HVR-L2 comprising the amino acid sequence of SEQ ID NO: 13; and an HVR-L3 comprising the amino acid sequence of SEQ ID NO: 14.

In some embodiments, the anti-CD3 antibody comprises at least one (e.g., 1, 2, 3, or 4) of heavy chain framework regions FR-H1, FR-H2, FR-H3, and FR-H4 comprising the sequences of SEQ ID NOs: 25-28, respectively, and/or at least one (e.g., 1, 2, 3, or 4) of the light chain framework regions FR-L1, FR-L2, FR-L3, and FR-L4 comprising the sequences of SEQ ID NOs: 29-32, respectively. In some embodiments, the CD3-binding domain comprises (a) a heavy chain variable (VH) domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 15; (b) a light chain variable (VL) domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 16; or (c) a VH domain as in (a) and a VL domain as in (b). In certain embodiments, the VH domain of the CD3-binding domain comprises the amino acid sequence of SEQ ID NO: 15 and the VL domain of the CD3-binding domain comprises the amino acid sequence of SEQ ID NO: 16. In particular instances, the CD3-binding domain can have the amino acid sequences of 40G5c, or a derivative or clonal relative thereof. Structural and functional characteristics of 40G5c are described, for example, in PCT Pub. No. WO 2015/095392, which is incorporated herein by reference in its entirety.

In other embodiments, the anti-CD3 arm includes a CD3-binding domain having one or more (e.g., one, two, three, four, five, or all six) hypervariable region (HVR) amino acid sequences of 38E4v1. For example, in some embodiments, the CD3-binding domain has an HVR-H1 comprising the amino acid sequence of SEQ ID NO: 65; an HVR-H2 comprising the amino acid sequence of SEQ ID NO: 66; an HVR-H3 comprising the amino acid sequence of SEQ ID NO: 67; an HVR-L1 comprising the amino acid sequence of SEQ ID NO: 68; an HVR-L2 comprising the amino acid sequence of SEQ ID NO: 69; and an HVR-L3 comprising the amino acid sequence of SEQ ID NO: 70.

In some embodiments, the anti-CD3 antibody comprises at least one (e.g., 1, 2, 3, or 4) of heavy chain framework regions FR-H1, FR-H2, FR-H3, and FR-H4 comprising the sequences of SEQ ID NOs: 81-84, respectively, and/or at least one (e.g., 1, 2, 3, or 4) of the light chain framework regions FR-L1, FR-L2, FR-L3, and FR-L4 comprising the sequences of SEQ ID NOs: 85-88, respectively. In some embodiments, the CD3-binding domain comprises (a) a heavy chain variable (VH) domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 71 ; (b) a light chain variable (VL) domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 72; or (c) a VH domain as in (a) and a VL domain as in (b). In certain embodiments, the VH domain of the CD3-binding domain comprises the amino acid sequence of SEQ ID NO: 71 and the VL domain of the CD3-binding domain comprises the amino acid sequence of SEQ ID NO: 72. In particular instances, the CD3-binding domain can have the amino acid sequences of 38E4v1, or a derivative or clonal relative thereof. Structural and functional characteristics of 38E4v1 are described, for example, in PCT Pub. No. WO 2015/095392, which is incorporated herein by reference in its entirety.

Anti-CD3 bispecific antibodies useful in the methods of the present disclosure include any of the anti-CD3 T cell activating bispecific antigen-binding molecules (e.g., a TCB, e.g., a 2+1 TCB) described in U.S. Patent No. 9,914,776, which is incorporated herein by reference in its entirety. In some embodiments, the anti-CD3 antibody includes a CD3-binding domain having one or more (e.g., one, two, three, four, five, or all six) of the following hypervariable regions (HVRs): an HVR-H1 comprising the amino acid sequence of SEQ ID NO: 45; an HVR-H2 comprising the amino acid sequence of SEQ ID NO: 46; an HVR-H3 comprising the amino acid sequence of SEQ ID NO: 47; an HVR-L1 comprising the amino acid sequence of SEQ ID NO: 48; an HVR-L2 comprising the amino acid sequence of SEQ ID NO: 49; and an HVR-L3 comprising the amino acid sequence of SEQ ID NO: 50, For example, in some embodiments, the CD3-binding domain has an HVR-H1 comprising the amino acid sequence of SEQ ID NO: 45; an HVR-H2 comprising the amino acid sequence of SEQ ID NO: 46; an HVR-H3 comprising the amino acid sequence of SEQ ID NO: 47; an HVR-L1 comprising the amino acid sequence of SEQ ID NO: 48; an HVR-L2 comprising the amino acid sequence of SEQ ID NO: 49; and an HVR-L3 comprising the amino acid sequence of SEQ ID NO: 50. In some embodiments, the CD3-binding domain comprises (a) a heavy chain variable (VH) domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 51; (b) a light chain variable (VL) domain comprising an amino acid sequence having at least 95% sequence identity (e.g., at least 96% sequence identity, at least 97% sequence identity, at least 98% sequence identity, at least 99% sequence identity, or 100% sequence identity) to the amino acid sequence of SEQ ID NO: 52; or (c) a VH domain as in (a) and a VL domain as in (b). In certain embodiments, the VH domain of the CD3-binding domain comprises the amino acid sequence of SEQ ID NO: 51 and the VL domain of the CD3-binding domain comprises the amino acid sequence of SEQ ID NO: 52.

In certain embodiments, the CD3-binding domain binds to an epitope within a fragment of CD3 (e.g., human CD3ε) consisting of amino acids 1-26 or 1-27 of human CD3ε.

CD3 binding domains of the any of the antibodies (e.g., TDBs or TCBs) described above may bind a particular CD3 epitope. For example, a CD3-binding domain can make contact with amino acids of human CD3ε at a distance of about 3.5 Angstroms, about 3.25 Angstroms, about 3.0 Angstroms, about 2.75 Angstroms, or less. In certain embodiments, an antibody binds to an epitope consisting of one, two, three, four, or five amino acids of human CD3ε at a distance of about 3.5 Angstroms, about 3.25 Angstroms, about 3.0 Angstroms, about 2.75 Angstroms or less. In one embodiment, the antibody makes contacts with amino acids of human CD3ε at a distance of about 3.5 Angstroms or less. In certain embodiments, an antibody binds to an epitope consisting of one, two, three, four, or five amino acids of human CD3ε at a distance of about 3.5 Angstroms or less. For example, in certain embodiments, an antibody binds to an epitope consisting of amino acids of human CD3ε selected from Gln1, Asp2, Asn4, Glu6, and Met7. In one particular embodiment, the CD3-binding domain binds to an epitope that specifically includes Glu6. In certain other embodiments, an antibody does not bind to an epitope that includes human CD3ε amino acid Glu5. In certain other embodiments, an antibody does not bind to an epitope that includes human CD3ε amino acids Gly3 and Glu5.

An anti-CD3 epitope may be determined by anti-CD3 antibody binding to peptide fragments of the epitope. Alternatively, an anti-CD3 epitope may be determined by alanine scanning mutagenesis. In particular instances, reduction in binding of an anti-CD3 antibody to mutated CD3 by about 20%, about 30%, about 50%, about 80% or more indicates the amino acid residue of CD3 mutated in an alanine scanning mutagenesis assay is an epitope residue for that anti-CD3 antibody. Alternatively, an anti-CD3 epitope may be determined by mass spectrometry. In some embodiments, the epitope is determined by crystallography.

In some embodiments, the epitope as determined by crystallography is determined using amino acids Q1-M7 of CD3.

In some embodiments, the epitope as determined by crystallography may be performed by combining the anti-CD3 antibody Fab, dissolved in 0.15 M NaCl, 25 mM tris, pH 7.5 at 10 mg/ml, with a 2-fold molar excess (1 mg) of CD3ε peptide and initially screening a sparse matrix of precipitants in a sitting drop vapor diffusion format. Optimized crystals may be grown from a 1:1 mixture with reservoir solution containing 70% v/v methyl-pentanediol and 0.1 M HEPES buffer at pH 7.5. The reservoir may be used as a cryoprotectant. The crystals may be transferred to cryogenic temperature by sudden immersion into liquid nitrogen. The diffraction data for crystals may be collected at Advanced Photon Source beam line 221D, using a MAR300 CCD detector (Argonne National Laboratory; Lemont, IL). The recorded diffractions may be integrated and scaled using the program HKL2000 (Z. Otwinowski and W. Minor, " Processing of X-ray Diffraction Data Collected in Oscillation Mode ", Methods in Enzymology, Volume 276: Macromolecular Crystallography, part A, p.307-326, 1997, C.W. Carter, Jr. & R. M. Sweet, Eds., Academic Press (New York)).

The structure may be phased by molecular replacement (MR) method using program Phaser (McCoy, A., et al. Phaser crystallographic software. J Appl Crystallogr. 2007 Aug 1 ;40(Pt 4):658-674). For example, the MR search model is a Fab subunit derived from a crystal structure of HGFA/Fab complex (Protein Data Bank (PDB; Berman H. M., Westbrook J., Feng Z., Gilliland G., Bhat T. N., Weissig H., Shindyalov I. N., Bourne P. E. (2000) The Protein Data Bank. Nucleic Acids Res. 28, 235-242) code: 2R0L (Wu Y., Eigenbrot C., Liang W.-C., Stawicki S., Shia S., Fan B., Ganesan R., Lipari M. T., Kirchhofer D. (2007) Structural insight into distinct mechanisms of protease inhibition by antibodies. Proc. Natl. Acad. Sci. U.S.A. 104, 19784-19789). The CD3ε peptide is built into the structure based on a Fo-Fc map. The structure may be subsequently refined with programs REFMAC5 (Murshudov GN, Vagin AA, Dodson EJ (1997) Acta Crystallogr D53:240-255) and PHENIX (D. Liebschner, P. V. Afonine, M. L. Baker, G. Bunkóczi, V. B. Chen, T. I. Croll, B. Hintze, L.-W. Hung, S. Jain, A. J. McCoy, N. W. Moriarty, R. D. Oeffner, B. K. Poon, M. G. Prisant, R. J. Read, J. S. Richardson, D. C. Richardson, M. D. Sammito, O. V. Sobolev, D. H. Stockwell, T. C. Terwilliger, A. G. Urzhumtsev, L. L. Videau, C. J. Williams, and P. D. Adams. Macromolecular structure determination using X-rays, neutrons and electrons: recent developments in Phenix. Acta Cryst. (2019). D75, 861-877) using the maximum likelihood target functions, anisotropic individual B-factor refinement method, and TLS (Translation-Libration-Screw-rotation) refinement method, to achieve convergence.

In certain other embodiments, a pharmaceutical composition includes an antibody that includes a paratope that binds to the same epitope as an anti-CD3 antibody described above.

### Anti-CD20/Anti-CD3 Bispecific Antibodies

In embodiments of the disclosure in which the therapeutic protein is an anti-CD20/anti-CD3 bispecific antibody, the anti-CD20/anti-CD3 bispecific antibody may incorporate any of the features, singly or in combination, as described herein.

### Mosunetuzumab

In some embodiments, the anti-CD20/anti-CD3 bispecific antibody (e.g., anti-CD20/anti-CD3 TDB; e.g., mosunetuzumab) comprises an anti-CD20 arm and an anti-CD3 arm. In some embodiments, the anti-CD20/anti-CD3 bispecific antibody includes (1) an anti-CD20 arm having a first binding domain comprising at least one, two, three, four, five, or six HVRs selected from (a) an HVR-H1 comprising the amino acid sequence of GYTFTSYNMH (SEQ ID NO: 1); (b) an HVR-H2 comprising the amino acid sequence of AIYPGNGDTSYNQKFKG (SEQ ID NO: 2); (c) an HVR-H3 comprising the amino acid sequence of VVYYSNSYWYFDV (SEQ ID NO: 3); (d) an HVR-L1 comprising the amino acid sequence of RASSSVSYMH (SEQ ID NO: 4); (e) an HVR-L2 comprising the amino acid sequence of APSNLAS (SEQ ID NO: 5); and (f) an HVR-L3 comprising the amino acid sequence of QQWSFNPPT (SEQ ID NO: 6); and (2) an anti-CD3 arm having a second binding domain comprising at least one, two, three, four, five, or six HVRs selected from (a) an HVR-H1 comprising the amino acid sequence of NYYIH (SEQ ID NO: 9); (b) an HVR-H2 comprising the amino acid sequence of WIYPGDGNTKYNEKFKG (SEQ ID NO: 10); (c) an HVR-H3 comprising the amino acid sequence of DSYSNYYFDY (SEQ ID NO: 11); (d) an HVR-L1 comprising the amino acid sequence of KSSQSLLNSRTRKNYLA (SEQ ID NO: 12); (e) an HVR-L2 comprising the amino acid sequence of WASTRES (SEQ ID NO: 13); and (f) an HVR-L3 comprising the amino acid sequence of TQSFILRT (SEQ ID NO: 14). In some instances, mosunetuzumab comprises (1) an anti-CD20 arm comprising at least one (e.g., 1, 2, 3, or 4) of heavy chain framework regions FR-H1, FR-H2, FR-H3, and FR-H4 comprising the sequences of SEQ ID NOs: 17-20, respectively, and/or at least one (e.g., 1, 2, 3, or 4) of the light chain framework regions FR-L1, FR-L2, FR-L3, and FR-L4 comprising the sequences of SEQ ID NOs: 21-24, respectively, and (2) an anti-CD3 arm comprising at least one (e.g., 1, 2, 3, or 4) of heavy chain framework regions FR-H1, FR-H2, FR-H3, and FR-H4 comprising the sequences of SEQ ID NOs: 25-28, respectively, and/or at least one (e.g., 1, 2, 3, or 4) of the light chain framework regions FR-L1, FR-L2, FR-L3, and FR-L4 comprising the sequences of SEQ ID NOs: 29-32, respectively. In some instances, mosunetuzumab comprises (1) an anti-CD20 arm comprising a first binding domain comprising (a) a VH domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 7; (b) a VL domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 8; or (c) a VH domain as in (a) and a VL domain as in (b), and (2) an anti-CD3 arm comprising a second binding domain comprising (a) a VH domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 15; (b) a VL domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 16; or (c) a VH domain as in (a) and a VL domain as in (b). In some instances, mosunetuzumab comprises (1) an anti-CD20 arm comprising a first binding domain comprising a VH domain comprising an amino acid sequence of SEQ ID NO: 7 and a VL domain comprising an amino acid sequence of SEQ ID NO: 8 and (2) an anti-CD3 arm comprising a second binding domain comprising a VH domain comprising an amino acid sequence of SEQ ID NO: 15 and a VL domain comprising an amino acid sequence of SEQ ID NO: 16.

In some instances, mosunetuzumab has the International Nonproprietary Names for Pharmaceutical Substances (INN) List 117 (WHO Drug Information, Vol. 31, No. 2, 2017, p. 303), or CAS Registry No. 1905409-39-3, and having (1) an anti-CD20 arm comprising the heavy chain and light chain sequences of SEQ ID NOs: 33 and 34, respectively; and (2) an anti-CD3 arm comprising the heavy chain and light chain sequences of SEQ ID NOs: 35 and 36, respectively.

In some instances, mosunetuzumab comprises (1) an anti-CD20 arm comprising a first binding domain comprising (a) a heavy chain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 33; (b) a light chain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 34; or (c) a heavy chain as in (a) and a light chain as in (b), and (2) an anti-CD3 arm comprising a second binding domain comprising (a) a heavy chain comprising an amino acid sequence having at least 90% sequence identity (e.g., at least 91%, 92%, 93%, 94%, 95% 96%, 97%, 98%, or 99% sequence identity) to, or the sequence of, SEQ ID NO: 35; (b) a light chain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 36; or (c) a heavy chain as in (a) and a light chain as in (b). In some instances, mosunetuzumab comprises (1) an anti-CD20 arm comprising a first binding domain comprising a heavy chain comprising an amino acid sequence of SEQ ID NO: 33 and a light chain comprising an amino acid sequence of SEQ ID NO: 34 and (2) an anti-CD3 arm comprising a second binding domain comprising a heavy chain comprising an amino acid sequence of SEQ ID NO: 35 and a light chain comprising an amino acid sequence of SEQ ID NO: 36.

Amino acid sequences of mosunetuzumab are summarized in Table 1 below.

**Table 1. Sequence IDs for Mosunetuzumab**

| CD3 Arm | | CD20 Arm | |
|---|---|---|---|
| SEQ ID NO: | Description | SEQ ID NO: | Description |
| 9 | CD3 HVR-H1 | 1 | CD20 HVR-H1 |
| 10 | CD3 HVR-H2 | 2 | CD20 HVR-H2 |
| 11 | CD3 HVR-H3 | 3 | CD20 HVR-H3 |
| 12 | CD3 HVR-L1 | 4 | CD20 HVR-L1 |
| 13 | CD3 HVR-L2 | 5 | CD20 HVR-L2 |
| 14 | CD3 HVR-L3 | 6 | CD20 HVR-L3 |
| 15 | CD3 VH | 7 | CD20 VH |
| 16 | CD3 VL | 8 | CD20 VL |
| 35 | CD3 heavy chain | 33 | CD20 heavy chain |
| 36 | CD3 light chain | 34 | CD20 light chain |

Mosunetuzumab may be produced using recombinant methods and compositions, for example, as described in U.S. Patent No. 4,816,567.

### Glofitamab

In some other embodiments, the anti-CD20/anti-CD3 bispecific antibody (e.g., anti-CD20/anti-CD3 TCB, e.g., glofitamab) comprises: at least one antigen binding domain that specifically binds to CD20 comprising a heavy chain variable region comprising: an HVR-H1 comprising the amino acid sequence of YSWIN (SEQ ID NO: 37); an HVR-H2 comprising the amino acid sequence of RIFPGDGDTDYNGKFKG (SEQ ID NO: 38); and an HVR-H3 comprising the amino acid sequence of NVFDGYWLVY (SEQ ID NO: 39); and a light chain variable region comprising: an HVR-L1 comprising the amino acid sequence of RSSKSLLHSNGITYLY (SEQ ID NO: 40); an HVR-L2 comprising the amino acid sequence of QMSNLVS (SEQ ID NO: 41); and an HVR-L3 comprising the amino acid sequence of AQNLELPYT (SEQ ID NO: 42); and at least one antigen binding domain that specifically binds to CD3 comprising a heavy chain variable region comprising: an HVR-H1 comprising the amino acid sequence of TYAMN (SEQ ID NO: 45); an HVR-H2 comprising the amino acid sequence of RIRSKYNNYATYYADSVKG (SEQ ID NO: 46); and an HVR-H3 comprising the amino acid sequence of HGNFGNSYVSWFAY (SEQ ID NO: 47); and a light chain variable region comprising: an HVR-L1 comprising the amino acid sequence of GSSTGAVTTSNYAN (SEQ ID NO: 48); an HVR-L2 comprising the amino acid sequence of GTNKRAP (SEQ ID NO: 49); and an HVR-L3 comprising the amino acid sequence of ALWYSNLWV (SEQ ID NO: 50).

In one embodiment, the anti-CD20/anti-CD3 bispecific antibody (e.g., anti-CD20/anti-CD3 TCB, e.g., glofitamab) comprises: at least one antigen binding domain that specifically binds to CD20 comprising a VH domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 43 and a VL domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 44, and at least one antigen binding domain that specifically binds to CD3 comprising a VH domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 51 and a VL domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 52.

In one embodiment, the antigen binding domain that specifically binds to CD3 of the anti-CD20/anti-CD3 bispecific antibody (e.g., anti-CD20/anti-CD3 TCB, e.g., glofitamab) is an antibody fragment, particularly a Fab molecule or a scFv molecule, more particularly a Fab molecule. In a particular embodiment, the antigen binding domain that specifically binds to CD3 of the anti-CD20/anti-CD3 bispecific antibody (e.g., anti-CD20/anti-CD3 TCB, e.g., glofitamab) is a crossover Fab molecule wherein the variable domains or the constant domains of the Fab heavy and light chain are exchanged (i.e., replaced by each other).

In one embodiment, the antigen binding domain that specifically binds to CD20 of the anti-CD20/anti-CD3 bispecific antibody (e.g., anti-CD20/anti-CD3 TCB, e.g., glofitamab) is an antibody fragment, particularly a Fab molecule or a scFv molecule, more particularly a Fab molecule. In a particular embodiment, the antigen binding domain that specifically binds to CD20 of the anti-CD20/anti-CD3 bispecific antibody (e.g., anti-CD20/anti-CD3 TCB, e.g., glofitamab) is a conventional Fab molecule.

In one embodiment, the anti-CD20/anti-CD3 bispecific antibody (e.g., anti-CD20/anti-CD3 TCB, e.g., glofitamab) comprises at least one antigen binding domain that specifically binds to CD20, and one antigen binding domain that specifically binds to CD3. In one embodiment, the anti-CD20/anti-CD3 bispecific antibody (e.g., anti-CD20/anti-CD3 TCB, e.g., glofitamab) comprises a first antigen binding domain that specifically binds to CD3, and a second and a third antigen binding domain that specifically bind to CD20. In one embodiment, the first antigen binding domain is a crossover Fab molecule, and the second and the third antigen binding domain are each a conventional Fab molecule. In one embodiment, the anti-CD20/anti-CD3 bispecific antibody (e.g., anti-CD20/anti-CD3 TCB, e.g., glofitamab) further comprises an Fc domain. The anti-CD20/anti-CD3 bispecific antibody may comprise modifications in the Fc region and/or the antigen binding domains as described herein. In one embodiment, the anti-CD20/anti-CD3 bispecific antibody (e.g., anti-CD20/anti-CD3 TCB, e.g., glofitamab) comprises an IgG1 Fc domain comprising one or more amino acid substitutions that reduce binding to an Fc receptor and/or effector function. In one embodiment the anti-CD20/anti-CD3 bispecific antibody (e.g., anti-CD20/anti-CD3 TCB, e.g., glofitamab) comprises an IgG1 Fc domain comprising the amino acid substitutions L234A, L235A and P329G (EU numbering).

In one embodiment the anti-CD20/anti-CD3 bispecific antibody (e.g., anti-CD20/anti-CD3 TCB, e.g., glofitamab) comprises: an antigen binding domain that specifically binds to CD3 which is fused at the C-terminus of the Fab heavy chain to the N-terminus of the first subunit of the Fc domain; a first antigen binding domain that specifically binds to CD20 which is fused at the C-terminus of the Fab heavy chain to the N-terminus of the Fab heavy chain of the antigen binding domain that specifically binds to CD3; and a second antigen binding domain that specifically binds to CD20 which is fused at the C-terminus of the Fab heavy chain to the N-terminus of the second subunit of the Fc domain.

In a particular embodiment, the anti-CD20/anti-CD3 bispecific antibody (e.g., anti-CD20/anti-CD3 TCB, e.g., glofitamab) comprises: (a) a first Fab molecule which specifically binds to CD3, particularly CD3 epsilon; and wherein the variable domains VL and VH of the Fab light chain and the Fab heavy chain are replaced by each other; (b) a second and a third Fab molecule which specifically bind to CD20, wherein in the constant domain CL of the second and third Fab molecule the amino acid at position 124 is substituted by lysine (K) (numbering according to Kabat) and the amino acid at position 123 is substituted by lysine (K) or arginine (R), particularly by arginine (R) (numbering according to Kabat), and wherein in the constant domain CH1 o of the second Fab and third Fab molecule the amino acid at position 147 is substituted by glutamic acid (E) (EU numbering) and the amino acid at position 213 is substituted by glutamic acid (E) (EU numbering); and (c) a Fc domain composed of a first and a second subunit capable of stable association.

In one embodiment, the anti-CD20/anti-CD3 bispecific antibody (e.g., anti-CD20/anti-CD3 TCB, e.g., glofitamab) comprises two antigen binding domains that specifically bind to CD20 and one antigen binding domain that specifically binds to CD3.

In one embodiment, the anti-CD20/anti-CD3 bispecific antibody (e.g., anti-CD20/anti-CD3 TCB, e.g., glofitamab) is bivalent for CD20 and monovalent for CD3.

In one embodiment the first Fab molecule (i.e., which binds CD3) is fused at the C-terminus of the Fab heavy chain to the N-terminus of one of the subunits of the Fc domain, the second Fab molecule (i.e., which binds CD20) is fused at the C-terminus of the Fab heavy chain to the N-terminus of the heavy chain of the first Fab molecule, and the third Fab molecule (i.e., which binds CD20) is fused at the C-terminus of the Fab heavy chain to the N-terminus of the other subunit of the Fc domain. In one embodiment, the first Fab molecule comprises a VH domain that is at least 95%, 96%, 97%, 98%, or 99% identical to the sequence of SEQ ID NO: 51, and a VL domain that is at least 95%, 96%, 97%, 98%, or 99% identical to the sequence of SEQ ID NO: 52. In still a further embodiment, the first Fab molecule comprises a heavy chain variable region sequence comprising the amino acid sequence of SEQ ID NO: 51, and a light chain variable region comprising the amino acid sequence of SEQ ID NO: 52.

In one embodiment, the second Fab molecule and the third Fab molecule each comprise a heavy chain variable region that is at least 95%, 96%, 97%, 98%, or 99% identical to the sequence of SEQ ID NO: 43, and a light chain variable region that is at least 95%, 96%, 97%, 98%, or 99% identical to the sequence of SEQ ID NO: 44.

In one embodiment, the second Fab molecule and the third Fab molecule each comprise the heavy chain variable region sequence of SEQ ID NO: 43, and the light chain variable region sequence of SEQ ID NO: 44.

In a particular embodiment, the anti-CD20/anti-CD3 bispecific antibody (e.g., anti-CD20/anti-CD3 TCB; e.g., glofitamab) comprises a polypeptide that is at least 95%, 96%, 97%, 98%, or 99% identical to the sequence of SEQ ID NO: 53, a polypeptide that is at least 95%, 96%, 97%, 98%, or 99% identical to the sequence of SEQ ID NO: 54, a polypeptide that is at least 95%, 96%, 97%, 98%, or 99% identical to the sequence of SEQ ID NO: 55, and a polypeptide that is at least 95%, 96%, 97%, 98%, or 99% identical to the sequence of SEQ ID NO: 56. In a further particular embodiment, the bispecific antibody comprises a polypeptide sequence of SEQ ID NO: 53, a polypeptide sequence of SEQ ID NO: 54, a polypeptide sequence of SEQ ID NO: 55 and a polypeptide sequence of SEQ ID NO: 56. In a further particular embodiment, the bispecific antibody comprises one polypeptide chain comprising the amino acid sequence of SEQ ID NO: 53, one polypeptide chain comprising the amino acid sequence of SEQ ID NO: 54, one polypeptide chain comprising the amino acid sequence of SEQ ID NO: 55, and two polypeptide chains each comprising the amino acid sequence of SEQ ID NO: 56.

Particular anti-CD20/anti-CD3 bispecific antibodies are described in PCT publication no. WO 2016/020309 and European patent application nos. EP15188093 and EP16169160 (each incorporated herein by reference in its entirety).

The sequences of glofitamab are summarized in Table 2.

**Table 2. Sequence IDs for Glofitamab**

| **Sequence IDs for glofitamab** | | | |
|---|---|---|---|
| CD3 Heavy Chain | | CD3 Light Chain | |
| SEQ ID NO: | Description | SEQ ID NO: | Description |
| 45 | HVR-H1 (Kabat) | 48 | HVR-L1 (Kabat) |
| 46 | HVR-H2 (Kabat) | 49 | HVR-L2 (Kabat) |
| 47 | HVR-H3 (Kabat) | 50 | HVR-L3 (Kabat) |
| 51 | VH | 52 | VL |

| CD20 Heavy Chain | | CD20 Light Chain | |
|---|---|---|---|
| 37 | HVR-H1 (Kabat) | 40 | HVR-L1 (Kabat) |
| 38 | HVR-H2 (Kabat) | 41 | HVR-L2 (Kabat) |
| 39 | HVR-H3 (Kabat) | 42 | HVR-L3 (Kabat) |
| 43 | VH | 44 | VH |

| Full-length antibody | | | |
|---|---|---|---|
| 53 | HC-knob | 54 | HC-hole |
| 56 | LC-CD3 | 55 | LC-CD20 |

In one embodiment the anti-CD20/anti-CD3 bispecific antibody of the pharmaceutical composition of the disclosure is glofitamab.

### Anti-FcRH5/Anti-CD3 Bispecific Antibodies

In embodiments of the disclosure in which the therapeutic protein is an anti-FcRH5/anti-CD30 bispecific antibody, the anti-FcRH5/anti-CD3 bispecific antibody may incorporate any of the features, singly or in combination, as described herein.

### Cevostamab

In some embodiments, the anti-FcRH5/anti-CD3 bispecific antibody (e.g., anti-FcRH5/anti-CD3 TDB; e.g., cevostamab) comprises an anti-FcRH5 arm and an anti-CD3 arm. In some embodiments, the anti-FcRH5/anti-CD3 bispecific antibody includes (1) an anti-FcRH5 arm having a first binding domain comprising at least one, two, three, four, five, or six HVRs selected from (a) an HVR-H1 comprising the amino acid sequence of RFGVH (SEQ ID NO: 57); (b) an HVR-H2 comprising the amino acid sequence of VIWRGGSTDYNAAFVS (SEQ ID NO: 58); (c) an HVR-H3 comprising the amino acid sequence of HYYGSSDYALDN (SEQ ID NO: 59); (d) an HVR-L1 comprising the amino acid sequence of KASQDVRNLVV (SEQ ID NO: 60); (e) an HVR-L2 comprising the amino acid sequence of SGSYRYS (SEQ ID NO: 61); and (f) an HVR-L3 comprising the amino acid sequence of QQHYSPPYT (SEQ ID NO: 62); and (2) an anti-CD3 arm having a second binding domain comprising at least one, two, three, four, five, or six HVRs selected from (a) an HVR-H1 comprising the amino acid sequence of SYYIH (SEQ ID NO: 65); (b) an HVR-H2 comprising the amino acid sequence of WIYPENDNTKYNEKFKD (SEQ ID NO: 66); (c) an HVR-H3 comprising the amino acid sequence of DGYSRYYFDY (SEQ ID NO: 67); (d) an HVR-L1 comprising the amino acid sequence of KSSQSLLNSRTRKNYLA (SEQ ID NO: 68); (c) an HVR-L2 comprising the amino acid sequence of WTSTRKS (SEQ ID NO: 69); and (f) an HVR-L3 comprising the amino acid sequence of KQSFILRT (SEQ ID NO: 70). In some instances, cevostamab comprises (1) an anti-FcRH5 arm comprising at least one (e.g., 1, 2, 3, or 4) of heavy chain framework regions FR-H1, FR-H2, FR-H3, and FR-H4 comprising the sequences of SEQ ID NOs: 73-76, respectively, and/or at least one (e.g., 1, 2, 3, or 4) of the light chain framework regions FR-L1, FR-L2, FR-L3, and FR-L4 comprising the sequences of SEQ ID NOs: 77-80, respectively, and (2) an anti-CD3 arm comprising at least one (e.g., 1, 2, 3, or 4) of heavy chain framework regions FR-H1, FR-H2, FR-H3, and FR-H4 comprising the sequences of SEQ ID NOs: 81-84, respectively, and/or at least one (e.g., 1, 2, 3, or 4) of the light chain framework regions FR-L1, FR-L2, FR-L3, and FR-L4 comprising the sequences of SEQ ID NOs: 85-88, respectively. In some instances, cevostamab comprises (1) an anti-FcRH5 arm comprising a first binding domain comprising (a) a VH domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 63; (b) a VL domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 64; or (c) a VH domain as in (a) and a VL domain as in (b), and (2) an anti-CD3 arm comprising a second binding domain comprising (a) a VH domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 71; (b) a VL domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 72; or (c) a VH domain as in (a) and a VL domain as in (b). In some instances, cevostamab comprises (1) an anti-FcRH5 arm comprising a first binding domain comprising a VH domain comprising an amino acid sequence of SEQ ID NO: 63 and a VL domain comprising an amino acid sequence of SEQ ID NO: 64 and (2) an anti-CD3 arm comprising a second binding domain comprising a VH domain comprising an amino acid sequence of SEQ ID NO: 71 and a VL domain comprising an amino acid sequence of SEQ ID NO: 72.

In some instances, cevostamab is described in WHO Drug Information (International Nonproprietary Names for Pharmaceutical Substances), Recommended INN: List 84, Vol. 34, No. 3, published 2020 (see page 701), has the CAS Registry No. 1905409-39-3, and has (1) an anti-FcRH5 arm comprising the heavy chain and light chain sequences of SEQ ID NOs: 89 and 90, respectively; and (2) an anti-CD3 arm comprising the heavy chain and light chain sequences of SEQ ID NOs: 91 and 92, respectively.

In some instances, cevostamab comprises (1) an anti-FcRH5 arm comprising a first binding domain comprising (a) a heavy chain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 89; (b) a light chain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 90; or (c) a heavy chain as in (a) and a light chain as in (b), and (2) an anti-CD3 arm comprising a second binding domain comprising (a) a heavy chain comprising an amino acid sequence having at least 90% sequence identity (e.g., at least 91%, 92%, 93%, 94%, 95%. 96%, 97%, 98%, or 99% sequence identity) to, or the sequence of, SEQ ID NO: 91 ; (b) a light chain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 92; or (c) a heavy chain as in (a) and a light chain as in (b). In some instances, cevostamab comprises (1) an anti-FcRH5 arm comprising a first binding domain comprising a heavy chain comprising an amino acid sequence of SEQ ID NO: 89 and a light chain comprising an amino acid sequence of SEQ ID NO: 90 and (2) an anti-CD3 arm comprising a second binding domain comprising a heavy chain comprising an amino acid sequence of SEQ ID NO: 91 and a light chain comprising an amino acid sequence of SEQ ID NO: 92.

Amino acid sequences of cevostamab are summarized in Table 3 below.

**Table 3. Sequence IDs for Cevostamab**

| CD3 Arm | | FcRH5 Arm | |
|---|---|---|---|
| SEQ ID NO: | Description | SEQ ID NO: | Description |
| 65 | CD3 HVR-H1 | 57 | FcRH5 HVR-H1 |
| 66 | CD3 HVR-H2 | 58 | FcRH5 HVR-H2 |
| 67 | CD3 HVR-H3 | 59 | FcRH5 HVR-H3 |
| 68 | CD3 HVR-L1 | 60 | FcRH5 HVR-L1 |
| 69 | CD3 HVR-L2 | 61 | FcRH5 HVR-L2 |
| 70 | CD3 HVR-L3 | 62 | FcRH5 HVR-L3 |
| 71 | CD3 VH | 63 | FcRH5 VH |
| 72 | CD3 VL | 64 | FcRH5 VL |
| 91 | CD3 heavy chain | 89 | FcRH5 heavy chain |
| 92 | CD3 light chain | 90 | FcRH5 light chain |

Cevostamab may be produced using recombinant methods and compositions, for example, as described in U.S. Patent No. 4,816,567.

### Anti-HER2/Anti-CD3 Bispecific Antibodies

In embodiments of the disclosure in which the therapeutic protein is an anti-HER2/anti-CD3 bispecific antibody, the anti-HER2/anti-CD3 bispecific antibody may incorporate any of the features, singly or in combination, as described herein.

### Runimotamab

In some embodiments, the anti-HER2/anti-CD3 bispecific antibody (e.g., anti-HER2/anti-CD3 TDB; e.g., runimotamab) comprises an anti-HER2 arm and an anti-CD3 arm. In some embodiments, the anti-HER2/anti-CD3 bispecific antibody includes (1) an anti-HER2 arm having a first binding domain comprising at least one, two, three, four, five, or six HVRs selected from (a) an HVR-H1 comprising the amino acid sequence of DTYIH (SEQ ID NO: 93); (b) an HVR-H2 comprising the amino acid sequence of RIYPTNGYTRYADSVKG (SEQ ID NO: 94); (c) an HVR-H3 comprising the amino acid sequence of WGGDGFYAMDY (SEQ ID NO: 95); (d) an HVR-L1 comprising the amino acid sequence of RASQDVNTAVA (SEQ ID NO: 96); (e) an HVR-L2 comprising the amino acid sequence of SASFLYS (SEQ ID NO: 97); and (f) an HVR-L3 comprising the amino acid sequence of QQHYTTPPT (SEQ ID NO: 98); and (2) an anti-CD3 arm having a second binding domain comprising at least one, two, three, four, five, or six HVRs selected from (a) an HVR-H1 comprising the amino acid sequence of NYYIH (SEQ ID NO: 109); (b) an HVR-H2 comprising the amino acid sequence of WIYPGDGNTKYNEKFKG (SEQ ID NO: 110); (c) an HVR-H3 comprising the amino acid sequence of DSYSNYYFDY (SEQ ID NO: 111); (d) an HVR-L1 comprising the amino acid sequence of KSSQSLLNSRTRKNYLA (SEQ ID NO: 112); (e) an HVR-L2 comprising the amino acid sequence of WASTRES (SEQ ID NO: 113); and (f) an HVR-L3 comprising the amino acid sequence of TQSFILRT (SEQ ID NO: 114). In some instances, runimotamab comprises (1) an anti-HER2 arm comprising at least one (e.g., 1, 2, 3, or 4) of heavy chain framework regions FR-H1, FR-H2, FR-H3, and FR-H4 comprising the sequences of SEQ ID NOs: 101-104, respectively, and/or at least one (e.g., 1, 2, 3, or 4) of the light chain framework regions FR-L1, FR-L2, FR-L3, and FR-L4 comprising the sequences of SEQ ID NOs: 105-108, respectively, and (2) an anti-CD3 arm comprising at least one (e.g., 1, 2, 3, or 4) of heavy chain framework regions FR-H1, FR-H2, FR-H3, and FR-H4 comprising the sequences of SEQ ID NOs: 117-120, respectively, and/or at least one (e.g., 1, 2, 3, or 4) of the light chain framework regions FR-L1, FR-L2, FR-L3, and FR-L4 comprising the sequences of SEQ ID NOs: 121-124, respectively. In some instances, runimotamab comprises (1) an anti-HER2 arm comprising a first binding domain comprising (a) a VH domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 99; (b) a VL domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 100; or (c) a VH domain as in (a) and a VL domain as in (b), and (2) an anti-CD3 arm comprising a second binding domain comprising (a) a VH domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 115; (b) a VL domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 116; or (c) a VH domain as in (a) and a VL domain as in (b). In some instances, runimotamab comprises (1) an anti-HER2 arm comprising a first binding domain comprising a VH domain comprising an amino acid sequence of SEQ ID NO: 99 and a VL domain comprising an amino acid sequence of SEQ ID NO: 100 and (2) an anti-CD3 arm comprising a second binding domain comprising a VH domain comprising an amino acid sequence of SEQ ID NO: 115 and a VL domain comprising an amino acid sequence of SEQ ID NO: 116.

In some instances, runimotamab has the International Nonproprietary Names for Pharmaceutical Substances (INN) List 124 (WHO Drug Information, Vol. 34, No. 4, 2020, p. 1031), or CAS Registry No. 2361325-98-4, and having (1) an anti-HER2 arm comprising the heavy chain and light chain sequences of SEQ ID NOs: 125 and 126, respectively; and (2) an anti-CD3 arm comprising the heavy chain and light chain sequences of SEQ ID NOs: 128 and 127, respectively.

In some instances, runimotamab comprises (1) an anti-HER2 arm comprising a first binding domain comprising (a) a heavy chain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 125; (b) a light chain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 126; or (c) a heavy chain as in (a) and a light chain as in (b), and (2) an anti-CD3 arm comprising a second binding domain comprising (a) a heavy chain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 127; (b) a light chain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 128; or (c) a heavy chain as in (a) and a light chain as in (b). In some instances, runimotamab comprises (1) an anti-HER2 arm comprising a first binding domain comprising a heavy chain comprising an amino acid sequence of SEQ ID NO: 125 and a light chain comprising an amino acid sequence of SEQ ID NO: 126 and (2) an anti-CD3 arm comprising a second binding domain comprising a heavy chain comprising an amino acid sequence of SEQ ID NO: 127 and a light chain comprising an amino acid sequence of SEQ ID NO: 128.

Amino acid sequences of runimotamab are summarized in Table 4 below.

**Table 4. Sequence IDs for Runimotamab**

| CD3 Arm | | HER2 Arm | |
|---|---|---|---|
| SEQ ID NO: | Description | SEQ ID NO: | Description |
| 109 | CD3 HVR-H1 | 93 | HER2 HVR-H1 |
| 110 | CD3 HVR-H2 | 94 | HER2 HVR-H2 |
| 111 | CD3 HVR-H3 | 95 | HER2 HVR-H3 |
| 112 | CD3 HVR-L1 | 96 | HER2 HVR-L1 |
| 113 | CD3 HVR-L2 | 97 | HER2 HVR-L2 |
| 114 | CD3 HVR-L3 | 98 | HER2 HVR-L3 |
| 115 | CD3 VH | 99 | HER2 VH |
| 116 | CD3 VL | 100 | HER2 VL |
| 127 | CD3 heavy chain | 125 | HER2 heavy chain |
| 128 | CD3 light chain | 126 | HER2 light chain |

Runimotamab may be produced using recombinant methods and compositions, for example, as described in U.S. Patent No. 4,816,567.

### IV. Antibody Properties, Modifications, and Methods of Characterization

The pharmaceutical compositions provided herein may further include any of the antibodies described above, wherein the antibody comprises a VH as in any of the embodiments provided above, and a VL as in any of the embodiments provided above, wherein one or both of the variable domain sequences include post-translational modifications. Additionally or alternatively, an antibody (e.g., a bispecific antibody, e.g., a TDB or a TCB) according to any of the above embodiments may incorporate any of the features, singly or in combination, as described below.

### 1. Antibody Affinity

In certain embodiments, antibodies of the pharmaceutical compositions and methods provided herein have a dissociation constant (K_{D}) of ≤ 1 µM, ≤ 100 nM, ≤ 10 nM, ≤ 1 nM, ≤ 0.1 nM, ≤ 0.01 nM, or ≤ 0.001 nM (e.g., 10⁻⁸ M or less, e.g., from 10⁻⁸ M to 10⁻¹³ M, e.g., from 10⁻⁹ M to 10⁻¹³ M), e.g., with respect to an anti-CD3 arm or an anti-target arm (e.g., an anti-CD20 arm, an anti-FcRH5 arm, or an anti-HER2 arm).

In one embodiment, K_{D} is measured by a radiolabeled antigen binding assay (RIA). In one embodiment, an RIA is performed with the Fab version of an antibody of interest and its antigen. For example, solution binding affinity of Fabs for antigen is measured by equilibrating Fab with a minimal concentration of (¹²⁵I)-labeled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER^{®} multi-well plates (Thermo Scientific) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23 °C). In a non-adsorbent plate (Nunc #269620), 100 pM or 26 pM [¹²⁵I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN^{®} 20) in PBS. When the plates have dried, 150 µL/well of scintillant (MICROSCINT-20^{™}; Packard) is added, and the plates are counted on a TOPCOUNT^{™} gamma counter (Packard) for ten minutes. Concentrations of each Fab that give less than or equal to 20% of maximal binding are chosen for use in competitive binding assays.

According to another embodiment, K_{D} is measured using a BIACORE^{®} surface plasmon resonance assay. For example, an assay using a BIACORE^{®}-2000 or a BIACORE^{®}-3000 (BIACORE^{®}, Inc., Piscataway, NJ) is performed at 25 °C with immobilized antigen CM5 chips at ~10 response units (RU). In one embodiment, carboxymethylated dextran biosensor chips (CM5, BIACORE^{®}, Inc.) arc activated with *N*-ethyl-*N*'- (3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (~0.2 µM) before injection at a flow rate of 5 µL/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN^{®} 20) surfactant (PBST) at 25 °C at a flow rate of approximately 25 µL/min. Association rates (kₒₙ) and dissociation rates (k_{off}) are calculated using a simple one-to-one Langmuir binding model (BIACORE^{®} Evaluation Software version 3.2) by simultaneously fitting the association and dissociation sensorgrams. The equilibrium dissociation constant (K_{D}) is calculated as the ratio k_{off}/kₒₙ. See, for example, Chen et al., J. Mol. Biol. 293:865-881 (1999). If the on-rate exceeds 10⁶ m^{- 1}s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25 °C of a 20 nM anti-antigen antibody (Fab form) in PBS, pH 7.2, in the presence of increasing concentrations of antigen as measured in a spectrometer, such as a stop-flow equipped spectrophotometer (Aviv Instruments) or a 8000-series SLM-AMINCO^{™} spectrophotometer (ThermoSpectronic) with a stirred cuvette.

### 2. Antibody Fragments

In certain embodiments, a therapeutic protein is an antibody fragment, e.g., an antibody fragment that binds to CD3 and CD20. Antibody fragments include Fab, Fab', Fab'-SH, F(ab')₂, Fv, and scFv fragments, and other fragments described below. For a review of certain antibody fragments, see Hudson et al. Nat. Med. 9:129-134 (2003). For a review of scFv fragments, see, e.g., Pluckthün, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and F(ab')₂ fragments comprising salvage receptor binding epitope residues and having increased *in vivo* half-life, see U.S. Patent No. 5,869,046.

Diabodies are antibody fragments with two antigen-binding sites that may be bivalent or bispecific. See, for example, EP 404,097; WO 1993/01161; Hudson et al. Nat. Med. 9:129-134 (2003); and Hollinger et al. Proc. Natl. Acad. Sci. USA 90: 6444-6448 (1993). Triabodies and tetrabodies are also described in Hudson et al. Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see, e.g., U.S. Patent No. 6,248,516).

Antibody fragments can be made by various techniques, including but not limited to proteolytic digestion of an intact antibody as well as production by recombinant host cells (e.g., *E. coli* or phage), as described herein.

### 3. Chimeric and Humanized Antibodies

In certain embodiments, the therapeutic protein is a chimeric antibody. Certain chimeric antibodies are described, e.g., in U.S. Patent No. 4,816,567; and Morrison et al. Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments. some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Natl Acad. Sci. USA 86:10029-10033 (1989); U.S. Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### 4. Knob-in-Hole Antibody Engineering

Antibodies (e.g., bispecific antibodies, e.g., TDBs or TCBs) may be prepared as a full-length antibody or an antibody fragment. Techniques for making bispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). "Knob-in-hole" engineering of bispecific antibodies may be utilized to generate a first subunit of an Fc domain (e.g., the subunit of the Fc domain of a first arm) containing a knob and a second subunit of an Fc domain (e.g., the subunit of the Fc domain of a second arm( containing the hole into which the knob of the first arm may bind. The knob of the bispecific antibodies may be on one subunit of the Fc domain (e.g., the Fc subunit of the anti-CD3 arm) in one embodiment. Alternatively, the knob of the bispecific antibodies of the disclosure may on the other subunit of the Fc domain (e.g., the Fc subunit of the anti-target arm; e.g., of the anti-CD20, anti-FcRH5, or anti-HER2 arm). The hole of the bispecific antibodies of the disclosure may be on the anti-CD3 arm in one embodiment. Alternatively, the hole of the bispecific antibodies of the disclosure may be on the anti-target arm (e.g., the anti-CD20, anti-FcRH5, or anti-HER2 arm). In some instances, the anti-CD20/anti-CD3 bispecific antibody produced using knob-in-hole technology may comprise one or more heavy chain constant domains, wherein the one or more heavy chain constant domains are selected from a first CH1 (CH1*₁*) domain, a first CH2 (CH2*₁*) domain, a first CH3 (CH3*₁*) domain, a second CH1 (CH1*₂*) domain, second CH2 (CH2*₂*) domain, and a second CH3 (CH3*₂*) domain. In some instances, at least one of the one or more heavy chain constant domains is paired with another heavy chain constant domain. In some instances, the CH3*₁* and CH3*₂* domains each comprise a protuberance or cavity, and wherein the protuberance or cavity in the CH3*₁* domain is positionable in the cavity or protuberance, respectively, in the CH3*₂* domain. In some instances, the CH3*₁* and CH3*₂* domains meet at an interface between the protuberance and cavity. In some instances, the CH2*₁* and CH2₂ domains each comprise a protuberance or cavity, and wherein the protuberance or cavity in the CH2₁ domain is positionable in the cavity or protuberance, respectively, in the CH2*₂* domain. In some instances, the CH2*₁* and CH2*₂* domains meet at an interface between said protuberance and cavity.

Bispecific antibodies may also be engineered using immunoglobulin crossover (also known as Fab domain exchange or CrossMab format) technology (see e.g., WO 2009/080253; Schaefer et al., Proc. Natl. Acad. Sci. USA, 108:11187-11192 (2011)). Bispecific antibodies may also be made by engineering electrostatic steering effects for making antibody Fc-heterodimeric molecules (WO 2009/089004); cross-linking two or more antibodies or fragments (see, e.g., U.S. Patent No. 4,676,980, and Brennan et al., Science, 229: 81 (1985)); using leucine zippers to produce bispecific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (scFv) dimers (see, e.g., Gruber et al., J. Immunol., 152:5368 (1994)); and preparing trispecific antibodies as described, e.g., in Tutt et al. J. Immunol. 147: 60 (1991).

Bispecific antibodies, or antibody fragments thereof, may also include a "Dual Action Fab" or "DAF" (see, e.g., U.S. Pub. No. 2008/0069820, Bostrom et al., 2009, Science 323:1610-14) comprising an antigen binding site that binds to CD3 as well as a target antigen, such as CD20, FcRH5, or HER2.

### 5. Antibody Variants

In some instances, amino acid sequence variants of the antibodies described above are envisioned. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, for example, antigen-binding.

### (i) Substitution, Insertion, and Deletion Variants

In certain embodiments, antibody variants having one or more amino acid substitutions are provided. Sites of interest for substitutional mutagenesis include the HVRs and framework regions (FRs). Conservative substitutions are shown in Table 5 under the heading of "preferred substitutions." More substantial changes are provided in Table 5 under the heading of "exemplary substitutions," and as further described below in reference to amino acid side chain classes. Amino acid substitutions may be introduced into an antibody of interest and the products screened for a desired activity, for example, retained/improved antigen binding, decreased immunogenicity, or improved ADCC or CDC.

**TABLE 5. Exemplary and preferred amino acid substitutions**

| **Original Residue** | **Exemplary Substitutions** | **Preferred Substitutions** |
|---|---|---|
| Ala (A) | Val; Leu; Ile | Val |
| Arg (R) | Lys; Gin; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gin; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region (HVR) residues of a parent antibody (e.g., a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g., binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, e.g., in Hoogenboom et al. in Methods in Molecular Biology 178:1-37 (O'Brion et al., ed., Human Press, Totowa, NJ, (2001)). In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as Arg, Asp, His, Lys, and Glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigenantibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g., for ADEPT) or a polypeptide which increases the serum half-life of the antibody.

### (ii) Glycosylation variants

In some instances, antibodies of the pharmaceutical compositions provided herein have been modified to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. **In** some embodiments, modifications of the oligosaccharide in an antibody of the disclosure may be made in order to create antibody variants with certain improved properties.

In some instances, antibodies of the pharmaceutical compositions have a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn297 (e. g. complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., U.S. Pat. Pub. Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO 2005/053742; WO 2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); U.S. Pat. Pub. No. US 2003/0157108, Presta, L; and PCT Pub. No. WO 2004/056312, Adams et al.*,* especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO 2003/085107).

In view of the above, in some instances, antibodies useful in the present disclosure include an aglycosylation site mutation. In some instances, the aglycosylation site mutation reduces effector function of the bispecific antibody. In some instances, the aglycosylation site mutation is a substitution mutation. In some instances, the bispecific antibody comprises a substitution mutation in the Fc region that reduces effector function. In some instances, the substitution mutation is at amino acid residue N297, L234, L235, D265, and/or P329 (EU numbering). In some instances, the substitution mutation is selected from the group consisting of N297G, N297A, L234A, L235A, D265A, and P329G. In some instances, the substitution mutation is at amino acid residue N297. In a preferred embodiment, the substitution mutation is N297A.

In other instances, antibody variants with bisected oligosaccharides are used in accordance with the methods of the disclosure, for example, in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); U.S. Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

### (iii) Fc region variants

In some instances, an antibody variant that has one or more amino acid modifications introduced into the Fc region (i.e., an Fc region variant (see e.g., U.S. 2012/0251531)) of the antibody (e.g., bispecific antibody, e.g., TDB or a TCB) may be administered to a subject having cancer (e.g., a B cell proliferation disorder) in accordance with the methods of the disclosure. The Fc region variant may comprise a human Fc region sequence (e.g., a human IgG₁, IgG₂, IgG₃ or IgG₄ Fc region) comprising an amino acid modification (e.g., a substitution) at one or more amino acid positions.

In some instances, the bispecific Fc region antibody variant possesses some but not all effector functions, which makes it a desirable candidate for applications in which the half-life of the antibody in vivo is important yet certain effector functions (such as complement and ADCC) are unnecessary or deleterious. In vitro and/or in vivo cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, e.g., Hellstrom, I. et al. Proc. Natl Acad. Sci. USA 83:7059-7063 (1986)) and Hellstrom, I et al., Proc. Natl Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI^{™} non-radioactive cytotoxicity assay for flow cytometry (CellTechnology, Inc. Mountain View, CA; and CytoTox 96^{®} non-radioactive cytotoxicity assay (PROMEGA^{®}, Madison, WI). Useful effector cells for such assays include peripheral blood mononuclear cells (PBMC) and Natural Killer (NK) cells. Alternatively, or additionally, ADCC activity of the molecule of interest may be assessed in vivo, e.g., in a animal model such as that disclosed in Clynes et al. Proc. Natl Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al. J. Immunol. Methods 202:163 (1996); Cragg, M.S. et al. Blood. 101:1045-1052 (2003); and Cragg, M.S. and M.J. Glennie Blood. 103:2738-2743 (2004)). FcRn binding and *in vivo* clearance/half-life determinations can also be performed using methods known in the art (see, e.g., Petkova, S.B. et al. Int'l. Immunol. 18(12):1759-1769 (2006)).

Antibodies with reduced effector function include those with substitution of one or more of Fc region residues 238, 265, 269, 270, 297, 327 and 329 (U.S. Patent Nos. 6,737,056 and 8,219,149). Such Fc mutants include Fc mutants with substitutions at two or more of amino acid positions 265, 269, 270, 297 and 327, including the so-called "DANA" Fc mutant with substitution of residues 265 and 297 to alanine (U.S. Patent Nos. 7,332,581 and 8,219,149).

In certain instances, the proline at position 329 of a wild-type human Fc region in the antibody is substituted with glycine or arginine or an amino acid residue large enough to destroy the proline sandwich within the Fc/Fcγ receptor interface that is formed between the proline 329 of the Fc and tryptophan residues Trp 87 and Trp 110 of FcγRIII (Sondermann et al. Nature. 406, 267-273 (2000)). In certain embodiments, the bispecific antibody comprises at least one further amino acid substitution. In one embodiment, the further amino acid substitution is S228P, E233P, L234A, L235A, L235E, N297A, N297D, or P331S, and still in another embodiment the at least one further amino acid substitution is L234A and L235A of the human IgG1 Fc region or S228P and L235E of the human IgG4 Fc region (see e.g., US 2012/0251531), and still in another embodiment the at least one further amino acid substitution is L234A and L235A and P329G of the human IgG1 Fc region.

Certain antibody variants with improved or diminished binding to FcRs are described. (See, e.g., U.S. Patent No. 6,737,056; WO 2004/056312, and Shields et al., J. Biol. Chem. 9(2): 6591-6604 (2001).)

In certain instances, the therapeutic protein is an antibody having an Fc region with one or more amino acid substitutions which improve ADCC, e.g., substitutions at positions 298, 333, and/or 334 of the Fc region (EU numbering of residues).

In some instances, alterations are made in the Fc region that result in altered (i.e., either improved or diminished) C1q binding and/or Complement Dependent Cytotoxicity (CDC), e.g., as described in U.S. Patent No. 6,194,551, WO 99/51642, and Idusogie et al. J. Immunol. 164: 4178-4184 (2000).

Antibodies with increased half-lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US 2005/0014934 (Hinton et al.). Those antibodies comprise an Fc region with one or more substitutions therein which improve binding of the Fc region to FcRn. Such Fc variants include those with substitutions at one or more of Fc region residucs: 238, 256, 265, 272, 286, 303, 305, 307, 311, 312, 317, 340, 356, 360, 362, 376, 378, 380, 382, 413, 424 or 434, e.g., substitution of Fc region residue 434 (U.S. Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

### (iv) Cysteine engineered antibody variants

In certain embodiments, it may be desirable to create cysteine engineered antibodies, e.g., "thioMAbs," in which one or more residues of the bispecific antibody are substituted with cysteine residues. In a particular embodiment, the substituted residues occur at accessible sites of the antibody. By substituting those residues with cysteine, reactive thiol groups are thereby positioned at accessible sites of the bispecific antibody and may be used to conjugate the antibody to other moieties, such as drug moieties or linker-drug moieties, to create an immunoconjugate. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, for example, in U.S. Patent No. 7,521,541.

Therefore, immunoconjugates of an antibody conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes, are specifically contemplated.

In some instances, an immunoconjugate is an antibody-drug conjugate (ADC) in which an bispecific antibody is conjugated to one or more drugs, including but not limited to a maytansinoid (see U.S. Patent Nos. 5,208,020, 5,416,064 and European Patent EP 0 425 235 B1); an auristatin such as monomethylauristatin drug moieties DE and DF (MMAE and MMAF) (see U.S. Patent Nos. 5,635,483 and 5,780,588, and 7,498,298); a dolastatin; a calicheamicin or derivative thereof (see U.S. Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin (see Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002); King et al., J. Med. Chem. 45:4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

In some instances, an immunoconjugate comprises the antibody (e.g., bispecific antibody) conjugated to an enzymatically active toxin or fragment thereof, including but not limited to diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), *Momordica charantia* inhibitor, curcin, crotin, *Sapaonaria officinalis* inhibitor, gelonin, mitogellin, restrictocin, phenomycin, enomycin, and the tricothecenes.

In another embodiment, an immunoconjugate comprises an antibody (e.g., a bispecific antibody, e.g., a TDB or a TCB) conjugated to a radioactive atom to form a radioconjugate. A variety of radioactive isotopes are available for the production of radioconjugates. Examples include ²¹¹AT, ¹³¹I, ¹²⁵I, ⁹⁰Y, ¹⁸⁶Re, 188Re, 153Sm, ²¹²Bi, ³²P, ²¹²Pb and radioactive isotopes of Lu. When the radioconjugate is used for detection, it may comprise a radioactive atom for scintigraphic studies, for example ^{99m}Tc or ¹²³I, or a spin label for nuclear magnetic resonance (NMR) imaging (also known as magnetic resonance imaging, MRI), such as ¹²³I again, ¹³¹I, ¹¹¹In, ¹⁹F, ¹³C, ¹⁵N, ¹⁷O, gadolinium, manganese or iron.

Conjugates of the antibody (e.g., bispecific antibody, e.g., TDB or a TCB) and a cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). ¹⁴C-labeled 1-isothiocyanatobenzyl-3-methyldiethylene triaminepentaacetic acid (MX-DTPA) is an exemplary chelating agent for conjugation of radionucleotide to the antibody. See WO 94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari et al., Cancer Res. 52:127-131 (1992); U.S. Patent No. 5,208,020) may be used.

The immunoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate), which are commercially available (e.g., from Pierce Biotechnology, Inc., Rockford, IL., U.S.A.).

### (v) Other antibody derivatives

In some instances, a therapeutic protein can be an antibody that has been modified to contain additional nonproteinaceous moieties that are known in the art and readily available and administered to the subject in accordance with the methods described herein. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, polypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, they can be the same or different molecules. **In** general, the number and/or type of polymers used for derivatization can be determined based on considerations including, but not limited to, the particular properties or functions of the antibody to be improved, whether the antibody derivative will be used in a therapy under defined conditions, etc.

In some instances, conjugates of an antibody and nonproteinaceous moiety that may be selectively heated by exposure to radiation are provided. **In** one instance, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibodynonproteinaceous moiety are killed.

### V. Recombinant Methods and Compositions

Therapeutic proteins (e.g., antibodies; e.g., bispecific antibodies; e.g., anti-CD3 bispecific antibodies; e.g., TDBs or TCBs; e.g., mosunetuzumab, glofitamab, cevostamab, or runimotamab) useful in the pharmaceutical compositions and methods provided herein may be produced using recombinant methods and compositions, for example, as described in U.S. Patent No. 4,816,567. In one embodiment, isolated nucleic acids encoding therapeutic protein (e.g., antibody; e.g., bispecific antibody; e.g., anti-CD3 bispecific antibodies; e.g., TDBs or TCBs; e.g., mosunetuzumab, glofitamab, cevostamab, or runimotamab) described herein are provided. In embodiments in which the therapeutic protein is an antibody, such nucleic acid may encode an amino acid sequence comprising the VL(s) and/or an amino acid sequence comprising the VH(s) of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL(s) of the antibody and an amino acid sequence comprising the VH(s) of the antibody, or (2) a first vector comprising a nucleic acid that encodes an amino acid sequence comprising the VL(s) of the antibody and a second vector comprising a nucleic acid that encodes an amino acid sequence comprising the VH(s) of the antibody. In one embodiment, the host cell is eukaryotic, e.g., a Chinese Hamster Ovary (CHO) cell or lymphoid cell (e.g., Y0, NS0, or Sp2/0 cell). Antibodies can be made by culturing a host cell comprising a nucleic acid encoding the antibody, as provided above, under conditions suitable for expression of the antibody, and optionally recovering the antibody from the host cell (or host cell culture medium).

For recombinant production of a therapeutic protein (e.g., antibodies; e.g.. bispecific antibodies; e.g., anti-CD3 bispecific antibody; e.g., TDB or TCB; e.g., mosunetuzumab, glofitamab, cevostamab, or runimotamab), nucleic acid encoding the therapeutic protein, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of an antibody).

Suitable host cells for cloning or expression of therapeutic protein-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. See also Charlton, Methods in Molecular Biology, Vol. 248 (B.K.C. Lo, ed., Humana Press, Totowa, NJ, 2003), pp. 245-254, describing expression of antibody fragments in *E. coli.* After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. See, e.g., U.S. Patent Nos. 5,959,177, 6,040,498, 6,420,548, 7,125,978, and 6,417,429 (describing PLANTIBODIES^{™} technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293T cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

### VI. Assays

Therapeutic proteins (e.g., antibodies; e.g., bispecific antibodies; e.g., anti-CD3 bispecific antibodies; e.g., TDBs or TCBs; e.g., mosunetuzumab, glofitamab, cevostamab, or runimotamab) of the pharmaceutical compositions of the disclosure may be identified, screened for, or characterized for their physical/chemical properties and/or biological activities by various assays known in the art and described herein.

### (i) Binding assays

In one aspect, a therapeutic protein (e.g., antibody; e.g., bispecific antibody; e.g., anti-CD3 bispecific antibody; e.g., TDB or TCB; e.g., mosunetuzumab, glofitamab, cevostamab, or runimotamab) of the disclosure is tested for its antigen binding activity, for example, by known methods such as ELISA, Western blot, etc.

For example, competition assays can be used to identify an antibody that competes with an antibody of the disclosure for binding to its antigen. In an exemplary competition assay configured to characterize binding to CD3, immobilized CD3 is incubated in a solution comprising a first labeled antibody that binds to CD3 and a second unlabeled antibody that is being tested for its ability to compete with the first antibody for binding to CD3. The second antibody may be present in a hybridoma supernatant. As a control, immobilized CD3 is incubated in a solution comprising the first labeled antibody but not the second unlabeled antibody. After incubation under conditions permissive for binding of the first antibody to CD3, excess unbound antibody is removed, and the amount of label associated with immobilized CD3 is measured. If the amount of label associated with immobilized CD3 is substantially reduced in the test sample relative to the control sample, then that indicates that the second antibody is competing with the first antibody for binding to CD3. See, e.g., Harlow and Lane (1988) Antibodies: A Laboratory Manual. Ch.14 (Cold Spring Harbor Laboratory, Cold Spring Harbor, NY).

### (ii) Activity assays

In one aspect, assays are provided for identifying antibodies thereof having biological activity. Biological activity may include, for example, binding to an antigen, such as CD3 (e.g., CD3 on the surface of a T cell), or a peptide fragment thereof, either *in vivo, in vitro,* or *ex vivo.* In the case of a bispecific antibody (e.g., a TDB having one anti-CD3 arm and one anti-target arm that recognizes a second biological molecule, e.g., a cell surface antigen, e.g., a tumor antigen), biological activity may also include, for example, effector cell activation (e.g., T cell (c.g., CD8+ and/or CD4+ T cell) activation), effector cell population expansion (i.e., an increase in T cell count), target cell population reduction (i.e., a decrease in the population of cells expressing the second biological molecule on their cell surfaces), and/or target cell killing. Antibodies having such biological activity in vivo and/or in vitro are provided.

### Activity Assay for Killing of B Cells

In some embodiments, antibody activity includes the ability to support B cell killing and/or the activation of the cytotoxic T cells. In certain embodiments, an anti-B cell targeting anti-CD3 antibody of the disclosure (e.g., an anti-CD20/CD3 bispecific antibody or an anti-FcRH5/anti-CD3 bispecific antibody;, e.g., an anti-CD20/anti-CD3 TDB or TCB or an anti-FcRH5 TDB; e.g., mosunetuzumab, glofitamab, or cevostamab) is tested for such B cell killing and/or the activation of the cytotoxic effect of T cells biological activity by any of the methods described herein. In some embodiments of any of these activity assays, PBMCs may be isolated from whole blood of healthy donors by FICOLL^{®} separation. In particular, human blood may be collected in heparinized syringes, and PBMCs isolated using Leucosep and FICOLL-PAQUE^{®} Plus. If needed CD4+ T and CD8+ T cells may be separated with MILTENYI^{®} kits according to manufacturer's instructions.

Cells may be washed in RPMI medium containing 10% FBS, supplemented with GLUTAMAX^{®}, penicillin & streptomycin, and ~0.2 million suspended cells added to a 96-well U-bottom plate. Cells may be cultured in RPMI1640 supplemented with 10% FBS at 37 °C in a humidified standard cell culture incubator. For BJAB cell killing assays, 20,000 BJAB cells may be incubated with effector cells, either as huPBMCs or purified T cells, as indicated ratios per assay, in the presence of various concentrations of TDB antibodies for 24 hours. For endogenous B cell killing assays, 200,000 huPBMCs may be incubated with various concentrations of TDB antibodies for 24 hours.

After culturing, cells can be washed with FACS buffer (0.5% BSA, 0.05% Na azide in PBS). Cells may then be stained in FACS buffer, washed with FACS buffer and suspended in 100µl of FACS buffer containing 1µg/ml propidium iodide. Data may be collected on a FACSCalibur flow cytometer and analyzed using FLOWJO^{®}. Live B cells may be gated out as PI-CD19+ or PI-CD20+ B cells by FACS, and absolute cell count may be obtained with FITC beads added to reaction mix as an internal counting control. The percent (%) of cell killing may be calculated based on non-TDB treated controls. Activated T cells may be detected by CD69 and CD25 surface expression using anti-CD69-FITC and anti-CD25-PE.

### Activity Assay for Killing of FcRH5-Expressing Cells

In some embodiments, antibody activity includes the ability to support killing of FcRH5-expressing MOLP-2 target cells and the activation of the cytotoxic effect of T cells. In some embodiments, *in vitro* cytotoxicity may be monitored by flow cytometry. Target cells may be labeled with CFSE according to manufacturer's protocol (Invitrogen, #C34554). The carboxyfluorescein succinimidyl ester (CFSE)-labeled target cells and purified CD8+ T cells from human PBMC may be mixed in a 3:1 ratio, with or without the bispecific antibody (e.g., anti-FcRH5/anti-CD3 bispecific antibody; e.g., anti-FcRH5/antiCD-3 TDB; e.g., cevostamab) for 48 hours. The cells may be resuspended in equal volume of PBS + 2% FBS + 1 mM EDTA + propidium iodine (PI). Flow cytometry analysis may be done on a FACSCalibur in automation format. The number of live target cells can be counted by gating on CFSE+/PI negative cells. The percentage of cytotoxicity can be calculated as follows: % cytotoxicity (live target cell number w/o bispecific antibody - live target cell number w/ bispecific antibody) / (live target cell number w/o bispecific antibody) x 100.

Activated T cells may be detected by staining with CD8-FITC (BD Bioscience, 555634), CD69-PE (BD Bioscience, 555531), and CD107a-Alexa-Fluor647 (eBioscience, 51-1079). Optionally, activated T cells may be detected by staining with CD8-FITC and CD69-PE. After staining, cells can be fixed and permeabilized with CYTOFlX/CYTOPERM^{®} solution (BD Bioscience, 554722) and intracellular stained with anti-granzyme B-Alexa-Fluor647 (BD Bioscience, 560212). T cell activation can be evaluated by the percentage of CD8+CD69+, CD8+CD107a+, and CD8+CD69+GranzymeB+ cells.

### Activity Assay for Killing of HER2-Expressing Cells

In some embodiments, antibody activity includes the ability to support killing of HER2-expressing SKBR3 target cells and the activation of the cytotoxic effect of T cells. *In vitro* cytotoxicity was monitored by flow cytometry. Target cells were labeled with carboxyfluorescein succinimidyl ester (CFSE) according to manufacturer's protocol (Invitrogen, #C34554). The CFSE -labeled target cells and purified CD8+ T cells from human PBMC were mixed in certain E:T ratio (as indicated in figure legends), with or without TDB for 24 hours. At the end of the incubation, the cells were lifted by trypsin and collected from the plate. The cells were resuspended in equal volume of PBS + 2% FBS + 1 mM EDTA + propidium iodine (PI). Flow cytometry analysis was done on a FACSCalibur in automation format. The number of live target cells was counted by gating on CFSE+/PI negative cells. The percentage of cytotoxicity was calculated as follows: % cytotoxicity (live target cell number w/o TDB - live target cell number w/TDB) / (live target cell number w/o TDB) x 100.

Activated T cells may be detected by staining with CD8-FITC (BD Bioscience, 555634), CD69-PE (BD Bioscience, 555531), and CD107a-Alexa-Fluor647 (eBioscience, 51-1079). Optionally, activated T cells may be detected by staining with CD8-FITC and CD69-PE. After staining, cells can be fixed and permeabilized with CYTOFlX/CYTOPERM^{®} solution (BD Bioscience, 554722) and intracellular stained with anti-granzyme B-Alexa-Fluor647 (BD Bioscience, 560212). T cell activation can be evaluated by the percentage of CD8+CD107a+ cells.

### 3. Stability assays

Suitable assays for determining the stability of a pharmaceutical composition (e.g., of a therapeutic protein, such as an antibody, e.g., a bispecific antibody; e.g., an anti-CD3 bispecific antibody; e.g., a TDB or a TCB) are known in the art and are described herein. For example, a pharmaceutical composition can be evaluated qualitatively and/or quantitatively in a variety of different ways, including evaluation of aggregate formation (for example, using size exclusion chromatography, by measuring turbidity, and/or by visual inspection); evaluation of reactive oxygen species (ROS) formation (for example, by using a light stress assay or an 2,2'-azobis(2-amidinopropane) dihydrochloride (AAPH) stress assay); oxidation of specific amino acid residues of the protein (for example, a Met residue of an antibody); by assessing charge heterogeneity using cation exchange chromatography, image capillary isoelectric focusing (iclEF) or capillary zone electrophoresis; amino-terminal or carboxy-terminal sequence analysis; mass spectrometric analysis; SDS-PAGE analysis to compare reduced and intact polypeptides (e.g., antibodies); peptide map (for example, tryptic or LYS-C) analysis; evaluating biological activity or target binding function of the protein (e.g., binding of an antibody to its antigen); and the like. Instability may involve any one or more of: aggregation, deamidation (e.g., Asn deamidation), oxidation (e.g., Met oxidation), isomerization (e.g., Asp isomerization), clipping/hydrolysis/fragmentation (e.g., hinge region fragmentation), succinimide formation, unpaired cysteine(s), N-terminal extension, C-terminal processing, glycosylation differences, and the like. Exemplary assays are described in the Examples below.

### VII. Therapeutic Methods and Uses

The pharmaceutical compositions described herein can be formulated for use as a medicament for treating various diseases and disorders. Thus, the disclosure features methods involving administration of the pharmaceutical composition to a subject in need thereof, e.g., a subject having a disease or disorder, such as cancer. A pharmaceutical composition of the present disclosure may be used to treat or delay progression of a cell proliferative disorder in a subject in need thereof (e.g., a human subject in need thereof) or to enhance immune function in a subject having a cell proliferative disorder (e.g., cancer).

In one aspect, the disclosure provides a pharmaceutical composition as described herein for use in treating or delaying progression of a cell proliferative disorder. In one aspect, the disclosure provides the use of a pharmaceutical composition as described herein in the manufacture of a medicament for treating or delaying progression of a cell proliferative disorder. In one aspect, the disclosure provides a method of treating or delaying progression of a cell proliferative disorder in a subject in need thereof, comprising administering to the subject a pharmaceutical composition as described herein.

In some embodiments, the therapeutic protein is a bispecific antibody formulated to bind to a CD3 molecule located on an immune effector cell and a target molecule located on a target cell other than the immune effector cell (e.g., a CD20 molecule, an FcRH5 molecule, or a HER2 located on (e.g., expressed by) a target cell, such as a B cell or a tumor cell (e.g., a HER2-expressing tumor cell)). In some embodiments, the bispecific antibody activates the immune effector cell following binding to the CD3 molecule and to the target molecule. Upon activation, the immune effector cell can exert a cytotoxic effect and/or an apoptotic effect on the target cell.

In some embodiments, e.g., in which the therapeutic protein is an anti-CD20/anti-CD3 bispecific antibody (e.g., anti-CD20/anti-CD3 TDB or TCB; e.g., mosunetuzumab or glofitamab), the cell proliferative disorder is a cancer that is a non-Hodgkin's lymphoma (NHL). In some embodiments, the NHL is selected from the group consisting of chronic lymphoid leukemia (CLL), B cell lymphoma, splenic diffuse red pulp small B cell lymphoma, B cell lymphoma with features intermediate between diffuse large B-cell lymphoma and Burkitt lymphoma, Burkitt-like lymphoma with 11q aberration, B cell lymphoma with features intermediate between diffuse large B-cell lymphoma and classical Hodgkin lymphoma, germinal center B cell-like (GCB) diffuse large B cell lymphoma (DLBCL), activated B cell-like (ABC) DLBCL, primary cutaneous follicle center lymphoma, T-cell/histiocyte-rich large B-cell lymphoma, primary DLBCL of the central nervous system, primary cutaneous DLBCL (leg type). Epstein-Barr virus (EBV)-positive DLBCL of the elderly, DLBCL associated with chronic inflammation, primary mediastinal (thymic) large B cell lymphoma, intravascular large B cell lymphoma, ALK-positive large B-cell lymphoma, large B-cell lymphoma arising in HHV8-associated multicentric Castleman disease, B cell leukemia, follicular lymphoma (FL), *in situ* follicular neoplasia, mantle cell lymphoma (MCL), *in situ* mantle cell neoplasia, acute myeloid leukemia (AML), marginal zone lymphoma (MZL), small lymphocytic leukemia (SLL), lymphoplasmacytic lymphoma (LL), Waldenstrom macroglobulinemia (WM), central nervous system lymphoma (CNSL), Burkitt's lymphoma (BL), B cell prolymphocytic leukemia, splenic marginal zone lymphoma, hairy cell leukemia, splenic lymphomalleukemia, hairy cell leukemia variant, α heavy chain disease, γ heavy chain disease, µ heavy chain disease, plasma cell myeloma, solitary plasmacytoma of bone, extraosseous plasmacytoma, extranodal marginal zone lymphoma of mucosa-associated lymphoid tissue (MALT lymphoma), nodal marginal zone lymphoma, pediatric nodal marginal zone lymphoma, pediatric follicular lymphoma, lymphomatoid granulomatosis, plasmablastic lymphoma, and primary effusion lymphoma. In a particular embodiment, the cancer is germinal center B cell-like (GCB) DLBCL, activated B-cell-like (ABC) DLBCL, follicular lymphoma (FL), mantle cell lymphoma (MCL), acute myeloid leukemia (AML), chronic lymphoid leukemia (CLL), marginal zone lymphoma (MZL), small lymphocytic leukemia (SLL), lymphoplasmacytic lymphoma (LL), Waldenstrom macroglobulinemia (WM), central nervous system lymphoma (CNSL), or Burkitt's lymphoma (BL).

In one embodiment the NHL is a diffuse large B cell lymphoma (DLBCL), a high-grade B cell lymphoma (HGBCL), a DLBCL arising from follicular lymphoma (FL) [transformed FL; trFL], a primary mediastinal large B-cell lymphoma (PMBCL), or marginal zone lymphoma (MZL). MZL can be categorized as splenic, nodal and extra-nodal MZL. In one embodiment the NHL is a mantle cell lymphoma (MCL). In one embodiment, the NHL is a Grades 1-3a Follicular Lymphoma (FL). In one embodiment, the CD20-positive B cell proliferative disorder is a relapsed or refractory B cell proliferative disorder . In one embodiment, the relapsed or refractory B cell proliferative disorder is relapsed or refractory NHL (e.g., a relapsed or refractory DLBCL, a relapsed or refractory FL, or a relapsed or refractory MCL).

In some embodiments, e.g., when the therapeutic is an anti-FcRH5/anti-CD3 bispecific antibody (e.g., anti-FcRH5/anti-CD3 TDB; e.g., cevostamab), the cell proliferative disorder is a cancer that is a multiple myeloma (MM), chronic lymphoid leukemia (CLL), mantle cell lymphoma (MCL), diffuse large B-cell lymphoma (DLBCL), follicular lymphoma (FL), acute myeloid leukemia (AML), myelodysplastic syndrome (MDS), chronic myelogenous leukemia (CML), chronic myelomonocytic leukemia, acute promyelocytic leukemia (APL), chronic myeloproliferative disorder, thrombocytic leukemia, precursor B-cell acute lymphoblastic leukemia (pre-B-ALL), precursor T cell acute lymphoblastic leukemia (pre-TALL), mast cell disease, mast cell leukemia, mast cell sarcoma, myeloid sarcomas, lymphoid leukemia, or undifferentiated leukemia. In a particular embodiment, the cancer is a multiple myeloma (MM), which may be relapsed or refractory (R/R) MM. In some embodiments, the cancer is a FcRH5-positive cancer.

In some embodiments, the cancer is selected from the group consisting of breast cancer, colorectal cancer, gastric cancer, non-small cell lung cancer (NSCLC), multiple myeloma, renal cancer, prostate cancer, liver cancer, head and neck cancer, melanoma, ovarian cancer, mesothelioma, and glioblastoma. In some embodiments, the cancer is a HER2-positive cancer.

In some embodiments, e.g., when the therapeutic protein is an anti-HER2/anti-CD3 bispecific antibody (e.g., anti-HER2/anti-CD3 TDB; e.g., runimotamab) the cancer is a HER2-positive cancer (e.g., a HER2-positive breast cancer or a HER2-positive gastric cancer). In a particular embodiment, the HER2-positive cancer is a HER2-positive breast cancer or HER2-positive gastric cancer. The HER2-positive cancer (e.g., the HER2-positive breast cancer or the HER2-positive gastric cancer) may be characterized by tumor cells that express HER2 at a copy number (e.g., an average copy number) of at least 200,000 per cell (e.g., at least 250,000 HER2 copies per cell, at least 300,000 HER2 copies per cell, at least 400,000 HER2 copies per cell, at least 500,000 HER2 copies per cell, at least 600,000 HER2 copies per cell, at least 700,000 HER2 copies per cell, at least 750,000 HER2 copies per cell, at least 800,000 HER2 copies per cell, at least 900,000 HER2 copies per cell, at least 1,000,000 HER2 copies per cell, at least 1,200,000 HER2 copies per cell, at least 1,500,000 HER2 copies per cell, at least 2,000,000 HER2 copies per cell, at least 2,500,000 HER2 copies per cell, at least 3,000,000 HER2 copies per cell, or more, e.g., from 200,000 to 2,000,000 HER2 copies per cell, from 300,000 to 1,500,000 HER2 copies per cell, from 400,000 to 1,200,000 HER2 copies per cell, or from 500,000 to 1,000,000 HER2 copies per cell, e.g., from 200,000 to 1,000,000 HER2 copies per cell (e.g., from 200,000 to 250,000 HER2 copies per cell, from 250,000 to 300,000 HER2 copies per cell, from 300,000 to 400,000 HER2 copies per cell, from 400,000 to 500,000 HER2 copies per cell, from 500,000 to 750,000 HER2 copies per cell, or from 750,000 to 1,000,000 HER2 copies per cell) or from 1,000,000 to 3,000,000 HER2 copies per cell (e.g., from 1,000,000 to 1,500,000 HER2 copies per cell, from 1,500,000 to 2,000,000 HER2 copies per cell, from 2,000,000 to 2,500,000 HER2 copies per cell, or from 2,500,000 to 3,000,000 HER2 copies per cell).

The therapeutic protein (e.g., anti-CD3 bispecific antibody; e.g., TDB or TCB; e.g., mosunetuzumab, glofitamab, cevostamab, or runimotamab) can be formulated for administration to the subject at a dosage from about 10 µg to about 100 mg (e.g., from 100 µg to 80 mg, from 500 µg to 50 mg, or from 1 mg to 20 mg, e.g., from 10 µg to 50 µg, from 50 µg to 100 µg, from 100 µg to 200 µg, from 200 µg to 500 µg, from 500 µg to 1 mg, from 1 mg to 5 mg, from 5 mg to 10 mg, from 10 mg to 20 mg, to 20 mg to 30 mg, from 30 mg to 40 mg, from 40 mg to 50 mg, from 50 mg to 60 mg, from 60 mg to 70 mg, from 70 mg to 80 mg, from 80 mg to 90 mg, or from 90 to 100 mg, e.g., about 10 µg, about 20 µg about 25 µg, about 30 µg, about 40 µg, about 50 µg, about 60 µg, about 70 µg, about 75 µg, about 80 µg, about 90 µg, about 100 µg, about 200 µg, about 250 µg, about 300 µg, about 400 µg, about 500 µg, about 600 µg, about 700 µg, about 750 µg, about 800 µg, about 900 µg, about 1 mg, about 2 mg, about 2.5 mg, about 3 mg, about 4 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 25 mg, about 30 mg, about 40 mg, about 50 mg, about 60 mg, about 70 mg, about 75 mg, about 80 mg, about 90 mg, or about 100 mg).

In some embodiments, the therapeutic protein is a bispecific antibody (e.g., a TDB or TCB) formulated for administration to the subject at a dosage from about 10 µg to about 100 mg (e.g., from 100 µg to 80 mg, from 500 µg to 50 mg, or from 1 mg to 20 mg, e.g., from 10 µg to 50 µg, from 50 µg to 100 µg, from 100 µg to 200 µg, from 200 µg to 500 µg, from 500 µg to 1 mg, from 1 mg to 5 mg, from 5 mg to 10 mg, from 10 mg to 20 mg, to 20 mg to 30 mg, from 30 mg to 40 mg, from 40 mg to 50 mg, from 50 mg to 60 mg, from 60 mg to 70 mg, from 70 mg to 80 mg, from 80 mg to 90 mg, or from 90 to 100 mg, e.g., about 10 µg, about 20 µg about 25 µg, about 30 µg, about 40 µg, about 50 µg, about 60 µg, about 70 µg, about 75 µg, about 80 µg, about 90 µg, about 100 µg, about 200 µg, about 250 µg, about 300 µg, about 400 µg, about 500 µg, about 600 µg, about 700 µg, about 750 µg, about 800 µg, about 900 µg, about 1 mg, about 2 mg, about 2.5 mg, about 3 mg, about 4 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 25 mg, about 30 mg, about 40 mg, about 50 mg, about 60 mg, about 70 mg, about 75 mg, about 80 mg, about 90 mg, or about 100 mg). In a particular embodiment, the therapeutic protein is a bispecific antibody formulated for administration to the subject at a dosage from about 1 mg to about 60 mg.

In some embodiments, the pharmaceutical composition (e.g., comprising an anti-FcRH5/anti-CD3 bispecific antibody; e.g., anti-FcRH5/anti-CD3 TDB; e.g., cevostamab) is administered to the subject without dilution (e.g., at a concentration of about 1 mg/ml or about 3 ml/ml). In some embodiments, the pharmaceutical composition is administered to the subject after dilution with a saline solution. In some embodiments, the saline solution is a normal saline solution. In some embodiments, the normal saline solution comprises sodium chloride (NaCl). In some embodiments, the normal saline solution comprises between 0.1-1.5% (e.g., between 0.1-1.2%, between 0.3-1.5%, between 0.4-0.5%, between 0.3-1%, between 0.8-1 %, between 0.85-0.95%; e.g., about 0.1%, about 0.3%, about 0.4%, about 0.45%, about 0.5%, about 0.6%, about 0.7%, about 0.8%, about 0.85%, about 0.9%, about 0.95%, about 1%, or about 1.2%) (w/v) NaCl). In particular embodiments, the normal saline solution comprises 0.45% or 0.9% (w/v) NaCl.

In some embodiments, after dilution with the normal saline solution, the concentration of the therapeutic protein (e.g., antibody; e.g., bispecific antibody; e.g., TDB or TCB; e.g., mosunetuzumab, glofitamab, cevostamab, or runimotamab) is from about 0.001 mg/ml to about 0.6 mg/ml (e.g., about 0.001 mg/ml, about 0.002 mg/ml, about 0.003 mg/ml, about 0.004 mg/ml, about 0.005 mg/ml, about 0.01 mg/ml, about 0.02 mg/ml, about 0.03 mg/ml, about 0.04 mg/ml, about 0.05 mg/ml, about 0.75 mg/ml, about 0.1 mg/ml, about 0.11 mg/ml, about 0.12 mg/ml, about 0.13 mg/ml, about 0.14 mg/ml, about 0.15 mg/ml, about 0.16 mg/ml, about 0.17 mg/ml, about 0.18 mg/ml, about 0.19 mg/ml, about 0.2 mg/ml, about 0.21 mg/ml, about 0.22 mg/ml, about 0.23 mg/ml, about 0.24 mg/ml, about 0.25 mg/ml, about 0.26 mg/ml, about 0.27 mg/ml, about 0.28 mg/ml, about 0.29 mg/ml, about 0.3 mg/ml, about 0.35 mg/ml, about 0.4 mg/ml about 0.45 mg/ml, about 0.5 mg/ml, about 0.55 mg/ml, or about 0.6 mg/ml). In particular embodiments, after dilution with the normal saline solution, the concentration of therapeutic protein (e.g., antibody; e.g., bispecific antibody; e.g., TDB or TCB; e.g., mosunetuzumab, glofitamab, cevostamab, or runimotamab) is about 0.003 mg/ml, about 0.01 mg/ml, about 0.02 mg/ml, about 0.03 mg/ml, about 0.04 mg/ml, about 0.12 mg/ml, about 0.24 mg/ml, or about 0.3 mg/ml. In a particular embodiment, after dilution with the normal saline solution, the concentration of the therapeutic antibody (e.g., antibody; bispecific antibody; anti-CD20/anti-CD3 bispecific antibody; anti-CD20/anti-CD3 TDB; e.g., mosunetuzumab) is about 0.01 mg/ml, about 0.02 mg/ml, about 0.04 mg/ml, about 0.12 mg/ml, about 0.24 mg/ml or about 0.3 mg/ml. In a particular embodiment, after dilution with the normal saline solution, the concentration of the therapeutic antibody (e.g., antibody; bispecific antibody; anti-CD20/anti-CD3 bispecific antibody; anti-CD20/anti-CD3 TCB; e.g., glofitamab) is about 0.1 mg/ml or about 0.6 mg/ml. In a particular embodiment, after dilution with the normal saline solution, the concentration of the therapeutic antibody (e.g., antibody; bispecific antibody; anti-FcRH5/anti-CD3 bispecific antibody; anti-FcRH5/anti-CD3 TDB; e.g., cevostamab) is about 0.003 mg/ml, 0.03 mg/ml, or 0.3 mg/ml.

The disclosure further provides methods for co-administration of a therapeutic protein (e.g., anti-CD3 bispecific antibody; e.g., TDB or TCB; e.g., mosunetuzumab, glofitamab, cevostamab, or runimotamab) with at least one additional therapeutic agent (e.g., one, two, three, four, or more additional therapeutic agents) are also disclosed. For example, additional therapeutic agents include PD-1 axis binding antagonists, such as PD-L1 binding antagonists, PD-1 binding antagonists, and a PD-L2 binding antagonists. PD-L1 binding antagonists useful in the methods and pharmaceutical compositions include, e.g., atezolizumab (MPDL3280A), MDX-1105 (BMS-936559), and MEDI4736 (durvalumab). For example, in some particular instances, the anti-PD-L1 antibody is atezolizumab (CAS Registry Number: 1422185-06-5). MDX-1 105, also known as BMS-936559, is an anti-PD-L1 antibody described in PCT Pub. No. WO 2007/005874 and PCT Pub. No. WO 2016/201425. MEDI4736 (durvalumab) is an anti-PD-L1 monoclonal antibody described in PCT Pub. No. WO 2011/066389 and U.S. Pub. No. 2013/034559. Examples of anti-PD-L1 antibodies useful for the methods of this disclosure, and methods for making them are described in PCT Pub. Nos. WO 2010/077634, WO 2007/005874, and WO 2011/066389, and also in U.S. Patent No. 8,217,149, and U.S. Pub. No. 2013/034559, which are incorporated herein by reference.

PD-1 binding antagonists include anti-PD-1 antibodies, such as an anti-PD-1 antibody selected from the group consisting of MDX-1106 (nivolumab), MK-3475 (pembrolizumab), MEDI-0680 (AMP-514), PDR001, AMG 404, REGN2810 (cemiplimab; LIBTAYO^{®}), and BGB-108. MDX-1106, also known as MDX-1106-04, ONO-4538, BMS-936558, or nivolumab, is an anti-PD-1 antibody described in PCT Pub. No. WO 2006/121168. MK-3475, also known as pembrolizumab or lambrolizumab, is an anti-PD-1 antibody described in PCT Pub. No. WO 2009/114335. In other instances, the PD-1 binding antagonist is an immunoadhesin (e.g., an immunoadhesin comprising an extracellular or PD-1 binding portion of PD-L1 or PD-L2 fused to a constant region (e.g., an Fc region of an immunoglobulin sequence). In other instances, the PD-1 binding antagonist is REGN2810 (LIBTAYO^{®}), also known as cemiplimab. In other instances, the PD-1 binding antagonist is AMP-224, a B7-DC Fc or PD-L2 Fc fusion protein described in PCT Pub. No. WO 2017/058780.

PD-L2 binding antagonists include, e.g., antibodies (e.g., an anti-PD-L2 antibody) and immunoadhesins. In some embodiments, an additional therapeutic agent includes obinutuzumab (an anti-CD20 antibody), rituximab (an anti-CD20 antibody), an antibody-drug conjugate (ADC), a corticosteroid, or tocilizumab (an anti-IL-6R antibody).

For example, in instances involving a bispecific antibody having an anti-CD3 arm and an anti-CD20 arm, an additional therapeutic agent useful for co-administration can be an ADC, such as an anti-CD79b ADC (e.g., polatuzumab vedotin).

In instances for which the methods described herein involve a combination therapy, such as a particular combination therapy noted above, the combination therapy encompasses the co-administration the therapeutic protein with one or more additional therapeutic agents, and such co-administration may be combined administration or separate administration. Additionally, the two or more therapeutic agents may be formulated together or separately. In cases where two or more therapeutic agents are administered separately, administration of the therapeutic protein can occur prior to, simultaneously, and/or following, administration of the additional therapeutic agent or agents.

The therapeutic agents may be administered, for example, intravenously, subcutaneously, intradermally, intramuscularly, percutaneously, intraarterially, intraperitoneally, intralesionally, intracranially, intraarticularly, intraprostatically, intrapleurally, intratracheally, intranasally, intravitreally, intravaginally, intrarectally, topically, intratumorally, peritoneally, subconjunctivally, intravesicularlly, mucosally, intrapericardially, intraumbilically, intraocularly, orally, topically, locally, by inhalation, by injection, by infusion, by continuous infusion, by localized perfusion bathing target cells directly, by catheter, by lavage, in cremes, or in lipid compositions.

In one embodiment, administration of the therapeutic protein and administration of an additional therapeutic agent can occur within about one month, or within about one, two or three weeks, or within about one, two, three, four, five, or six days, of each other.

The methods described herein may result in an improved benefit-risk profile for patients having cancer being treated with a therapeutic protein (e.g., anti-CD3 bispecific antibody; e.g., TDB or TCB; e.g., mosunetuzumab, glofitamab, cevostamab, or runimotamab). In some instances, treatment using the methods described herein that result in administering the therapeutic protein in the context of a fractionated, dose-escalation dosing regimen may result in a reduction (e.g., by 20% or greater, 25% or greater, 30% or greater, 35% or greater, 40% or greater, 45% or greater, 50% or greater, 55% or greater, 60% or greater, 65% or greater, 70% or greater, 75% or greater, 80% or greater, 85% or greater, 90% or greater, 95% or greater, 96% or greater, 97% or greater, 98% or greater, or 99% or greater) or complete inhibition (100% reduction) of undesirable events, such as cytokine-driven toxicities (e.g., cytokine release syndrome (CRS)), infusion-related reactions (IRRs), macrophage activation syndrome (MAS), neurologic toxicities, severe tumor lysis syndrome (TLS), neutropenia, thrombocytopenia, elevated liver enzymes, and/or central nervous system (CNS) toxicities, following treatment with a therapeutic protein using the fractionated, dose-escalation dosing regimen of the disclosure relative to treatment with an anti-CD20/anti-CD3 bispecific antibody using an non-fractioned dosing regimen.

For all the methods and pharmaceutical formulations described herein, the therapeutic protein would be formulated, dosed, and administered consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The therapeutic protein need not be, but is optionally formulated with, one or more agents currently used to prevent or treat the disorder in question. The effective amount of such other agents depends on the amount of the therapeutic protein present in the formulation, the type of disorder or treatment, and other factors discussed above. The therapeutic protein may be suitably administered to the patient over a series of treatments.

### VIII. Articles of Manufacture

In another aspect of the disclosure, an article of manufacture containing materials useful for the treatment, prevention, and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a pharmaceutical composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is a therapeutic protein (e.g., anti-CD3 bispecific antibody; e.g., TDB or TCB; e.g., mosunetuzumab, glofitamab, cevostamab, or runimotamab), as described herein. The label or package insert indicates that the composition is used for treating the condition of choice (e.g., a cancer) and further includes information related to at least one of the dosing regimens described herein.

The pharmaceutical composition can be supplied in a container having a volume from 1 ml to 100 ml (e.g., from 1 ml to 5 ml, from 5 ml to 10 ml, from 10 ml to 15 ml, from 15 ml to 20 ml, from 20 ml to 25 ml, from 25 ml to 30 ml, from 30 ml to 40 ml, from 40 ml to 50 ml, from 50 ml to 60 ml, from 60 ml to 70 ml, from 70 ml to 80 ml, from 80 ml to 90 ml, or from 90 ml to 100 ml, e.g., about 1 ml, about 1.5 ml, about 2 ml, about 2.5 ml, about 3 ml, about 3.5 ml, about 4 ml, about 4.5 ml, about 5 ml, about 10 ml, about 15 ml, about 20 ml, about 25 ml, about 30 ml, about 40 ml, about 50 ml, about 60 ml, about 70 ml, about 80 ml, about 90 ml, or about 100 ml). In a particular embodiment, the container has a volume of about 50 ml (e.g., about 40 ml, about 45 ml, about 46 ml, about 47 ml, about 48 ml, about 49 ml, about 50 ml, about 51 ml, about 52 ml, about 53 ml, about 54 ml, about 55 ml, or about 60 ml). In another particular embodiment, the container has a volume of about 2 ml (e.g., about 1 ml, about 1.5 ml, about 1.6 ml, about 1.7 ml, about 1.8 ml, about 1.9 ml, about 2 ml, about 2.1 ml, about 2.2 ml, about 2.3 ml, about 2.4 ml, about 2.5 ml, or about 3 ml). In yet another particular embodiment, the container has a volume of about 15 ml (e.g., about 10 ml, about 11 ml, about 12 ml, about 13 ml, about 14 ml, about 15 ml, about 16 ml, about 17 ml, about 18 ml, about 19 ml, or about 20 ml). In yet another embodiment, the container has a volume of about 2.5 ml or about 10 ml.

In some embodiments, the container is a stainless-steel container or a nickel-steel alloy container (e.g., HASTELLOY^{®}), such as a tank, mini-tank, canister, can, etc. In some instances, the pharmaceutical composition in such a container is a DS, which can be further diluted prior to use, e.g., into a DP (e.g., in final vial configuration). Alternatively, the pharmaceutical composition in the container is a DP. In some embodiments, if the DP is to be administered with a diluent, or if it is intended to be administered in combination with other therapeutic reagents, the DP may be at a higher concentration than the concentration at which it is to be administered to the subject. In some embodiments, the DP is in a container such as an IV bag or a syringe (e.g., for delivery via syringe pump).

In some embodiments, the article of manufacture includes a vial having a volume of about 1 ml or more, for example, about 1 ml, about 2 ml, about 3 ml, about 4 ml, about 5 ml, about 6 ml, about 7 ml, about 8 ml, about 9 ml, about 10 ml, about 11 ml, about 12 ml, about 13 ml, about 14 ml, about 15 ml, about 16 ml, about 17 ml, about 18 ml, about 19 ml, about 20 ml, about 25 ml, about 30 ml, about 35 ml, about 40 ml, about 50 ml, or more. In a particular embodiment, the vial has a volume of about 50 ml (e.g., about 40 ml, about 45 ml, about 46 ml, about 47 ml, about 48 ml, about 49 ml, about 50 ml, about 51 ml, about 52 ml, about 53 ml, about 54 ml, about 55 ml, or about 60 ml). In another particular embodiment, the vial has a volume of about 2 ml (e.g., about 1 ml, about 1.5 ml, about 1.6 ml, about 1.7 ml, about 1.8 ml, about 1.9 ml, about 2 ml, about 2.1 ml, about 2.2 ml, about 2.3 ml, about 2.4 ml, about 2.5 ml, or about 3 ml). In yet another particular embodiment, the vial has a volume of about 15 ml (e.g., about 10 ml, about 11 ml, about 12 ml, about 13 ml, about 14 ml, about 15 ml, about 16 ml, about 17 ml, about 18 ml, about 19 ml, or about 20 ml). In a particular embodiment, the vial has a volume of 2.5 ml or 10 ml. In some embodiments, the vial is for single use. In some embodiments, the vial contains about 1 mg, about 2 mg, about 3 mg, about 4 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 25 mg, about 30 mg, about 35 mg, about 40 mg, about 45 mg, about 50 mg, about 55 mg, about 60 mg, or more therapeutic protein. In some embodiments, the vial includes a pharmaceutical composition comprising a therapeutic protein (e.g., anti-CD3 bispecific antibody; e.g., TDB or TCB; e.g., mosunetuzumab, glofitamab, cevostamab, or runimotamab), polysorbate 20 (PS20), methionine, a buffering agent, and a carrier, wherein the molar ratio of the PS20 to the therapeutic protein is 100 or less, the PS20 is at a concentration from 0.01% to 0.12% weight-by-volume (w/v), the methionine is at a concentration from 1 mM to 50 mM, and the buffering agent is at a concentration from 5 mM to 20 mM. In some embodiments, the container closure system comprises one or more, or all, of a glass vial, a stopper, and a cap.

Moreover, the article of manufacture may comprise (a) a first container with a composition contained therein, wherein the pharmaceutical composition comprises a therapeutic protein described herein; and (b) a second container with a pharmaceutical composition contained therein, wherein the pharmaceutical composition comprises a further cytotoxic or otherwise therapeutic agent. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

### EXAMPLES

The following are examples of methods and compositions of the disclosure. It is understood that various other embodiments may be practiced, given the general description provided above, and the examples are not intended to limit the scope of the claims.

### Example 1. Pharmaceutical Development for Mosunetuzumab

This example shows a method of formulating and using mosunetuzumab (BTCT4465A). For mosunetuzumab, the researchers observed that in the formulation, a relatively low protein concentration and relatively high surfactant concentration were considered preferable to ensure accurate delivery of low doses of mosunetuzumab.

Mosunetuzumab is based on a human IgG1 isotype and is intended for the treatment of B cell malignancies by T cell recruitment and activation. The CD20 arm of mosunetuzumab is directed against B-lymphocyte antigen CD20, a glycosylated phosphoprotein expressed on the surface of B cells. The CD3 arm binds to and recruits T cells, which are activated upon target engagement with CD20, resulting in robust T cell proliferation and cell killing.

The amino acid sequences comprising mosunetuzumab (BTCT4465A) are summarized in the Table 1 above.

Recombinant mosunetuzumab was produced in two separate CHO cell lines using the knob-in-hole technology, including the glycosylation mutation N297G to reduce or eliminate ADCC function. Mosunetuzumab was composed of one anti-CD20 half antibody (an anti-CD20 arm) with a knob and one anti-CD3 half antibody (an anti-CD3 arm) with a hole, which half antibodies were assembled using a glutathione reduction. Mosunetuzumab was active against indolent (e.g., non-dividing) and chemo-resistant cells, and prior immune response to tumor was not a prerequisite for activity.

The mosunetuzumab Drug Substance (DS) and Drug Product (DP) were formulated as 60 mg/ml mosunetuzumab in 20 mM histidine acetate (HisOAc), 240 mM sucrose, and 0.02% (w/v) polysorbate 20 (PS20) at pH 5.8. To produce mosunetuzumab DP, 1.0 ml nominal fill of the DS was filled into 2.0 ml vials. In this example, mosunetuzumab DS had a molar ratio of surfactant to therapeutic protein of about 0.4.

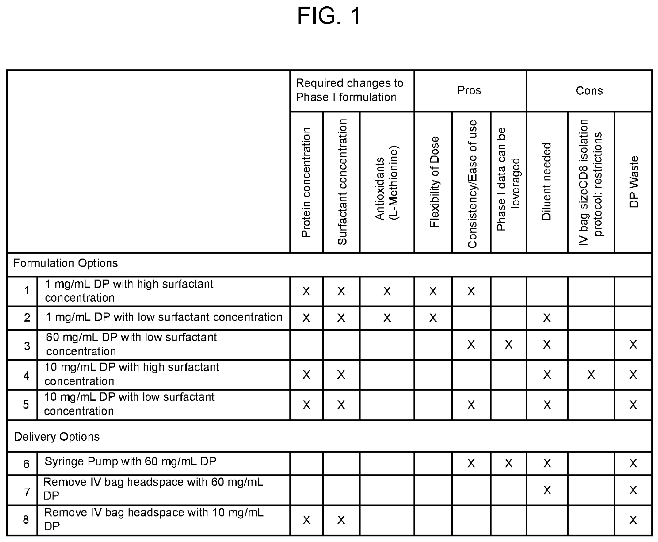

The 60 mg/ml protein concentration previously supported delivering in the clinic a wide range of doses (50 µg to 30 mg) using a syringe pump, with the lowest dose composition concentration diluted in two steps using both formulation buffer and saline as diluent. The dose range in subsequent and ongoing clinical trials varied due to the use of a step-fractionated dosing scheme in which, during cycle 1, patients received a low dose (e.g., 1 mg) on day 1, an intermediate dose (e.g., 2 mg) on day 8, and a full dose (e.g., 20 mg) on day 15. Several formulation options were considered when pairing protein concentration with low or high surfactant level **(****FIG. 1****).** In particular, a relatively low protein concentration and relatively high surfactant concentration were considered preferable to ensure accurate delivery of low doses using an intravenous fluid (IV) bag, to reduce or avoid the use of diluents, to permit flexibility of dosing, and to reduce or eliminate waste.

### Example 2. Knowledge Building Study for Mosunetuzumab Formulation Development

A knowledge building study was executed after early-stage development to evaluate the impact of formulation parameters on stability of the formulations and to identify major risks of the formulations. Importantly, the results of this study suggested that oxidative degradation (e.g., oxidative degradation related to other forms of degradation, such as aggregation, fragmentation, and surfactant degradation) is a primary risk factor for clinical phase III DP formulation (i.e., a low-concentration mosunetuzumab DP formulation). In addition, the results of this study suggested that low-protein concentration mosunetuzumab formulations can be susceptible to oxidative degradation, particularly those formulations that have a high histidine concentration, low sucrose concentration, and a high PS20 concentration. Further, the results of this study suggested that the addition of methionine (L-methionine; L-Met) inhibited oxidative degradation, thereby reducing the risk of other forms of degradation (e.g., aggregation, fragmentation, and/or surfactant degradation).

### Example 3. Mosunetuzumab Formulation Development

The purpose of the studies in this Example was to characterize risks associated with pharmaceutical compositions having mosunetuzumab, and to identify formulation parameters that reduce these risks. The studies are summarized and characterize the effects of various components of the DS and DP formulations and the containers in which they can be stored. The studies also found that an important characteristic for such formulations was the molar ratio of mosunetuzumab to surfactant, where the protein was present at low molar concentrations and the surfactant at higher molar concentrations.

### 3.1. IV Bag Agitation Study

To determine the appropriate amount and composition of surfactant for use in formulations delivered using IV bags and without the use of a diluent which support a wide dose range, IV bag agitation studies were performed. Several factors were characterized, including protein concentration, dose, IV bag size, surfactant composition, and surfactant concentration.

Mosunetuzumab was formulated at various protein concentrations (1 mg/ml, 5 mg/ml, and 10 mg/ml) in 20 mM HisOAc, 240 mM sucrose, pH 5.8. A range of doses (1 mg, 2 mg, 5 mg, 20 mg, and 30 mg) was tested, and 50-ml and 100-ml polyoxyethelene (PO) bags were assessed. 50-ml and 100-ml PO IV bags had been previously determined to be high-risk IV bags because of protein aggregation due to the relatively large headspace and high wall rigidity relative to polyvinyl chloride (PVC) IV bags of corresponding size. Three surfactants were evaluated: polysorbate 20 (PS20), poloxamer 188 (P188), and super-refined polysorbate 20 (srPS20). PS20 and P188 were tested over the full dose range, and limited testing was performed with srPS20, in view of its comparability to PS20 in agitation studies. SrPS20 was subsequently discontinued to simplify the studies.

In these experiments, DP surfactant concentration was varied to determine the minimum surfactant concentration in the IV bag required to prevent aggregation and particle formation during IV bag agitation stress. For each test condition, mosunetuzumab DP was injected into the IV bag and shaken at 100 rpm on an orbital shaker at 2-8 °C for up to 24 hours. Samples were taken at 0, 1, 6, and 24 hours and assessed for sub-visible particles by high accuracy liquid particle counting (HIAC), and soluble aggregates by size-exclusion high performance liquid chromatography (SE-HPLC). A condition was considered to pass if there was no substantial increase in soluble aggregate or particle counts. Increase in soluble aggregate was assessed by observed qualitative changes in the SE-HPLC chromatogram compared to time 0. Particle formation was observed as a significant increase in sub-visible particle counts compared to time 0 (an increase of > 1000 in cumulative particle counts/ml at ≥ 2 µm).

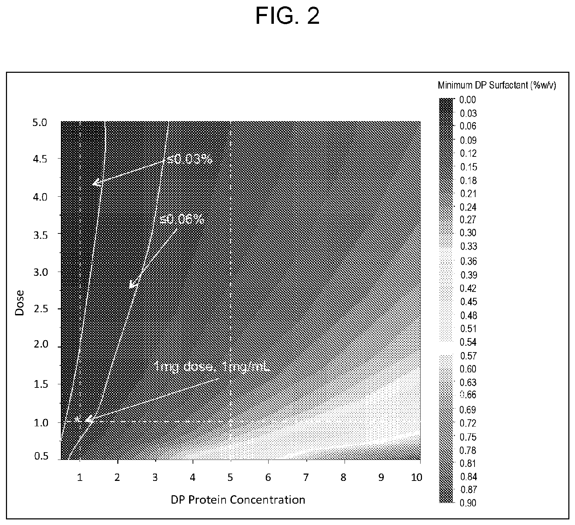

Data from this study were analyzed to determine the minimum amount of surfactant in the IV bag required to support phase III and commercial dose administration without a diluent. **FIG. 2** shows how DP protein concentration and dose together affected the minimum required PS20 concentration in a 100-ml PO IV bag. Similar results were observed for 50-ml PO IV bags. A relatively high ratio of PS20 concentration to protein concentration (e.g., for low doses, such as 1 mg/ml) ensured that there was a sufficient surfactant level in the final dose solution in IV bags. These results suggest that a high ratio of PS20 to protein concentrations may be better for ensuring protein stability, e.g., in IV bags, at low DP doses (e.g., 1 mg/ml).

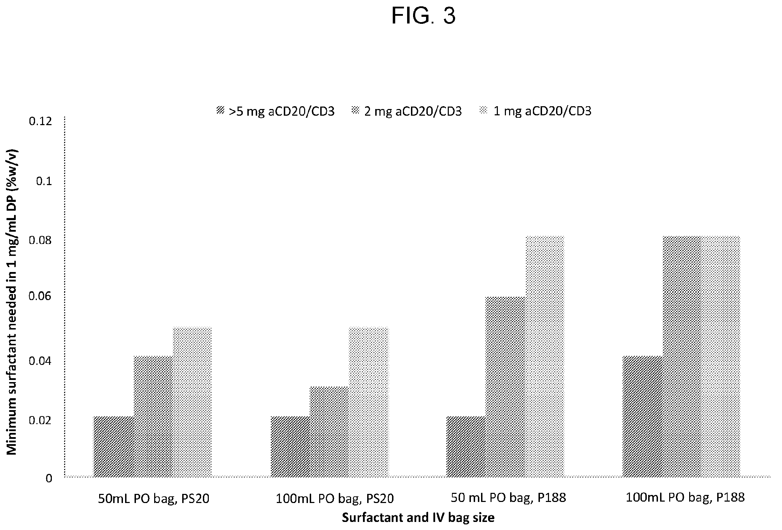

Minimum suitable surfactant levels were determined for each surfactant composition for 1 mg/ml DP, which was identified as an effective protein concentration to support low dose administration, as shown in **FIG. 3****.** It was determined that a minimum of 0.05% (w/v) PS20 (molar ratio of surfactant to mosunetuzumab of about 59) and super refined PS20 (srPS20), and a minimum of 0.08% (w/v) P188 (molar ratio of surfactant to mosunetuzumab of about 14), were necessary to prevent aggregation and particle formation during the shaking stress conditions described above. To support a manufacturing range, the PS20 target level was set at 0.06% (w/v) (molar ratio of surfactant to mosunetuzumab of about 71) and P188 target level was set at 0.10% (w/v) (molar ratio of surfactant to mosunetuzumab of about 17) for the following DP formulation development.

### 3.2. Product quality impact of surfactant

Product quality impact (PQI) of different surfactant types was evaluated in a stability study. Oxidation risk for mosunetuzumab was of particular interest due to the observed oxidation effects in the knowledge building study (Example 2). Additionally, PS20 is known to be susceptible to oxidation, and high levels of PS20 (e.g., 0.06%, selected by the IV bag agitation study) may increase the risk of protein oxidation. In this study, protein concentration (60 mg/ml, 10 mg/ml, and 1 mg/ml) and surfactant composition (PS20, srPS20, and P188) were evaluated in a full-factorial study. All materials were formulated at the respective protein concentration with 20 mM HisOAc, 240 mM sucrose, and 0.1 % (w/v) surfactant, at pH 5.8. For PS20 or srPS20 as the surfactant, with protein concentrations of 60 mg/ml, 10 mg/ml, and 1 mg/ml, the molar ratios of surfactant to mosunetuzumab were about 2, 12, and 119, respectively. For P188 as the surfactant, with protein concentrations of 60 mg/ml, 10 mg/ml, and 1 mg/ml, the molar ratios of surfactant to mosunetuzumab were about 0.3, 1.7, and 17, respectively. The samples were hand filled into 6-ml glass vials at a fill volume of 3 ml, stoppered and capped using standard components, and stored for up to one month at 40 °C and up to three months at 30 °C. Assays included peptide mapping to quantify oxidation, HIAC to quantify aggregation and particle formation, evaporative light scattering detector (ELSD) to quantify surfactant degradation, and SE-HPLC to quantify size heterogeneity.

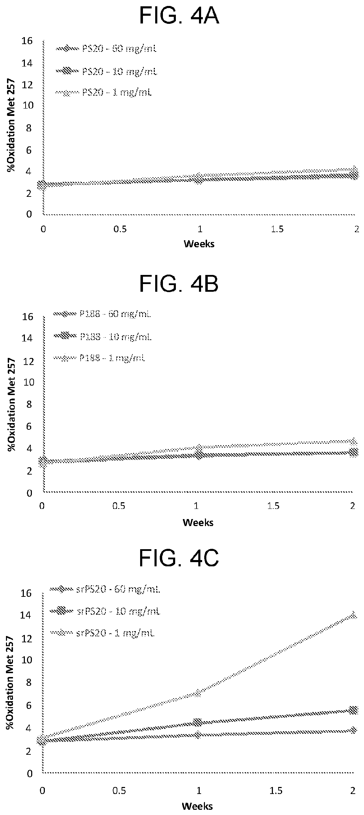

In compositions including PS20 or P188, Met 257 (M257) oxidation increased only slightly as protein concentration decreased from 60 mg/ml to 1 mg/ml **(****FIG. 4A** and **FIG. 4B****).** However, for srPS20, oxidation increased significantly at 1 mg/ml protein concentration **(****FIG. 4C****).** In the 1 mg/ml srPS20 sample, increases in fragmentation and aggregation and loss of surfactant were also observed. Based on these results, srPS20 was excluded as a surfactant choice for mosunetuzumab formulation.

### 3.3. Histidine concentration, ambient light exposure, and antioxidant screen

Histidine is susceptible to oxidation and its presence in the formulation buffer can induce protein oxidation. In this example, the impact of histidine concentration was further characterized. The knowledge building study described in Example 2 and surfactant type studies described above revealed that mosunetuzumab is sensitive to oxidation from thermal stress. However, the impact of light stress on mosunetuzumab had not been characterized. Here, the sensitivity of tryptophan residues of mosunetuzumab to oxidation was quantified.

Two formulations were evaluated for thermal stress and four formulations were evaluated for light stress (Table 6). All formulations included 1 mg/ml mosunetuzumab, 160 mM sucrose, 0.04% (w/v) PS20, pH of 5.5, in addition to the specified composition. In this example, the molar ratio of surfactant to mosunetuzumab is about 48. Formulation 7 (F7) was identified in the knowledge building study as being at risk for high oxidation by thermal stress. A low histidine F7 formulation (F7-low His) was included in this study to evaluate the impact of decreasing histidine concentration. In light stress studies, F7 was supplemented with the antioxidant methionine, alone or in combination with N-acetyl tryptophan (NAT), to evaluate the effect of antioxidant on light stress-mediated degradation. The formulations were filled into 6-ml vials with a fill volume of 3 ml, stoppered, capped, and placed under thermal stress (up to one month at 40 °C and up to three months at 25 °C) and ambient light stress (300,000 lux-hours at 25 °C). Ambient light exposure was performed by incubating samples in a fluorescent light box maintained at room temperature and 5500 lux light intensity for 54 hours. Assays included peptide mapping to quantify oxidation, HIAC to quantify aggregation and particle formation, ELSD to quantify surfactant degradation, and SE-HPLC to quantify size heterogeneity (e.g., as a result of fragmentation and/or aggregation).

**TABLE 6**

| Formulation summary for oxidation study | | | | | |
|---|---|---|---|---|---|
| **Formulation** | **His (mM)** | **Met (mM)** | **NAT (mM)** | **Thermal stress** | **Light Stress** |
| F7 | 30 | 0 | 0 | X | X |
| F7 + Met | 30 | 5 | 0 | | X |
| F7 + Met + NAT | 30 | 5 | 5 | | X |
| F7-low His | 10 | 0 | 0 | X | X |

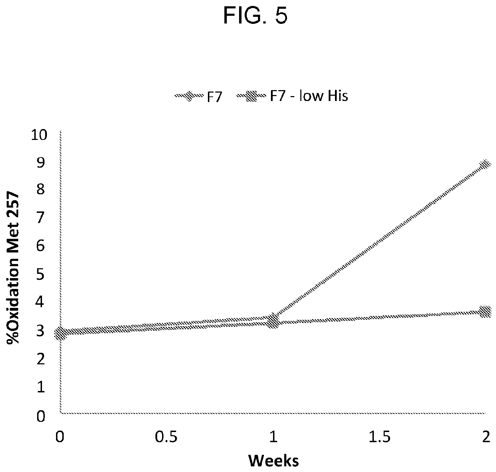

Compositions containing different levels of histidine (F7 and F7-low His) under 40 °C thermal stress conditions were compared. Both samples contained no antioxidant. As shown in **FIG. 5****,** the 30 mM histidine composition exhibited approximately 6% M257 oxidation after two weeks at 40 °C, whereas the 10 mM histidine composition exhibited less than 1% M257 oxidation. In addition, the aggregation level for both dimer and high molecular weight species (HMWS) was also higher in the 30 mM histidine sample. Results suggest that higher surfactant level and lower HisOAc concentration may be better for preventing oxidative stress under thermal stress conditions.

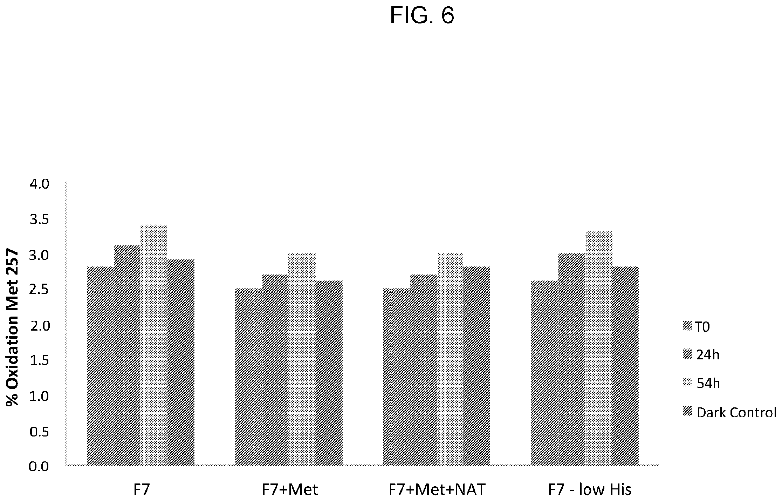

Effects of histidine concentration and antioxidant were evaluated under ambient light exposure, a sensitive stress type that can induce oxidation. As shown in **FIG. 6****,** no substantial change in oxidation at any site (Met or Trp) was observed in any sample tested, indicating that F7 is stable under the relevant ambient light stress, despite being at high-risk for oxidation. NAT provided no additional benefit and was therefore not included in subsequent formulation development.

### 3.4. Methionine Concentration Optimization

Hydrogen peroxide spiking studies were performed to identify preferable concentrations of methionine, which was identified as an important excipient. Samples containing 1 mg/ml mosunetuzumab, 15 mM histidine acetate, 0.08% (w/v) PS20, 160 mM sucrose, at pH of 5.8 (control formulation) were prepared with or without hydrogen peroxide (H₂O₂) and different levels of Met (Table 7). In these samples the molar ratio of surfactant to mosunetuzumab is about 95. The control formulation has a high-risk for oxidation, as it contains a relatively high concentration of histidine, a relatively high concentration of PS20, and a relatively low concentration of sucrose.

Hydrogen peroxide (2,000 ng/ml) was used as an oxidative stress challenge to antioxidant protection. Samples were filled into 20 cc vials with a fill volume of 3 ml to represent container conditions at high risk for oxidation due to a large headspace. Vials were stoppered, capped, and placed on thermal stability for one month at 40 °C or six months at 25 °C, or on real-time stability, i.e., at 2-8 °C. In ambient light studies, samples were subject to ambient light exposure up to 300,000 lux-hours (54 hours at 5,500 lux) at room temperature. Samples were obtained from the ambient light study at time points of 0 hours, 24 hours, 48 hours, and 54 hours, and an aluminum foil-wrapped dark control was included. The AMPLEX^{®} hydrogen peroxide/peroxidase assay (ThermoFisher Scientific; Waltham, MA) was used to measure hydrogen peroxide concentration. Assays used to monitor product quality (e.g., stability) included color, pH, turbidity (by ultraviolet spectrometry (UV-spec)), strength (by UV-spec), charge variants (by imaged capillary isoelectric focusing (icIEF) and microchip sieving electrophoresis (MCE-SDS)), size heterogeneity (by SE-HPLC), visible and sub-visible particles (by HIAC), potency, oxidation (by peptide mapping), methionine concentration (by mass spectrometry), and polysorbate concentration (by ELSD).

| TABLE 7 | | |
|---|---|---|
| Formulation summary for methionine study | | |
| **Sample^{a}** | **Met (mM)** | **H₂O₂ (ng/ml)** |
| Control | 0 | 0 |
| + H₂O₂ | 0 | 2000 |
| + H₂O₂ + Met 2.5 | 2.5 | 2000 |
| + Met 5 | 5 | 0 |
| + H₂O₂ + Met 5 | 5 | 2000 |
| + H₂O₂ + Met 10 | 10 | 2000 |

| | | |
|---|---|---|
| ^{a} All samples contained 1 mg/ml mosunetuzumab, 15 mM histidine acetate, 0.08% (w/v) PS20, 160 mM sucrose, and a pH of 5.8 in addition to the composition specified in the table. | | |

After storage for 12 months at 2-8 °C (e.g., 5 °C), the H₂O₂ present in formulations containing no Met was completely depleted **(****FIG. 7A****).** At 12 months, 41.5% M257 oxidation was observed, as opposed to 3.7% M257 oxidation in the control sample **(****FIG. 7B****).** With a minimum of 2.5 mM Met added to the formulation, the H₂O₂ was consumed completely after one month **(****FIG. 7A****),** and M257 oxidation remained equivalent with the control sample throughout the duration of the study **(****FIG. 7B****).** No differences were observed between formulations containing 2.5 mM, 5 mM, and 10 mM Met. No changes in other attributes were observed.

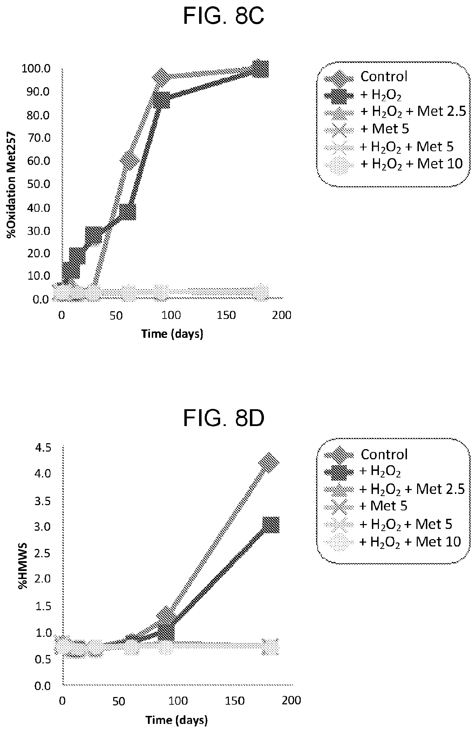

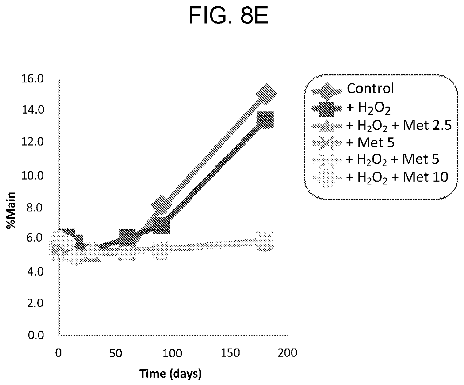

At accelerated thermal conditions (i.e., 25 °C), the level of H₂O₂ in samples containing no Met decreased to 130 ng/ml at two months and subsequently accumulated back to a level of 1,000 ng/ml by six months **(****FIG. 8A****).** The control sample also exhibited an increase of H₂O₂ after two months **(****FIG. 8A****).** This observation is consistent with a slow phase of Met oxidation increase represented by M257 in the first two months for the spiked sample containing no Met and a fast phase of Met oxidation **(****FIG. 8C****)** in the later part up to 6 months for both the spiked sample with no Met and the control. This observation is also consistent with the increase in tryptophan 107 (i.e., Trp107 or W107) oxidation in the CD20 arm **(****FIG. 8B****),** protein aggregation **(****FIG. 8D****),** and fragmentation **(****FIG. 8E****)** between two and six months for both the spiked sample containing no Met and control sample. This observed H₂O₂ production is likely related to oxidative PS20 degradation in both the control and spiked sample with no Met, which can trigger further protein degradation. In contrast, upon addition of 2.5-10 mM Met, H₂O₂ was consumed completely after one week and did not re-accumulate over the course of the six-month incubation **(****FIG. 8A****).** Increases in M257 oxidation, W107 oxidation, aggregation, fragmentation, and PS20 degradation were greatly reduced by 2.5 mM Met and completely inhibited by 5 mM and 10 mM Met through the six-month incubation (FIG. 8B - FIG. 8E), suggesting that 5 mM Met was the minimal requirement as antioxidant for this formulation under these conditions. A similar trend was observed from stress stability results at 40 °C on a shorter time scale.

The samples in Table 8 were all also subjected to ambient light stress (up to 300,000 lux-hours) at 25 °C. Consistent with the histidine concentration study, ambient light did not cause extensive oxidation, and the minor M257 oxidation generated in the sample without Met was completely inhibited by 2.5 mM Met addition.

| TABLE 8 | | | | | |
|---|---|---|---|---|---|
| DP formulation summary | | | | | |
| **Formulation^{a}** | **His acetate (mM)** | **pH** | **PS20 (%w/v)** | **Sucrose (mM)** | **Met (mM)** |
| 1 (Target) | 10 | 5.8 | 0.06 | 240 | 5 |
| 2 | 15 | 5.5 | 0.08 | 160 | 2.5 |
| 3 | 15 | 5.8 | 0.08 | 160 | 2.5 |
| 4 | 15 | 6.1 | 0.08 | 160 | 2.5 |
| 5 | 15 | 5.8 | 0.08 | 160 | 5 |

| | | | | | |
|---|---|---|---|---|---|
| ^{a} All formulations contained 1 mg/ml mosunetuzumab. | | | | | |

### 3.5 DP Formulation Screen

A DP formulation screening study was performed based on information obtained from the studies described above. The target formulation and high-risk oxidation (high His, high PS20, low sucrose) were evaluated with varied pH and Met concentrations. Each formulation was sterile-filtered and filled into 15-ml glass vials with a 5-ml fill volume. Vials were stoppered with 20-mm Daikyo D777-1 liquid stoppers (Daikyo; Tochigi, Japan), capped with aluminum flip-top caps, and stored upright on stability according to the program specified in Table 9. Assays were configured to observe color, pH, turbidity (by UV-spec), strength (by UV-spec), charge variants (by icIEF and mCE-SDS), size heterogeneity (by SE-HPLC), visible and sub-visible particles (by HIAC), potency, oxidation (by peptide mapping), methionine concentration (by mass spectrometry), and polysorbate concentration (by ELSD).

| TABLE 9 | | | | | | | | | | | | | |
|---|---|---|---|---|---|---|---|---|---|---|---|---|---|
| DP formulation sample time points | | | | | | | | | | | | | |
| **Storage temperature / Time Point** | **T₀** | **3D** | **1W** | **2W** | **3W** | **1M** | **2M** | **3M** | **6M** | **9M** | **12M** | **18M** | **24M** |
| 5 °C | X* | | | | | X | X | X | X | X | X | X | X |
| 25 °C, 65% RH | | | | X | | X | X | X | X | | | | |
| 40 °C, 75% RH | | X | X | X | X | X | | | | | | | |

| | | | | | | | | | | | | | |
|---|---|---|---|---|---|---|---|---|---|---|---|---|---|
| X = 3 vials; X* = 5 vials; D=days, W=weeks, M=months | | | | | | | | | | | | | |

Time zero (T₀) characterization of the formulations is summarized in Table 10. No change was observed for any formulation after 12 months at 5 °C (Table 11). After storage at 5 °C for 24 months, consistent changes through all five formulation were observed by iCIEF and CE-SDS. There is a small increase in percent acidic variants by icIEF, and the CE-SDS results for pre-peaks appear to show a slight increase after 24 months at 5 °C, but were observed to increase the same across all formulations. No changes were observed by any other assay.

After storage at 25 °C, 60% relative humidity (RH; accelerated condition) for six months (Table 12), similar changes were observed across all five formulations for SE-HPLC main peak decrease (0.3-0.4%), icIEF acidic increase and main peak decrease (12.6-17%), and mCE-SDS main peak decrease and pre-peaks increase (1.0%). No changes were observed by any other assay conducted.

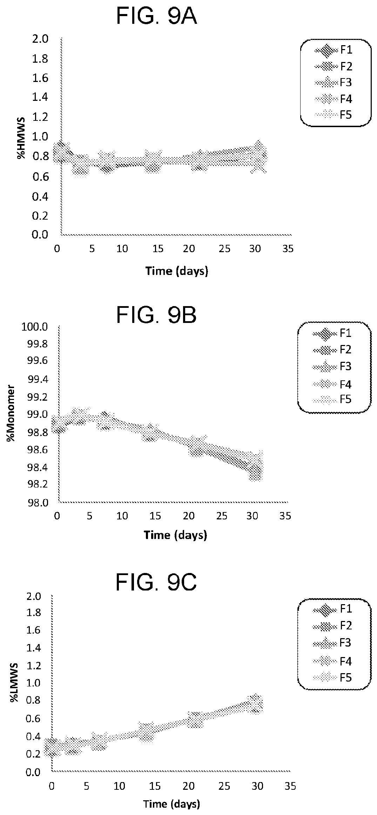

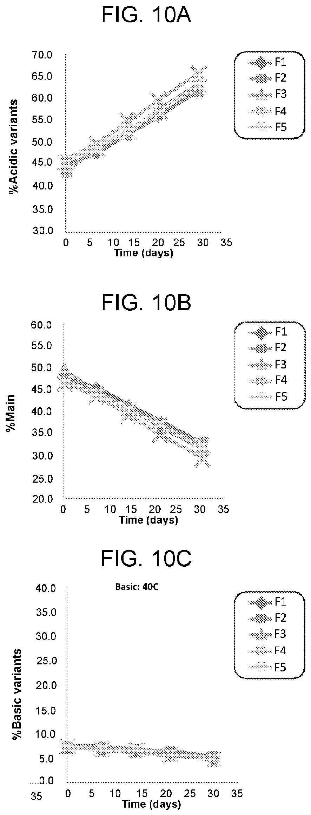

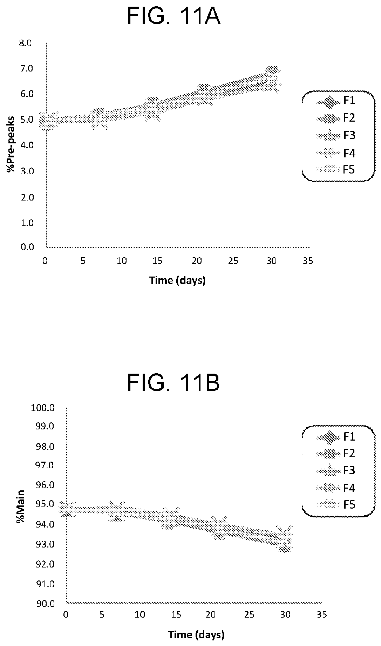

After storage at 40 °C, 75% RH (stress condition) for one month (Table 13), similar changes were observed across all five formulations for SE-HPLC main peak decrease (0.4-0.6%, **FIG. 9A** **-** **FIG.9C****),** icIEF acidic increase and main peak decrease (17.1-19.8%, **FIG. 10A** **-** **FIG. 10C****),** and mCE-SDS main peak decrease and pre-peak increase (1.4-1.9%, **FIG. 11A and FIG. 11B****).** No change was observed for other assay conducted.

Results of this study indicated that mosunetuzumab DP was stable within pH 5.5-6.1, even at the high-risk condition for oxidation (high L-histidine, low sucrose, low L-methionine, high polysorbate).

| TABLE 10 | | | | | | | | | | | | |
|---|---|---|---|---|---|---|---|---|---|---|---|---|
| Formulation screen - T₀ | | | | | | | | | | | | |
| Formulation | Time (months) | CAC | | | | | icIEF | | | SE-HPLC | | |
| | | (Color / Appearance) | Turbidity | Osmolality (mOsm/kg) | pH | Strength (mg/ml) | (% Acidic) | (% Main Peak) | (% Basic) | (% HMWS) | (% Main Peak) | (% LMWS) |
| 1 (Target) | T = 0 | ≤ BY7 | 0.03 | 286 | 5.8 | 1.01 | 45.3 | 47.5 | 7.2 | 0.9 | 98.9 | 0.3 |
| 2 | T = 0 | ≤ BY7 | 0.02 | 203 | 5.5 | 1.02 | 44.3 | 48.2 | 7.5 | 0.8 | 98.9 | 0.3 |
| 3 | T = 0 | ≤ BY7 | 0.02 | 201 | 5.8 | 0.99 | 43.7 | 49.2 | 7.1 | 0.8 | 98.9 | 0.3 |
| 4 | T = 0 | ≤ BY7 | 0.03 | 195 | 6.2 | 0.98 | 46.4 | 46.5 | 7.1 | 0.8 | 98.9 | 0.3 |
| 5 | T = 0 | ≤ BY7 | 0.01 | 203 | 5.8 | 1.02 | 46.2 | 46.9 | 6.9 | 0.8 | 98.9 | 0.3 |

**TABLE 6, continued**

| Formulation | Time (months) | Non-Reduced CE-SDS | | Visible Particulates | Subvisible Particulates (Cumulative Counts/ml) | | | | Potency | L-Met (mM) | Oxidation Met 257 (%) | PS20 (% w/v) |
|---|---|---|---|---|---|---|---|---|---|---|---|---|
| | | Sum of Pre-Peaks (%) | (% Main Peak) | | ≥ 2 µm | ≥ 5 µm | ≥ 10 µm | ≥ 25 µm | | | | |
| 1 (Target) | T = 0 | 4.9 | 94.8 | PFVP | 16 | 7 | 1 | 0 | 103 | 4.6 | 2.8 | 0.06 |
| 2 | T = 0 | 4.9 | 94.8 | PFVP | 73 | 26 | 13 | 4 | 106 | 2.3 | 2.6 | 0.08 |
| 3 | T = 0 | 4.9 | 94.8 | PFVP | 8 | 3 | 0 | 0 | 113 | 2.3 | 2.6 | 0.08 |
| 4 | T = 0 | 4.9 | 94.8 | PFVP | 28 | 3 | 1 | 0 | 102 | 2.4 | 2.5 | 0.08 |
| 5 | T = 0 | 4.9 | 94.8 | PFVP | 137 | 25 | 7 | 1 | 109 | 4.8 | 2.7 | 0.08 |

| TABLE 11 | | | | | | | | | | | |
|---|---|---|---|---|---|---|---|---|---|---|---|
| DP formulation screen results at 2-8 °C | | | | | | | | | | | |
| Formulation | Time (months) | Color | Turbidity | pH | Strength (mg/ml) | iclEF | | | SE-HPLC | | |
| | | | | | | (% Acidic) | (% Main Peak) | (% Basic) | (% HMWS) | (% Main Peak) | (% LMWS) |
| 1 (Target) | 1 | NT | 0.01 | 5.8 | NT | 45.0 | 47.8 | 7.2 | 0.8 | 98.9 | 0.3 |
| | 2 | ≤ BY7 | 0.02 | 5.8 | NT | 47.1 | 45.4 | 7.5 | 0.7 | 99.0 | 0.3 |
| | 3 | ≤ BY7 | NT | 5.8 | NT | 45.2 | 47.9 | 6.9 | 0.8 | 98.9 | 0.4 |
| | 6 12 | ≤ B9* | 0.01 | 5.8 | 1.02 | 44.6 | 47.0 | 8.4 | 0.7 | 99.0 | 0.3 |
| | 9 | NT | 0.00 | 5.9 | NT | NT | NT | NT | 0.7 | 99.0 | 0.3 |
| | | ≤ B9* | 0.01 | 5.8 | NT | 45.3 | 46.7 | 8.0 | 0.7 | 99.0 | 0.3 |
| 2 | 1 | NT | 0.00 | 5.5 | NT | 43.1 | 49.3 | 7.6 | 0.8 | 99.0 | 0.2 |
| | 2 | ≤ BY7 | 0.02 | 5.5 | NT | 45.0 | 46.6 | 8.4 | 0.8 | 99.0 | 0.3 |
| | 3 | ≤ BY7 | NT | 5.5 | NT | 45.2 | 47.5 | 7.3 | 0.6 | 99.1 | 0.3 |
| | 6 | ≤ B9* | 0.01 | 5.5 | 1.01 | 45.7 | 46.2 | 8.1 | 0.7 | 99.0 | 0.3 |
| | 9 | NT | 0.01 | 5.5 | NT | NT | NT | NT | 0.7 | 99.0 | 0.3 |
| | 12 | ≤ B9* | 0.01 | 5.5 | NT | 43.7 | 46.7 | 9.7 | 0.7 | 99.0 | 0.3 |
| 3 | 1 | NT | 0.01 | 5.8 | NT | 44.4 | 48.2 | 7.4 | 0.8 | 99.0 | 0.3 |
| | 2 | ≤ BY7 | 0.01 | 5.8 | NT | 45.7 | 46.7 | 7.6 | 0.8 | 98.9 | 0.3 |
| | 3 | ≤ BY7 | NT | 5.8 | NT | 45.9 | 47.5 | 6.6 | 0.8 | 99.0 | 0.3 |
| | 6 | ≤ B9* | 0.01 | 5.8 | 1.04 | 45.4 | 47.3 | 7.3 | 0.7 | 99.0 | 0.3 |
| | 9 | NT | 0.01 | 5.8 | NT | NT | NT | NT | 0.7 | 99.0 | 0.3 |
| | 12 | ≤ B9* | 0.01 | 5.8 | NT | 45.7 | 46.3 | 8.1 | 0.7 | 99.0 | 0.3 |
| 4 | 1 | NT | 0.01 | 6.2 | NT | 45.1 | 48.0 | 6.9 | 0.8 | 98.9 | 0.3 |
| | 2 | ≤ BY7 | 0.02 | 6.2 | NT | 46.8 | 46.0 | 7.3 | 0.8 | 98.9 | 0.3 |
| | 3 12 | ≤ BY7 | NT | 6.2 | NT | 46.7 | 46.4 | 6.9 | 0.7 | 99.0 | 0.3 |
| | 6 | ≤ B9* | 0.01 | 6.2 | 1.04 | 45.7 | 46.8 | 7.5 | 0.7 | 99.0 | 0.3 |
| | 9 | NT | 0.01 | 6.2 | NT | NT | NT | NT | 0.7 | 99.0 | 0.3 |
| | | ≤ B9* | 0.01 | 6.2 | NT | 47.1 | 45.1 | 7.8 | 0.8 | 99.0 | 0.3 |
| 5 | 1 | NT | -0.01 | 5.7 | NT | 44.8 | 48.2 | 7.0 | 0.8 | 98.9 | 0.3 |
| | 2 | ≤ BY7 | 0.00 | 5.8 | NT | 46.0 | 46.3 | 7.7 | 0.8 | 99.0 | 0.2 |
| | 3 | ≤ BY7 | NT | 5.8 | NT | 45.5 | 47.4 | 7.0 | 0.8 | 98.9 | 0.4 |
| | 6 | ≤ B9* | 0.00 | 5.8 | 1.02 | 45.9 | 46.0 | 8.1 | 0.7 | 99.0 | 0.3 |
| | 9 | NT | 0.01 | 5.8 | NT | NT | NT | NT | 0.7 | 99.0 | 0.3 |
| | 12 | ≤ B9* | 0.01 | 5.8 | NT | 46.5 | 45.3 | 8.2 | 0.7 | 99.0 | 0.3 |

**TABLE 11, continued:**

| Formulatio n | Time (months) | Non-Reduced CE- SDS | | Visible Particulates | Subvisible Particulates (Cumulative Counts/ml) | | | | Potency (%) | L-Met (mM) | Oxidation Met 257 (%) | PS20 (% w/v) |
|---|---|---|---|---|---|---|---|---|---|---|---|---|
| | | Sum of Pre- Peaks (%) | (% Main Peak) | | ≥ 2 µm | ≥ 5 µm | ≥ 10 µm | ≥ 25 µm | | | | |
| 1 | 1 | 5.1 | 94.6 | PFVP | 356 | 73 | 9 | 2 | NT | 4.9 | 2.6 | 0.06 |
| | 2 | 5.0 | 94.7 | PFVP | 301 | 79 | 8 | 2 | NT | 4.5 | 2.6 | 0.06 |
| | 3 | 5.0 | 94.8 | PFVP | 493 | 38 | 7 | 1 | NT | 3.9 | 2.5 | 0.06 |
| | 6 12 | 4.9 | 94.8 | PFVP | 333 | 33 | 3 | 0 | NT | 4.8 | 2.6 | 0.06 |
| | 9 | NT | NT | PFVP | 479 | 62 | 8 | 2 | NT | NT | NT | 0.06 |
| | | 5.0 | 94.7 | PFVP | 447 | 58 | 4 | 0 | 102 | 5.2 | 2.2 | 0.06 |
| 2 | 1 | 5.1 | 94.6 | PFVP | 42 | 13 | 6 | 2 | NT | 2.4 | 2.8 | 0.08 |
| | 2 | 4.9 | 94.9 | PFVP | 351 | 68 | 9 | 2 | NT | 2.3 | 2.6 | 0.08 |
| | 3 | 5.0 | 94.7 | PFVP | 738 | 163 | 23 | 0 | NT | 1.9 | 2.5 | 0.08 |
| | 6 12 | 4.8 | 94.8 | PFVP | 271 | 73 | 6 | 0 | NT | 2.4 | 2.6 | 0.08 |
| | 9 | NT | NT | PFVP | 34 | 9 | 3 | 1 | NT | NT | NT | 0.08 |
| | | 5.0 | 94.8 | PFVP | 787 | 107 | 2 | 0 | 107 | 2.6 | 2.3 | 0.08 |
| 3 | 1 | 5.2 | 94.4 | PFVP | 202 | 19 | 2 | 0 | NT | 2.5 | 2.7 | 0.08 |
| | 2 | 5.0 | 94.7 | PFVP | 435 | 127 | 23 | | NT | 2.3 | 2.5 | 0.08 |
| | 3 | 4.9 | 94.8 | PFVP | 593 | 72 | | 4 1 | NT | 2.1 | 2.5 | 0.08 |
| | 6 | 4.8 | 94.9 | PFVP | 427 | 52 | 0 | 0 | NT | 2.4 | 2.7 | 0.08 |
| | 9 | NT | NT | PFVP | 15 | 4 | 3 1 | 0 | NT | NT | NT | 0.08 |
| | 12 | 5.0 | 94.7 | PFVP | 25 | 4 | 1 | 0 | 100 | 2.6 | 2.3 | 0.08 |
| 4 | 1 | 5.1 | 94.5 | PFVP | 395 | 67 | 13 | 2 | NT | 2.6 | 2.6 | 0.08 |
| | 2 | 5.0 | 94.8 | PFVP | 242 | 38 | 3 | 0 | NT | 2.3 | 2.8 | 0.08 |
| | 3 | 4.9 | 94.8 | PFVP | 242 | 27 | 4 | 1 | NT | 2.2 | 2.5 | 0.08 |
| | 6 12 | 4.8 | 94.8 | PFVP | 755 | 135 | 15 | 0 | NT | 2.4 | 2.6 | 0.08 |
| | 9 | NT | NT | PFVP | 143 | 12 | 2 | 0 | NT | NT | NT | 0.08 |
| | | 5.1 | 94.4 | PFVP | 733 | 60 | 3 | 0 | 93 | 2.6 | 2.3 | 0.08 |
| 5 | 1 | 5.2 | 94.4 | PFVP | 377 | 88 | 12 | 0 | NT | 4.8 | 2.6 | 0.08 |
| | 2 | 5.0 | 94.8 | PFVP | 370 | 112 | 25 | 1 | NT | 4.5 | 2.6 | 0.08 |
| | 3 | 4.9 | 94.8 | PFVP | 199 | 58 | 11 | 1 | NT | 4.0 | 2.5 | 0.08 |
| | 6 | 4.8 | 94.9 | PFVP | 351 | 48 | 5 | | NT | 4.9 | 2.7 | 0.08 |
| | 9 | NT | NT | PFVP | 1047 | 148 | 2 | 0 0 | NT | NT | NT | 0.08 |
| | 12 | 4.9 | 94.8 | PFVP | 229 | 20 | 0 | 0 | 2.2 | 0.08 | 4.9 | 94.8 |

| TABLE 12 | | | | | | | | | | | |
|---|---|---|---|---|---|---|---|---|---|---|---|
| DP formulation screen results at 25 °C/60% RH | | | | | | | | | | | |
| Formulation | Time (months) | Color | Turbidity | pH | Strength (mg/ml) | iclEF | | | SE-HPLC | | |
| | | | | | | (% Acidic) | (% Main Peak) | (% Basic) | (% HMWS) | (% Main Peak) | (% LMWS) |
| 1 | 0.5 | NT | NT | 5.8 | NT | 45.7 | 47.4 | 6.9 | 0.8 | 99.0 | 0.3 |
| | 1 | NT | 0.02 | 5.8 | NT | 46.3 | 47.1 | 6.6 | 0.7 | 99.0 | 0.3 |
| | 2 | NT | -0.01 | 5.8 | NT | 50.1 | 42.1 | 7.8 | 0.7 | 98.9 | 0.4 |
| | 3 | NT | 0.01 | 5.8 | NT | 51.9 | 41.6 | 6.5 | 0.7 | 98.9 | 0.5 |
| | 6 | ≤ B9* | 0.00 | 5.8 | 1.04 | 57.9 | 35.3 | 6.8 | 0.8 | 98.6 | 0.7 |
| 2 | 0.5 | NT | NT | 5.5 | NT | 44.7 | 47.9 | 7.4 | 0.7 | 99.0 | 0.3 |
| | 1 | NT | 0.03 | 5.5 | NT | 45.8 | 47.5 | 6.8 | 0.7 | 98.9 | 0.4 |
| | 2 | NT | 0.02 | 5.5 | NT | 50.3 | 41.9 | 7.7 | 0.8 | 98.9 | 0.4 |
| | 3 | NT | 0.00 | 5.5 | NT | 51.6 | 41.9 | 6.6 | 0.7 | 98.8 | 0.5 |
| | 6 | ≤ B9* | 0.01 | 5.5 | 1.04 | 57.3 | 35.1 | 7.6 | 0.8 | 98.5 | 0.7 |
| 3 | 0.5 | NT | NT | 5.8 | NT | 45.2 | 47.9 | 6.9 | 0.7 | 99.0 | 0.3 |
| | 1 | NT | 0.07 | 5.7 | NT | 46.6 | 47.0 | 6.5 | 0.7 | 99.0 | 0.3 |
| | 2 | NT | 0.00 | 5.8 | NT | 50.3 | 42.2 | 7.5 | 0.8 | 98.9 | 0.4 |
| | 3 | NT | 0.00 | 5.8 | NT | 53.4 | 39.9 | 6.7 | 0.6 | 98.9 | 0.5 |
| | 6 | ≤ B9* | 0.00 | 5.8 | 1.04 | 60.3 | 33.3 | 6.4 | 0.8 | 98.5 | 0.7 |
| 4 | 0.5 | NT | NT | 6.2 | NT | 46.8 | 46.6 | 6.6 | 0.7 | 99.0 | 0.3 |
| | 1 | NT | 0.05 | 6.2 | NT | 47.3 | 46.3 | 6.4 | 0.7 | 98.9 | 0.3 |
| | 2 | NT | 0.01 | 6.2 | NT | 52.5 | 40.3 | 7.2 | 0.7 | 98.9 | 0.4 |
| | 3 | NT | 0.01 | 6.2 | NT | 56.0 | 38.0 | 6.0 | 0.7 | 98.8 | 0.5 |
| | 6 | ≤ B9* | 0.00 | 6.2 | 1.04 | 63.4 | 30.4 | 6.2 | 0.8 | 98.5 | 0.7 |
| 5 | 0.5 | NT | NT | 5.8 | NT | 45.1 | 47.8 | 7.2 | 0.8 | 99.0 | 0.3 |
| | 1 | NT | 0.04 | 5.8 | NT | 47.6 | 45.7 | 6.8 | 0.7 | 98.9 | 0.3 |
| | 2 | NT | -0.01 | 5.8 | NT | 50.2 | 42.6 | 7.2 | 0.8 | 98.9 | 0.4 |
| | 3 | NT | 0.01 | 5.8 | NT | 52.7 | 41.8 | 5.6 | 0.7 | 98.8 | 0.6 |
| | 6 | ≤ B9* | 0.01 | 5.8 | 1.04 | 59.1 | 35.0 | 5.9 | 0.8 | 98.5 | 0.7 |

**TABLE 12, continued**

| Formulation | Time (months) | Non-Reduced CE-SDS | | Visible Particulates | Subvisible Particulates (Cumulative Counts/ml) | | | | Potency (%) | L- Met (mM) | Oxidation Met257 (%) | PS20 (% w/v) |
|---|---|---|---|---|---|---|---|---|---|---|---|---|
| | | Sum of Pre-Peaks (%) | (% Main Peak) | | ≥ 2 µm | ≥ 5 µm | ≥ 10 µm | ≥ 25 µm | | | | |
| 1 | 0.5 | 5.0 | 94.7 | PFVP | 133 | 30 | 5 | 0 | NT | 4.8 | 2.7 | 0.06 |
| | 1 | 5.1 | 94.6 | PFVP | 286 | 48 | 8 | 0 | NT | 4.8 | 2.6 | 0.06 |
| | 2 | 5.1 | 94.7 | PFVP | 675 | 150 | 26 | 0 | NT | 4.5 | 2.7 | 0.06 |
| | 3 | 5.3 | 94.5 | PFVP | 230 | 43 | 7 | 0 | NT | 3.8 | 2.5 | 0.06 |
| | 6 | 5.8 | 94.0 | PFVP | 39 | 6 | 2 | 0 | 88 | 4.6 | 2.7 | 0.06 |
| 2 | 0.5 | 5.0 | 94.7 | PFVP | 42 | 13 | 6 | 2 | NT | 2.4 | 2.8 | 0.08 |
| | 1 | 5.2 | 94.5 | PFVP | 351 | 68 | 9 | 2 | NT | 2.4 | 2.8 | 0.08 |
| | 2 | 5.3 | 94.5 | PFVP | 738 | 163 | 23 | 0 | NT | 2.3 | 2.6 | 0.08 |
| | 3 | 5.4 | 94.3 | PFVP | 366 | 64 | 18 | 0 | NT | 2.0 | 2.6 | 0.08 |
| | 6 | 5.8 | 94.0 | PFVP | 217 | 73 | 8 | 0 | 86 | 2.4 | 2.7 | 0.08 |
| 3 | 0.5 | 4.9 | 94.8 | PFVP | 268 | 89 | 22 | 0 | NT | 2.5 | 2.8 | 0.08 |
| | 1 | 5.1 | 94.6 | PFVP | 45 | 14 | 3 | 0 | NT | 2.4 | 3.0 | 0.08 |
| | 2 | 5.2 | 94.6 | PFVP | 395 | 101 | 23 | 2 | NT | 2.4 | 2.8 | 0.08 |
| | 3 | 5.4 | 94.4 | PFVP | 355 | 80 | 8 | 0 | NT | 1.7 | 2.7 | 0.08 |
| | 6 | 5.8 | 94.0 | PFVP | 409 | 78 | 11 | 0 | 93 | 2.4 | 2.9 | 0.08 |
| 4 | 0.5 | 5.0 | 94.8 | PFVP | 98 | 40 | 6 | 0 | NT | 2.5 | 2.9 | 0.08 |
| | 1 | 5.0 | 94.7 | PFVP | 36 | 8 | 2 | 0 | NT | 2.5 | 2.7 | 0.08 |
| | 2 | 5.1 | 94.7 | PFVP | 653 | 148 | 20 | 0 | NT | 2.3 | 2.6 | 0.08 |
| | 3 | 5.3 | 94.5 | PFVP | 578 | 128 | 23 | 0 | NT | 1.9 | 2.6 | 0.08 |
| | 6 | 5.8 | 94.0 | PFVP | 77 | 18 | 3 | 0 | 79 | 2.4 | 2.9 | 0.08 |
| 5 | 0.5 | 5.0 | 94.7 | PFVP | 243 | 78 | 21 | 1 | NT | 5.2 | 2.7 | 0.08 |
| | 1 | 5.1 | 94.7 | PFVP | 194 | 37 | 3 | 0 | NT | 4.9 | 2.7 | 0.08 |
| | 2 | 5.2 | 94.6 | PFVP | 448 | 118 | 28 | 0 | NT | 4.5 | 2.6 | 0.08 |
| | 3 | 5.4 | 94.4 | PFVP | 333 | 104 | 14 | 0 | NT | 4.1 | 2.6 | 0.08 |
| | 6 | 4.8 | 94.8 | PFVP | 335 | 79 | 12 | 0 | 82 | 4.8 | 2.7 | 0.08 |

| TABLE 13 | | | | | | | | | | |
|---|---|---|---|---|---|---|---|---|---|---|
| DP formulation screen results at 40 °C/75% RH | | | | | | | | | | |
| Formulation | Time (months) | Color | Turbidity | pH | icIEF | | | SE-HPLC | | |
| | | | | | (% Acidic) | (% Main Peak) | (% Basic) | (% HMWS) | (% Main Peak) | (% LMWS) |
| 1 | 0.1 | NT | NT | 5.8 | NT | NT | NT | 0.7 | 99.0 | 0.3 |
| | 0.25 | NT | NT | 5.8 | 47.7 | 45.5 | 6.8 | 0.7 | 99.0 | 0.3 |
| | 0.5 | NT | NT | 5.8 | 52.2 | 41.5 | 6.4 | 0.7 | 98.8 | 0.5 |
| | 0.75 | NT | NT | 5.8 | 56.2 | 37.8 | 6.0 | 0.7 | 98.7 | 0.6 |
| | 1 | ≤ BY7 | 0.01 | 5.8 | 62.3 | 32.7 | 5.0 | 0.8 | 98.5 | 0.7 |
| 2 | 0.1 | NT | NT | 5.5 | NT | NT | NT | 0.7 | 99.0 | 0.3 |
| | 0.25 | NT | NT | 5.5 | 48.3 | 44.2 | 7.4 | 0.8 | 98.9 | 0.3 |
| | 0.5 | NT | NT | 5.5 | 52.1 | 41.1 | 6.8 | 0.8 | 98.8 | 0.5 |
| | 0.75 | NT | NT | 5.5 | 56.5 | 37.2 | 6.4 | 0.8 | 98.6 | 0.6 |
| | 1 | ≤ BY7 | 0.01 | 5.5 | 61.9 | 32.8 | 5.4 | 0.8 | 98.4 | 0.8 |
| 3 | 0.1 | NT | NT | 5.8 | NT | NT | NT | 0.7 | 99.0 | 0.3 |
| | 0.25 | NT | NT | 5.8 | 48.8 | 44.5 | 6.7 | 0.8 | 98.9 | 0.4 |
| | 0.5 | NT | NT | 5.8 | 52.3 | 40.8 | 6.9 | 0.7 | 98.8 | 0.4 |
| | 0.75 | NT | NT | 5.7 | 57.8 | 36.4 | 5.8 | 0.8 | 98.6 | 0.6 |
| | 1 | ≤ BY7 | 0.00 | 5.7 | 63.5 | 31.5 | 4.9 | 0.9 | 98.3 | 0.8 |
| 4 | 0.1 | NT | NT | 6.2 | NT | NT | NT | 0.7 | 99.0 | 0.3 |
| | 0.25 | NT | NT | 6.2 | 50.0 | 43.5 | 6.5 | 0.7 | 98.9 | 0.3 |
| | 0.5 | NT | NT | 6.2 | 54.6 | 39.3 | 6.1 | 0.8 | 98.8 | 0.5 |
| | 0.75 | NT | NT | 6.2 | 59.8 | 34.7 | 5.5 | 0.7 | 98.7 | 0.6 |
| | 1 | ≤ BY7 | 0.01 | 6.2 | 66.0 | 29.3 | 4.6 | 0.7 | 98.5 | 0.8 |
| 5 | 0.1 | NT | NT | 5.8 | NT | NT | NT | 0.7 | 99.0 | 0.3 |
| | 0.25 | NT | NT | 5.8 | 48.9 | 44.3 | 6.8 | 0.8 | 98.9 | 0.4 |
| | 0.5 | NT | NT | 5.8 | 53.1 | 40.4 | 6.6 | 0.8 | 98.8 | 0.5 |
| | 0.75 | NT | NT | 5.8 | 57.4 | 36.9 | 5.7 | 0.8 | 98.7 | 0.6 |
| | 1 | ≤ BY7 | 0.00 | 5.8 | 63.4 | 31.6 | 5.0 | 0.8 | 98.5 | 0.7 |

**TABLE 13, continued**

| Formulation | Time (months) | Non-Reduced CE-SDS | | Visible Particulates | Subvisible Particulates (Cumulative Counts/ml) | | | | Potency (%) | L-Met (mM) | Oxidation Met257 (%) | PS20 (% w/v) |
|---|---|---|---|---|---|---|---|---|---|---|---|---|
| | | Sum of Pre-Peaks (%) | (% Main Peak) | | ≥ 2 µm | ≥ 5 µm | ≥ 10 µm | ≥ 25 µm | | | | |
| 1 | 0.1 | NT | NT | PFVP | NT | NT | NT | NT | NT | NT | NT | 0.06 |
| | 0.25 | 5.1 | 94.7 | PFVP | 38 | 6 | 0 | 0 | NT | 4.8 | 2.6 | 0.06 |
| | 0.5 | 5.4 | 94.3 | PFVP | 344 | 83 | 23 | 0 | NT | 4.9 | 2.7 | 0.06 |
| | 0.75 | 5.8 | 93.9 | PFVP | 26 | 3 | 0 | 0 | NT | 5.0 | 2.7 | 0.06 |
| | 1 | 6.5 | 93.3 | PFVP | 16 | 3 | 1 | 0 | 82 | 5.1 | 2.7 | 0.06 |
| 2 | 0.1 | NT | NT | PFVP | NT | NT | NT | NT | NT | NT | NT | 0.08 |
| | 0.25 | 5.2 | 94.6 | PFVP | 296 | 80 | 12 | 0 | NT | 2.4 | 2.7 | 0.08 |
| | 0.5 | 5.6 | 94.2 | PFVP | 411 | 99 | 25 | 0 | NT | 2.5 | 2.9 | 0.08 |
| | 0.75 | 6.1 | 93.6 | PFVP | 928 | 273 | 38 | 0 | NT | 2.5 | 2.8 | 0.08 |
| | 1 | 6.8 | 92.9 | PFVP | 133 | 55 | 11 | 0 | 90 | 2.4 | 2.8 | 0.08 |
| 3 | 0.1 | NT | NT | PFVP | NT | NT | NT | NT | NT | NT | NT | 0.08 |
| | 0.25 | 5.1 | 94.9 | PFVP | 31 | 8 | 1 | 0 | NT | 2.4 | NR | 0.08 |
| | 0.5 | 5.5 | 94.5 | PFVP | 172 | 46 | 18 | 0 | NT | 2.5 | 2.8 | 0.08 |
| | 0.75 | 6.0 | 94.0 | PFVP | 403 | 93 | 9 | 0 | NT | 2.6 | 2.8 | 0.08 |
| | 1 | 6.7 | 93.4 | PFVP | 167 | 59 | 8 | 0 | 101 | 2.4 | 2.9 | 0.08 |
| 4 | 0.1 | NT | NT | PFVP | NT | NT | NT | NT | NT | NT | NT | 0.08 |
| | 0.25 | 4.9 | 94.9 | PFVP | 123 | 44 | 4 | 0 | NT | 2.5 | 2.8 | 0.08 |
| | 0.5 | 5.3 | 94.5 | PFVP | 246 | 60 | 12 | 2 | NT | 2.5 | 2.9 | 0.08 |
| | 0.75 | 5.8 | 94.0 | PFVP | 489 | 114 | 9 | 0 | NT | 2.5 | 2.7 | 0.08 |
| | 1 | 6.3 | 93.4 | PFVP | 61 | 18 | 1 | 0 | 79 | 2.4 | 2.9 | 0.08 |
| 5 | 0.1 | NT | NT | PFVP | NT | NT | NT | NT | NT | NT | NT | 0.08 |
| | 0.25 | 5.1 | 94.6 | PFVP | 237 | 60 | 8 | 0 | NT | 4.9 | 2.7 | 0.09 |
| | 0.5 | 5.4 | 94.3 | PFVP | 312 | 68 | 20 | 0 | NT | 5.0 | 2.8 | 0.08 |
| | 0.75 | 6.0 | 93.8 | PFVP | 322 | 124 | 27 | 0 | NT | 4.9 | 2.9 | 0.08 |
| | 1 | 6.6 | 93.1 | PFVP | 105 | 36 | 8 | 0 | 99 | 4.8 | 2.8 | 0.08 |

### 3.6 DS Formulation Screen

DS stability was assessed in stainless steel mini-cans. Samples included the target formulation (Formulation 1), Formulation 2, and Formulation 3, having compositions shown in Table 14. All formulations contained 10 mg/ml mosunetuzumab and 0.06% (w/v) PS20. In these examples the molar ratio of surfactant to mosunetuzumab is about 7. Formulations 2 and 3 are high-risk formulations for oxidation at a relatively low pH and high pH, respectively. The risk of accelerated protein oxidation by metal leachables was assessed by incubating DS in surface-worn mini-cans with various fill volumes to quantify the impact of headspace.

| TABLE 14 | | | | |
|---|---|---|---|---|
| DS formulation summary | | | | |
| **Formulation** | **His acetate (mM)** | **pH** | **Sucrose (mM)** | **Methionine (mM)** |
| 1 (Target) | 10 | 5.8 | 240 | 5 |
| 2 | 15 | 5.5 | 160 | 2.5 |
| 3 | 15 | 6.1 | 160 | 2.5 |

Each formulation was sterile-filled and filled into 316L 25 ml stainless steel mini-cans with a 15 ml fill volume. The mini-cans were stored upright on stability at real-time (-20 °C), accelerated (2 °C -8 °C), stressed (25 °C, 60% relative humidity; RH) conditions, and selectively at -40 °C. Mini-cans were stored under frozen conditions underwent no more than three freeze-thaw cycles. In order to control for mini-can variability, two cans were placed on stability for most temperature conditions.

To test for protein oxidation induced by metal leachables, a total of four 45-ml mini-cans that exhibited surface wear (pitting, scratches, etc.) were selected and filled with the low pH formulation (pH 5.5), which was considered high-risk for protein oxidation. These mini-cans were placed on stability at 2-8 °C and 25 °C, 60% RH (two mini-cans at each temperature). The DS fill volumes were varied with a high fill volume (40 ml) and low fill volume (15 ml) in the mini-cans to generate variability in headspace.

Assays were performed to observe pH, oxidation (by peptide mapping), size variations (by SE-HPLC), charge variants (by mCE-SDS and iCIEF), color and appearance, sub-visible particles (by HIAC), visible particles, concentration (by UV-Spec Scan), turbidity, potency, and PS20 concentration (by ELSD). No changes were observed for any formulation tested after storage for 12 months at -40 °C or -20 °C, and no changes were observed for any formulation tested after storage at 5 °C for six months. The high and low fill samples in the surface-worn 45-ml mini-cans showed no difference compared to samples stored in the 25-ml mini-cans at 5 °C.

In summary, mosunetuzumab DS was stable within pH 5.5-6.1, even at the high-risk formulations for oxidation formulation conditions (high His and low Met for oxidation, low sucrose for frozen stability), and when challenging for metal-leachable induced oxidation (low fill volume in surface worn mini-can). Thus, these data show that such compositions (e.g., compositions having high His, low Met, and low sucrose) can also be used with levels of protein up to about 10 mg/ml, as necessary in some DS formulations.

### 3.7 DS formulations confirmatory stability study (additional formulations)

Additional DS formulations were tested to assess whether low histidine and high Met concentrations had an impact on DS stability. Stability had not previously been tested for histidine acetate concentrations less than the targeted 10 mM, or for methionine concentrations greater than 10 mM. Formulations are shown in Table 15, below. All formulations contained 10 mg/ml mosunetuzumab and 0.06% (w/v) PS20. In these formulations the molar ratio of surfactant mosunetuzumab is about 7.

| TABLE 15 | | | | |
|---|---|---|---|---|
| DS formulation confirmatory stability study (additional formulations) | | | | |
| **Formulation** | **His acetate (mM)** | **pH** | **Sucrose (mM)** | **Methionine (mM)** |
| 1 (Target) | 10 | 5.8 | 240 | 10 |
| 2 | 5 | 5.8 | 240 | 15 |

Each formulation was sterile-filtered and filled into 316L 25-ml stainless steel mini-cans with a 16 ml fill volume. The mini-cans were stored upright on stability at accelerated (2-8 °C) and stressed (25 °C/60%RH). In order to control for mini-can variability, two cans were placed on stability.

Assays were conducted to test for pH, oxidation (by peptide mapping), methionine concentration, potency, size variants (by SE-HPLC), charge variants (by mCE-SDS and iCIEF), color and appearance, visible particles, concentration (by UV-Spec Scan), turbidity, and PS20 concentration (ELSD).

No change was observed for any formulation tested after storage for one month at 5 °C. High methionine and low histidine formulations showed no difference compared to target samples. After storage at 25 °C for one month, consistent changes through both DS formulations were observed, with a slight increase in LMWS by SE-HPLC (0.2%) and CE-SDS and a decrease in main peak and increase in acidic peak by iclEF. No change was observed for other assays. This study demonstrated that mosunetuzumab DS was stable within a methionine concentration range of 10-15 mM and a histidine acetate concentration range of 5-10 mM.

### 3.8 DS freeze-thaw stability

Freeze-thaw stability of DS was evaluated by stressing each formulation specified in Table 10 with seven freeze-thaw cycles in mini-cans. The 25 ml 316L stainless steel mini-cans were filled with 16 ml of sterile-filtered material. One mini-can was prepared for each formulation. A freeze-thaw cycle consisted of two hours at -40 °C, followed by at least 8 hours at -20 °C, followed by storage at room temperature until the samples are completely thawed (approximately 2-3 hours). Before sampling, material was mixed by gently inverting the can approximately ten times. Each mini-can was sampled for 2.5 ml aseptically after the third, fourth, fifth, and seventh cycles.

Assays tested for sub-visible particles (HIAC), visible particles, turbidity (UV spec), oxidation (peptide map), and sizing (SE-HPLC). As shown in Table 16, no changes were observed. These data confirmed that the DS was stable through seven freeze-thaw cycles.

| TABLE 16 | | | | | | | | | | | | |
|---|---|---|---|---|---|---|---|---|---|---|---|---|
| Freeze and thaw stability of DS formulation screen samples | | | | | | | | | | | | |
| | Freeze Thaw Cycle | Color | Turbidity | Oxidation (%Met257) | SE-HPLC | | | Visible Particles | Subvisible Particulates (Cumulative Counts/ml) | | | |
| | | | | | (% HMWS) | (% Main Peak) | (% LMWS) | | ≥ 2 µm | ≥ 5 µm | ≥ 10 µm | ≥ 25 µm |
| 1 (Target) | 0 | ≤ BY7 | 0.05 | 2.8 | 1.0 | 98.7 | 0.3 | PFVP | 33 | 8 | 3 | 0 |
| | 3 | ≤ BY7 | 0.07 | NT | 1.0 | 98.7 | 0.3 | PFVP | 19 | 9 | 8 | 2 |
| | 4 | ≤ BY7 | 0.05 | NT | 1.0 | 98.8 | 0.3 | PFVP | 240 | 83 | 40 | 1 |
| | 5 | ≤ BY7 | 0.05 | NT | 1.0 | 98.8 | 0.3 | PFVP | 317 | 90 | 37 | 2 |
| | 7 | ≤ BY7 | 0.03 | 2.8 | 1.0 | 98.8 | 0.3 | PFVP | 543 | 150 | 49 | 2 |
| 2 | 0 | ≤ BY7 | 0.05 | 2.8 | 0.9 | 98.8 | 0.3 | PFVP | 14 | 8 | 8 | 3 |
| | 3 | ≤ BY7 | 0.04 | NT | 1.0 | 98.8 | 0.3 | PFVP | 165 | 50 | 22 | 4 |
| | 4 | ≤ BY7 | 0.07 | NT | 1.0 | 98.7 | 0.3 | PFVP | 353 | 117 | 44 | 2 |
| | 5 | ≤ BY7 | 0.05 | NT | 0.9 | 98.8 | 0.3 | PFVP | 532 | 162 | 73 | 3 |
| | 7 | ≤ BY7 | 0.03 | 2.8 | 1.0 | 98.8 | 0.3 | PFVP | 806 | 252 | 73 | 4 |
| 3 | 0 | ≤ BY7 | 0.05 | 2.8 | 1.0 | 98.7 | 0.3 | PFVP | 23 | 3 | 1 | 0 |
| | 3 | ≤ BY7 | 0.04 | NT | 1.0 | 98.7 | 0.3 | PFVP | 132 | 20 | 3 | 0 |
| | 4 | ≤ BY7 | 0.04 | NT | 1.0 | 98.7 | 0.3 | PFVP | 181 | 55 | 15 | 0 |
| | 5 | ≤ BY7 | 0.02 | NT | 1.0 | 98.7 | 0.3 | PFVP | 403 | 123 | 33 | 1 |
| | 7 | ≤ BY7 | 0.04 | 2.8 | 1.0 | 98.8 | 0.2 | PFVP | 643 | 210 | 67 | 2 |

### 3.9 Donnan Effect

Donnan Effect (an unequal distribution of permeant charged ions on either side of a semipermeable membrane which occurs in the presence of impermeant charged ions) was assessed by measuring pH of in-process samples taken from each diavolume during diafiltration (DF). The pH of DF buffer, conditioning buffer, recovered pool, diluted pool, and conditioned material was also measured. L-histidine concentration was measured in LSPD for select samples using a UPLC analysis for free histidine assay.

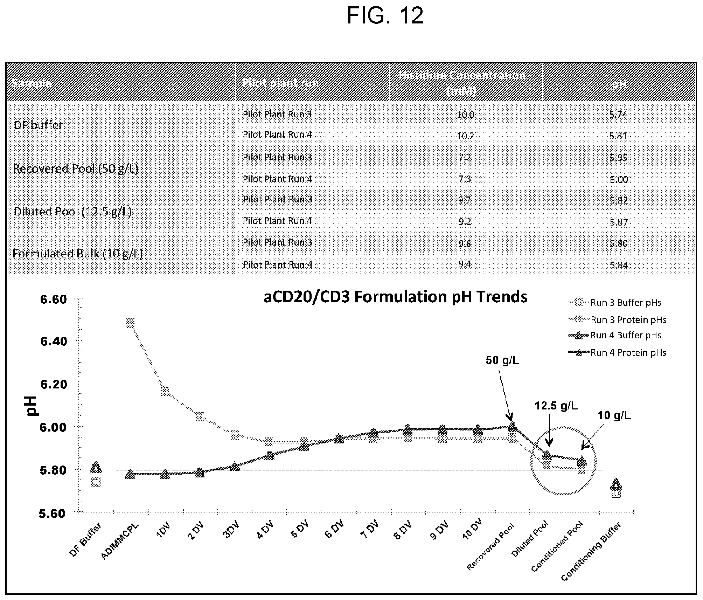

Results of the experiment are summarized in **FIG. 12****.** Donnan Effect was observed with a pH increase of about 0.2 (relative to the DF buffer pH of 5.8) in the recovered pool at a protein centration of about 50 g/L. With a 5× dilution factor after dilution and conditioning to a protein concentration of 10 g/L, the pH returned to the target of pH 5.8. The histidine concentration in the diluted pool and conditioned pool was approximately 10 mM.

### Example 4: Component Selection based on Mosunetuzumab Formulation Development

The formulation development studies described in Example 3 above informed selection of various components (e.g., protein, surfactant, antioxidant, buffer, pH, sucrose, and containers) as described below.

### 4.1. Protein

Protein concentration was selected to deliver the Phase III dose accurately via IV bag without the use of a diluent. The day-one fractionated dose was anticipated to be as low as 0.8-1.0 mg. IV bag shaking studies of Example 3.1 demonstrated that, to deliver such a low dose, a low DP protein concentration is necessary to ensure there is sufficient surfactant in the diluted dose solution to protect the protein against mechanical agitation. Further, a lower protein concentration allows a larger dose volume and, accordingly, more accurate dose delivery. For these reasons, DP protein concentration was selected as 1 mg/ml. The DS concentration was selected as 10 mg/ml to balance a reasonable dilution factor from DS to DP and the facility fit for DS storage.

### 4.2. Surfactant

Three surfactant types were evaluated: PS20, super refined PS20 (srPS20), and P188. SrPS20 was ruled out due to its increased risk of inducing protein oxidation, fragmentation, and aggregation in low protein concentration formulations (Example 3.2). The minimum required level of PS20 and P188 was determined to be 0.05% (w/v) and 0.08% (w/v), respectively from the IV bag agitation study (Example 3.1). Both PS20 and P188 showed no impact on protein quality under thermal stress (Example 3.2). PS20 was selected as the surfactant type, in view of the relatively low concentration required, the low risk of ester bond hydrolysis due to low protein concentration (and, accordingly, low abundance of any lipase impurities), and low risk of surfactant oxidation due to the inclusion of antioxidant. Based on the minimum required level determined from IV bag shaking studies (Example 3.1), 0.06% (w/v) was selected as PS20 target concentration (molar ratio of surfactant to mosunetuzumab of about 71 for formulations containing 1 mg/ml mosunetuzumab, and 0.05-0.07% (w/v) was determined as DS release specification for PS20.

### 4.3. Antioxidant

Oxidation was identified as a major risk for the low protein concentration formulation of mosunetuzumab in the knowledge building study, and L-Met protected the formulation from oxidation. During formulation development, oxidation was considered the major risk for evaluation, and the worst-case formulation for oxidation was challenged under thermal stress and ambient light stress conditions (Example 3.3). These studies indicated that Met is required, whereas NAT does not add additional benefit with respect to light-stress induced oxidation.

Met concentration was optimized in a hydrogen peroxide spiking study with thermal stress (Example 3.4). In this stress model, 5 mM Met was required to fully protect the formulation from protein oxidation. Met-mediated quenching of oxidation immediately after addition of hydrogen peroxide is beneficial for formulation stability, since DS and DP may experience residual hydrogen peroxide challenge during the manufacturing process, given that vaporized hydrogen peroxide is a common sterilization reagent for equipment, such as isolators. To ensure the DS and DP formulation stability against oxidation stress from multiple sources, and considering manufacturing variability of Met concentration, 10 mM was selected as the concentration of Met in the DS and DP formulations.

### 4.4. Buffer

Histidine acetate (His) was used as the buffer species in the formulation 1 and demonstrated capability of buffering the DS and DP formulation at pH 5.5-6.1. Therefore, His was selected as the buffer species for the formulation. The knowledge building study showed His concentration positively impacted acidic peak formation in the range of 10-30 mM. Further studies demonstrated that formulations with 10 mM His are less prone to oxidation compared to those with 30 mM His, in the absence of antioxidant (Example 3.3). His concentrations of 10 mM and lower (e.g., 5 mM) were sufficient to buffer the DS and DP solution at pH 5.5-6.1. The pH was confirmed to be stable on long term real-time, accelerated, and stress stability in formulations containing 10 mM His (Examples 3.5 and 3.6). From the manufacturing perspective, given the pH shift observed in the Donnan Effect evaluation (Example 3.8), His concentration may shift in the recovered pool at 50 g/L. However, given the high dilution factor from recovered pool to diluted pool (12.5 g/L) and DS (10 g/L), the His concentration returned to close to 10 mM. Therefore, the impact of Donnan Effect on the final DS and DP His concentration is minimal. With all these considerations, 10 mM histidine acetate was selected as the target buffer concentration.

### 4.5. pH

Long-term stability of the early stage formulation demonstrated that the target pH 5.8 is adequate for stability. The knowledge building study showed that pH has a small positive impact on acidic peak formation and no impact on aggregation or fragmentation. DP and DS screening studies on target formulation and high-risk oxidation formulations showed that the impact of pH (5.5-6.1) is minimal on real-time, accelerated, and stress stability (Examples 3.5 and 3.6). Based on these results, the formulation pH was selected as pH 5.8.

### 4.6. Sucrose

The early stage formulation included 240 mM sucrose for tonicity to accommodate subcutaneous administration. Knowledge-building studies did not show any significant impact of sucrose concentration on product quality attributes when the high-risk oxidation formulation was excluded from statistical analysis. A relatively low concentration of sucrose (160 mM) was evaluated in the DP and DS screen study, as well as the multiple freeze and thaw study for DS, and showed no impact on stability when compared to target formulation (Examples 3.5, 3.6, and 3.7).

### 4.7. Containers

316L stainless steel mini-cans were used in the DS formulation screen. Multiple freeze-thaw studies demonstrated adequate stability (Examples 3.6 and 3.7). Stainless steel is considered a high-risk container for metal leachable-induced degradation of protein (e.g., compared to HASTELLOY^{®}). These results suggest that the DS formulation is stable in stainless steel and HASTELLOYO. 316L stainless steel and HASTELLOY^{®} mini-cans will be evaluated in the DS representative study to provide additional supportive data for allowing use of either type of container for DS storage.

It is necessary that DP vial configuration supports delivery of a wide range of doses in clinical trials, according to the double step-fractionated dosing scheme, which calls for two low fractional doses (e.g., 1 mg and 2 mg) and one full dose (e.g., 5-20 mg). To best support this range of doses, a 20 ml vial with a 13.5 ml nominal fill was selected.

### Example 5: Glofitamab in silico analysis

RO7082859 / Glofitamab is a T-cell bispecific humanized monoclonal antibody (TCB) that binds to human CD20 on tumor cells and to the human CD3 epsilon subunit (CD3 ε) of the T cell receptor complex (TCR) on T cells. It is comprised of two different heavy chains and two different light chains. Point mutations in the CH3 domain ("Knobs-into-holes") promote the assembly of two different heavy chains. Exchange of the VH and VL domains in the CD3 binding Fab ("CrossMab approach") and point mutations in the CH and CL domains ("charged variants") in the CD20 binding Fabs promote the correct assembly of the two different light chains with the corresponding heavy chains. The "Knobs-into-holes" mutations consist of amino exchanges Y349C, T366S, L368A and Y407V in the heavy chain HC1 and of amino exchanges S354C and T366W in the heavy chain HC2 (Kabat EU index numbering). The "charged variants" mutations consist of amino acid exchanges E123R and Q124K in the light chain LC2 (Kabat numbering) and K147E and K213E in the heavy chains HC1 and HC2 (Kabat EU index numbering).

The binding to human CD20 occurs with high affinity and in a bivalent binding mode, whereas the binding to CD3 ε is monovalent and of low affinity. RO7082859 is a human IgG1 with the Fc region bearing a modification ("PG LALA" mutation) which abrogates its binding in vitro to Fc gamma receptors (FcyR), and prevents FcyR-mediated co-activation of innate immune effector cells, including natural killer (NK) cells, monocytes/macrophages and neutrophils without changes in functional binding to FcRn (neonatal Fc receptor). The "PG LALA" mutations consist of amino acid exchanges P329G, L234A, and L235A in the heavy chain HC1 and in the heavy chain HC2 ("PG LALA", Kabat EU index numbering).

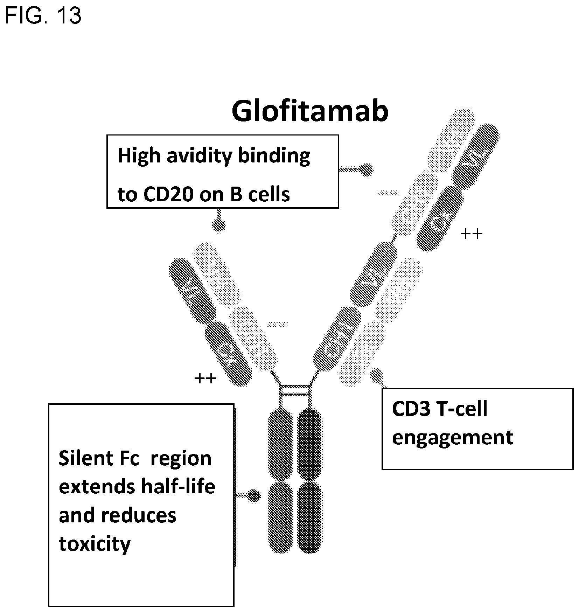

The recombinant antibody is produced in CHO cells and consists of two heavy chains (449 and 674 amino acid residues, respectively) and three light chains (232 and 219 (two copies) amino acid residues, respectively), arranged in an asymmetric configuration as illustrated in **FIG. 13****.**

### Summary active hot spots

For the CD3 binding moiety of the molecule, in silico prediction indicated 2 degradation prone Asn residues and 1 exposed Trp residues in CDR3 of the heavy chain. In a stress experiment over 14 days, no major change in target binding activity was observed after incubation at pH 6.0 but a strong loss of target binding activity was observed after incubation at physiological pH (PBS pH 7.4, data not shown).

### Example 6: Glofitamab Formulation Development GLP Tox and Entry into Human Study

The screen was performed according to the scheme displayed in Table 17. During the screen, the formulations were exposed to the following conditions: 3 and 6-week storage (at 5°C, 25°C and 40°C), shaking at 5°C and 25°C for 1 week and F/T (5 cycles). The nominated formulation is then followed up to 52 weeks.

**Table 17: Adapted platform screen study design with formulation codes**

| **Formulation** | **Protein conc. (mg/ml)** | **Buffer** | **pH** | **Excipient 1** | **Excipient 2** | **Surfactant** |
|---|---|---|---|---|---|---|
| **F1** | 5 | 20 mM His/His-Cl | 5.5 | 240 mM Sucrose | 10 mM Methionine | 0.05 (w/v)% PS20 |
| **F2** | 5 | 20 mM His/His-Cl | 5.5 | 240 mM Sucrose | - | 0.05 (w/v)% PS20 |
| **F3** | 5 | 20 mM His/His-Cl | 5.5 | 240 mM Sucrose | 10 mM Methionine | 0.05 (w/v)% Px188 |
| **F4** | 5 | 20 mM His/His-Cl | 5.5 | 240 mM Sucrose | - | 0.05 (w/v)% Px188 |
| **F5** | 5 | 20 mM His/His-Cl | 6.0 | 240 mM Sucrose | 10 mM Methionine | 0.05 (w/v)% PS20 |

After 6 weeks of storage at 5°C, 25°C, and 40°C, all formulations remained without significant changes in most of the tested physical properties, namely visible and sub-visible particles, color, turbidity, pH, and protein content. CE-SDS data is not shown as it was not critical for the nomination.

Visible particle analysis by the Seidenader method demonstrated no formation of visible particles for either of the formulation at all storage conditions. Subvisible particle count was low (not shown). Under mechanical stress conditions, F2-F5 showed many particles at both 5 and 25°C. F1 was free of particles in both conditions. Using EP and Optima, all compositions were practically free of particles (0 particles) apart from F3 and F4 (both with P188) show particles but below the limit (not shown). Sub-visible particles were significantly worse in F3 (P188 + Met) than in F4 (P188) at 5°C shake, all other formulations have similar counts at each condition (not shown).

Turbidity and color showed no significant changes in all formulations under all conditions after 6 weeks. Surfactant contents were stable at 5 and 25°C, and for both P188 containing formulations (F3, F4) also at 40°C. For all PS20 containing active formulations (F1, F2, and F5), a loss in surfactant content was observed at 40°C, regardless of whether the formulation contained methionine or not.

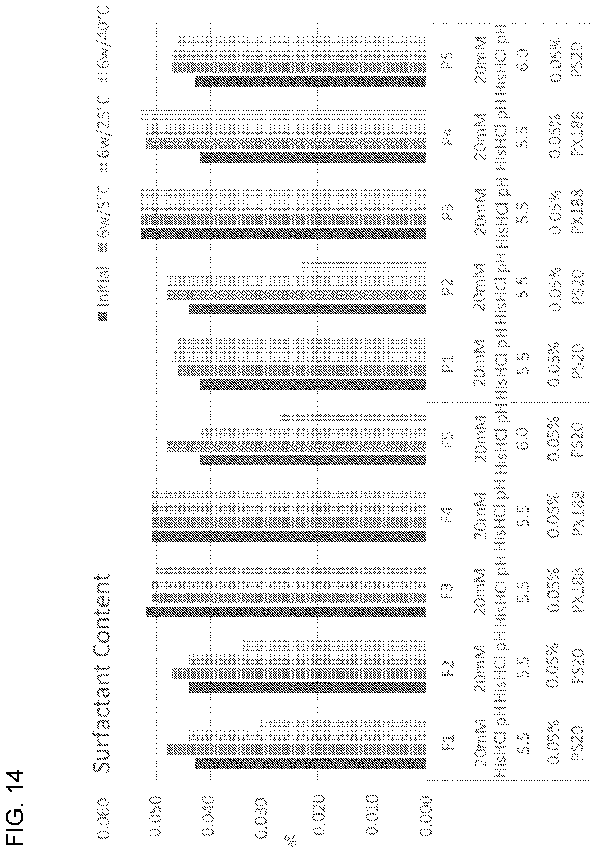

A beneficial effect of methionine could only be seen in the PS20 containing placebo formulations, where only P2 dropped in PS content at 40°C **(****FIG. 14****).** Biochemical characterization revealed differences in formulations only after storage at 40°C.

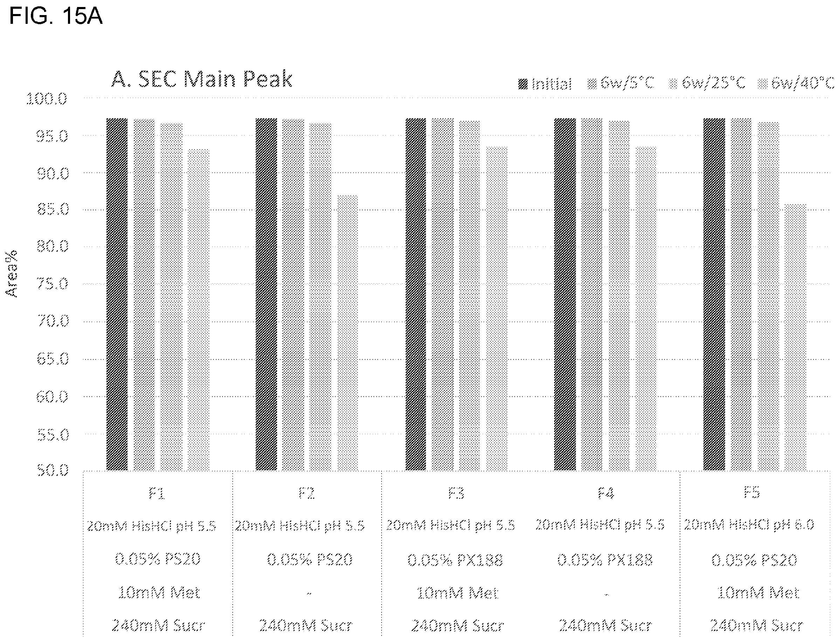

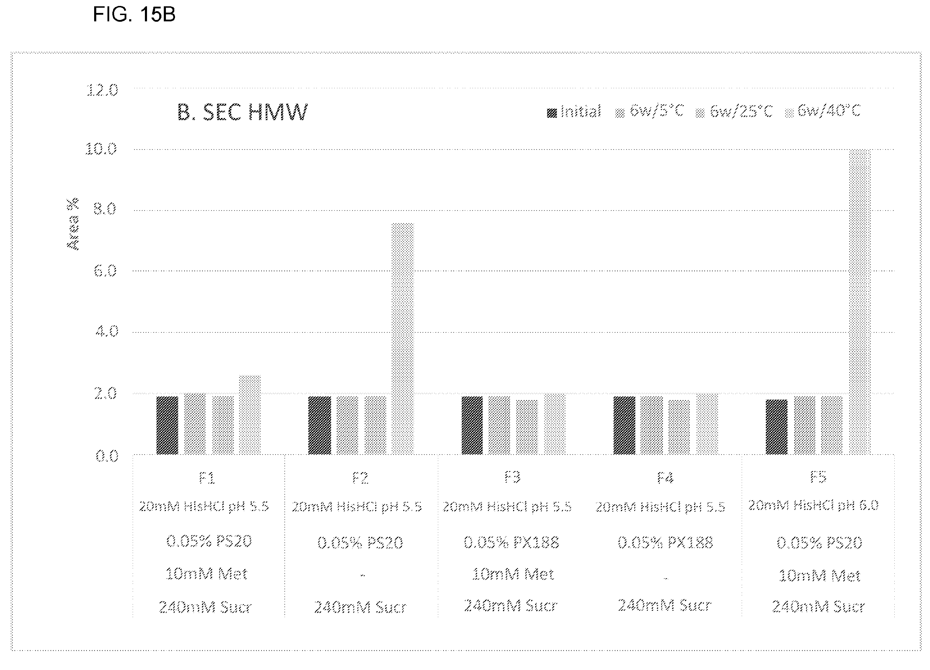

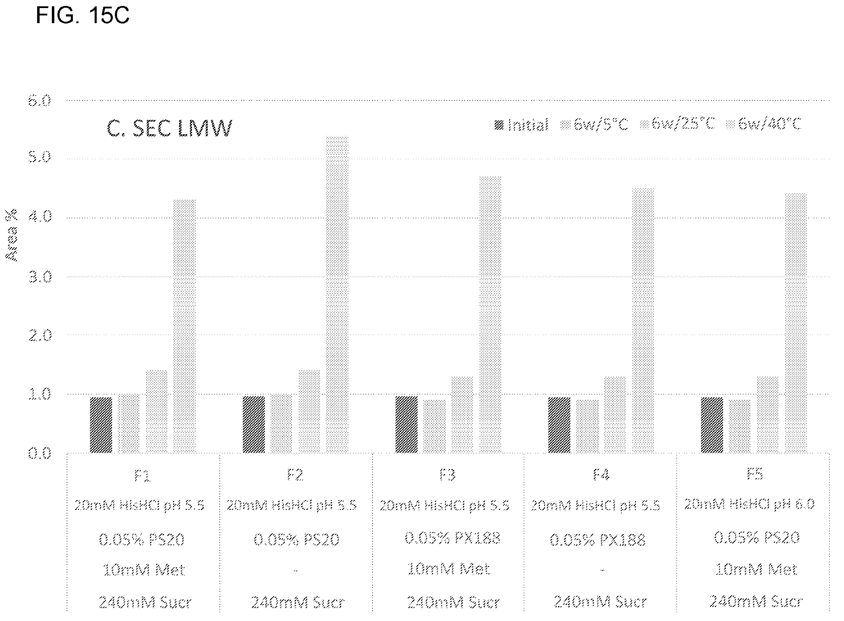

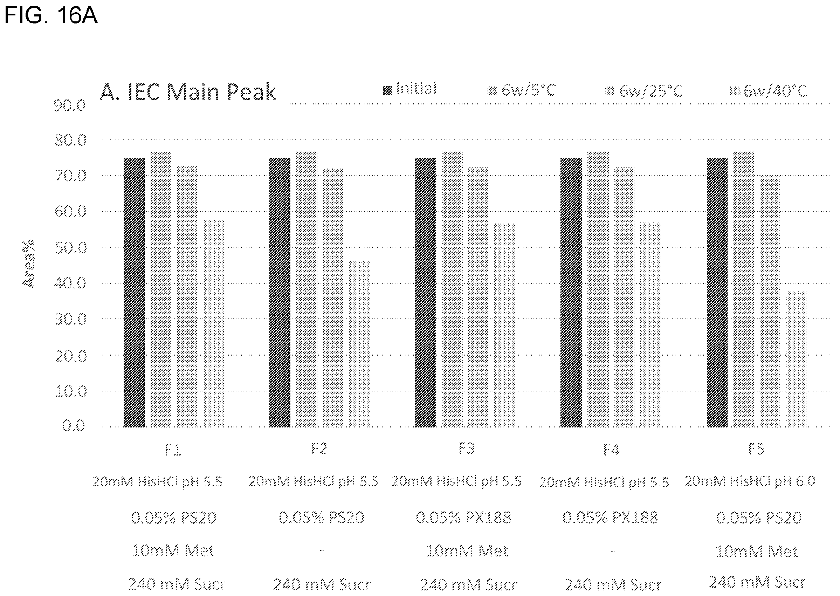

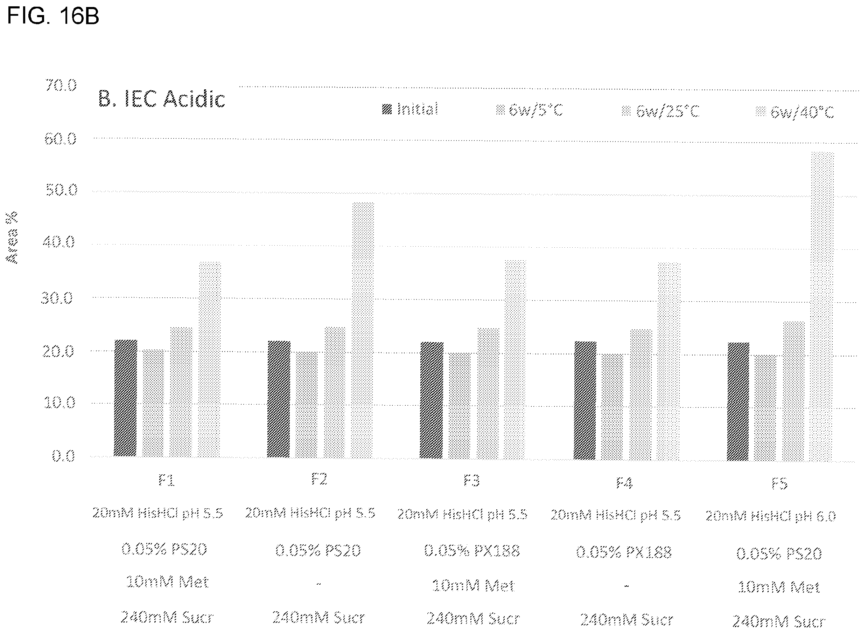

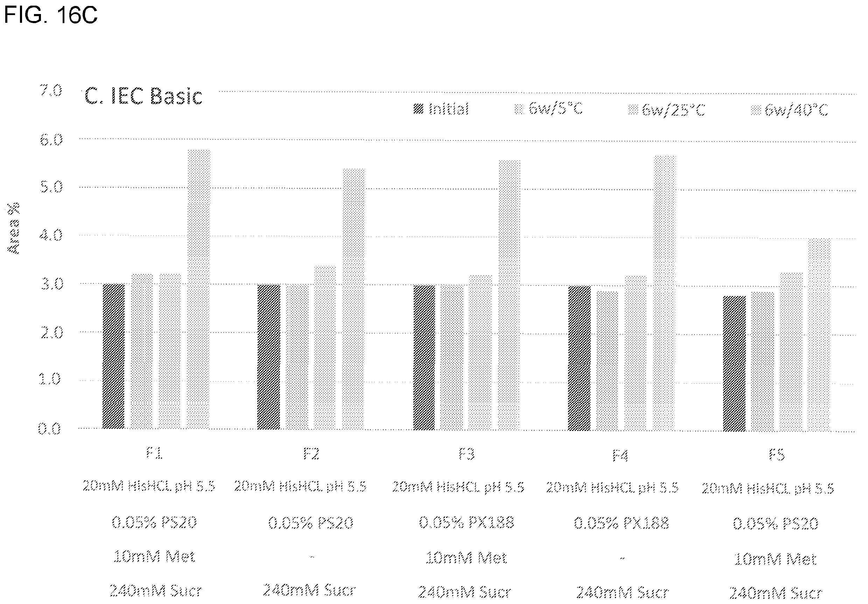

In size exclusion chromatography (SEC), the loss of monomer was more pronounced for F2 and F5, correlating with an increase in HMW area. A new HMW (high molecular weight) species could be seen to emerge, only minor in F3 and F4, stronger in F1, and dominant in F2 and F5. LMW (low molecular weight) species were found to increase in all formulations at approximately the same rate **(****FIG. 15****).** The similar trend could be observed in ion exchange chromatography (IEC), with an overall increase in Basic peak area, and with the increase of Acidic area being more pronounced in F2 and F5 **(****FIG. 16****).**

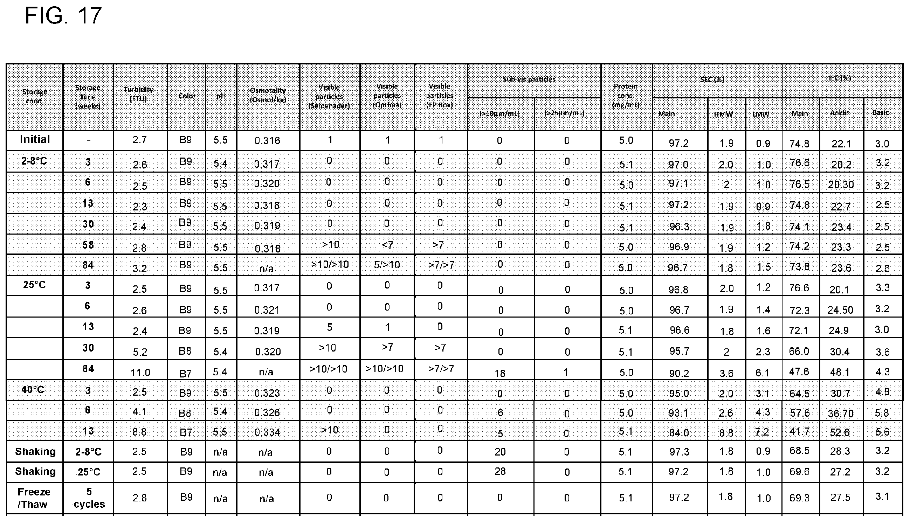

In summary, the data clearly ruled out F2 and F5, and showed F1, F3, and F4 to be equally stable, with no clear preference for any of the three. F1 (20 mM His/His-Cl, pH 5.5, 240 mM Sucrose, 10 mM Methionine, 0.05 (w/v) % PS20) was nominated. A summary of all analytical results for F1 can be found in **FIG. 17****.**

### Example 7: Glofitamab GLP Tox / Entry into Human Study

### Binding by Biacore

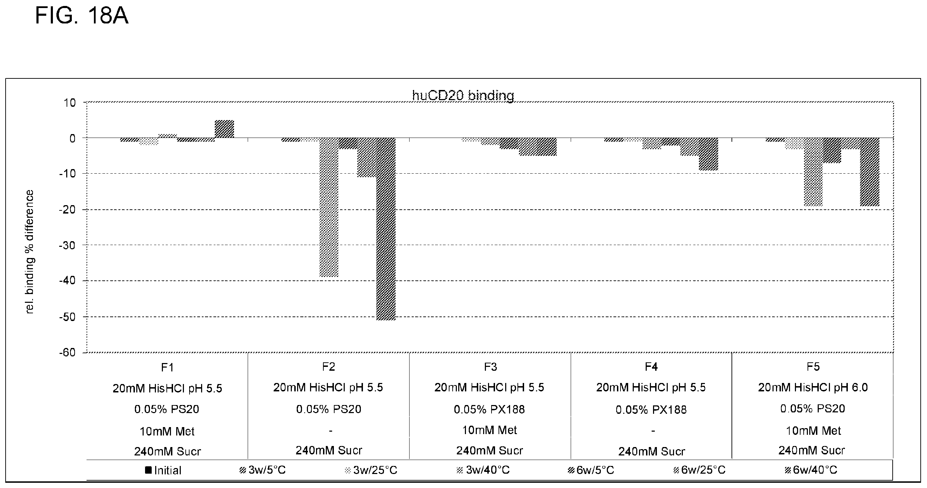

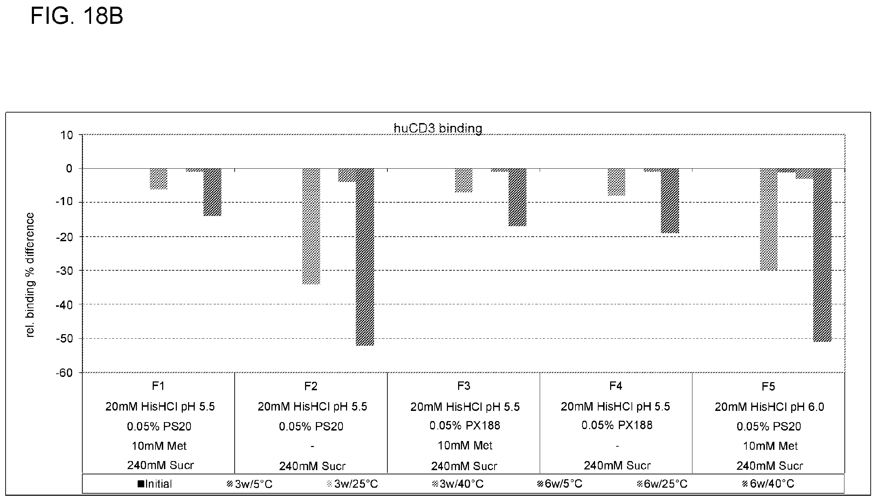

The aforementioned purity results are also reflected in a loss of CD20 binding at 40°C for F2 and F5, and a strong loss of CD3 binding in those formulations of up to 50%, compared to a loss between 10 to 20% for the remaining formulations **(****FIG. 18A** and **FIG. 18B****).**

### Example 8: Development Studies for Phase III and commercial formulation of Glofitamab

This Example provides an overview of the pharmaceutical development of the glofitamab formulation. As a result of this development, glofitamab drug product is provided as a sterile liquid concentrate for solution for IV infusion. The drug product is composed of 1 mg/ml glofitamab in 20 mM L-histidine/ L-histidine hydrochloride (HCl) buffer, 240 mM sucrose, 10 mM L-methionine, 0.5 mg/ml polysorbate 20, pH 5.5. Glofitamab is the only active ingredient in the drug substance and drug product. Formulation development studies established that the dosage form and formulation are suitable for the intended use. The formulation is sufficiently robust to ensure that the drug product is stable during manufacture, storage, transportation, and administration.

Formulations having higher protein concentrations (e.g., 5, 25, or 50 mg/ml glofitamab) were tested, but were not pursued because of sub-visible and visible particle formation due to PS20 degradation. The release of free fatty acids (lauric and myristic acids) at levels increasing together with protein concentration, confirmed the root cause for sub-visible and visible particle formation as being due to hydrolytic PS20 degradation.

A liquid dosage form was selected enabling few handling steps while ensuring product quality during manufacturing and through end of drug product shelf life.

Glofitamab drug product will be commercially available in two strengths provided in two vial configurations: 2.5 mg/vial filled in a 6-ml single-use glass vial and 10 mg/vial filled in a 15-ml single-use glass vial, to match the required clinical doses of 2.5, 10, and 30 mg, while minimizing product wastage. For commercial drug product formulation, the concentration of glofitamab was reduced to 1 mg/ml while keeping the excipient composition unchanged.

Formulation development studies informed the rationale for the selection of the appropriate dosage form, protein concentration, surfactant concentration, buffer species, solution pH, stabilizer, tonicity agent, and vial configuration for the drug product. The drug substance formulation was optimized to account for facility fit, dilution, and storage considerations.

### Selection of Dosage Form

A liquid dosage form was selected to provide a concentrate for solution for infusion requiring few handling steps while ensuring product quality during manufacturing and through end of drug product shelf life.

### Selection of Protein Concentration

A protein concentration of 5 mg/ml was selected for the phase I and retained until phase III. A protein concentration of 1 mg/ml was subsequently selected as commercial formulation based on formulation development studies and updated clinical dosing requirements.

The stability of formulations containing 20 mM L-histidine / L-histidine hydrochloride, 10 mM L-methionine, 240 mM D-sucrose and 0.5 mg/ml polysorbate 20 (PS20), at pH 5.5, was tested at glofitamab concentrations of 1 mg/ml, 5 mg/ml, and 25 mg/ml in order to be prepared to adapt the protein concentration to clinical needs. These formulations were evaluated at the initial time point (T0), at several intermediate time points and at the end of the study after 104 weeks of storage at 2°C-8°C by assessing purity of glofitamab by SE-HPLC and IE-HPLC, PS20 content and visible/subvisible particle formation.

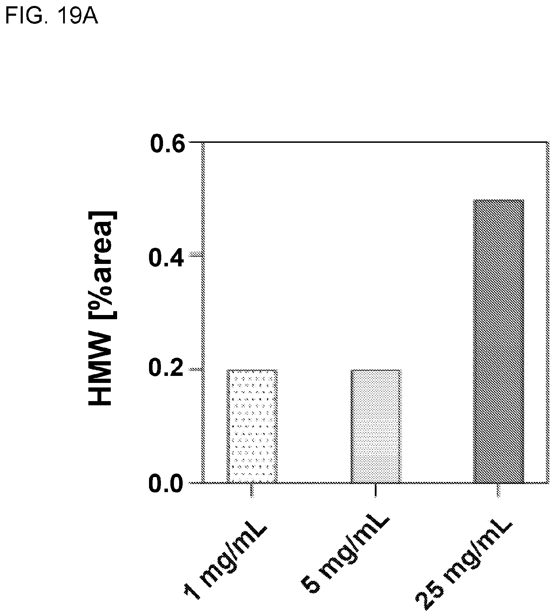

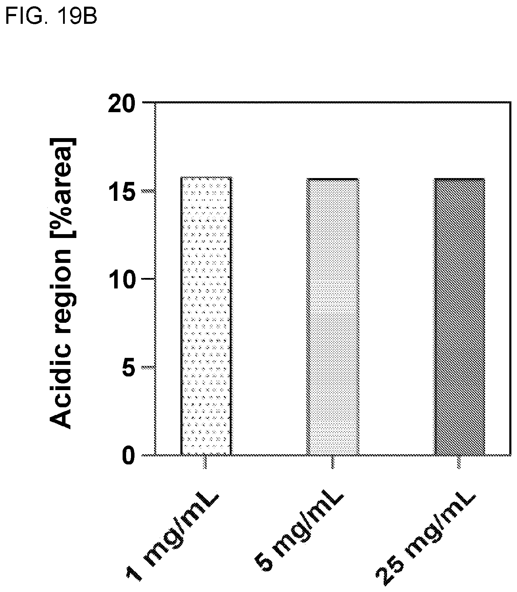

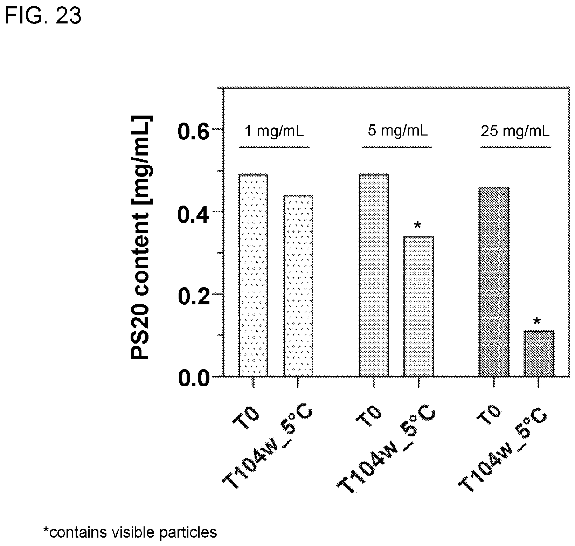

Purity by SE-HPLC and IE-HPLC were comparable between the 1 mg/ml and 5 mg/ml formulations throughout the study **(****FIG. 19A** and **FIG. 19B****).** The subvisible particle counts were also comparable. Furthermore, the 1 mg/ml formulation exhibits no PS20 degradation beyond method variability (FIG. 23, also see below, Assessment of Polysorbate 20 Degradation) compared to the 5 mg/ml and 25 mg/ml formulations. Based on these results and the updated clinical dose regimen of 2.5, 10 and 30 mg, the 1 mg/ml formulation was selected as commercial formulation.

A concentration range of 0.9-1.1 mg/ml protein was further assessed in a subsequent multivariate formulation robustness study (see Example 9, Formulation Robustness Studies). The study confirmed the acceptable stability behavior over this concentration range.

### Selection of pH, Buffer, Stabilizer, and Tonicity Agent

Based on formulation development studies, a 20 mM solution of L-histidine/ L-histidine hydrochloride at pH 5.5 was selected as the buffer in combination with 10 mM L-methionine as stabilizer and 240 mM D-sucrose as tonicity agent for the phase I and retained for the phase III and commercial formulation.

A study at 5 mg/ml glofitamab was set up to test a pH range of 5.5 to 6.0 of a 20 mM L-histidine/L-histidine hydrochloride buffer as well as L-methionine levels of 0 and 10 mM. Additionally, a comparison between 240 mM D-sucrose and 130 mM sodium chloride was performed.

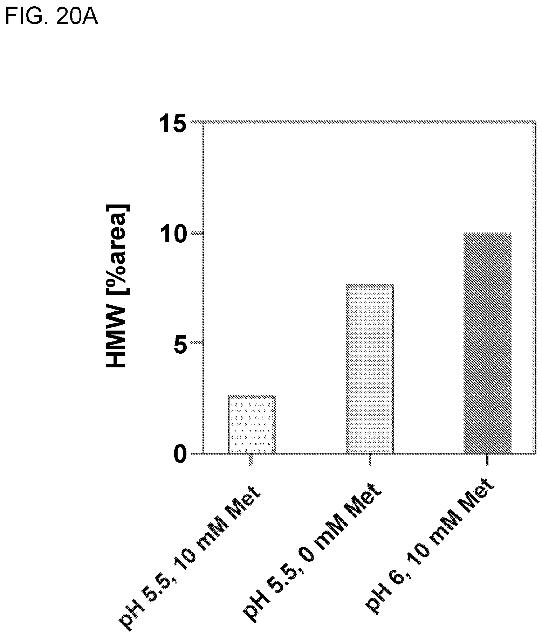

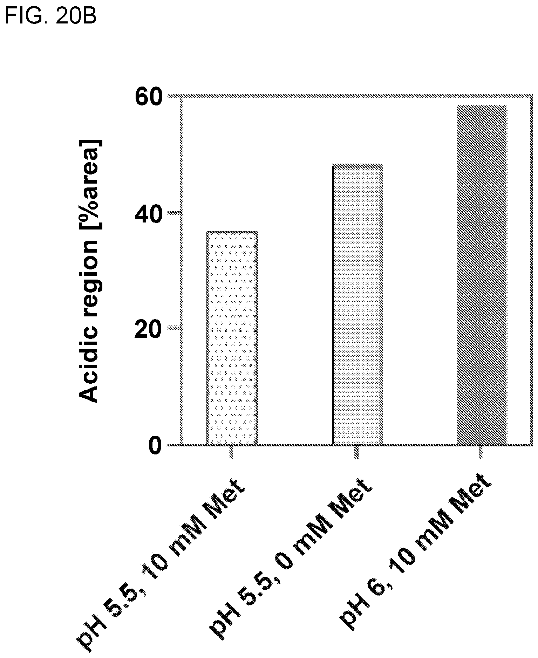

The effect of pH and stabilizer was evaluated at the initial time point (T0) and after 6 weeks of storage at 40°C by assessing purity of glofitamab by SE-HPLC and IE-HPLC, and visible/subvisible particle formation. The choice of tonicity agent was assessed at the initial time point (T0) and after 26 weeks of storage at 25°C by measuring SE-HPLC, IE-HPLC, and determine visible/subvisible particle formation. A 20 mM L-histidine/ L-histidine hydrochloride buffer at pH 5.5 in combination with 10 mM L-methionine showed lowest formation of high molecular weight species (HMWS) **(****FIG. 20A****)** and change in charge variants **(****FIG. 20B****)** compared to the corresponding formulation without stabilizer addition or a 20 mM L-histidine/L-histidine hydrochloride buffer/10 mM L-methionine combination at pH 6. A L histidine/L histidine hydrochloride monohydrate concentration of 20 mM was shown to be sufficient to maintain the formulation pH during the manufacturing of the drug product as well as during storage of the drug substance and drug product.

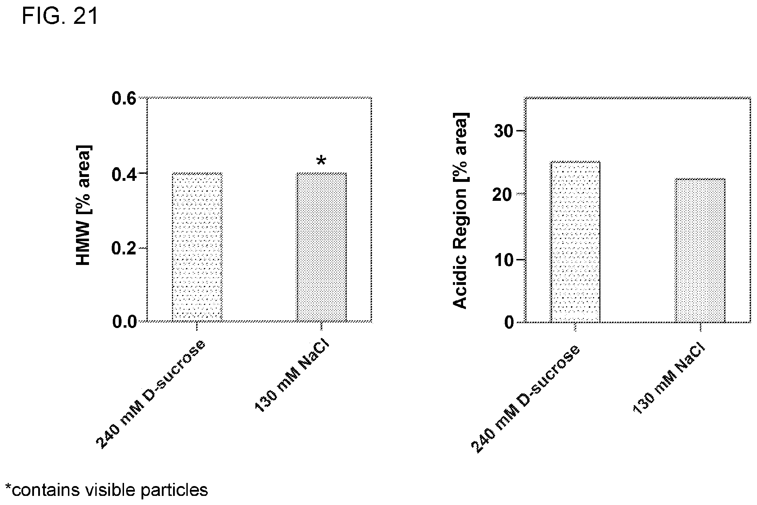

240 mM D-sucrose was chosen based on the comparison between 240 mM D-sucrose and 130 mM sodium chloride. The subvisible particle counts were comparable between the formulations. No visible particle formation was observed after 26 weeks storage at 25 °C for the D-sucrose containing formulation whereas visible particles were observed for the NaCl containing formulation **(****FIG. 21****).**

### Selection of Surfactant

PS20, at a concentration of 0.5 mg/ml, was selected for the phase I and retained until commercial formulation based on the results of the stability studies. A study at 50 mg/ml glofitamab in a 20 mM L-histidine / L-histidine hydrochloride buffer, pH 5.5 with 10 mM L-methionine and 240 mM D-sucrose was set up to investigate the stabilizing effect of poloxamer 188 (P188) versus PS20. P188 was tested at levels of 0.5, 0.7, and 1.0 mg/ml; PS20 at levels of 0.1, 0.3, and 0.5 mg/ml.

The effect of the added surfactant was evaluated at the initial time point (T0) and after 7 days of shaking at 25°C by assessing the purity of glofitamab by SE-HPLC and IE-HPLC, and visible/subvisible particle formation.

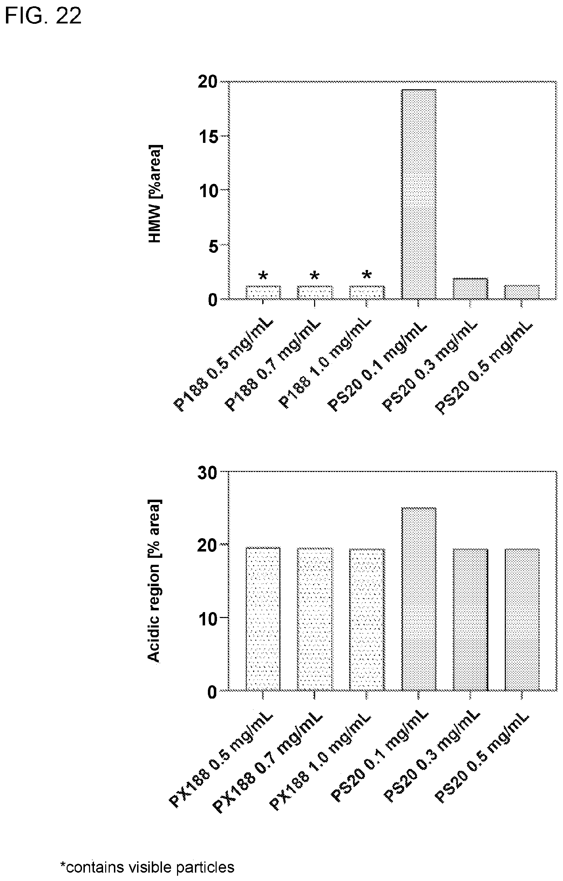

Visible particle formation was observed for all P188 concentrations. It was therefore ruled out as a suitable surfactant for glofitamab **(****FIG. 22****).** No visible particles were detected in the PS20 containing formulations after 7 days of shaking at 25°C **(****FIG. 22****).** For the 0.1 mg/ml PS20 containing formulation a substantial increase in HMWS and charge variants was observed, whereas for the 0.3 mg/ml PS20 containing formulation, a slightly increased level of HMWS and charge variants was observed, compared to the 0.5 mg/ml PS20 containing formulation after 7 days of shaking at 25°C **(****FIG. 22****).** The subvisible particle counts were comparable across the different PS20 concentrations. For the 0.1 mg/mL PS20 containing formulation, a substantial increase in HMWS and charge variants was observed, whereas for the 0.3 mg/mL PS20 containing formulation, a slightly increased level of HMWS and charge variants was observed, compared to the 0.5 mg/mL PS20 containing formulation after 7 days of shaking at 25°C **(****FIG. 22****).** Therefore, the 0.5 mg/ml PS20 containing formulation was selected. A polysorbate 20 level of 0.5 mg/ml was shown to be sufficient to protect glofitamab against stresses that may occur during processing (e.g., agitation, freezing and thawing, or shear stress), handling, storage, and transportation. A concentration range of 0.2-0.8 mg/ml PS20 was further assessed in a subsequent multivariate formulation robustness study (see Example 9, Formulation Robustness Studies). The study confirmed the acceptable stability behavior over this concentration range.

### Example 9: Formulation Robustness Studies for Glofitamab

The composition of the drug substance and the drug product can vary within a range based on manufacturing factors such as weighing tolerances of the buffer components. A multivariate formulation robustness study was performed, and it demonstrated that the relevant quality attributes (QAs) of glofitamab are acceptable at the edges of these composition ranges. A multivariate stability study at two levels was conducted on three factors that had been identified as having a potential impact on critical quality attributes (CQAs) during drug product storage. The following three formulation parameters were assessed:
1. Protein concentration
2. pH
3. PS20 concentration

In addition, three formulation parameters were assessed individually in a univariate stability study:
4. Buffer strength
5. L-methionine concentration
6. D-sucrose concentration

The multivariate formulation robustness study demonstrated that the relevant CQAs of glofitamab are acceptable throughout the entire claimed formulation composition ranges.

### Design of Study

A risk assessment was performed to identify formulation parameters in the drug substance and drug product that are important for maintaining product quality over shelf life. A multivariate study and a univariate study have been set up accordingly.

### Multivariate study (F6 to F12)

A fractional factorial design (resolution III) stability study at two levels was conducted using the three identified formulation parameters protein concentration, pH, and PS20 concentration, as input factors.

### Univariate study (F13 to F20)

L-Methionine and D-sucrose concentration (low and high level), as well as a buffer strength (low and high level) was tested.

One formulation with low protein concentration, low pH and low PS20 concentration was assessed as direct comparison to the corresponding formulation at high pH, high protein concentration and high PS20 concentration.

One formulation with 0.3 mg/ml PS20 concentration was included to support acceptance criteria setting.

The tested formulation parameter ranges are defined to cover either the drug product specification acceptance criteria and/or manufacturing acceptable ranges, as described in Table 18. Table 19 shows the design plan comprising 15 experiments including 3 center points, with the 3 center points corresponding to the target commercial formulation composition.

**Table 18: Formulation Robustness Study: Target Formulation and Multivariate and Univariate Study Range**

| | | Target | Lower Level | Upper Level |
|---|---|---|---|---|
| Glofitamab Concentration (mg/ml) | Mab | 1 | 0.9 | 1.1 |
| L-Histidine/ L-histidine hydrochloride (mM) | His | 20 | 15 | 25 |
| pH | pH | 5.5 | 5.0 | 6.0 |
| PS20 Concentration (mg/ml) | PS20 | 0.5 | 0.2 (0.3) | 0.8 |
| D-sucrose Concentration (mM) | Suc | 240 | 200 | 280 |
| L-Methionine Concentration (mM) | Met | 10 | 5 | 15 |

**Table 19: Formulation Robustness Study Design Plan: Evaluated Glofitamab Formulations**

| Multivariate Study | | | | Tested in the Univariate Study | | |
|---|---|---|---|---|---|---|
| Formulation | Protein Concentration (mg/ml) | pH | PS20 Concentration (mg/ml) | Buffer Strength (mM) | D-Sucrose Concentration (mM) | L-Methionine Concentration (mM) |
| F6 | 0.90 | 5.0 | 0.80 | | | |
| F7 | 1.10 | 5.0 | 0.20 | | | |
| F8 | 0.90 | 6.0 | 0.20 | | | |
| F9 | 1.10 | 6.0 | 0.80 | 20 | 240 | 10 |
| F10 (target) | 1.00 | 5.5 | 0.50 | | | |
| F11 (target) | 1.00 | 5.5 | 0.50 | | | |
| F12 (target) Univariate Study | 1.00 | 5.5 | 0.50 | | | |
| F13 | 0.90 | 5.0 | 0.20 | 20 | 240 | 10 |
| F14 | 1.00 | 5.5 | 0.50 | 20 | 200 | 10 |
| F15 | 1.00 | 5.5 | 0.50 | 20 | 280 | 10 |
| F16 | 1.00 | 5.5 | 0.50 | 20 | 240 | 5 |
| F17 | 1.00 | 5.5 | 0.50 | 20 | 240 | 15 |
| F18 | 1.00 | 5.5 | 0.50 | 15 | 240 | 10 |
| F19 | 1.00 | 5.5 | 0.50 | 25 | 240 | 10 |
| F20 | 1.00 | 5.5 | 0.30 | 20 | 240 | 10 |

The stability of glofitamab in the formulation compositions described in Table 19 was evaluated as:
   - Stability study:
      ∘ Storage conditions: Real time (2°C-8°C), and accelerated (25°C)
      ∘ Testing frequency: 0, 4, 13, 26 (end of 25°C storage), 39, 52, 78 and 104 weeks of storage at the above storage conditions
   - Stress tests:
      ∘ 5 freeze-thaw cycles,
      o Shaking for one week at 2-8°C and shaking for one week at 25°C
   - Stability to support DS: storage at -40°C for 0, 26, 52 and 104 weeks
Assessed QAs:
   o HMWS and Main Peak by SE-HPLC
   ∘ LMWS and Main Peak by non-reduced CE-SDS,
   ∘ Acidic Peak 2 and 3, Acidic Region, Basic Region and Main Peak by IE-HPLC
   o Protein content by ultraviolet-visible spectroscopy
   ∘ Polysorbate 20 content by HPLC-ELSD
   ∘ L-Methionine and L-histidine concentration by RP-HPLC
   ∘ Oxidation, and isomerization by peptide mapping (LC-MS)
   ∘ Potency by bioassay
   ∘ Visible particles
   ∘ Subvisible particles
   ∘ Color, Clarity/Opalescence
   ∘ pH
   ∘ Osmolality
   ∘ Density

### Overall Data Analysis Procedure

Data for all quality attributes was collected over time for each formulation. The relative change of each QA over time was evaluated.

### Multivariate study:

A simple linear regression is fitted for each quality attribute and for each formulation over time. Thus, a degradation rate for each quality attribute and each formulation is calculated. If not mentioned explicitly, degradation rates are reported as degradation per week. These degradation rates are evaluated as responses in a Design of Experiment (DoE) study and the effect of the three parameters, protein concentration, pH, and PS20 concentration, on these degradations was investigated. If a quality attribute showed no meaningful change compared to target formulation over time, regression analysis and effect estimates was not performed. For quality attributes that showed a meaningful change over time, a linear regression was used to estimate the main effects of the three factors on the degradation rates. In addition, main effect plots are shown to illustrate these effects graphically.

### Univariate study:

For the parameters tested in the univariate study, the results after 39 weeks storage at 2°C-8°C were evaluated in comparison to the T0 to identify potential changes. If changes were identified, degradation rates are calculated and compared to the degradation of the target formulation in order to estimate the impact of the investigated formulation parameter at the edges. In some cases, the degradation rate per week was transformed to a degradation observed over 104 weeks by multiplying it with a factor of 104. Regression analysis was performed using JMP software (SAS Institute, Cary, NC, Version 10.0 or higher).

### Stability of Robustness Formulations at Recommended Storage Condition (2°C-8°C):

An overview of the evaluation of the relative change in comparison to the target formulation after 39 weeks storage at 2°C-8°C is provided in Table 20. Increased acidic variant levels (Acidic Region and Acidic Peak 2 by IE-HPLC) were observed for all formulations formulated at pH 6 (F8, F9, F20). The observed increase in acidic variants is reflected by a corresponding decrease of the IE-HPLC Main Peak in the impacted formulations. No changes were observed for all other CQAs across all other formulations after 39 weeks at 2°C-8°C storage. In conclusion, pH was identified as critical formulation parameter. All other formulation parameters, protein content, PS20, L-methionine, and D-sucrose concentration, as well as buffer strengths did not show an impact on the tested CQAs over the investigated range.

### Stability of Robustness Formulations at Accelerated Storage Conditions (25°C):

Comparable to the 2°C-8°C data, increased acidic variant levels due to deamidation (Acidic Region and Acidic Peak 2 by IE-HPLC) were observed for all formulations formulated at pH 6 (F8, F9, F20), which is reflected in a decrease of the IE-HPLC Main Peak in the impacted formulations. Additionally, increased fragmentation levels were observed by an increase in LMWS by CE-SDS for F1 and F2 which are formulated at pH 5. This increase is reflected in a decrease of the CE-SDS Main Peak. No changes were observed for any other CQAs across all other formulations after 26 weeks at 25°C storage.

In conclusion, the 25°C data confirmed that pH is a critical formulation parameter. All other formulation parameters did not show an impact on CQAs.

**Table 20: Relative Change After Storage of 39 Weeks at 2°C-8°C of Relevant CQA**

| | Relative Change in Comparison to Target Formulation | Description in Comparison to Target Formulation |
|---|---|---|
| Purity by SE-HPLC | | |
| Sum of HMWs | No Change | |
| Main Peak | No Change | |
| Purity by NR-CE-SDS | | |
| Sum of LMWS | No Change | |
| Main Peak | No Change | |
| Purity by IE-HPLC | | |
| Acidic Peak 2 | Increase | Increase for all Formulations at pH 6 (F8, F9, F20) |
| Acidic Peak 3 | No Change | |
| Acidic Region | Increase | Increase for all Formulations at pH 6 (F8, F9, F20) |
| Main Peak | Decrease | Decrease for all Formulations at pH 6 (F8, F9, F20) |
| Protein Concentration | No Change | |
| Polysorbate 20 Concentration | No Change | |
| L-Methionine and L-Histidine Concentration by RP-HPLC^{a} | NA | |
| Tryptophan and Methionine Oxidation | No Change | |
| Aspartic Acid Isomerization^{b} | No Change | |
| Potency by Bioassay | No Change | |
| Visible Particles | No Change | |
| Subvisible Particles | No Change | |
| Color, Clarity/Opalescence | No Change | |
| Solution pH | No Change | |
| Osmolality^{a} | NA | |

| | | |
|---|---|---|
| ^{a} Measurement at t=0 and at the end of the study after 104 weeks. ^{b} Measurement at t=0 and after 52 and 104 weeks of storage at 2°C-8°C only. | | |

### Stability of Robustness Formulations at Recommended Drug Substance Storage Condition (-40°C):

To support the stability of the drug substance over the entire claimed formulation composition ranges, a stability study of drug product robustness formulations stored at - 40°C was performed. The study results confirmed that no significant change in the tested quality attributes was observed when formulations were stored at the recommended drug substance storage condition of -40°C for 26 weeks.

### Stability of Robustness Formulations after Shaking and Freeze/Thaw Stress:

Formulations were subjected to one week of shaking at 2°C-8°C or 25°C. Additionally, the formulations were evaluated after undergoing five freeze/thaw cycles between -40°C and 5°C. All samples were practically free of visible particles upon shaking or freeze/thaw stress.

Subvisible particles did not change upon shaking and freeze/thaw stress for all formulations. The formulations with low PS20 content (0.2 mg/ml, F7, F8, F13), did not show any product quality impact after shaking and freeze/thaw stress compared to all other formulations containing levels of 0.3-0.8 mg/ml of PS20.

This result confirms that a level of ≥0.2 mg/ml of polysorbate 20 is sufficient to protect the protein against shaking and freeze/thaw stress. Comparably, the formulation with low D-sucrose content (200 mM, F19) did not show any product quality impact after shaking and freeze/thaw stress compared to all other formulations containing levels of 240-280 mM of D-sucrose. This result confirms that a level of ≥200 mM D-sucrose is sufficient to protect the protein against freeze/thaw stress. No substantial changes to any other quality attributes were observed upon shaking or freeze/thaw stress when compared to control samples.

### Linear Regression Analysis of Identified CQAs Based on Data at Recommended Storage Conditions (2°C-8°C)

A simple linear regression analysis was performed for the impacted CQAs: solution pH, protein concentration and PS20 concentration. pH was identified to have the main impact. The calculated degradation rates per week were extrapolated to end of shelf-life (EoS) by multiplication with 104 weeks ( = 24 months). The extrapolated results are summarized in Table 21.

Linear regression analysis demonstrated that there is no meaningful impact of the tested pH range on the identified CQAs, because all CQAs are within the stability acceptance criteria. However, in order to control the increase in Acidic Region, the pH acceptance criterion at drug product release was tightened to 5.2-5.8.

**Table 21: Results of Linear Regression Analysis Based on 2°C-8°C Data**

| CQA | pH 5^{a} Extrapolated Degradation Rate after 104 weeks | pH 5^{a} Calculated Value at EoS^{b} | pH 6^{a} Extrapolated Degradation Rate after 104 weeks | pH 6^{a} Calculated Value at EoS^{b} | Target (pH 5.5) Extrapolated Degradation Rate after 104 weeks | Target (pH 5.5) calculated value at EoS^{b} |
|---|---|---|---|---|---|---|
| Acidic Peak 2 (area %) | 0.298 | 5.9 | 1.829 | 7.4 | 1.064 | 6.7 |
| Acidic Peak 3 (area %) | 0.147 | 2.8 | 0.481 | 3.1 | 0.314 | 2.9 |
| Acidic Region (area %) | 1.509 | 15.7 | 3.996 | 18.2 | 2.753 | 16.9 |
| LMWS (area %) | 0.574 | 2.4 | 0.134 | 2.0 | 0.354 | 2.2 |

| | | | | | | |
|---|---|---|---|---|---|---|
| ^{a} All other parameters are set to target for the linear regression analysis. ^{b} Calculated by t = 0 + degradation rate after 104 weeks (average t = 0 over all formulations was used). | | | | | | |

### Conclusion

The extrapolated data suggest an impact of a high pH of 6.0 on the level of acidic variants after 24 months (claimed drug product shelf life). Therefore, the pH acceptance criterion at drug product release was tightened to 5.2-5.8 to limit the formation of acidic forms during drug product stability.

The formulation is considered robust until the end of shelf life since:
- CQAs meet the release acceptance criteria at t = 0 and after 9 months of storage at 2°C-8°C for all formulations at the edges of the formulation ranges
- CQAs meet the stability acceptance criteria when using degradation rates for extrapolation to EoS for all formulations at the edges of the formulation ranges.

### Example 10: Assessment of Polysorbate 20 Degradation for Formulations of Glofitamab

Polysorbate 20 can degrade via oxidative or hydrolytic mechanisms. Hydrolytic degradation of polysorbate 20 results in the formation of free fatty acids (FFAs), such as lauric acid. At certain high concentrations, the FFAs may form subvisible or visible particles. Moreover, polysorbate 20 degradation is also a concern if this leads to less polysorbate in the formulation than what is necessary to protect the protein from agitation stress.

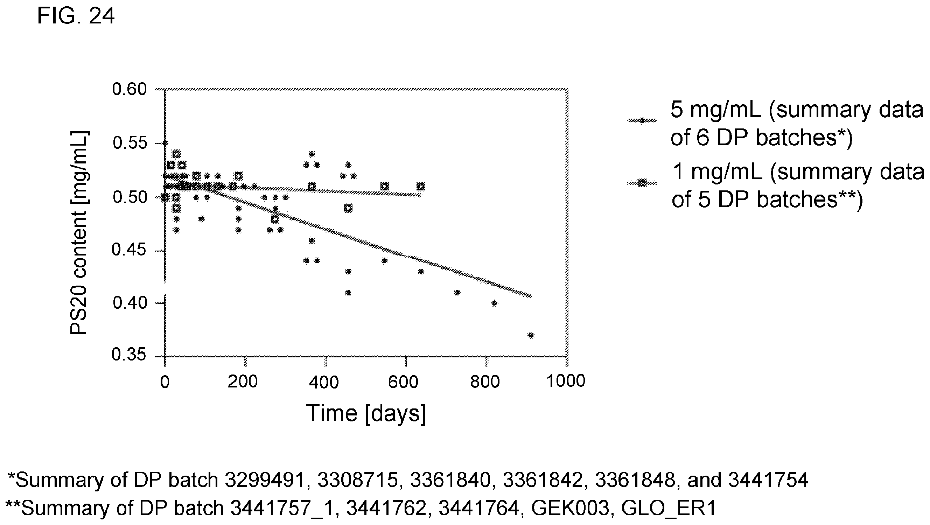

Due to these concerns, polysorbate 20 degradation was monitored during formulation development. PS20 degradation was observed in glofitamab formulations during formulation development with dependence on the protein concentration. Significant PS20 degradation was observed for the 25 mg/ml formulation **(****FIG. 23****)** with observation of visible particles at 2°C-8°C. For the 5 mg/ml formulation, PS20 degradation was less prominent, with observation of visible particles after 20 months. The subvisible particle counts were not impacted. Visible particles were isolated and characterized by fourier transform infrared (FTIR) analysis and were found to be FFA. The 1 mg/ml formulation showed no PS20 degradation (beyond method precision) with absence of visible particle formation throughout the study time of 24 months. The subvisible particle counts were consistently low. Long-term stability data of 9 drug product (DP) batches which were derived from 4 different drug substance (DS) batches confirmed the absence of visible particle. **FIG. 24** provides a visualization of long-term stability data of example DP batches.

### Example 11: Physicochemical In-Use Stability Study for Glofitamab

Glofitamab drug product is provided as a sterile liquid concentrate for solution for IV infusion. The drug product is composed of 1 mg/ml glofitamab in 20 mM L-histidine/ L-histidine hydrochloride buffer, 240 mM sucrose, 10 mM L-methionine, 0.5 mg/ml polysorbate 20, pH 5.5. Glofitamab is a preservative-free drug product supplied in single-dose 2.5-ml and 10-ml glass vials. Glofitamab is intended for IV administration after dilution in 0.9% or 0.45% sodium chloride via IV bag infusion. The proposed registration dose and schedule based on the step-up dosing schedule is 2.5/10/30 mg. The doses are enabled in the IV bag by two dose solution concentrations, 0.1 mg/ml and 0.6 mg/ml. In a bracketing approach, both dose solutions were tested for compatibility to cover the full dose range (Table 22).

Stability and compatibility studies were conducted to confirm the physicochemical stability of the solutions for infusion under recommended in-use conditions. The studies demonstrated that the glofitamab solutions for infusion are stable during typical preparation and administration procedures and may be held for 72 hours at 2°C-8°C and an additional 24 hours at 30°C at ambient room light conditions followed by an infusion at ≤ 25°C taking no longer than 16 hours. The nominal protein concentration range over which the solutions for infusion are demonstrated to be stable is 0.1-0.6 mg/ml.

### Study Materials and Setup:

The physicochemical stability of glofitamab was evaluated after dilution into 100 ml IV bags containing 0.9% sodium chloride solution and 0.45% sodium chloride solution, mimicking the handling procedures to be used in the commercial setting. For each diluent, the product quality of glofitamab was evaluated at diluted concentrations of approximately 0.1 mg/ml (low dose) and 0.6 mg/ml (high dose), which bracket the expected concentration range of the product as outlined in Table 22,

For 0.9% sodium chloride, two different bag types with drug product contact surfaces made of polyvinylchloride (PVC) or polyolefin-polyethylene-polypropylene (PO-PE-PP) were tested. For 0.45% sodium chloride bags with drug product contact surfaces made of PVC were tested. For each diluent, three drug product batches were set up in a matrix approach for this stability assessment. The drug product batches had been stored for 20 months, or 7 months at 2°C-8°C.

**Table 22: Simulated In-Use Study Setup (0.9% Sodium Chloride Solution in PVC or PO-PE-PP IV Bags and 0.45% Sodium Chloride Solution in PVC IV Bags)**

| | Dose | Nominal Protein Concentration in the Bag after Dilution | 0.9% NaCl/ 0.45% NaCl Removed from Bag | Drug Product Injected into Bag | Hold Time | Infusion Volume | Infusion Speed |
|---|---|---|---|---|---|---|---|
| Low Dose | 2.5 mg | 0.1 mg/ml | 10 ml | 10 ml | 2°C-8°C: 72 h | 100 ml | 0.3 mg/h |
| | | | | | 30°C: 24 h | | 3 ml/h |
| High Dose | 30 mg | 0.6 mg/ml | 60 ml | 60 ml | 2°C-8°C: 72 h | 100 ml | 0.72 mg/h |
| | | | | | 30°C: 24 h | | 1.2 ml/h |

Infusion of the dosing solution was simulated by passing the diluted glofitamab solutions through the following:
1. Infusion sets with product-contacting surfaces of PVC, polyethylene (PE), polybutadiene (PBD), polyurethane (PUR), silicone, and acrylonitrile butadiene styrene (ABS) with/without 0.2 µm in-line filters made of polysulfone or polyethersulfone (PES).
2. A three-way stopcock infusion aid made from polycarbonate (PC).
3. Catheters made from polyetherurethane (PEU), or polytetrafluoroethylene (PTFE)

The simulated infusion was performed over a period of 16 hours, which is longer than the intended infusion duration of 4-8 hours to ensure compatibility of the dosing solution during extended contact with the materials of construction of the infusion sets and aids.

Samples were collected for analysis from each IV bag after dilution and after the cumulative hold time, as well as at the end of the simulated infusion.

The samples were tested using appropriate stability-indicating methods including purity by SE-HPLC, IE-HPLC and CE-SDS, content of protein by UV, subvisible particles by light obscuration, color, clarity/opalescence, pH, and potency by bioassay. LMW by CE-SDS was measured for high dose (0.6 mg/ml) only, because at a sample concentration of ≤ 0.1 mg/ml the signal intensity was too low to allow for meaningful interpretation of the data. However the product quality was ensured by the presented potency data.

### Results:

The in-use studies demonstrated that glofitamab is physicochemically stable after dilution into 0.9% or 0.45% sodium chloride solution and after holding for 72 hours at 2°C-8°C and for an additional 24 hours at 30°C at ambient room light conditions, followed by simulated infusion at ≤ 25°C taking no longer than 16 hours.

The drug product batches used in these compatibility studies had previously been stored at the recommended storage temperature (2°C-8°C) for 7-20 months, demonstrating that drug product age does not impact stability during in-use handling and administration.

### Example 12: Microbiological Stability of Glofitamab Formulations

The drug product must be diluted before administration using aseptic technique. Solutions of glofitamab for IV administration are prepared by dilution of the drug product into an infusion bag containing 0.9% sodium chloride or 0.45% sodium chloride. The prepared infusion solution should be used immediately. The drug product does not contain any antimicrobial preservative; therefore, sterility of the solution must be ensured during in-use handling by maintaining appropriate aseptic conditions.

Microbiological challenge studies were performed to evaluate the propensity of the solutions to support microbiological proliferation, in case an accidental contamination was to occur. The proliferation of seven different test microorganisms (listed in USP <51>) at 2°C-8°C for up to 96 hours and at 20°C-25°C for up to 48 hours was assessed. The results met the acceptance criterion of "no growth," when a difference of not more than 0.5 log₁₀ unit higher than the initial value was measured.

### Example 13. Formulations for Cevostamab

### i. Overview

During clinical development, additional formulations and vial configurations of cevostamab are used, as outlined in Tables 23 and 24. Nominal content of formulation components for each vial configuration of cevostamab are provided in Table 23.

**Table 23. Overview of Cevostamab Drug Product Formulation Development**

| Concentration | 20 mg/ml | 3 mg/ml |
|---|---|---|
| Description | 40 mg/vial | 90 mg/vial |
| Cevostamab (mg) | 40 | 90.0 |
| L-Histidine (mg) | 6.21 | 93.0 |
| Glacial Acetic Acid (mg) | 1.56 | 24.0 |
| Sucrose (mg) | 164 | 2466 |
| Polysorbate 20 (mg) | 0.40 | 9.00 |
| L-Methionine (mg) | 1.49 | 44.7 |
| N-Acetyl-DL-Tryptophan (mg) | 0.148 | 2.22 |
| Water for Injection (ml) | QS to 2.00 | QS to 30.0 |
| Primary Packaging Configuration | 6 ml vials | 50 ml vials |

**Table 24. Overview of Cevostamab Drug Product Configurations**

| **Component** | **20 mg/ml Toxicology** | **20 mg/ml Clinical** | **3 mg/ml Clinical** | **3 mg/ml Clinical** |
|---|---|---|---|---|
| Vial | 6 ml Type I Glass | 6 ml Type I Glass | 2 ml Type I Glass | 50 ml Type I Glass |
| Stopper | 20 mm butyl rubber fluororesin laminated, serum-type, USP/Ph. Eur. | 20 mm butyl rubber fluororesin laminated, serum-type, USP/Ph. Eur. | 13 mm butyl rubber fluororesin laminated, serum-type, USP/Ph. Eur. | 20 mm butyl rubber fluororesin laminated, serum-type, USP/Ph. Eur. |
| Cap | 20 mm aluminum seal with plastic flip-off cap | 20 mm aluminum seal with plastic flip-off cap | 13 mm aluminum seal with plastic flip-off cap | 20 mm aluminum seal with plastic flip-off cap |
| Fill Volume | 2 ml | 2 ml | 0.4 ml | 20 ml |

### ii. Components of the drug product

### Drug substance

Cevostamab is the only active ingredient in the drug product. The drug substance manufacturing process, testing procedures, and release criteria used to control the drug substance are given in the corresponding drug substance sections. The drug product cevostamab, polysorbate 20, and methionine concentrations in the drug substance are altered during drug product manufacturing through a dilution step. No incompatibility exists between the excipients in the formulation and the active drug, as demonstrated by the drug substance and drug product stability data.

### Excipients

The 3 mg/ml and 20 mg/ml drug products are formulated with the same buffer and excipients at a target pH of 5.8. The 3 mg/ml drug product is formulated with a greater amount of polysorbate 20 and methionine than in the 20 mg/ml drug product, as described below. The rationale for all formulation excipients is listed below and is the same for both 3 mg/ml and 20 mg/ml formulations.

### L-Histidine/Glacial Acetic Acid [5.8]

Function: Buffer to maintain solution pH at 5.8.

Concentration: 20 mM in drug substance and drug product.

L-histidine provides buffering capacity at target pH 5.8. A L-histidine concentration of 20 mM was shown to be sufficient to maintain the formulation pH through the manufacturing of the drug product, as well as during storage of the drug substance and drug product.

The total concentration of the buffering system (histidine acetate) is 20 mM.

### Sucrose

Function: Tonicity agent.

Concentration: 240 mM in drug substance and drug product.

A sucrose concentration of 240 mM is sufficient to achieve isotonicity and provide stability for the drug substance and drug product.

### Polysorbate 20

Function: Surfactant to prevent losses due to surface adsorption as well as to minimize the potential formation of soluble aggregates and/or insoluble proteinaceous particles.

Concentration: 0.2 mg/ml in drug substance and 1.2 mg/ml in drug product.

A polysorbate 20 level of 0.2 mg/ml in the drug substance and 1.2 mg/ml in the drug product was shown to be sufficient to protect cevostamab against stresses that may occur during processing (e.g., freezing and thawing), handling, and storage and in-use administration.

### L-Methionine

Function: Stabilizer.

Concentration: 5 mM L-methionine in the drug substance and 10mM L-methionine in drug product.

An L-methionine drug substance concentration of 5mM and drug product concentration of 10mM are sufficient to provide stability for the cevostamab drug substance and drug product.

### N-Acetyl-DL-Tryptophan

Function: Antioxidant.

Concentration: 0.3 mM N-acetyl-DL-tryptophan in drug substance and drug product.

An N-acetyl-DL-tryptophan concentration of 0.3 mM is sufficient to provide stability for the cevostamab drug substance and drug product.

### iii. Drug product

### Formulation Development

A single-dose formulation designed as a solution for intravenous (IV) infusion or subcutaneous (SC) injection was developed for the initiation of Phase 1 cevostamab clinical trials. The drug substance and drug product were composed of 50 mg/ml and 20 mg/ml cevostamab, respectively, in 20 mM L-histidine acetate, 240 mM sucrose, 5 mM L-methionine, 0.3 mM N-acetyl-DL-tryptophan, 0.2 mg/ml polysorbate 20, pH 5.8. The protein concentration in drug product differs from that of drug substance due to a dilution step during drug product manufacturing.

A 3 mg/ml drug product formulation was developed to enable delivery of a wider dose range expected during subsequent clinical trials as an IV infusion. This drug product formulation is composed of 3 mg/ml cevostamab in 20 mM L-histidine acetate, 240 mM sucrose, 10 mM L-methionine, 0.3 mM N-acetyl-DL-tryptophan, and 1.2 mg/ml polysorbate 20, pH 5.8. The formulation differs from drug substance due to a dilution step, which alters protein, L-methionine and polysorbate 20 concentrations during dilution to drug product. The drug substance composition was not altered during the development of the 3 mg/ml drug product.

All the excipients and excipient concentrations of the 20 mg/ml and 3 mg/ml formulations are the same, with the exception of the polysorbate 20 and L-methionine. The surfactant concentration was determined based on studies designed to determine stability of diluted drug product into saline-containing IV bags. Based on the results of this study, 1.2 mg/ml polysorbate 20 was found to be sufficient to ensure drug product stability and was therefore selected for the 3 mg/ml drug product formulation. L-methionine was added to the drug product formulation as a stabilizer. Formulation development studies demonstrated that 10mM L-methionine was sufficient to ensure stability of the 3 mg/ml drug product formulation. Formulation studies were performed to demonstrate acceptable stability in the 3 mg/ml cevostamab drug product.

Based on the results from these formulation development studies, a liquid formulation consisting of 3 mg/ml cevostamab in 20 mM histidine acetate, 240 mM sucrose, 10 mM L-methionine, 0.3 mM N-Acetyl-DL-Tryptophan, 1.2 mg/ml polysorbate 20, with a target pH of 5.8 was selected as the drug product formulation.

For the initiation of the clinical studies, cevostamab 40 mg/vial (20 mg/ml) was used. Current patients are transitioned to and new patients begin using the newly developed 1.2 mg/vial and 60 mg/vial drug product.

### Physicochemical and Biological Properties

All characterization testing was performed on the drug substance. Extended characterization of drug product lots are provided in Table 25 below.

### Table 25. Extended Characterization of Cevostamab Drug Product Batches

| Analytical Procedure | Batch 1 | Batch 2 |
|---|---|---|
| Mass Spectrometry-Based Anti-CD3 Homodimer (%) | < 0.5 | < 0.5 |
| T-Cell Activation Assay (%) | < 0.5 | < 0.5 |

The formulation remains stable at the recommended storage conditions of 2°C - 8°C when protected from light.

There was no increase in visible or subvisible particles (≥10 µm and ≥25 µm) at the recommended storage temperature (2°C - 8°C), as shown by the 3 mg/ml drug product representative stability study.

Subvisible particles ≥ 2 µm and ≥ 5 µm in size (in addition to ≥ 10 µm and ≥ 25 µm, which are part of the control system) are monitored using the light-obscuration method through development. These evaluations are conducted as part of extended characterization performed at the time of drug product release and during stability.

### Intravenous (IV)

An in-line filter (0.2 µm) is used for administration of clinical material at this stage of development, as a measure of precaution.

### iv. Manufacturing Process Development

Changes in the drug product manufacturing process are highlighted in Table 26. No changes have been made to the manufacturing process of the drug substance. The drug product manufacturing process for cevostamab is a standard, aseptic manufacturing procedure. For the 40 mg/vial drug product (DP), thawed drug substance is diluted with formulation buffer to 20 mg/ml followed by processing through bioburden reduction and sterile filtration steps. Next, 2 ml of diluted solution is filled into 6-ml glass vials, stoppered, capped, labeled and packaged. For the 1.2 mg/vial and 60 mg/vial DP, thawed drug substance is diluted to 3 mg/ml with a dilution buffer, followed by processing through bioburden reduction and sterile filtration steps. Next, 0.4 ml of diluted solution is filled into 2-ml glass vials or 20 ml diluted solution is filled into 50-ml vials. Vials are then stoppered, capped, labeled, and packaged.

**Table 26. Comparison of the Manufacturing Process of Cevostamab 20 mg/ml DP and 3 mg/ml DP**

| Process Step | Cevostamab Solution for Injection in Vial | Cevostamab Solution for Injection in Vial |
|---|---|---|
| 1 | Thaw the drug substance solution | Thaw the drug substance solution |
| 2 | Prepare buffer solution | Prepare buffer solution |
| 3 | Mix drug substance solution with buffer solution to obtain a bulk drug product solution with a concentration of 20 mg/ml cevostamab | Mix drug substance solution with buffer solution to obtain a bulk drug product solution with a concentration of 3 mg/ml cevostamab |
| 4 | Bioburden reduction filtration and sterile filtration (in-line filtration) | Bioburden reduction filtration and sterile filtration (in-line filtration) |
| 5 | Aseptically fill into 6-ml vials | Aseptically fill into 2-ml or 50-ml vials |
| 6 | Close vials with stoppers | Close vials with stoppers |
| 7 | Cap with aluminum seal | Cap with aluminum seal |

### Example 14: In-Use Formulations for Mosunetuzumab

Mosunetuzumab drug product is provided as a sterile liquid concentrate for solution for IV infusion. The drug product is composed of 1 mg/ml mosunetuzumab in 10 mM histidine acetate buffer, 240 mM sucrose, 10 mM methionine, 0.6% (w/v) polysorbate 20, pH 5.8. Mosunetuzumab is a preservative-free drug product supplied in single-dose 2-ml and 50-ml glass vials. Mosunetuzumab is intended for IV administration after dilution in 0.9% or 0.45% sodium chloride (normal saline solution) via IV bag infusion. The proposed the step-load-base dosing schedule is 112/60/60/30 mg. For example, the following dilutions can be used to deliver the indicated dose using a drug product of 1 mg/ml mosunetuzumab (Table 27):

**Table 27. Dilution of mosunetuzumab formulations (1 mg/ml drug product)**

| **Dose of mosunetuzumab** | **Volume of mosunetuzumab in 0.9% or 0.45% sodium chloride solution** | **Size of infusion bag** | **Final concentration of mosunetuzumab** |
|---|---|---|---|
| 1 mg | 1 ml | 50 ml or 100 ml | 0.02 mg/ml or 0.01 mg/ml |
| 2 mg | 2 ml | 50 ml or 100 ml | 0.04 mg/ml or 0.02 mg/ml |
| 30 mg | 30 ml | 100 ml or 250 ml | 0.3 mg/ml or 0.12 mg/ml |
| 60 mg | 60 ml | 250 ml | 0.24 mg/ml |

### Embodiments

Some embodiments of the technology described herein can be defined according to any of the following numbered embodiments:
1. A pharmaceutical composition comprising a therapeutic protein, polysorbate 20 (PS20), methionine, a buffering agent, and a carrier, wherein the molar ratio of the PS20 to the therapeutic protein is 100 or less, the PS20 is at a concentration from 0.01% to 0.12% weight-by-volume (w/v), the methionine is at a concentration from 1 mM to 50 mM, and the buffering agent is at a concentration from 5 mM to 20 mM.
2. The pharmaceutical composition of embodiment 1, wherein the therapeutic protein is at a concentration of about 10 mg/ml or less.
3. The pharmaceutical composition of embodiment 1 or 2, wherein the concentration of the therapeutic protein is between about 0.1 mg/ml to about 10 mg/ml.
4. The pharmaceutical composition of embodiment 3, wherein the concentration of the therapeutic is about 3 mg/ml or about 1 mg/ml.
5. The pharmaceutical composition of any one of embodiments 1-4, wherein the molar ratio of the PS20 to the therapeutic protein is from 45 to 100.
6. The pharmaceutical composition of embodiment 5, wherein the molar ratio of the PS20 to the therapeutic protein is about 48, about 71, or about 79.
7. The pharmaceutical composition of any one of embodiments 1-6, wherein the pharmaceutical composition is formulated as a drug product (DP).
8. The pharmaceutical composition of any one of embodiments 1-7, wherein the concentration of the methionine is from about 2.5 mM to about 20 mM.
9. The pharmaceutical composition of embodiment 7, wherein the concentration of the methionine is about 10 mM.
10. The pharmaceutical composition of any one of embodiments 1-9, wherein the concentration of the buffering agent is from about 5 mM to about 25 mM.
11. The pharmaceutical composition of embodiment 10, wherein the concentration of the buffering agent is about 10 mM or about 20 mM.
12. The pharmaceutical composition of any one of embodiments 1-11, wherein the buffering agent is a histidine, a phosphate, a succinate, an acetate, or a combination thereof.
13. The pharmaceutical composition of embodiment 12, wherein the buffering agent is histidine.
14. The pharmaceutical composition of embodiment 13, wherein the histidine is histidine acetate or histidine HCl.
15. The pharmaceutical composition of any one of embodiments 10-14, wherein the buffering agent is histidine acetate at a concentration of about 10 mM or about 20 mM.
16. The pharmaceutical composition of any one of embodiments 10-14, wherein the buffering agent is histidine HCl at a concentration of about 20 mM.
17. The pharmaceutical composition of any one of embodiments 1-16, further comprising a tonicity agent.
18. The pharmaceutical composition of embodiment 17, wherein the tonicity agent is a sugar, an amino acid, or a salt.
19. The pharmaceutical composition of embodiment 18, wherein the tonicity agent is a sugar.
20. The pharmaceutical composition of embodiment 19, wherein the sugar is sucrose, glucose, glycerol, or trehalose.
21. The pharmaceutical composition of embodiment 20, wherein the sugar is sucrose.
22. The pharmaceutical composition of any one of embodiments 17-21, wherein the tonicity agent is at a concentration from about 100 mM to about 500 mM.
23. The pharmaceutical composition of embodiment 22, wherein the concentration of the tonicity agent is from about 200 mM to about 300 mM.
24. The pharmaceutical composition of embodiment 23, wherein the concentration of the tonicity agent is about 240 mM.
25. The pharmaceutical composition of any one of embodiments 1-24, wherein the pharmaceutical composition has a pH from about 4.5 to about 8.
26. The pharmaceutical composition of embodiment 25, wherein the pH of the pharmaceutical composition is from about 5.1 to about 6.1.
27. The pharmaceutical composition of embodiment 26, wherein the pH of the pharmaceutical composition is about 5.5 or about 5.8.
28. The pharmaceutical composition of any one of embodiments 1-27, wherein the pharmaceutical composition further comprises an antioxidant.
29. The pharmaceutical composition of embodiment 28, wherein the antioxidant is N-acetyl-DL-tryptophan.
30. The pharmaceutical composition of embodiment 29, wherein the concentration of N-acetyl-DL-tryptophan is between 0.1 and 0.5 mM.
31. The pharmaceutical composition of embodiment 30, wherein the concentration of N-acetyl-DL-tryptophan is about 0.3 mM.
32. The pharmaceutical composition of any one of embodiments 1-31, wherein the therapeutic protein is an antibody.
33. The pharmaceutical composition of embodiment 32, wherein the antibody is a bispecific antibody.
34. The pharmaceutical composition of embodiment 33, wherein the bispecific antibody comprises at least one antigen-binding domain that specifically binds to CD3 and at least one antigen-binding domain that specifically binds to a target antigen.
35. The pharmaceutical composition of embodiment 34, wherein the bispecific antibody has a methionine at position 257 of the Fc region (EU numbering), and wherein oxidation of the methionine at position 257 of the Fc region is less than about 10% over two weeks at 40 °C.
36. The pharmaceutical composition of embodiment 35, wherein the oxidation of methionine at position 257 of the Fc region is no more than about 6% over two weeks at 40 °C.
37. A pharmaceutical composition comprising a bispecific antibody, a surfactant, methionine, and a carrier, wherein the pharmaceutical composition has a pH of about 5.5 or 5.8, and wherein:
   (i) the bispecific antibody comprises at least one antigen-binding domain that specifically binds to CD3 and at least one antigen-binding domain that specifically binds to a target antigen and wherein the bispecific antibody is at a concentration of about 10 mg/ml or less,
   (ii) the surfactant is at a concentration from about 0.05% to about 0.12% w/v, and
   (iii) the methionine is at a concentration of about 10 mM.
38. The pharmaceutical composition of embodiment 37, wherein the molar ratio of the surfactant to the bispecific antibody is 100 or less.
39. The pharmaceutical composition of embodiment 37 or 38, wherein the surfactant is PS20 or poloxamer 188 (P188).
40. The pharmaceutical composition of embodiment 39, wherein the surfactant is PS20 and the concentration of the PS20 is about 0.05%, 0.06%, or about 0.12% w/v.
41. The pharmaceutical composition of embodiment 40, wherein the molar ratio of the PS20 to the bispecific antibody is from about 45 to about 100.
42. The pharmaceutical composition of embodiment 41, wherein the molar ratio of the PS20 to the bispecific antibody is about 48, about 71, or about 79.
43. The pharmaceutical composition of embodiment 39, wherein the surfactant is P188 and the concentration of the P188 is about 0.1% w/v.
44. The pharmaceutical composition of embodiment 43, wherein the molar ratio of the P188 to the bispecific antibody is from about 5 to about 25.
45. The pharmaceutical composition of embodiment 44, wherein the molar ratio of the P188 to the bispecific antibody is about 17.
46. The pharmaceutical composition of any one of embodiments 37-45, wherein the bispecific antibody is at a concentration of between about 0.1 mg/ml to about 5 mg/ml.
47. The pharmaceutical composition of embodiment 46, wherein the bispecific antibody is at a concentration of about 1 mg/ml or about 3 mg/ml.
48. The pharmaceutical composition of any one of embodiments 37-47, wherein the pharmaceutical composition is formulated as a DP.
49. The pharmaceutical composition of any one of embodiments 37-48, wherein the pharmaceutical composition further comprises histidine at a concentration of about 10 mM or about 20 mM.
50. The pharmaceutical composition of embodiment 49, wherein the histidine is histidine acetate or histidine HCl.
51. The pharmaceutical composition of any one of embodiments 37-50, wherein the pharmaceutical composition further comprises sucrose at a concentration of about 240 mM.
52. The pharmaceutical composition of any one of embodiments 37-51, wherein the carrier is water.
53. The pharmaceutical composition of any one of embodiments 32-52, wherein the pharmaceutical composition further comprises an antioxidant.
54. The pharmaceutical composition of embodiment 53, wherein the antioxidant is N-acetyl-DL-tryptophan.
55. The pharmaceutical composition of embodiment 54, wherein the concentration of N-acetyl-DL-tryptophan is between 0.1 and 0.5 mM.
56. The pharmaceutical composition of embodiment 55, wherein the concentration of N-acetyl-DL-tryptophan is about 0.3 mM.
57. The pharmaceutical composition of any one of embodiments 32-56, wherein the bispecific antibody comprises an anti-CD3 arm and an anti-target arm.
58. The pharmaceutical composition of embodiment 57, wherein the anti-target arm is an anti-CD20 arm.
59. The pharmaceutical composition of embodiment 58, wherein the anti-CD3 arm comprises a CD3-binding domain comprising:
   (i) a hypervariable region (HVR)-H1 comprising the amino acid sequence of SEQ ID NO: 9;
   (ii) an HVR-H2 comprising the amino acid sequence of SEQ ID NO: 10;
   (iii) an HVR-H3 comprising the amino acid sequence of SEQ ID NO: 11;
   (iv) an HVR-L1 comprising the amino acid sequence of SEQ ID NO: 12;
   (v) an HVR-L2 comprising the amino acid sequence of SEQ ID NO: 13; and
   (vi) an HVR-L3 comprising the amino acid sequence of SEQ ID NO: 14.
60. The pharmaceutical composition of embodiment 59, wherein the CD3-binding domain comprises (a) a heavy chain variable (VH) domain comprising an amino acid sequence having at least 95% sequence identity to the amino acid sequence of SEQ ID NO: 15; (b) a light chain variable (VL) domain comprising an amino acid sequence having at least 95% sequence identity to the amino acid sequence of SEQ ID NO: 16; or (c) a VH domain as in (a) and a VL domain as in (b).
61. The pharmaceutical composition of embodiment 60, wherein the VH domain of the CD3-binding domain comprises the amino acid sequence of SEQ ID NO: 15 and the VL domain of the CD3-binding domain comprises the amino acid sequence of SEQ ID NO: 16.
62. The pharmaceutical composition of any one of embodiments 58-61, wherein the anti-CD20 arm comprises a CD20-binding domain comprising:
   (i) an HVR-H1 comprising the amino acid sequence of SEQ **ID** NO: 1;
   (ii) an HVR-H2 comprising the amino acid sequence of SEQ ID NO: 2;
   (iii) an HVR-H3 comprising the amino acid sequence of SEQ ID NO: 3;
   (iv) an HVR-L1 comprising the amino acid sequence of SEQ ID NO: 4;
   (v) an HVR-L2 comprising the amino acid sequence of SEQ ID NO: 5; and
   (vi) an HVR-L3 comprising the amino acid sequence of SEQ ID NO: 6.
63. The pharmaceutical composition of embodiment 62, wherein the CD20-binding domain comprises (a) a VH domain comprising an amino acid sequence having at least 95% sequence identity to the amino acid sequence of SEQ ID NO: 7; (b) a VL domain comprising an amino acid sequence having at least 95% sequence identity to the amino acid sequence of SEQ ID NO: 8; or (c) a VH domain as in (a) and a VL domain as in (b).
64. The pharmaceutical composition of embodiment 63, wherein the VH domain of the CD20-binding domain comprises the amino acid sequence of SEQ ID NO: 7 and the VL domain of the CD20-binding domain comprises the amino acid sequence of SEQ ID NO: 8.
65. A pharmaceutical composition comprising a bispecific antibody, PS20, methionine, a buffering agent, and a carrier, wherein the molar ratio of the PS20 to the bispecific antibody is about 100 or less, the PS20 is at a concentration from about 0.01% to about 0.12% w/v, the methionine is at a concentration from 1 mM to 50 mM, and the buffering agent is at a concentration from 5 mM to 20 mM, wherein the bispecific antibody comprises an anti-CD3 arm and an anti-CD20 arm, and wherein:
   (a) the anti-CD3 arm comprises a CD3-binding domain comprising:
      an HVR-H1 comprising the amino acid sequence of SEQ ID NO: 9;
      an HVR-H2 comprising the amino acid sequence of SEQ ID NO: 10;
      an HVR-H3 comprising the amino acid sequence of SEQ ID NO: 11;
      an HVR-L1 comprising the amino acid sequence of SEQ ID NO: 12;
      an HVR-L2 comprising the amino acid sequence of SEQ ID NO: 13; and
      an HVR-L3 comprising the amino acid sequence of SEQ ID NO: 14; and
   (b) the anti-CD20 arm comprises a CD20-binding domain comprising:
      an HVR-H1 comprising the amino acid sequence of SEQ ID NO: 1;
      an HVR-H2 comprising the amino acid sequence of SEQ ID NO: 2;
      an HVR-H3 comprising the amino acid sequence of SEQ ID NO: 3;
      an HVR-L1 comprising the amino acid sequence of SEQ ID NO: 4;
      an HVR-L2 comprising the amino acid sequence of SEQ ID NO: 5; and
      an HVR-L3 comprising the amino acid sequence of SEQ ID NO: 6.
66. The pharmaceutical composition of embodiment 65, wherein
   the CD3-binding domain comprises:
      (a) a VH domain comprising an amino acid sequence having at least 95% sequence identity to the amino acid sequence of SEQ ID NO: 15; (b) a VL domain comprising an amino acid sequence having at least 95% sequence identity to the amino acid sequence of SEQ **ID** NO: 16; or (c) a VH domain as in (a) and a VL domain as in (b);
   and the CD20-binding domain comprises:
      (a) a VH domain comprising an amino acid sequence having at least 95% sequence identity to the amino acid sequence of SEQ ID NO: 7; (b) a VL domain comprising an amino acid sequence having at
   least 95% sequence identity to the amino acid sequence of SEQ ID NO: 8; or (c) a VH domain as in (a) and a VL domain as in (b).
67. The pharmaceutical composition of embodiment 66, wherein:
   (a) the VH domain of the CD3-binding domain comprises the amino acid sequence of SEQ ID NO: 15 and the VL domain of the CD3-binding domain comprises the amino acid sequence of SEQ ID NO: 16, and
   (b) the VH domain of the CD20-binding domain comprises the amino acid sequence of SEQ **ID** NO: 7 and the VL domain of the CD20-binding domain comprises the amino acid sequence of SEQ ID NO: 8.
68. The pharmaceutical composition of embodiment 57, wherein the anti-target arm is an anti-FcRH5 arm.
69. The pharmaceutical composition of embodiment 68, wherein the anti-CD3 arm comprises a CD3-binding domain comprising:
   (i) an HVR-H1 comprising the amino acid sequence of SEQ **ID** NO: 65;
   (ii) an HVR-H2 comprising the amino acid sequence of SEQ **ID** NO: 66;
   (iii) an HVR-H3 comprising the amino acid sequence of SEQ ID NO: 67;
   (iv) an HVR-L1 comprising the amino acid sequence of SEQ ID NO: 68;
   (v) an HVR-L2 comprising the amino acid sequence of SEQ ID NO: 69; and
   (vi) an HVR-L3 comprising the amino acid sequence of SEQ ID NO: 70.
70. The pharmaceutical composition of embodiment 69, wherein the CD3-binding domain comprises (a) a VH domain comprising an amino acid sequence having at least 95% sequence identity to the amino acid sequence of SEQ ID NO: 71; (b) a VL domain comprising an amino acid sequence having at least 95% sequence identity to the amino acid sequence of SEQ **ID** NO: 72; or (c) a VH domain as in (a) and a VL domain as in (b).
71. The pharmaceutical composition of embodiment 70, wherein the VH domain of the CD3-binding domain comprises the amino acid sequence of SEQ ID NO: 71 and the VL domain of the CD3-binding domain comprises the amino acid sequence of SEQ ID NO: 72.
72. The pharmaceutical composition of any one of embodiments 68-71, wherein the anti-FcRH5 arm comprises a FcRH5-binding domain comprising:
   (i) an HVR-H1 comprising the amino acid sequence of SEQ ID NO: 57;
   (ii) an HVR-H2 comprising the amino acid sequence of SEQ ID NO: 58;
   (iii) an HVR-H3 comprising the amino acid sequence of SEQ ID NO: 59;
   (iv) an HVR-L1 comprising the amino acid sequence of SEQ ID NO: 60;
   (v) an HVR-L2 comprising the amino acid sequence of SEQ ID NO: 61; and
   (vi) an HVR-L3 comprising the amino acid sequence of SEQ ID NO: 62.
73. The pharmaceutical composition of embodiment 72, wherein the FcRH5-binding domain comprises (a) a VH domain comprising an amino acid sequence having at least 95% sequence identity to the amino acid sequence of SEQ ID NO: 63; (b) a VL domain comprising an amino acid sequence having at least 95% sequence identity to the amino acid sequence of SEQ ID NO: 64; or (c) a VH domain as in (a) and a VL domain as in (b).
74. The pharmaceutical composition of embodiment 73, wherein the VH domain of the FcRH5-binding domain comprises the amino acid sequence of SEQ ID NO: 63 and the VL domain of the FcRH5-binding domain comprises the amino acid sequence of SEQ ID NO: 64.
75. A pharmaceutical composition comprising a bispecific antibody, PS20, methionine, a buffering agent, and a carrier, wherein the molar ratio of the PS20 to the bispecific antibody is about 100 or less, the PS20 is at a concentration from about 0.01% to about 0.12% w/v, the methionine is at a concentration from 1 mM to 50 mM, and the buffering agent is at a concentration from 5 mM to 20 mM, wherein the bispecific antibody comprises an anti-CD3 arm and an anti-FcRH5 arm, and wherein:
   (a) the anti-CD3 arm comprises a CD3-binding domain comprising:
      an HVR-H1 comprising the amino acid sequence of SEQ ID NO: 65;
      an HVR-H2 comprising the amino acid sequence of SEQ ID NO: 66;
      an HVR-H3 comprising the amino acid sequence of SEQ ID NO: 67;
      an HVR-L1 comprising the amino acid sequence of SEQ ID NO: 68;
      an HVR-L2 comprising the amino acid sequence of SEQ ID NO: 69; and
      an HVR-L3 comprising the amino acid sequence of SEQ ID NO: 70; and
   (b) the anti-FcRH5 arm comprises a FcRH5-binding domain comprising:
      an HVR-H1 comprising the amino acid sequence of SEQ ID NO: 57;
      an HVR-H2 comprising the amino acid sequence of SEQ ID NO: 58;
      an HVR-H3 comprising the amino acid sequence of SEQ ID NO: 59;
      an HVR-L1 comprising the amino acid sequence of SEQ ID NO: 60;
      an HVR-L2 comprising the amino acid sequence of SEQ ID NO: 61 ; and
      an HVR-L3 comprising the amino acid sequence of SEQ ID NO: 62.
76. The pharmaceutical composition of embodiment 75, wherein the CD3-binding domain comprises:
   (a) a VH domain comprising an amino acid sequence having at least 95% sequence identity to the amino acid sequence of SEQ ID NO: 71; (b) a VL domain comprising an amino acid sequence having at least 95% sequence identity to the amino acid sequence of SEQ ID NO: 72; or (c) a VH domain as in (a) and a VL domain as in (b);
   and the FcRH5-binding domain comprises:
      (a) a VH domain comprising an amino acid sequence having at least 95% sequence identity to the amino acid sequence of SEQ ID NO: 63; (b) a VL domain comprising an amino acid sequence having at least 95% sequence identity to the amino acid sequence of SEQ ID NO: 64; or (c) a VH domain as in (a) and a VL domain as in (b).
77. The pharmaceutical composition of embodiment 76, wherein:
   (a) the VH domain of the CD3-binding domain comprises the amino acid sequence of SEQ ID NO: 71 and the VL domain of the CD3-binding domain comprises the amino acid sequence of SEQ ID NO: 72, and
   (b) the VH domain of the FcRH5-binding domain comprises the amino acid sequence of SEQ ID NO: 63 and the VL domain of the FcRH5-binding domain comprises the amino acid sequence of SEQ ID NO: 64.
78. The pharmaceutical composition of embodiment 57, wherein the anti-target arm is an anti-HER2 arm.
79. The pharmaceutical composition of embodiment 78, wherein the anti-CD3 arm comprises a CD3-binding domain comprising:
   (i) an HVR-H1 comprising the amino acid sequence of SEQ **ID** NO: 109;
   (ii) an HVR-H2 comprising the amino acid sequence of SEQ ID NO: 110;
   (iii) an HVR-H3 comprising the amino acid sequence of SEQ ID NO: 111;
   (iv) an HVR-L1 comprising the amino acid sequence of SEQ ID NO: 112;
   (v) an HVR-L2 comprising the amino acid sequence of SEQ ID NO: 113; and
   (vi) an HVR-L3 comprising the amino acid sequence of SEQ **ID** NO: 114.
80. The pharmaceutical composition of embodiment 79, wherein the CD3-binding domain comprises (a) a VH domain comprising an amino acid sequence having at least 95% sequence identity to the amino acid sequence of SEQ ID NO: 115; (b) a VL domain comprising an amino acid sequence having at least 95% sequence identity to the amino acid sequence of SEQ **ID** NO: 116; or (c) a VH domain as in (a) and a VL domain as in (b).
81. The pharmaceutical composition of embodiment 80, wherein the VH domain of the CD3-binding domain comprises the amino acid sequence of SEQ ID NO: 115 and the VL domain of the CD3-binding domain comprises the amino acid sequence of SEQ ID NO: 116.
82. The pharmaceutical composition of any one of embodiments 78-81, wherein the anti-HER2 arm comprises a HER2-binding domain comprising:
   (i) an HVR-H1 comprising the amino acid sequence of SEQ **ID** NO: 93;
   (ii) an HVR-H2 comprising the amino acid sequence of SEQ **ID** NO: 94;
   (iii) an HVR-H3 comprising the amino acid sequence of SEQ ID NO: 95;
   (iv) an HVR-L1 comprising the amino acid sequence of SEQ ID NO: 96;
   (v) an HVR-L2 comprising the amino acid sequence of SEQ ID NO: 97; and
   (vi) an HVR-L3 comprising the amino acid sequence of SEQ ID NO: 98.
83. The pharmaceutical composition of embodiment 82, wherein the HER2-binding domain comprises (a) a VH domain comprising an amino acid sequence having at least 95% sequence identity to the amino acid sequence of SEQ ID NO: 99; (b) a VL domain comprising an amino acid sequence having at least 95% sequence identity to the amino acid sequence of SEQ ID NO: 100; or (c) a VH domain as in (a) and a VL domain as in (b).
84. The pharmaceutical composition of embodiment 83, wherein the VH domain of the HER2-binding domain comprises the amino acid sequence of SEQ ID NO: 99 and the VL domain of the HER2-binding domain comprises the amino acid sequence of SEQ ID NO: 100.
85. A pharmaceutical composition comprising a bispecific antibody, PS20, methionine, a buffering agent, and a carrier, wherein the molar ratio of the PS20 to the bispecific antibody is about 100 or less, the PS20 is at a concentration from about 0.01% to about 0.12% w/v, the methionine is at a concentration from 1 mM to 50 mM, and the buffering agent is at a concentration from 5 mM to 20 mM, wherein the bispecific antibody comprises an anti-CD3 arm and an anti-HER2 arm, and wherein:
   (a) the anti-CD3 arm comprises a CD3-binding domain comprising:
      an HVR-H1 comprising the amino acid sequence of SEQ ID NO: 109;
      an HVR-H2 comprising the amino acid sequence of SEQ ID NO: 110;
      an HVR-H3 comprising the amino acid sequence of SEQ ID NO: 111;
      an HVR-L1 comprising the amino acid sequence of SEQ ID NO: 112;
      an HVR-L2 comprising the amino acid sequence of SEQ ID NO: 113; and
      an HVR-L3 comprising the amino acid sequence of SEQ ID NO: 114; and
   (b) the anti-HER2 arm comprises a HER2-binding domain comprising:
      an HVR-H1 comprising the amino acid sequence of SEQ ID NO: 93;
      an HVR-H2 comprising the amino acid sequence of SEQ ID NO: 94;
      an HVR-H3 comprising the amino acid sequence of SEQ ID NO: 95;
      an HVR-L1 comprising the amino acid sequence of SEQ ID NO: 96;
      an HVR-L2 comprising the amino acid sequence of SEQ ID NO: 97; and
      an HVR-L3 comprising the amino acid sequence of SEQ ID NO: 98.
86. The pharmaceutical composition of embodiment 85, wherein the CD3-binding domain comprises:
   (a) a VH domain comprising an amino acid sequence having at least 95% sequence identity to the amino acid sequence of SEQ ID NO: 115; (b) a VL domain comprising an amino acid sequence having at least 95% sequence identity to the amino acid sequence of SEQ ID NO: 116; or (c) a VH domain as in (a) and a VL domain as in (b);
   and the HER2-binding domain comprises:
      (a) a VH domain comprising an amino acid sequence having at least 95% sequence identity to the amino acid sequence of SEQ ID NO: 99; (b) a VL domain comprising an amino acid sequence having at least 95% sequence identity to the amino acid sequence of SEQ ID NO: 100; or (c) a VH domain as in (a) and a VL domain as in (b).
87. The pharmaceutical composition of embodiment 86, wherein:
   (a) the VH domain of the CD3-binding domain comprises the amino acid sequence of SEQ ID NO: 115 and the VL domain of the CD3-binding domain comprises the amino acid sequence of SEQ ID NO: 116, and
   (b) the VH domain of the HER2-binding domain comprises the amino acid sequence of SEQ ID NO: 99 and the VL domain of the HER2-binding domain comprises the amino acid sequence of SEQ ID NO: 100.
88. The pharmaceutical composition of embodiment 32, wherein the antibody is an IgG antibody, or the pharmaceutical composition of any one of embodiments 33, 34, and 57-87, wherein the bispecific antibody is an IgG antibody.
89. The pharmaceutical composition of embodiment 88, wherein the IgG antibody is an IgG₁ antibody.
90. The pharmaceutical composition of embodiment 88 or 89, wherein the bispecific antibody comprises one or more substitution mutations in the Fc region.
91. The pharmaceutical composition of any one of embodiments 88-90, wherein the bispecific antibody comprises an aglycosylation site mutation.
92. The pharmaceutical composition of embodiment 91, wherein the aglycosylation site mutation is a substitution mutation.
93. The pharmaceutical composition of embodiment 90, wherein the one or more substitution mutations reduces effector function of the bispecific antibody.
94. The pharmaceutical composition of embodiment 80, wherein the one or more substitution mutations is at one or more amino acid residues selected from the group consisting of N297, L234, L235, D265, and P329 (EU numbering).
95. The pharmaceutical composition of embodiment 94, wherein the substitution mutation is at least one selected from the group consisting of N297A, N297G, L234A, L235A, D265A, and P329G (EU numbering).
96. The pharmaceutical composition of embodiment 80, wherein the one or more substitution mutations in the Fc region comprises one or more knob-in-hole mutations.
97. The pharmaceutical composition of any one of embodiments 88-86, wherein the anti-target arm comprises T366W and N297G substitution mutations, and the anti-CD3 arm comprises T366S, L368A, Y407V, and N297G substitution mutations.
98. The pharmaceutical composition of any one of embodiments 58-67 and 88-97, wherein the bispecific antibody is mosunetuzumab.
99. The pharmaceutical composition of any one of embodiments 68-77 and 88-97, wherein the bispecific antibody is cevostamab.
100. The pharmaceutical composition of any one of embodiments 78-97, wherein the bispecific antibody is runimotamab.
101. The pharmaceutical composition of any one of embodiments 34-57, wherein the target antigen is CD20.
102. The pharmaceutical composition of embodiment 101, wherein the at least one antigen-binding domain that specifically binds to CD3 comprises:
   (a) a heavy chain variable region comprising:
      an HVR-H1 comprising the amino acid sequence of SEQ ID NO: 45;
      an HVR-H2 comprising the amino acid sequence of SEQ ID NO: 46; and
      an HVR-H3 comprising the amino acid sequence of SEQ ID NO: 47; and
   (b) a light chain variable region comprising:
      an HVR-L1 comprising the amino acid sequence of SEQ ID NO: 48;
      an HVR-L2 comprising the amino acid sequence of SEQ ID NO: 49; and
   an HVR-L3 comprising the amino acid sequence of SEQ ID NO: 50.
103. The pharmaceutical composition of embodiment 102, wherein the at least one antigen-binding domain that specifically binds to CD3 comprises (a) a VH domain comprising an amino acid sequence having at least 95% sequence identity to the amino acid sequence of SEQ ID NO: 51; (b) a VL domain comprising an amino acid sequence having at least 95% sequence identity to the amino acid sequence of SEQ **ID** NO: 52; or (c) a VH domain as in (a) and a VL domain as in (b).
104. The pharmaceutical composition of embodiment 103, wherein the VH domain of the at least one antigen-binding domain that specifically binds to CD3 comprises the amino acid sequence of SEQ ID NO: 51 and the VL domain of the at least one antigen-binding domain that specifically binds to CD3 comprises the amino acid sequence of SEQ ID NO: 52.
105. The pharmaceutical composition of any one of embodiments 102-104, wherein the at least one antigen-binding domain that specifically binds to CD20 comprises:
   (a) a heavy chain variable region comprising:
      an HVR-H1 comprising the amino acid sequence of SEQ ID NO: 37;
      an HVR-H2 comprising the amino acid sequence of SEQ ID NO: 38; and
      an HVR-H3 comprising the amino acid sequence of SEQ ID NO: 39; and
   (b) a light chain variable region comprising:
      an HVR-L1 comprising the amino acid sequence of SEQ ID NO: 40;
      an HVR-L2 comprising the amino acid sequence of SEQ ID NO: 41 ; and
   an HVR-L3 comprising the amino acid sequence of SEQ ID NO: 42.
106. The pharmaceutical composition of embodiment 105, wherein the at least one antigen-binding domain that specifically binds to CD20 comprises (a) a VH domain comprising an amino acid sequence having at least 95% sequence identity to the amino acid sequence of SEQ **ID** NO: 43; (b) a VL domain comprising an amino acid sequence having at least 95% sequence identity to the amino acid sequence of SEQ ID NO: 44; or (c) a VH domain as in (a) and a VL domain as in (b).
107. The pharmaceutical composition of embodiment 106, wherein the VH domain of the at least one antigen-binding domain that specifically binds to CD20 comprises the amino acid sequence of SEQ ID NO: 43 and the VL domain of the at least one antigen-binding domain that specifically binds to CD3 comprises the amino acid sequence of SEQ ID NO: 44.
108. A pharmaceutical composition comprising a bispecific antibody, PS20, methionine, a buffering agent, and a carrier, wherein the molar ratio of the PS20 to the bispecific antibody is about 100 or less, the PS20 is at a concentration from about 0.01% to about 0.12% w/v, the methionine is at a concentration from 1 mM to 50 mM, and the buffering agent is at a concentration from 5 mM to 20 mM, wherein the bispecific antibody comprises at least one antigen-binding domain that specifically binds to CD3 and at least one antigen-binding domain that specifically binds to CD20, and wherein:
   (a) the at least one antigen-binding domain that specifically binds to CD3 comprises:
      an HVR-H1 comprising the amino acid sequence of SEQ ID NO: 45;
      an HVR-H2 comprising the amino acid sequence of SEQ ID NO: 46;
      an HVR-H3 comprising the amino acid sequence of SEQ ID NO: 47;
      an HVR-L1 comprising the amino acid sequence of SEQ ID NO: 48;
      an HVR-L2 comprising the amino acid sequence of SEQ ID NO: 49; and
      an HVR-L3 comprising the amino acid sequence of SEQ ID NO: 50; and
   (b) the at least one antigen-binding domain that specifically binds to CD20 comprises:
      an HVR-H1 comprising the amino acid sequence of SEQ ID NO: 37;
      an HVR-H2 comprising the amino acid sequence of SEQ ID NO: 38;
      an HVR-H3 comprising the amino acid sequence of SEQ ID NO: 39;
      an HVR-L1 comprising the amino acid sequence of SEQ ID NO: 40;
      an HVR-L2 comprising the amino acid sequence of SEQ ID NO: 41 ; and
      an HVR-L3 comprising the amino acid sequence of SEQ ID NO: 42.
109. The pharmaceutical composition of embodiment 108, wherein:
   the at least one antigen-binding domain that specifically binds to CD3 comprises:
      (a) a VH domain comprising an amino acid sequence having at least 95% sequence identity to the amino acid sequence of SEQ ID NO: 51 ; (b) a VL domain comprising an amino acid sequence having at least 95% sequence identity to the amino acid sequence of SEQ ID NO: 52; or (c) a VH domain as in (a) and a VL domain as in (b);
   and the at least one antigen-binding domain that specifically binds to CD20 comprises:
      (a) a VH domain comprising an amino acid sequence having at least 95% sequence identity to the amino acid sequence of SEQ ID NO: 43; (b) a VL domain comprising an amino acid sequence having at least 95% sequence identity to the amino acid sequence of SEQ ID NO: 44; or (c) a VH domain as in (a) and a VL domain as in (b).
110. The pharmaceutical composition of embodiment 109, wherein:
   (a) the VH domain of the at least one antigen-binding domain that specifically binds to CD3 comprises the amino acid sequence of SEQ ID NO: 51 and the VL domain of at least one antigen-binding domain that specifically binds to CD3 comprises the amino acid sequence of SEQ ID NO: 52, and
   (b) the VH domain of the at least one antigen-binding domain that specifically binds to CD20 comprises the amino acid sequence of SEQ ID NO: 43 and the VL domain of the at least one antigen-binding domain that specifically binds to CD20 comprises the amino acid sequence of SEQ ID NO: 44.
111. The pharmaceutical composition of any one of embodiments 101-110, wherein the bispecific antibody is bivalent for CD20 and monovalent for CD3.
112. The pharmaceutical composition of any one of embodiments 101-110, wherein the bispecific antibody comprises two Fab molecules which specifically bind to CD20 and one Fab molecule which specifically binds to CD3.
113. The pharmaceutical composition of embodiment 112, wherein the Fab molecule which specifically binds to CD3 is a crossover Fab molecule, wherein the variable domains VL and VH of the Fab light chain and the Fab heavy chain are replaced by each other.
114. The pharmaceutical composition of embodiment 112 or 113, wherein in the constant domain CL of the second Fab and third Fab molecule the amino acid at position 124 is substituted by lysine (K) (numbering according to Kabat) and the amino acid at position 123 is substituted by lysine (K) or arginine (R), particularly by arginine (R) (numbering according to Kabat), and wherein in the constant domain CH1 of the second Fab and third Fab molecule the amino acid at position 147 is substituted by glutamic acid (E) (EU numbering) and the amino acid at position 213 is substituted by glutamic acid (E) (EU numbering).
115. The pharmaceutical composition of any one of embodiments 101-114, wherein the bispecific antibody comprises an Fc domain composed of a first and a second subunit capable of stable association.
116. The pharmaceutical composition of any one of embodiments 34-115, wherein the at least one antigen-binding domain that specifically binds to CD3 binds to a human CD3 polypeptide or a cynomolgus monkey (cyno) CD3 polypeptide.
117. The pharmaceutical composition of embodiment 116, wherein the human CD3 polypeptide or the cyno CD3 polypeptide is a human CD3ε polypeptide or a cyno CD3ε polypeptide, respectively.
118. The pharmaceutical composition of embodiment 116, wherein the human CD3 polypeptide or the cyno CD3 polypeptide is a human CD3γ polypeptide or a cyno CD3γ polypeptide, respectively.
119. The pharmaceutical composition of any one of embodiments 33-118, wherein the bispecific antibody is monoclonal.
120. The pharmaceutical composition of any one of embodiments 33-119, wherein the bispecific antibody is human, humanized, or chimeric.
121. The pharmaceutical composition of any one of embodiments 1-120, wherein the pharmaceutical composition is in a unit dosage form.
122. The pharmaceutical composition of embodiment 121, wherein the unit dosage form is a liquid formulation for dilution.
123. The pharmaceutical composition of embodiment 122, wherein the liquid formulation for dilution is supplied in a container having a volume of about 50 ml.
124. The pharmaceutical composition of embodiment 122, wherein the liquid formulation for dilution is supplied in a container having a volume of about 15 ml.
125. The pharmaceutical composition of embodiment 122, wherein the liquid formulation for dilution is supplied in a container having a volume of about 2 ml.
126. The pharmaceutical composition of embodiment 122, wherein the liquid formulation for dilution is supplied in a container having a volume of about 1 ml.
127. The pharmaceutical composition of embodiment 122 or 123, wherein the volume of the liquid formulation for dilution is about 15 ml or about 30 ml.
128. The pharmaceutical composition of embodiment 122 or 124, wherein the volume of the liquid formulation for dilution is about 8 ml or about 15 mL.
129. The pharmaceutical composition of embodiment 122 or 125, wherein the volume of the liquid formulation for dilution is about 0.5 ml or about 0.9 ml.
130. The pharmaceutical composition of embodiment 122 or 126, wherein the volume of the liquid formulation for dilution is about 0.5 ml or about 0.9 ml.
131. The pharmaceutical composition of any one of embodiments 122-130, wherein the liquid formulation is for dilution with a normal saline solution comprising 0.45% or 0.9% (w/v) NaCl.
132. The pharmaceutical composition of any one of embodiments 1-131, wherein the pharmaceutical composition comprises no more than 1,000 particles having a diameter ≥ 2 µm per ml as detected by high accuracy liquid particle counting (HIAC).
133. The pharmaceutical composition of any one of embodiments 1-132, wherein the pharmaceutical composition has a shelf-life of at least 36 months when stored at 5 °C ± 3 °C and protected from light.
134. The pharmaceutical composition of any one of embodiments 1-133, wherein the pharmaceutical composition is stable through one or more freeze-thaw cycles.
135. The pharmaceutical composition of embodiment 134, wherein the pharmaceutical composition is stable through three or more freeze-thaw cycles.
136. The pharmaceutical composition of any one of embodiments 1-135, wherein the pharmaceutical composition is stable for about two weeks or longer at about 25 °C.
137. The pharmaceutical composition of embodiment 136, wherein the pharmaceutical composition is stable for about four weeks or longer at about 25 °C.
138. The pharmaceutical composition of any one of embodiments 1-137, wherein the pharmaceutical composition is stable for about 48 months or longer at -20 °C.
139. The pharmaceutical composition of any one of embodiments 134-138, wherein stability is assessed by size-exclusion high-performance liquid chromatography (SE-HPLC).
140. The pharmaceutical composition of embodiment 139, wherein the pharmaceutical composition is determined to be stable if the pharmaceutical composition maintains a purity that is changed by less than 5% as measured by SE-HPLC.
141. The pharmaceutical composition of any one of embodiments 134-138, wherein stability is assessed by non-reduced capillary electrophoresis sodium dodecyl sulfate (CE-SDS) assay.
142. The pharmaceutical composition of embodiment 141, wherein the pharmaceutical composition is determined to be stable if the pharmaceutical composition maintains a purity that is changed by less than 5% as measured by non-reduced CE-SDS assay.
143. The pharmaceutical composition of embodiment 141 or 142, wherein the non-reduced CE-SDS assay is a microchip CE-SDS (mCE-SDS) assay.
144. The pharmaceutical composition of any one of embodiments 1-143, wherein the pharmaceutical composition has a purity of about 85% or higher as assessed by SE-HPLC.
145. The pharmaceutical composition of embodiment 144, wherein the pharmaceutical composition has a purity of about 90% or higher as assessed by SE-HPLC.
146. The pharmaceutical composition of embodiment 145, wherein the pharmaceutical composition has a purity of about 95% or higher as assessed by SE-HPLC.
147. The pharmaceutical composition of any one of embodiments 144-146, wherein the purity of the pharmaceutical composition as assessed by SE-HPLC is maintained about the same for about 36 months or longer at about 5 °C.
148. The pharmaceutical composition of embodiment 147, wherein the purity of the pharmaceutical composition as assessed by SE-HPLC is maintained about the same for about 42 months or longer at about 5 °C.
149. The pharmaceutical composition of embodiment 148, wherein the purity of the pharmaceutical composition as assessed by SE-HPLC is maintained about the same for about 64 months or longer at about 5 °C.
150. The pharmaceutical composition of any one of embodiments 1-143, wherein the pharmaceutical composition has a purity of about 75% or higher as assessed by non-reduced CE-SDS assay.
151. The pharmaceutical composition of embodiment 150, wherein the pharmaceutical composition has a purity of about 80% or higher as assessed by non-reduced CE-SDS assay.
152. The pharmaceutical composition of embodiment 151, wherein the pharmaceutical composition has a purity of about 85% or higher as assessed by non-reduced CE-SDS assay.
153. The pharmaceutical composition of any one of embodiments 150-152, wherein the purity of the pharmaceutical composition as assessed by non-reduced CE-SDS assay is maintained for about 36 months or longer at about 5 °C.
154. The pharmaceutical composition of embodiment 153, wherein the purity of the pharmaceutical composition as assessed by non-reduced CE-SDS assay is maintained for about 42 months or longer at about 5 °C.
155. The pharmaceutical composition of any one of embodiments 150-154, wherein the non-reduced CE-SDS assay is a microchip CE-SDS (mCE-SDS) assay.
156. The pharmaceutical composition of any one of embodiments 1-155, wherein the pharmaceutical composition is formulated for intravenous administration.
157. The pharmaceutical composition of any one of embodiments 1-156, wherein the pharmaceutical composition does not contain a preservative.
158. The pharmaceutical composition of any one of embodiments 1-157, wherein the pharmaceutical composition is formulated for administration by infusion after dilution with a normal saline solution comprising 0.45% or 0.9% NaCl.
159. The pharmaceutical composition for any one of embodiments 1-157, wherein the pharmaceutical composition is formulated for administration by infusion without dilution.
160. The pharmaceutical composition of any one of embodiments 1-159 for use as a medicament.
161. Use of the pharmaceutical composition of any one of embodiments 1-159 in the manufacture of a medicament for:
   (i) treating or delaying progression of a cell proliferative disorder in a subject in need thereof; or
   (ii) enhancing immune function in a subject having a cell proliferative disorder.
162. The pharmaceutical composition of any one of embodiments 1-159 for use in:
   (i) treating or delaying progression of a cell proliferative disorder in a subject in need thereof; or
   (ii) enhancing immune function in a subject having a cell proliferative disorder.
163. The use or pharmaceutical composition for use of embodiment 161 or 162, wherein the cell proliferative disorder is a cancer.
164. The use or pharmaceutical composition for use of any one of embodiments 160-163, wherein the therapeutic protein is a bispecific antibody formulated to bind to a CD3 molecule located on an immune effector cell and a target molecule located on a target cell other than the immune effector cell.
165. The use or pharmaceutical composition for use of embodiment 164, wherein the bispecific antibody activates the immune effector cell following binding to the CD3 molecule and to the target molecule.
166. The use or pharmaceutical composition for use of embodiment 165, wherein the activated immune effector cell is capable of exerting a cytotoxic effect and/or an apoptotic effect on the target cell.
167. Use of the pharmaceutical composition of any one of embodiments 1-67, 88-98, and 101-159 in the manufacture of a medicament for treating or delaying progression of a cell proliferative disorder, or for enhancing immune function in a subject having a cell proliferative disorder, wherein the cell proliferative disorder is a cancer selected from the group consisting of non-Hodgkin's lymphoma (NHL), chronic lymphoid leukemia (CLL), B cell lymphoma, splenic diffuse red pulp small B cell lymphoma, B cell lymphoma with features intermediate between diffuse large B-cell lymphoma and Burkitt lymphoma, B cell lymphoma with features intermediate between diffuse large B-cell lymphoma and classical Hodgkin lymphoma, diffuse large B cell lymphoma (DLBCL), germinal center B cell-like (GCB) diffuse large B cell lymphoma (DLBCL), activated B cell-like (ABC) DLBCL, primary cutaneous follicle center lymphoma, T-cell/histiocyte rich large B-cell lymphoma, primary DLBCL of the central nervous system, primary cutaneous DLBCL (leg type), Epstein-Barr virus (EBV)-positive DLBCL of the elderly, DLBCL associated with chronic inflammation, primary mediastinal (thymic) large B cell lymphoma, intravascular large B cell lymphoma, ALK-positive large B-cell lymphoma, large B-cell lymphoma arising in HHV8-associated multicentric Castleman disease, B cell leukemia, follicular lymphoma (FL), mantle cell lymphoma (MCL), acute myeloid leukemia (AML), marginal zone lymphoma (MZL), small lymphocytic leukemia (SLL), lymphoplasmacytic lymphoma (LL), Waldenstrom macroglobulinemia (WM), central nervous system lymphoma (CNSL), Burkitt's lymphoma (BL), B cell prolymphocytic leukemia, splenic marginal zone lymphoma, hairy cell leukemia, splenic lymphoma/leukemia, hairy cell leukemia variant, α heavy chain disease, γ heavy chain disease, µ heavy chain disease, plasma cell myeloma, solitary plasmacytoma of bone, extraosseous plasmacytoma, extranodal marginal zone lymphoma of mucosa-associated lymphoid tissue (MALT lymphoma), nodal marginal zone lymphoma, pediatric nodal marginal zone lymphoma, pediatric follicular lymphoma, lymphomatoid granulomatosis, plasmablastic lymphoma, and primary effusion lymphoma.
168. The pharmaceutical composition of any one of embodiments 1-67, 88-98, and 101-159 for use in treating or delaying progression of cell proliferative disorder, or for use in enhancing immune function in a subject having a cell proliferative disorder, wherein the cell proliferative disorder is a cancer selected from the group consisting of non-Hodgkin's lymphoma (NHL), chronic lymphoid leukemia (CLL), B cell lymphoma, splenic diffuse red pulp small B cell lymphoma, B cell lymphoma with features intermediate between diffuse large B-cell lymphoma and Burkitt lymphoma, B cell lymphoma with features intermediate between diffuse large B-cell lymphoma and classical Hodgkin lymphoma, diffuse large B cell lymphoma (DLBCL), germinal center B cell-like (GCB) diffuse large B cell lymphoma (DLBCL), activated B cell-like (ABC) DLBCL, primary cutaneous follicle center lymphoma, T-cell/histiocyte rich large B-cell lymphoma, primary DLBCL of the central nervous system, primary cutaneous DLBCL (leg type), Epstein-Barr virus (EBV)-positive DLBCL of the elderly, DLBCL associated with chronic inflammation, primary mediastinal (thymic) large B cell lymphoma, intravascular large B cell lymphoma, ALK-positive large B-cell lymphoma, large B-cell lymphoma arising in HHV8-associated multicentric Castleman disease, B cell leukemia, follicular lymphoma (FL), mantle cell lymphoma (MCL), acute myeloid leukemia (AML), marginal zone lymphoma (MZL), small lymphocytic leukemia (SLL), lymphoplasmacytic lymphoma (LL), Waldenstrom macroglobulinemia (WM), central nervous system lymphoma (CNSL), Burkitt's lymphoma (BL), B cell prolymphocytic leukemia, splenic marginal zone lymphoma, hairy cell leukemia, splenic lymphoma/leukemia, hairy cell leukemia variant, α heavy chain disease, γ heavy chain disease, µ heavy chain disease, plasma cell myeloma, solitary plasmacytoma of bone, extraosseous plasmacytoma, extranodal marginal zone lymphoma of mucosa-associated lymphoid tissue (MALT lymphoma), nodal marginal zone lymphoma, pediatric nodal marginal zone lymphoma, pediatric follicular lymphoma, lymphomatoid granulomatosis, plasmablastic lymphoma, and primary effusion lymphoma.
169. The use or pharmaceutical composition for use of embodiment 168 or 169, wherein the cancer is DLBCL, GCB DLBCL, ABC DLBCL, FL, MCL, AML, CLL, MZL, SLL, LL, WM, CNSL, or BL.
170. Use of the pharmaceutical composition of any one of embodiments 1-57, 78-97, 100, and 116-159 in the manufacture of a medicament for treating or delaying progression of a cell proliferative disorder, or for enhancing immune function in a subject having a cell proliferative disorder, wherein the cell proliferative disorder is a cancer selected from the group consisting of breast cancer, colorectal cancer, gastric cancer, non-small cell lung cancer, multiple myeloma (MM), renal cancer, prostate cancer, liver cancer, head and neck cancer, melanoma, ovarian cancer, mesothelioma, and glioblastoma.
171. The pharmaceutical composition of any one of embodiments 1-57, 78-97, 100, and 116-159 for use in treating or delaying progression of cell proliferative disorder, or for use in enhancing immune function in a subject having a cell proliferative disorder, wherein the cell proliferative disorder is a cancer selected from the group consisting of breast cancer, colorectal cancer, gastric cancer, non-small cell lung cancer, multiple myeloma (MM), renal cancer, prostate cancer, liver cancer, head and neck cancer, melanoma, ovarian cancer, mesothelioma, and glioblastoma.
172. The use or pharmaceutical composition for use of embodiment 170 or 171, wherein the cancer is a HER2-positive cancer.
173. Use of the pharmaceutical composition of any one of embodiments 1-57, 68-77, 88-97, 99, and 116-159 in the manufacture of a medicament for treating or delaying progression of a cell proliferative disorder, or for enhancing immune function in a subject having a cell proliferative disorder, wherein the cell proliferative disorder is a cancer selected from multiple myeloma (MM), chronic lymphoid leukemia (CLL), mantle cell lymphoma (MCL), diffuse large B-cell lymphoma (DLBCL), follicular lymphoma (FL), acute myeloid leukemia (AML), myelodysplastic syndrome (MDS), chronic myelogenous leukemia (CML), chronic myelomonocytic leukemia, acute promyelocytic leukemia (APL), chronic myeloproliferative disorder, thrombocytic leukemia, precursor B-cell acute lymphoblastic leukemia (pre-B-ALL), precursor T cell acute lymphoblastic leukemia (pre-T-ALL), mast cell disease, mast cell leukemia, mast cell sarcoma, myeloid sarcomas, lymphoid leukemia, and undifferentiated leukemia.
174. The pharmaceutical composition of any one of embodiments 1-57, 68-77, 88-97, 99, and 116-159 for use in treating or delaying progression of a cell proliferative disorder, or for use in enhancing immune function in a subject having a cell proliferative disorder, wherein the cell proliferative disorder is a cancer selected from multiple myeloma (MM), chronic lymphoid leukemia (CLL), mantle cell lymphoma (MCL), diffuse large B-cell lymphoma (DLBCL), follicular lymphoma (FL), acute myeloid leukemia (AML), myelodysplastic syndrome (MDS), chronic myelogenous leukemia (CML), chronic myelomonocytic leukemia, acute promyelocytic leukemia (APL), chronic myeloproliferative disorder, thrombocytic leukemia, precursor B-cell acute lymphoblastic leukemia (pre-B-ALL), precursor T cell acute lymphoblastic leukemia (pre-T-ALL), mast cell disease, mast cell leukemia, mast cell sarcoma, myeloid sarcomas, lymphoid leukemia, and undifferentiated leukemia.
175. The use or pharmaceutical composition for use of embodiment 173 or 174, wherein the cancer is an FcRH5-positive cancer.
176. The use or pharmaceutical composition for use of any one of embodiments 161-175, wherein the therapeutic protein is a bispecific antibody formulated for administration to the subject at a dosage from about 10 µg to about 100 mg.
177. The use or pharmaceutical composition for use of embodiment 176, wherein the therapeutic protein is a bispecific antibody formulated for administration to the subject at a dosage from about 1 mg to about 60 mg.
178. The use or pharmaceutical composition for use of any one of embodiments 161-177, wherein the subject is to be co-administered with at least one additional therapeutic agent.
179. The use or pharmaceutical composition for use of embodiment 178, wherein the at least one additional therapeutic agent comprises a PD-1 axis binding antagonist.
180. The use or pharmaceutical composition for use of embodiment 179, wherein the PD-1 axis binding antagonist is selected from the group consisting of a PD-L1 binding antagonist, a PD-1 binding antagonist, and a PD-L2 binding antagonist.
181. The use or pharmaceutical composition for use of embodiment 180, wherein the PD-1 axis binding antagonist is a PD-L1 binding antagonist.
182. The use or pharmaceutical composition for use of embodiment 181, wherein the PD-L1 binding antagonist is selected from the group consisting of atezolizumab (MPDL3280A), MDX-1105 (BMS-936559), and MEDI4736 (durvalumab).
183. The use or pharmaceutical composition for use of embodiment 180, wherein the PD-1 axis binding antagonist is a PD-1 binding antagonist.
184. The use or pharmaceutical composition for use of embodiment 183, wherein the PD-1 binding antagonist is selected from the group consisting of MDX-1106 (nivolumab), MK-3475 (lambrolizumab), AMG 404, REGN2810 (cemiplimab), and AMP-224.
185. The use or pharmaceutical composition for use of embodiment 180, wherein the PD-1 axis binding antagonist is a PD-L2 binding antagonist.
186. The use or pharmaceutical composition for use of embodiment 185, wherein the PD-L2 binding antagonist is an antibody or an immunoadhesin.
187. The use or pharmaceutical composition for use of embodiment 178, wherein the at least one additional therapeutic agent comprises obinutuzumab, rituximab, a corticosteroid, or tocilizumab.
188. The use or pharmaceutical composition for use of embodiment 178, wherein the at least one additional therapeutic agent comprises an antibody-drug conjugate (ADC)
189. The use or pharmaceutical composition for use of embodiment 188, wherein the ADC is an anti-CD79b ADC.
190. The use or pharmaceutical composition for use of embodiment 189, wherein the anti-CD79b ADC is polatuzumab vedotin.
191. The use or pharmaceutical composition for use of any one of embodiments 161-190, wherein the subject is a human.
192. A method of treating or delaying the progression of a cell proliferative disorder in a subject in need thereof, the method comprising administering to the subject an effective amount of the pharmaceutical composition of any one of embodiments 1-159.
193. A method of enhancing immune function in a subject having a cell proliferative disorder, the method comprising administering to the subject an effective amount of the pharmaceutical composition of any one of embodiments 1-159.
194. The method of embodiment 192 or 193, wherein the cell proliferative disorder is a cancer.
195. The method of any one of embodiments 192-194, wherein the therapeutic protein is a bispecific antibody formulated to bind to a CD3 molecule located on an immune effector cell and a target molecule located on a target cell other than the immune effector cell.
196. The method of embodiment 195, wherein the bispecific antibody activates the immune effector cell following binding to the CD3 molecule and to the target molecule.
197. The method of embodiment 196, wherein the activated immune effector cell is capable of exerting a cytotoxic effect and/or an apoptotic effect on the target cell.
198. A method of treating or delaying the progression of a cell proliferative disorder in a subject in need thereof or enhancing immune function in a subject having a cell proliferative disorder, the method comprising administering to the subject an effective amount of the pharmaceutical composition of any one of embodiments 1-67, 88-98, and 101-159, wherein the cell proliferative disorder is a cancer selected from the group consisting of NHL, CLL, B cell lymphoma, splenic diffuse red pulp small B cell lymphoma, B cell lymphoma with features intermediate between diffuse large B-cell lymphoma and Burkitt lymphoma, B cell lymphoma with features intermediate between diffuse large B-cell lymphoma and classical Hodgkin lymphoma, DLBCL, GCB DLBCL, ABC DLBCL, primary cutaneous follicle center lymphoma, T-cell/histiocyte rich large B-cell lymphoma, primary DLBCL of the central nervous system, primary cutaneous DLBCL (leg type), EBV-positive DLBCL of the elderly, DLBCL associated with chronic inflammation, primary mediastinal (thymic) large B cell lymphoma, intravascular large B cell lymphoma, ALK-positive large B-cell lymphoma, large B-cell lymphoma arising in HHV8-associated multicentric Castleman disease, B cell leukemia, FL, MCL, AML, MZL, SLL, LL, WM, CNSL, BL, B cell prolymphocytic leukemia, splenic marginal zone lymphoma, hairy cell leukemia, splenic lymphoma/leukemia, hairy cell leukemia variant, α heavy chain disease, γ heavy chain disease, µ heavy chain disease, plasma cell myeloma, solitary plasmacytoma of bone, extraosseous plasmacytoma, MALT lymphoma, nodal marginal zone lymphoma, pediatric nodal marginal zone lymphoma, pediatric follicular lymphoma, lymphomatoid granulomatosis, plasmablastic lymphoma, and primary effusion lymphoma.
199. The method of embodiment 197, wherein the cancer is DLBCL, GCB DLBCL, ABC DLBCL, FL, MCL, AML, CLL, MZL, SLL, LL, WM, CNSL, or BL.
200. A method of treating or delaying the progression of a cell proliferative disorder in a subject in need thereof or enhancing immune function in a subject having a cell proliferative disorder, the method comprising administering to the subject an effective amount of the pharmaceutical composition of any one of embodiments 1-57, 78-97, 100, and 116-159, wherein the cell proliferative disorder is a cancer selected from the group consisting of breast cancer, colorectal cancer, gastric cancer, non-small cell lung cancer, multiple myeloma (MM), renal cancer, prostate cancer, liver cancer, head and neck cancer, melanoma, ovarian cancer, mesothelioma, and glioblastoma.
201. The method of embodiment 200, wherein the cancer is a HER2-positive cancer.
202. A method of treating or delaying the progression of a cell proliferative disorder in a subject in need thereof or enhancing immune function in a subject having a cell proliferative disorder, the method comprising administering to the subject an effective amount of the pharmaceutical composition of any one of embodiments 1-57, 68-77, 88-97, 99, and 116-159, wherein the cell proliferative disorder is a cancer selected from multiple myeloma (MM), chronic lymphoid leukemia (CLL), mantle cell lymphoma (MCL), diffuse large B-cell lymphoma (DLBCL), follicular lymphoma (FL), acute myeloid leukemia (AML), myelodysplastic syndrome (MDS), chronic myelogenous leukemia (CML), chronic myelomonocytic leukemia, acute promyelocytic leukemia (APL), chronic myeloproliferative disorder, thrombocytic leukemia, precursor B-cell acute lymphoblastic leukemia (pre-B-ALL), precursor T cell acute lymphoblastic leukemia (pre-T-ALL), mast cell disease, mast cell leukemia, mast cell sarcoma, myeloid sarcomas, lymphoid leukemia, and undifferentiated leukemia.
203. The method of embodiment 202, wherein the cancer is an FcRH5-positive cancer.
204. The method of any one of embodiments 192-203, wherein the therapeutic protein is a bispecific antibody and is administered to the subject at a dosage from about 10 µg to about 100 mg.
205. The method of embodiment 204, wherein the therapeutic protein is administered to the subject at a dosage from about 1 mg to about 60 mg.
206. The method of any one of embodiments 192-205, wherein the subject is co-administered with at least one additional therapeutic agent.
207. The method of embodiment 206, wherein the at least one additional therapeutic agent comprises a PD-1 axis binding antagonist.
208. The method of embodiment 207, wherein the PD-1 axis binding antagonist is selected from the group consisting of a PD-L1 binding antagonist, a PD-1 binding antagonist, and a PD-L2 binding antagonist.
209. The method of embodiment 208, wherein the PD-1 axis binding antagonist is a PD-L1 binding antagonist.
210. The method of embodiment 209, wherein the PD-L1 binding antagonist is selected from the group consisting of atezolizumab (MPDL3280A), MDX-1105 (BMS-936559), and MEDI4736 (durvalumab).
211. The method of embodiment 208, wherein the PD-1 axis binding antagonist is a PD-1 binding antagonist.
212. The method of embodiment 211, wherein the PD-1 binding antagonist is selected from the group consisting of MDX-1106 (nivolumab), MK-3475 (lambrolizumab), AMG 404, REGN2810 (cemiplimab), and AMP-224.
213. The method of embodiment 208, wherein the PD-1 axis binding antagonist is a PD-L2 binding antagonist.
214. The method of embodiment 213, wherein the PD-L2 binding antagonist is an antibody or an immunoadhesin.
215. The method of embodiment 206, wherein the at least one additional therapeutic agent comprises obinutuzumab, rituximab, a corticosteroid, or tocilizumab.
216. The method of embodiment 206, wherein the at least one additional therapeutic agent comprises an ADC.
217. The method of embodiment 216, wherein the ADC is an anti-CD79b ADC.
218. The method of embodiment 217, wherein the anti-CD79b ADC is polatuzumab vedotin.
219. The method of any one of embodiments 192-218, wherein the pharmaceutical composition is administered intravenously.
220. The method of any one of embodiments 192-219, wherein the subject is a human.

### Other Embodiments

Although the foregoing disclosure has been described in some detail by way of illustration and example for purposes of clarity of understanding, the descriptions and examples should not be construed as limiting the scope of the disclosure. The disclosures of all patent and scientific literature cited herein are expressly incorporated in their entirety by reference.

The following numbered paragraphs (paras.) contain further statements of various aspects and embodiments of the present invention:-
1. A pharmaceutical composition comprising a therapeutic protein, polysorbate 20 (PS20), methionine, a buffering agent, and a carrier, wherein the molar ratio of the PS20 to the therapeutic protein is 100 or less, the PS20 is at a concentration from 0.01% to 0.12% weight-by-volume (w/v), the methionine is at a concentration from 1 mM to 50 mM, and the buffering agent is at a concentration from 5 mM to 20 mM.
2. The pharmaceutical composition of para. 1, wherein the therapeutic protein is at a concentration of about 10 mg/ml or less.
3. The pharmaceutical composition of para. 1 or 2, wherein the concentration of the therapeutic protein is between about 0.1 mg/ml to about 10 mg/ml.
4. The pharmaceutical composition of para. 3, wherein the concentration of the therapeutic is about 3 mg/ml or about 1 mg/ml.
5. The pharmaceutical composition of any one of paras. 1-4, wherein the molar ratio of the PS20 to the therapeutic protein is from 45 to 100.
6. The pharmaceutical composition of para. 5, wherein the molar ratio of the PS20 to the therapeutic protein is about 48, about 71, or about 79.
7. The pharmaceutical composition of any one of paras. 1-6, wherein the pharmaceutical composition is formulated as a drug product (DP).
8. The pharmaceutical composition of any one of paras. 1-7, wherein the concentration of the methionine is from about 2.5 mM to about 20 mM.
9. The pharmaceutical composition of para. 7, wherein the concentration of the methionine is about 10 mM.
10. The pharmaceutical composition of any one of paras. 1-9, wherein the concentration of the buffering agent is from about 5 mM to about 25 mM.
11. The pharmaceutical composition of para. 10, wherein the concentration of the buffering agent is about 10 mM or about 20 mM.
12. The pharmaceutical composition of any one of paras. 1-11, wherein the buffering agent is a histidine, a phosphate, a succinate, an acetate, or a combination thereof.
13. The pharmaceutical composition of para.12, wherein the buffering agent is histidine.
14. The pharmaceutical composition of para. 13, wherein the histidine is histidine acetate or histidine HCl.
15. The pharmaceutical composition of any one of paras. 10-14, wherein the buffering agent is histidine acetate at a concentration of about 10 mM or about 20 mM.
16. The pharmaceutical composition of any one of paras. 10-14, wherein the buffering agent is histidine HCl at a concentration of about 20 mM.
17. The pharmaceutical composition of any one of paras. 1-16, further comprising a tonicity agent.
18. The pharmaceutical composition of para. 17, wherein the tonicity agent is a sugar, an amino acid, or a salt.
19. The pharmaceutical composition of para. 18, wherein the tonicity agent is a sugar.
20. The pharmaceutical composition of para. 19, wherein the sugar is sucrose, glucose, glycerol, or trehalose.
21. The pharmaceutical composition of para. 20, wherein the sugar is sucrose.
22. The pharmaceutical composition of any one of paras. 17-21, wherein the tonicity agent is at a concentration from about 100 mM to about 500 mM.
23. The pharmaceutical composition of para. 22, wherein the concentration of the tonicity agent is from about 200 mM to about 300 mM.
24. The pharmaceutical composition of para.23, wherein the concentration of the tonicity agent is about 240 mM.
25. The pharmaceutical composition of any one of paras. 1-24, wherein the pharmaceutical composition has a pH from about 4.5 to about 8.
26. The pharmaceutical composition of para. 25, wherein the pH of the pharmaceutical composition is from about 5.1 to about 6.1.
27. The pharmaceutical composition of para. 26, wherein the pH of the pharmaceutical composition is about 5.5 or about 5.8.
28. The pharmaceutical composition of any one of paras. 1-27, wherein the pharmaceutical composition further comprises an antioxidant.
29. The pharmaceutical composition of para. 28, wherein the antioxidant is N-acetyl-DL-tryptophan.
30. The pharmaceutical composition of para. 29, wherein the concentration of N-acetyl-DL-tryptophan is between 0.1 and 0.5 mM.
31. The pharmaceutical composition of para. 30, wherein the concentration of N-acetyl-DL-tryptophan is about 0.3 mM.
32. The pharmaceutical composition of any one of paras. 1-31, wherein the therapeutic protein is an antibody.
33. The pharmaceutical composition of para. 32, wherein the antibody is a bispecific antibody.
34. The pharmaceutical composition of para. 33, wherein the bispecific antibody comprises at least one antigen-binding domain that specifically binds to CD3 and at least one antigen-binding domain that specifically binds to a target antigen.
35. The pharmaceutical composition of para. 34, wherein the bispecific antibody has a methionine at position 257 of the Fc region (EU numbering), and wherein oxidation of the methionine at position 257 of the Fc region is less than about 10% over two weeks at 40 °C.
36. The pharmaceutical composition of para. 35, wherein the oxidation of methionine at position 257 of the Fc region is no more than about 6% over two weeks at 40 °C.
37. A pharmaceutical composition comprising a bispecific antibody, a surfactant, methionine, and a carrier, wherein the pharmaceutical composition has a pH of about 5.5 or 5.8, and wherein:
   (i) the bispecific antibody comprises at least one antigen-binding domain that specifically binds to CD3 and at least one antigen-binding domain that specifically binds to a target antigen and wherein the bispecific antibody is at a concentration of about 10 mg/ml or less,
   (ii) the surfactant is at a concentration from about 0.05% to about 0.12% w/v, and
   (iii) the methionine is at a concentration of about 10 mM.
38. The pharmaceutical composition of para. 37, wherein the molar ratio of the surfactant to the bispecific antibody is 100 or less.
39. The pharmaceutical composition of para. 37 or 38, wherein the surfactant is PS20 or poloxamer 188 (P188).
40. The pharmaceutical composition of para. 39, wherein the surfactant is PS20 and the concentration of the PS20 is about 0.05%, 0.06%, or about 0.12% w/v.
41. The pharmaceutical composition of para. 40, wherein the molar ratio of the PS20 to the bispecific antibody is from about 45 to about 100.
42. The pharmaceutical composition of para. 41, wherein the molar ratio of the PS20 to the bispecific antibody is about 48, about 71, or about 79.
43. The pharmaceutical composition of para. 39, wherein the surfactant is P188 and the concentration of the P188 is about 0.1% w/v.
44. The pharmaceutical composition of para. 43, wherein the molar ratio of the P188 to the bispecific antibody is from about 5 to about 25.
45. The pharmaceutical composition of para. 44, wherein the molar ratio of the P188 to the bispecific antibody is about 17.
46. The pharmaceutical composition of any one of paras. 37-45, wherein the bispecific antibody is at a concentration of between about 0.1 mg/ml to about 5 mg/ml.
47. The pharmaceutical composition of para. 46, wherein the bispecific antibody is at a concentration of about 1 mg/ml or about 3 mg/ml.
48. The pharmaceutical composition of any one of paras. 37-47, wherein the pharmaceutical composition is formulated as a DP.
49. The pharmaceutical composition of any one of paras. 37-48, wherein the pharmaceutical composition further comprises histidine at a concentration of about 10 mM or about 20 mM.
50. The pharmaceutical composition of para. 49, wherein the histidine is histidine acetate or histidine HCl.
51. The pharmaceutical composition of any one of paras. 37-50, wherein the pharmaceutical composition further comprises sucrose at a concentration of about 240 mM.
52. The pharmaceutical composition of any one of paras. 37-51, wherein the carrier is water.
53. The pharmaceutical composition of any one of paras. 32-52, wherein the pharmaceutical composition further comprises an antioxidant.
54. The pharmaceutical composition of para. 53, wherein the antioxidant is N-acetyl-DL-tryptophan.
55. The pharmaceutical composition of para. 54, wherein the concentration of N-acetyl-DL-tryptophan is between 0.1 and 0.5 mM.
56. The pharmaceutical composition of para. 55, wherein the concentration of N-acetyl-DL-tryptophan is about 0.3 mM.
57. The pharmaceutical composition of any one of paras. 32-56, wherein the bispecific antibody comprises an anti-CD3 arm and an anti-target arm.
58. The pharmaceutical composition of para. 57, wherein the anti-target arm is an anti-FcRH5 arm.
59. The pharmaceutical composition of para. 58, wherein the anti-CD3 arm comprises a CD3-binding domain comprising:
   (i) an HVR-H1 comprising the amino acid sequence of SEQ ID NO: 65;
   (ii) an HVR-H2 comprising the amino acid sequence of SEQ ID NO: 66;
   (iii) an HVR-H3 comprising the amino acid sequence of SEQ ID NO: 67;
   (iv) an HVR-L1 comprising the amino acid sequence of SEQ ID NO: 68;
   (v) an HVR-L2 comprising the amino acid sequence of SEQ ID NO: 69; and
   (vi) an HVR-L3 comprising the amino acid sequence of SEQ ID NO: 70.
60. The pharmaceutical composition of para. 59, wherein the CD3-binding domain comprises (a) a VH domain comprising an amino acid sequence having at least 95% sequence identity to the amino acid sequence of SEQ ID NO: 71; (b) a VL domain comprising an amino acid sequence having at least 95% sequence identity to the amino acid sequence of SEQ ID NO: 72; or (c) a VH domain as in (a) and a VL domain as in (b).
61. The pharmaceutical composition of para. 60, wherein the VH domain of the CD3-binding domain comprises the amino acid sequence of SEQ **ID** NO: 71 and the VL domain of the CD3-binding domain comprises the amino acid sequence of SEQ **ID** NO: 72.
62. The pharmaceutical composition of any one of paras. 58-61, wherein the anti-FcRH5 arm comprises a FcRH5-binding domain comprising:
   (i) an HVR-H1 comprising the amino acid sequence of SEQ ID NO: 57;
   (ii) an HVR-H2 comprising the amino acid sequence of SEQ ID NO: 58;
   (iii) an HVR-H3 comprising the amino acid sequence of SEQ ID NO: 59;
   (iv) an HVR-L1 comprising the amino acid sequence of SEQ ID NO: 60;
   (v) an HVR-L2 comprising the amino acid sequence of SEQ **ID** NO: 61; and
   (vi) an HVR-L3 comprising the amino acid sequence of SEQ ID NO: 62.
63. The pharmaceutical composition of para. 62, wherein the FcRH5-binding domain comprises (a) a VH domain comprising an amino acid sequence having at least 95% sequence identity to the amino acid sequence of SEQ ID NO: 63; (b) a VL domain comprising an amino acid sequence having at least 95% sequence identity to the amino acid sequence of SEQ ID NO: 64; or (c) a VH domain as in (a) and a VL domain as in (b).
64. The pharmaceutical composition of para. 63, wherein the VH domain of the FcRH5-binding domain comprises the amino acid sequence of SEQ **ID** NO: 63 and the VL domain of the FcRH5-binding domain comprises the amino acid sequence of SEQ ID NO: 64.
65. A pharmaceutical composition comprising a bispecific antibody, PS20, methionine, a buffering agent, and a carrier, wherein the molar ratio of the PS20 to the bispecific antibody is about 100 or less, the PS20 is at a concentration from about 0.01% to about 0.12% w/v, the methionine is at a concentration from 1 mM to 50 mM, and the buffering agent is at a concentration from 5 mM to 20 mM, wherein the bispecific antibody comprises an anti-CD3 arm and an anti-FcRH5 arm, and wherein:
   (a) the anti-CD3 arm comprises a CD3-binding domain comprising:
      an HVR-H1 comprising the amino acid sequence of SEQ ID NO: 65;
      an HVR-H2 comprising the amino acid sequence of SEQ ID NO: 66;
      an HVR-H3 comprising the amino acid sequence of SEQ ID NO: 67;
      an HVR-L1 comprising the amino acid sequence of SEQ ID NO: 68;
      an HVR-L2 comprising the amino acid sequence of SEQ ID NO: 69; and
      an HVR-L3 comprising the amino acid sequence of SEQ ID NO: 70; and
   (b) the anti-FcRH5 arm comprises a FcRH5-binding domain comprising:
      an HVR-H1 comprising the amino acid sequence of SEQ ID NO: 57;
      an HVR-H2 comprising the amino acid sequence of SEQ ID NO: 58;
      an HVR-H3 comprising the amino acid sequence of SEQ ID NO: 59;
      an HVR-L1 comprising the amino acid sequence of SEQ ID NO: 60;
      an HVR-L2 comprising the amino acid sequence of SEQ ID NO: 61 ; and
      an HVR-L3 comprising the amino acid sequence of SEQ ID NO: 62.
66. The pharmaceutical composition of para. 65, wherein the CD3-binding domain comprises:
   (a) a VH domain comprising an amino acid sequence having at least 95% sequence identity to the amino acid sequence of SEQ **ID** NO: 71; (b) a VL domain comprising an amino acid sequence having at least 95% sequence identity to the amino acid sequence of SEQ ID NO: 72; or (c) a VH domain as in (a) and a VL domain as in (b);
      and the FcRH5-binding domain comprises:
      (a) a VH domain comprising an amino acid sequence having at least 95% sequence identity to the amino acid sequence of SEQ ID NO: 63; (b) a VL domain comprising an amino acid sequence having at least 95% sequence identity to the amino acid sequence of SEQ ID NO: 64; or (c) a VH domain as in (a) and a VL domain as in (b).
67. The pharmaceutical composition of para. 66, wherein:
   (a) the VH domain of the CD3-binding domain comprises the amino acid sequence of SEQ ID NO: 71 and the VL domain of the CD3-binding domain comprises the amino acid sequence of SEQ ID NO: 72, and
   (b) the VH domain of the FcRH5-binding domain comprises the amino acid sequence of SEQ ID NO: 63 and the VL domain of the FcRH5-binding domain comprises the amino acid sequence of SEQ ID NO: 64.
68. The pharmaceutical composition of para. 57, wherein the anti-target arm is an anti-HER2 arm.
69. The pharmaceutical composition of para. 68, wherein the anti-CD3 arm comprises a CD3-binding domain comprising:
   (i) an HVR-H1 comprising the amino acid sequence of SEQ ID NO: 109;
   (ii) an HVR-H2 comprising the amino acid sequence of SEQ ID NO: 110;
   (iii) an HVR-H3 comprising the amino acid sequence of SEQ ID NO: 111 ;
   (iv) an HVR-L1 comprising the amino acid sequence of SEQ ID NO: 112;
   (v) an HVR-L2 comprising the amino acid sequence of SEQ **ID** NO: 113; and
   (vi) an HVR-L3 comprising the amino acid sequence of SEQ ID NO: 114.
70. The pharmaceutical composition of para. 69, wherein the CD3-binding domain comprises (a) a VH domain comprising an amino acid sequence having at least 95% sequence identity to the amino acid sequence of SEQ ID NO: 115; (b) a VL domain comprising an amino acid sequence having at least 95% sequence identity to the amino acid sequence of SEQ ID NO: 116; or (c) a VH domain as in (a) and a VL domain as in (b).
71. The pharmaceutical composition of para. 70, wherein the VH domain of the CD3-binding domain comprises the amino acid sequence of SEQ **ID** NO: 115 and the VL domain of the CD3-binding domain comprises the amino acid sequence of SEQ **ID** NO: 116.
72. The pharmaceutical composition of any one of paras. 68-71, wherein the anti-HER2 arm comprises a HER2-binding domain comprising:
   (i) an HVR-H1 comprising the amino acid sequence of SEQ ID NO: 93;
   (ii) an HVR-H2 comprising the amino acid sequence of SEQ ID NO: 94;
   (iii) an HVR-H3 comprising the amino acid sequence of SEQ ID NO: 95;
   (iv) an HVR-L1 comprising the amino acid sequence of SEQ ID NO: 96;
   (v) an HVR-L2 comprising the amino acid sequence of SEQ **ID** NO: 97; and
   (vi) an HVR-L3 comprising the amino acid sequence of SEQ ID NO: 98.
73. The pharmaceutical composition of para. 72, wherein the HER2-binding domain comprises (a) a VH domain comprising an amino acid sequence having at least 95% sequence identity to the amino acid sequence of SEQ ID NO: 99; (b) a VL domain comprising an amino acid sequence having at least 95% sequence identity to the amino acid sequence of SEQ ID NO: 100; or (c) a VH domain as in (a) and a VL domain as in (b).
74. The pharmaceutical composition of para. 73, wherein the VH domain of the HER2-binding domain comprises the amino acid sequence of SEQ ID NO: 99 and the VL domain of the HER2-binding domain comprises the amino acid sequence of SEQ ID NO: 100.
75. A pharmaceutical composition comprising a bispecific antibody, PS20, methionine, a buffering agent, and a carrier, wherein the molar ratio of the PS20 to the bispecific antibody is about 100 or less, the PS20 is at a concentration from about 0.01% to about 0.12% w/v, the methionine is at a concentration from 1 mM to 50 mM, and the buffering agent is at a concentration from 5 mM to 20 mM, wherein the bispecific antibody comprises an anti-CD3 arm and an anti-HER2 arm, and wherein:
   (a) the anti-CD3 arm comprises a CD3-binding domain comprising:
      an HVR-H1 comprising the amino acid sequence of SEQ ID NO: 109;
      an HVR-H2 comprising the amino acid sequence of SEQ ID NO: 110;
      an HVR-H3 comprising the amino acid sequence of SEQ ID NO: 111;
      an HVR-L1 comprising the amino acid sequence of SEQ ID NO: 112;
      an HVR-L2 comprising the amino acid sequence of SEQ ID NO: 113; and
      an HVR-L3 comprising the amino acid sequence of SEQ ID NO: 114; and
   (b) the anti-HER2 arm comprises a HER2-binding domain comprising:
      an HVR-H1 comprising the amino acid sequence of SEQ ID NO: 93;
      an HVR-H2 comprising the amino acid sequence of SEQ ID NO: 94;
      an HVR-H3 comprising the amino acid sequence of SEQ ID NO: 95;
      an HVR-L1 comprising the amino acid sequence of SEQ ID NO: 96;
      an HVR-L2 comprising the amino acid sequence of SEQ ID NO: 97; and
      an HVR-L3 comprising the amino acid sequence of SEQ ID NO: 98.
76. The pharmaceutical composition of para. 75, wherein the CD3-binding domain comprises:
   (a) a VH domain comprising an amino acid sequence having at least 95% sequence identity to the amino acid sequence of SEQ ID NO: 115; (b) a VL domain comprising an amino acid sequence having at least 95% sequence identity to the amino acid sequence of SEQ ID NO: 116; or (c) a VH domain as in (a) and a VL domain as in (b);
      and the HER2-binding domain comprises:
   (a) a VH domain comprising an amino acid sequence having at least 95% sequence identity to the amino acid sequence of SEQ ID NO: 99; (b) a VL domain comprising an amino acid sequence having at least 95% sequence identity to the amino acid sequence of SEQ ID NO: 100; or (c) a VH domain as in (a) and a VL domain as in (b).
77. The pharmaceutical composition of para. 76, wherein:
   (a) the VH domain of the CD3-binding domain comprises the amino acid sequence of SEQ ID NO: 115 and the VL domain of the CD3-binding domain comprises the amino acid sequence of SEQ ID NO: 116, and
   (b) the VH domain of the HER2-binding domain comprises the amino acid sequence of SEQ ID NO: 99 and the VL domain of the HER2-binding domain comprises the amino acid sequence of SEQ ID NO: 100.
78. The pharmaceutical composition of para. 32, wherein the antibody is an IgG antibody, or the pharmaceutical composition of any one of paras. 33, 34, and 57-77, wherein the bispecific antibody is an IgG antibody.
79. The pharmaceutical composition of para. 78, wherein the IgG antibody is an IgG₁ antibody.
80. The pharmaceutical composition of para. 78 or 79, wherein the bispecific antibody comprises one or more substitution mutations in the Fc region.
81. The pharmaceutical composition of para. 80, wherein the bispecific antibody comprises an aglycosylation site mutation.
82. The pharmaceutical composition of para. 81, wherein the aglycosylation site mutation is a substitution mutation.
83. The pharmaceutical composition of para. 80, wherein the one or more substitution mutations reduces effector function of the bispecific antibody.
84. The pharmaceutical composition of para. 80, wherein the one or more substitution mutations is at one or more amino acid residues selected from the group consisting of N297, L234, L235, D265, and P329 (EU numbering).
85. The pharmaceutical composition of para. 84, wherein the substitution mutation is at least one selected from the group consisting of N297A, N297G, L234A, L235A, D265A, and P329G (EU numbering).
86. The pharmaceutical composition of para. 80, wherein the one or more substitution mutations in the Fc region comprises one or more knob-in-hole mutations.
87. The pharmaceutical composition of any one of paras. 78-86, wherein the anti-target arm comprises T366W and N297G substitution mutations, and the anti-CD3 arm comprises T366S, L368A, Y407V, and N297G substitution mutations.
88. The pharmaceutical composition of any one of paras. 58-67 and 78-87, wherein the bispecific antibody is cevostamab.
89. The pharmaceutical composition of any one of paras. 68-87, wherein the bispecific antibody is runimotamab.
90. The pharmaceutical composition of any one of paras. 32-89, wherein the at least one antigen-binding domain that specifically binds to CD3 binds to a human CD3 polypeptide or a cynomolgus monkey (cyno) CD3 polypeptide.
91. The pharmaceutical composition of para. 90, wherein the human CD3 polypeptide or the cyno CD3 polypeptide is a human CD3ε polypeptide or a cyno CD3ε polypeptide, respectively.
92. The pharmaceutical composition of para. 90, wherein the human CD3 polypeptide or the cyno CD3 polypeptide is a human CD3γ polypeptide or a cyno CD3γ polypeptide, respectively.
93. The pharmaceutical composition of any one of paras. 32-92, wherein the bispecific antibody is monoclonal.
94. The pharmaceutical composition of any one of paras. 32-93, wherein the bispecific antibody is human, humanized, or chimeric.
95. The pharmaceutical composition of any one of paras. 1-94, wherein the pharmaceutical composition is in a unit dosage form.
96. The pharmaceutical composition of para. 95, wherein the unit dosage form is a liquid formulation for dilution.
97. The pharmaceutical composition of para. 96, wherein the liquid formulation for dilution is supplied in a container having a volume of about 50 ml.
98. The pharmaceutical composition of para. 96, wherein the liquid formulation for dilution is supplied in a container having a volume of about 15 ml.
99. The pharmaceutical composition of para. 96, wherein the liquid formulation for dilution is supplied in a container having a volume of about 2 ml.
100. The pharmaceutical composition of para. 96, wherein the liquid formulation for dilution is supplied in a container having a volume of about 1 ml.
101. The pharmaceutical composition of para. 96 or 97, wherein the volume of the liquid formulation for dilution is about 15 ml or about 30 ml.
102. The pharmaceutical composition of para. 96 or 98, wherein the volume of the liquid formulation for dilution is about 8 ml or about 15 mL.
103. The pharmaceutical composition of para. 96 or 99, wherein the volume of the liquid formulation for dilution is about 0.5 ml or about 0.9 ml.
104. The pharmaceutical composition of para. 96 or 100, wherein the volume of the liquid formulation for dilution is about 0.5 ml or about 0.9 ml.
105. The pharmaceutical composition of any one of paras. 96-104, wherein the liquid formulation is for dilution with a normal saline solution comprising 0.45% or 0.9% (w/v) NaCl.
106. The pharmaceutical composition of any one of paras. 1-105, wherein the pharmaceutical composition comprises no more than 1,000 particles having a diameter ≥ 2 µm per ml as detected by high accuracy liquid particle counting (HIAC).
107. The pharmaceutical composition of any one of paras. 1-106, wherein the pharmaceutical composition has a shelf-life of at least 36 months when stored at 5 °C ± 3 °C and protected from light.
108. The pharmaceutical composition of any one of paras. 1-107, wherein the pharmaceutical composition is stable through one or more freeze-thaw cycles.
109. The pharmaceutical composition of para. 108, wherein the pharmaceutical composition is stable through three or more freeze-thaw cycles.
110. The pharmaceutical composition of any one of paras. 1-109, wherein the pharmaceutical composition is stable for about two weeks or longer at about 25 °C.
111. The pharmaceutical composition of para. 110, wherein the pharmaceutical composition is stable for about four weeks or longer at about 25 °C.
112. The pharmaceutical composition of any one of paras. 1-111, wherein the pharmaceutical composition is stable for about 48 months or longer at -20 °C.
113. The pharmaceutical composition of any one of paras. 108-112, wherein stability is assessed by size-exclusion high-performance liquid chromatography (SE-HPLC).
114. The pharmaceutical composition of para. 113, wherein the pharmaceutical composition is determined to be stable if the pharmaceutical composition maintains a purity that is changed by less than 5% as measured by SE-HPLC.
115. The pharmaceutical composition of any one of paras. 108-112, wherein stability is assessed by non-reduced capillary electrophoresis sodium dodecyl sulfate (CE-SDS) assay.
116. The pharmaceutical composition of para. 115, wherein the pharmaceutical composition is determined to be stable if the pharmaceutical composition maintains a purity that is changed by less than 5% as measured by non-reduced CE-SDS assay.
117. The pharmaceutical composition of para. 115 or 116, wherein the non-reduced CE-SDS assay is a microchip CE-SDS (mCE-SDS) assay.
118. The pharmaceutical composition of any one of paras. 1-117, wherein the pharmaceutical composition has a purity of about 85% or higher as assessed by SE-HPLC.
119. The pharmaceutical composition of para. 118, wherein the pharmaceutical composition has a purity of about 90% or higher as assessed by SE-HPLC.
120. The pharmaceutical composition of para. 119, wherein the pharmaceutical composition has a purity of about 95% or higher as assessed by SE-HPLC.
121. The pharmaceutical composition of any one of paras. 118-120, wherein the purity of the pharmaceutical composition as assessed by SE-HPLC is maintained about the same for about 36 months or longer at about 5 °C.
122. The pharmaceutical composition of para. 121, wherein the purity of the pharmaceutical composition as assessed by SE-HPLC is maintained about the same for about 42 months or longer at about 5 °C.
123. The pharmaceutical composition of para. 122, wherein the purity of the pharmaceutical composition as assessed by SE-HPLC is maintained about the same for about 64 months or longer at about 5 °C.
124. The pharmaceutical composition of any one of paras. 1-117, wherein the pharmaceutical composition has a purity of about 75% or higher as assessed by non-reduced CE-SDS assay.
125. The pharmaceutical composition of para. 124, wherein the pharmaceutical composition has a purity of about 80% or higher as assessed by non-reduced CE-SDS assay.
126. The pharmaceutical composition of para. 125, wherein the pharmaceutical composition has a purity of about 85% or higher as assessed by non-reduced CE-SDS assay.
127. The pharmaceutical composition of any one of paras. 124-126, wherein the purity of the pharmaceutical composition as assessed by non-reduced CE-SDS assay is maintained for about 36 months or longer at about 5 °C.
128. The pharmaceutical composition of para. 127, wherein the purity of the pharmaceutical composition as assessed by non-reduced CE-SDS assay is maintained for about 42 months or longer at about 5 °C.
129. The pharmaceutical composition of any one of paras. 124-128, wherein the non-reduced CE-SDS assay is a microchip CE-SDS (mCE-SDS) assay.
130. The pharmaceutical composition of any one of paras. 1-129, wherein the pharmaceutical composition is formulated for intravenous administration.
131. The pharmaceutical composition of any one of paras. 1-130, wherein the pharmaceutical composition does not contain a preservative.
132. The pharmaceutical composition of any one of paras. 1-131, wherein the pharmaceutical composition is formulated for administration by infusion after dilution with a normal saline solution comprising 0.45% or 0.9% NaCl.
133. The pharmaceutical composition of any one of paras. 1-132 for use as a medicament.
134. Use of the pharmaceutical composition of any one of paras. 1-132 in the manufacture of a medicament for:
   (i) treating or delaying progression of a cell proliferative disorder in a subject in need thereof; or
   (ii) enhancing immune function in a subject having a cell proliferative disorder.
135. The pharmaceutical composition of any one of paras. 1-132 for use in:
   (i) treating or delaying progression of a cell proliferative disorder in a subject in need thereof; or
   (ii) enhancing immune function in a subject having a cell proliferative disorder.
136. The use or pharmaceutical composition for use of para. 134 or 135, wherein the cell proliferative disorder is a cancer.
137. The use or pharmaceutical composition for use of any one of paras. 133-136, wherein the therapeutic protein is a bispecific antibody formulated to bind to a CD3 molecule located on an immune effector cell and a target molecule located on a target cell other than the immune effector cell.
138. The use or pharmaceutical composition for use of para. 137, wherein the bispecific antibody activates the immune effector cell following binding to the CD3 molecule and to the target molecule.
139. The use or pharmaceutical composition for use of para. 138, wherein the activated immune effector cell is capable of exerting a cytotoxic effect and/or an apoptotic effect on the target cell.
140. Use of the pharmaceutical composition of any one of paras. 1-57, 78-87, and 90-132 in the manufacture of a medicament for treating or delaying progression of a cell proliferative disorder, or for enhancing immune function in a subject having a cell proliferative disorder, wherein the cell proliferative disorder is a cancer selected from the group consisting of non-Hodgkin's lymphoma (NHL), chronic lymphoid leukemia (CLL), B cell lymphoma, splenic diffuse red pulp small B cell lymphoma, B cell lymphoma with features intermediate between diffuse large B-cell lymphoma and Burkitt lymphoma, B cell lymphoma with features intermediate between diffuse large B-cell lymphoma and classical Hodgkin lymphoma, diffuse large B cell lymphoma (DLBCL), germinal center B cell-like (GCB) DLBCL, activated B cell-like (ABC) DLBCL, primary cutaneous follicle center lymphoma, T-cell/histiocyte rich large B-cell lymphoma, primary DLBCL of the central nervous system, primary cutaneous DLBCL (leg type), Epstein-Barr virus (EBV)-positive DLBCL of the elderly, DLBCL associated with chronic inflammation, primary mediastinal (thymic) large B cell lymphoma, intravascular large B cell lymphoma, ALK-positive large B-cell lymphoma, large B-cell lymphoma arising in HHV8-associated multicentric Castleman disease, B cell leukemia, follicular lymphoma (FL), mantle cell lymphoma (MCL), acute myeloid leukemia (AML), marginal zone lymphoma (MZL), small lymphocytic leukemia (SLL), lymphoplasmacytic lymphoma (LL), Waldenstrom macroglobulinemia (WM), central nervous system lymphoma (CNSL), Burkitt's lymphoma (BL), B cell prolymphocytic leukemia, splenic marginal zone lymphoma, hairy cell leukemia, splenic lymphoma/leukemia, hairy cell leukemia variant, α heavy chain disease, γ heavy chain disease, µ heavy chain disease, plasma cell myeloma, solitary plasmacytoma of bone, extraosseous plasmacytoma, extranodal marginal zone lymphoma of mucosa-associated lymphoid tissue (MALT lymphoma), nodal marginal zone lymphoma, pediatric nodal marginal zone lymphoma, pediatric follicular lymphoma, lymphomatoid granulomatosis, plasmablastic lymphoma, and primary effusion lymphoma.
141. The pharmaceutical composition of any one of paras. 1-57, 78-87, and 90-132 for use in treating or delaying progression of cell proliferative disorder, or for use in enhancing immune function in a subject having a cell proliferative disorder, wherein the cell proliferative disorder is a cancer selected from the group consisting of NHL, CLL, B cell lymphoma, splenic diffuse red pulp small B cell lymphoma, B cell lymphoma with features intermediate between diffuse large B-cell lymphoma and Burkitt lymphoma, B cell lymphoma with features intermediate between diffuse large B-cell lymphoma and classical Hodgkin lymphoma, DLBCL, GCB DLBCL, ABC DLBCL, primary cutaneous follicle center lymphoma, T-cell/histiocyte rich large B-cell lymphoma, primary DLBCL of the central nervous system, primary cutaneous DLBCL (leg type), EBV-positive DLBCL of the elderly, DLBCL associated with chronic inflammation, primary mediastinal (thymic) large B cell lymphoma, intravascular large B cell lymphoma, ALK-positive large B-cell lymphoma, large B-cell lymphoma arising in HHV8-associated multicentric Castleman disease, B cell leukemia, FL, MCL, AML, MZL, SLL, LL, WM, CNSL, BL, B cell prolymphocytic leukemia, splenic marginal zone lymphoma, hairy cell leukemia, splenic lymphoma/leukemia, hairy cell leukemia variant, α heavy chain disease, γ heavy chain disease, µ heavy chain disease, plasma cell myeloma, solitary plasmacytoma of bone, extraosseous plasmacytoma, MALT lymphoma, nodal marginal zone lymphoma, pediatric nodal marginal zone lymphoma, pediatric follicular lymphoma, lymphomatoid granulomatosis, plasmablastic lymphoma, and primary effusion lymphoma.
142. The use or pharmaceutical composition for use of para. 140 or 141, wherein the cancer is GCB DLBCL, ABC DLBCL, FL, MCL, AML, CLL, MZL, SLL, LL, WM, CNSL, or BL.
143. Use of the pharmaceutical composition of any one of paras. 1-57, 68-87, and 89-132 in the manufacture of a medicament for treating or delaying progression of a cell proliferative disorder, or for enhancing immune function in a subject having a cell proliferative disorder, wherein the cell proliferative disorder is a cancer selected from the group consisting of breast cancer, colorectal cancer, gastric cancer, non-small cell lung cancer, multiple myeloma (MM), renal cancer, prostate cancer, liver cancer, head and neck cancer, melanoma, ovarian cancer, mesothelioma, and glioblastoma.
144. The pharmaceutical composition of any one of paras.1-57, 68-87, and 89-132 for use in treating or delaying progression of cell proliferative disorder, or for use in enhancing immune function in a subject having a cell proliferative disorder, wherein the cell proliferative disorder is a cancer selected from the group consisting of breast cancer, colorectal cancer, gastric cancer, non-small cell lung cancer, multiple myeloma, renal cancer, prostate cancer, liver cancer, head and neck cancer, melanoma, ovarian cancer, mesothelioma, and glioblastoma.
145. The use or pharmaceutical composition for use of para. 143 or 144, wherein the cancer is a HER2-positive cancer.
146. Use of the pharmaceutical composition of any one of paras. 1-67, 78-88, and 90-132 in the manufacture of a medicament for treating or delaying progression of a cell proliferative disorder, or for enhancing immune function in a subject having a cell proliferative disorder, wherein the cell proliferative disorder is a cancer selected from MM, CLL, MCL, DLBCL, FL, AML, myelodysplastic syndrome (MDS), chronic myelogenous leukemia (CML), chronic myelomonocytic leukemia, acute promyelocytic leukemia (APL), chronic mycloproliferative disorder, thrombocytic leukemia, precursor B-cell acute lymphoblastic leukemia (pre-B-ALL), precursor T cell acute lymphoblastic leukemia (pre-T-ALL), mast cell disease, mast cell leukemia, mast cell sarcoma, myeloid sarcomas, lymphoid leukemia, and undifferentiated leukemia.
147. The pharmaceutical composition of any one of paras. 1-67, 78-88, and 90-132 for use in treating or delaying progression of cell proliferative disorder, or for use in enhancing immune function in a subject having a cell proliferative disorder, wherein the cell proliferative disorder is a cancer selected from MM, CLL, MCL, DLBCL, FL, AML, MDS, CML, chronic myelomonocytic leukemia, APL, chronic myeloproliferative disorder, thrombocytic leukemia, pre-B-ALL, pre-T-ALL, mast cell disease, mast cell leukemia, mast cell sarcoma, myeloid sarcomas, lymphoid leukemia, and undifferentiated leukemia.
148. The use or pharmaceutical composition for use of para. 146 or 147, wherein the cancer is an FcRH5-positive cancer.
149. The use or pharmaceutical composition for use of any one of paras. 134-148, wherein the therapeutic protein is a bispecific antibody formulated for administration to the subject at a dosage from about 10 µg to about 100 mg.
150. The use or pharmaceutical composition for use of para. 149, wherein the therapeutic protein is a bispecific antibody formulated for administration to the subject at a dosage from about 1 mg to about 60 mg.
151. The use or pharmaceutical composition for use of any one of paras. 134-150, wherein the subject is to be co-administered with at least one additional therapeutic agent.
152. The use or pharmaceutical composition for use of any one of paras. 134-151, wherein the subject is a human.
153. A method of treating or delaying the progression of a cell proliferative disorder in a subject in need thereof, the method comprising administering to the subject an effective amount of the pharmaceutical composition of any one of paras. 1-132.
154. A method of enhancing immune function in a subject having a cell proliferative disorder, the method comprising administering to the subject an effective amount of the pharmaceutical composition of any one of paras.1-132.
155. The method of para. 153 or 154, wherein the cell proliferative disorder is a cancer.
156. The method of any one of paras. 153-155, wherein the therapeutic protein is a bispecific antibody formulated to bind to a CD3 molecule located on an immune effector cell and a target molecule located on a target cell other than the immune effector cell.
157. The method of para. 156, wherein the bispecific antibody activates the immune effector cell following binding to the CD3 molecule and to the target molecule.
158. The method of para. 157, wherein the activated immune effector cell is capable of exerting a cytotoxic effect and/or an apoptotic effect on the target cell.
159. A method of treating or delaying the progression of a cell proliferative disorder in a subject in need thereof or enhancing immune function in a subject having a cell proliferative disorder, the method comprising administering to the subject an effective amount of the pharmaceutical composition of any one of paras. 1-57, 78-87, and 90-132, wherein the cell proliferative disorder is a cancer selected from the group consisting of NHL, CLL, B cell lymphoma, splenic diffuse red pulp small B cell lymphoma, B cell lymphoma with features intermediate between diffuse large B-cell lymphoma and Burkitt lymphoma, B cell lymphoma with features intermediate between diffuse large B-cell lymphoma and classical Hodgkin lymphoma, DLBCL, GCB DLBCL, ABC DLBCL, primary cutaneous follicle center lymphoma, T-cell/histiocyte rich large B-cell lymphoma, primary DLBCL of the central nervous system, primary cutaneous DLBCL (leg type), EBV-positive DLBCL of the elderly, DLBCL associated with chronic inflammation, primary mediastinal (thymic) large B cell lymphoma, intravascular large B cell lymphoma, ALK-positive large B-cell lymphoma, large B-cell lymphoma arising in HHV8-associated multicentric Castleman disease, B cell leukemia, FL, MCL, AML, MZL, SLL, LL, WM, CNSL, BL, B cell prolymphocytic leukemia, splenic marginal zone lymphoma, hairy cell leukemia, splenic lymphoma/leukemia, hairy cell leukemia variant, α heavy chain disease, γ heavy chain disease, µ heavy chain disease, plasma cell myeloma, solitary plasmacytoma of bone, extraosseous plasmacytoma, MALT lymphoma, nodal marginal zone lymphoma, pediatric nodal marginal zone lymphoma, pediatric follicular lymphoma, lymphomatoid granulomatosis, plasmablastic lymphoma, and primary effusion lymphoma.
160. The method of para. 159, wherein the cancer is GCB DLBCL, ABC DLBCL, FL, MCL, AML, CLL, MZL, SLL, LL, WM, CNSL, or BL.
161. A method of treating or delaying the progression of a cell proliferative disorder in a subject in need thereof or enhancing immune function in a subject having a cell proliferative disorder, the method comprising administering to the subject an effective amount of the pharmaceutical composition of any one of paras. 1-57, 68-87, and 89-132, wherein the cell proliferative disorder is a cancer selected from the group consisting of breast cancer, colorectal cancer, gastric cancer, non-small cell lung cancer, multiple myeloma, renal cancer, prostate cancer, liver cancer, head and neck cancer, melanoma, ovarian cancer, mesothelioma, and glioblastoma.
162. The method of para. 161, wherein the cancer is a HER2-positive cancer.
163. A method of treating or delaying the progression of a cell proliferative disorder in a subject in need thereof or enhancing immune function in a subject having a cell proliferative disorder, the method comprising administering to the subject an effective amount of the pharmaceutical composition of any one of paras. 1-67, 78-88, and 90-132, wherein the cell proliferative disorder is a cancer selected from MM, CLL, MCL, DLBCL, FL, AML, MDS, CML, chronic myelomonocytic leukemia, APL, chronic myeloproliferative disorder, thrombocytic leukemia, pre-B-ALL, pre-T-ALL, mast cell disease, mast cell leukemia, mast cell sarcoma, myeloid sarcomas, lymphoid leukemia, and undifferentiated leukemia.
164. The method of para. 163, wherein the cancer is an FcRH5-positive cancer.
165. The method of any one of paras. 153-164, wherein the therapeutic protein is a bispecific antibody and is administered to the subject at a dosage from about 10 µg to about 100 mg.
166. The method of para. 165, wherein the therapeutic protein is administered to the subject at a dosage from about 1 mg to about 60 mg.
167. The method of any one of paras. 153-166, wherein the subject is co-administered with at least one additional therapeutic agent.
168. The method of any one of paras. 153-167, wherein the subject is a human.

## Claims

1. A pharmaceutical composition comprising about 10 mg/ml mosunetuzumab, about 10 mM L-histidine, about 240 mM sucrose, about 0.06% (w/v) PS20, and about 10 mM methionine, wherein the pharmaceutical composition has a pH of 5.5 to 6.1.

2. The pharmaceutical composition of claim 1, wherein the pharmaceutical composition comprises about 5 mg mosunetuzumab, about 10 mM L-histidine, about 240 mM sucrose, about 0.06% (w/v) PS20, and about 10 mM methionine at a fill volume of about 0.5 ml, and wherein the pharmaceutical composition has a pH of 5.5 to 6.1.

3. A pharmaceutical composition comprising about 45 mg/ml mosunetuzumab, about 10 mM L-histidine, about 240 mM sucrose, about 0.06% (w/v) PS20, and about 10 mM methionine, wherein the pharmaceutical composition has a pH of 5.5 to 6.1.

4. The pharmaceutical composition of claim 3, wherein the pharmaceutical composition comprises about 45 mg mosunetuzumab, about 10 mM L-histidine, about 240 mM sucrose, about 0.06% (w/v) PS20, and about 10 mM methionine at a fill volume of about 1 ml, and wherein the pharmaceutical composition has a pH of 5.5 to 6.1.

5. The pharmaceutical composition of any one of claims 1-4, wherein the pH is about 5.8.

6. The pharmaceutical composition of any one of claims 1-5, wherein the pH is 5.8.

7. The pharmaceutical composition of any one of claims 1-6, wherein the pharmaceutical composition is in a unit dosage form.

8. The pharmaceutical composition of any one of claims 1-7, wherein mosunetuzumab has a methionine at position 257 of the Fc region (EU numbering), and wherein oxidation of the methionine at position 257 of the Fc region is less than about 10% over two weeks at 40 °C.

9. The pharmaceutical composition of any one of claims 1-8, wherein the pharmaceutical composition is a liquid formulation for dilution supplied in a single-dose glass vial.

10. The pharmaceutical composition of any one of claims 1-9, wherein the pharmaceutical composition further comprises histidine acetate.

11. The pharmaceutical composition of any one of claims 1-10, wherein the pharmaceutical composition is formulated as a drug product (DP).

12. The pharmaceutical composition of any one of claims 1-11, wherein the pharmaceutical composition is formulated for subcutaneous administration.

13. The pharmaceutical composition of claim 12, wherein the pharmaceutical composition is formulated for administration by subcutaneous injection.

14. The pharmaceutical composition of any one of claims 1-13, wherein the pharmaceutical composition does not contain a preservative.

15. The pharmaceutical composition of any one of claims 1-14 for use as a medicament.

16. The pharmaceutical composition of any one of claims 1-15 for use in treating a cancer in a subject in need thereof, wherein the pharmaceutical composition is to be subcutaneously administered to the subject.

17. The pharmaceutical composition for use of claim 16, wherein the cancer is a B cell proliferative disorder.

18. The pharmaceutical composition for use of claim 17, wherein the cancer is a non-Hodgkin's lymphoma (NHL).

19. The pharmaceutical composition for use of claim 18, wherein the NHL is a follicular lymphoma (FL), a mantle cell lymphoma (MCL), an acute myeloid leukemia (AML), a chronic lymphoid leukemia (CLL), a small lymphocytic leukemia (SLL), a lymphoplasmacytic lymphoma (LL), a Waldenstrom macroglobulinemia (WM), a central nervous system lymphoma (CNSL), a Burkitt's lymphoma (BL), a diffuse large B cell lymphoma (DLBCL), a high-grade B cell lymphoma (HGBCL), a transformed FL (trFL), a primary mediastinal large B-cell lymphoma (PMBCL), or a marginal zone lymphoma (MZL).

20. The pharmaceutical composition for use of claim 19, wherein the NHL is an FL.

21. The pharmaceutical composition for use of claim 19, wherein the NHL is a DLBCL.

22. The pharmaceutical composition of any one of claims 1-15 for use in treating an autoimmune disorder in a subject in need thereof, wherein the pharmaceutical composition is to be subcutaneously administered to the subject.
